(19) Europäisches Patentamt
European Patent Office
Office européen des brevets

(11) EP 0 952 149 B1

(12) EUROPEAN PATENT SPECIFICATION

(45) Date of publication and mention of the grant of the patent:
**09.06.2004 Bulletin 2004/24**

(51) Int Cl.[7]: **C07D 209/88**, C07D 209/86, C07C 205/59, C07C 229/48, C07C 229/60, A61K 31/40

(21) Application number: **99302941.2**

(22) Date of filing: **16.04.1999**

(54) **Substituted carbazoles, process for their preparation and their use as sPLA2 inhibitiors**

Substituierte Karbazole, Verfahren zu ihrer Herstellung und ihre Verwendung als sPLA2- Inhibitoren

Carbazoles substitués, procédé pour leur préparation et leur utilisation comme inhibiteurs de l' enzyme sPLA2

(84) Designated Contracting States:
**AT BE CH CY DE DK ES FI FR GB GR IE IT LI LU NL PT SE**
Designated Extension States:
**AL LT LV RO SI**

(30) Priority: **17.04.1998 US 62328**

(43) Date of publication of application:
**27.10.1999 Bulletin 1999/43**

(73) Proprietor: **ELI LILLY AND COMPANY**
**Indianapolis, Indiana 46285 (US)**

(72) Inventors:
- **Anderson, Benjamin Alan**
  **Zionsville, Indiana 46077 (US)**
- **Bach, Nicholas James**
  **Indianapolis, Indiana 46217 (US)**
- **Bastian, Jolie Anne**
  **Beech Grove, Indiana 46107 (US)**
- **Harn, Nancy Kay**
  **Indianapolis, Indiana 46228 (US)**
- **Harper, Richard Waltz**
  **Indianapolis, Indiana 46208 (US)**
- **Hite, Gary Alan**
  **Indianapolis, Indiana 46227 (US)**
- **Kinnick, Michael Dean**
  **South, Indianapolis, Indiana 46217 (US)**
- **Lin, Ho-Shen**
  **Indianapolis, Indiana 46217 (US)**
- **Loncharich, Richard James**
  **Carmel, Indiana 46033 (US)**
- **McGill, John McNeill**
  **Greenwood, Indiana 46143 (US)**
- **Mihelich, Edward David**
  **Carmel, Indiana 46032 (US)**
- **Morin jr., John Michael**
  **Brownsburg, Indian 46112 (US)**
- **Phillips, Michael LeRoy**
  **Indianapolis, Indiana 46217 (US)**
- **Richett, Michael Enrico**
  **Indianapolis, Indiana 26250 (US)**
- **Sall, Daniel Jon**
  **Greenwood, Indiana 46142 (US)**
- **Sawyer,Jason Scott**
  **Indianapolis, Indiana 46220 (US)**
- **Schevitz, Richard Walter**
  **Indianapolis, Indiana 46220 (US)**
- **Vasileff, Robert Theodore**
  **Indianapolis, Indiana 46228 (US)**

(74) Representative: **Vaughan, Jennifer Ann et al**
**Lilly Research Centre,**
**Erl Wood Manor**
**Windlesham, Surrey GU20 6PH (GB)**

(56) References cited:
**EP-A- 0 620 214**
**EP-A- 0 620 215**
**EP-A- 0 749 962**
**EP-A- 0 779 271**
**EP-A- 0 839 806**
**US-A- 3 979 391**
**US-A- 5 420 289**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 952 149 B1

## Description

This invention relates to novel substituted tricyclic organic compounds useful for inhibiting $sPLA_2$ mediated release of fatty acids for conditions such as septic shock.

The structure and physical properties of human non-pancreatic secretory phospholipase $A_2$ (hereinafter called, "$sPLA_2$") has been thoroughly described in two articles, namely, "Cloning and Recombinant Expression of Phospholipase $A_2$ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase $A_2$" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989; the disclosures of which are incorporated herein by reference.

It is believed that $sPLA_2$ is a rate limiting enzyme in the arachidonic acid cascade which hydrolyzes membrane phospholipids. Thus, it is important to develop compounds which inhibit $sPLA_2$ mediated release of fatty acids (e.g., arachidonic acid). Such compounds would be of value in general treatment of conditions induced and/or maintained by overproduction of $sPLA_2$ such as septic shock, adult respiratory distress syndrome, pancreatitis, trauma-induced shock, bronchial asthma, allergic rhinitis, rheumatoid arthritis, etc.

It is desirable to develop new compounds and treatments for $sPLA_2$ induced diseases.

Alexander, et al., United States Patent Nos. 3,939,177 and 3,979,391, disclose 1,2,3,4-tetrahydrocarbazoles useful as antibacterial agents.

EP-A-839806 discloses substituted tricyclic organic compounds useful for inhibiting $sPLA_2$ mediated release of fatty acids for conditions such as septic shock.

This invention provides the tricyclic compounds identified in Claims 1 and 2.

These substituted tricyclics are effective in inhibiting human $sPLA_2$ mediated release of fatty acids.

This invention is also a pharmaceutical formulation comprising a compound as identified in Claim 1 or 2 below in association with one or more pharmaceutically acceptable diluents, carriers and excipients.

This invention is also the use, for the manufacture of a medicament for inhibiting $sPLA_2$ in a mammal in need of such treatment, of a compound as identified in Claim 1 or 2 below.

According to a further aspect of the present invention, there is provided the use, for the manufacture of a medicament for selectively inhibiting $sPLA_2$ in a mammal in need of such treatment, of a compound as identified in Claim 1 or 2 below.

This invention, further provides a compound as identified in Claim 1 or 2 below for use as a medicament in the treatment of inflammatory diseases such as, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases.

The salts of the above tricyclics are an additional aspect of the invention. In those instances where the compounds of the invention possess acidic functional groups various salts may be formed which are more water soluble and physiologically suitable than the parent compound. Representative pharmaceutically acceptable salts include but are not limited to the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, *et al.*, "Pharmaceutical Salts," J. Phar. Sci., 66: 1-19 (1977)).

Compounds of the invention may have chiral centers and exist in optically active forms. R- and S-isomers and racemic mixtures are contemplated by this invention. A particular stereoisomer may be prepared by known methods using stereospecific reactions with starting materials containing asymmetric centers already resolved or, alternatively,

by subsequent resolution of mixtures of stereoisomers using known methods.

Prodrugs are derivatives of the compounds of the invention which have chemically or metabolically cleavable groups and become by solvolysis or under physiological conditions the compounds of the invention which are pharmaceutically active in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives, such as, esters prepared by reaction of the parent acidic compound with a suitable alcohol, or amides prepared by reaction of the parent acid compound with a suitable amine. Simple aliphatic esters (e.g., methyl, ethyl, propyl, isopropyl, butyl, sec-butyl, tert-butyl) or aromatic esters derived from acidic groups pendent on the compounds of this invention are preferred prodrugs. Other preferred esters include morpholinoethyloxy, diethylglycolamide and diethylaminocarbonylmethoxy. In some cases it is desirable to prepare double ester type prodrugs such as (acyloxy) alkyl esters or ((alkoxycarbonyl)oxy)alkyl esters.

The following list of abreviations are used in the Examples and Preparations.

| | |
|---|---|
| HCl = | hydrochloric acid |
| EtOAc = | ethyl acetate |
| DMF = | dimethyl formamide |
| THF = | tetrahydrofuran |
| $Et_2O$ = | diethyl ether |
| $H_2O$ = | water |
| NaOH = | sodium hydroxide |
| EtOH = | ethanol |
| $Na_2SO_4$ = | sodium sulfate |
| $NaHCO_3$ = | sodium bicarbonate |
| celite = | diatomaceous earth |
| $CH_2Cl_2$ = | methylene chloride |
| $H_2SO_4$ = | sulfuric acid |
| MeoH = | methanol |
| $Rh/Al_2O_3$ = | rhodium on alumina |
| DDQ = | 2,3-dichloro-5,6-dicyano-1,4-benzo-quinone |
| TLC = | thin layer chromotography |
| NaH = | sodium hydride |
| $NH_4OH$ = | ammonium hydroxide |
| LiOH = | lithium hydroxide |
| $NH_3$ = | ammonia |
| $Cs_2CO_3$ = | cesium carbonate |
| $NH_4oAc$ = | ammonium acetate |

The following preparations of intermediates and examples illustrate the preparation of the compounds of this invention.

Example 1

Preparation of 9-benzyl-5,7-dimethoxy-1,2,3,4-tetrahydrocarbazole-4-carboxylic acid hydrazide

A. Preparation of N-benzyl-3,5-dimethoxyaniline.

A solution of 25gm. (0.163 mol) of 3,5-dimethoxyaniline and 18.3ml. (0.18 mol) of benzaldehyde in 300ml. of methanol was cooled in ice-water and treated with 10.3gm. (0.18 mol) of sodium cyanoborohydride in portions. The solution was stirred and cooled for 3 hours, treated with 1-2gm. of sodium borohydride for 30 minutes, diluted with water and extracted with ethyl acetate. The organic phase was washed with brine, dried over sodium sulfate, and evaporated *in vacuo*. The residue was chromatographed on silica gel eluting with a gradient hexane/15-70% ether to give 9-benzyl-3,5-dimethoxyaniline, 28.0gm., 71%, as an oil.

| Elemental Analyses for $C_{15}H_{17}NO_2$: | | | |
|---|---|---|---|
| Calculated | C 74.05; | H 7.04; | N 5.76 |
| Found | C 74.30; | H 7.12; | N 5.70 |

B. Preparation of 9-benzyl-5,7-dimethoxy-1,2,3,4-tetrahydrocarbazole-4-carboxylic acid hydrazide.

A solution of 9.72gm of the compound of part A and 4.98gm of 2-carbethoxy-6-bromocyclohexanone (J. Sheehan and C.E.Mumaw, J.Am.Chem.Soc., 72, 2127-2129, (1950).) in 125ml of benzene was refluxed for 72 hours, cooled, filtered, and evaporated *in vacuo*. The residue (12gm) and 10gm of zinc chloride were refluxed in 250ml of benzene for 6 hours, cooled and evaporated *in vacuo*. The residue was taken up in ethyl acetate, washed with 1N hydrochloric acid, washed with water, dried over sodium sulfate, and evaporated *in vacuo*. The residue was chromatographed on silica gel eluting with a gradient toluene/0-5% ethyl acetate to give compound (5) ($R^2$=5-MeO $R^3$=7-MeO $R^4$=phenyl), 1.88gm which was dissolved in 100ml of ethanol containing 10ml of hydrazine hydrate and refluxed for 5 days, cooled, the solution decanted, diluted with ethyl acetate, washed with brine, dried over sodium sulfate, and evaporated *in vacuo* to give title compound, 1.1gm, 60%, mp 189-190°C/$CH_2Cl_2$-EtOH.

| Elemental Analyses for $C_{22}H_{25}N_3O_3$: | | | |
|---|---|---|---|
| Calculated | C 69.64; | H 6.64; | N 11.07 |
| Found | C 69.59; | H 6.74; | N 10.84 |

Example 2

Preparation of 9-benzyl-5,7-dimethoxy-1,2,3,4-tetrahydrocarbazole-4-carboxamide

A mixture of 980mg of the compound of example 1, 2gm of Raney nickel catalyst, 1-2ml of hydrazine hydrate, and 125ml of ethanol was refluxed 1 hour, the solution decanted, diluted with ethyl acetate, washed with water, washed with brine, dried over sodium sulfate, and evaporated *in vacuo*. The residue was chromatographed on silica gel eluting with a gradient methylene chloride/1-3% methanol to give title compound, 820mg, 84%, mp 190-192°C/EtOH.

| Elemental Analyses for $C_{22}H_{24}N_2O_3$: | | | |
|---|---|---|---|
| Calculated | C 72.51; | H 6.64; | N 7.69 |
| Found | C 71.88; | H 6.89; | N 7.81 |

Example 3

Preparation of [9-benzyl-4-carbamoyl-7-methoxy-1,2,3,4-tetrahydrocarbazol-5-yl]oxyacetic acid sodium salt

A. Preparation of 9-benzyl-5-hydroxy-7-methoxy-1,2,3,4-tetrahydrocarbazole-4-carboxamide.

A solution of 1.75gm. (4.8 mmol) of the compound of example 6 in 50ml. of dimethylformamide was mixed with a solution of sodium thioethoxide (13.5 mmol) in 75ml. of dimethylformamide and then heated at 100°C for 21 hours. The mixture was cooled, diluted with water, acidified with hydrochloric acid, and extracted with ethyl acetate. The organic phase was washed with brine, dried over sodium sulfate, and evaporated *in vacuo*. The residue was chromatographed on silica gel eluting with a gradient methylene chloride/0-4% methanol to give the sub-titled product, 825mg., 50%, mp 225-7°C/ethanol.

| Elemental Analyses for $C_{21}H_{22}N_2O_3$: | | | |
|---|---|---|---|
| Calculated | C 71.98; | H 6.33; | N 7.99 |
| Found | C 71.71; | H 6.37; | N 7.72 |

B. Preparation of [9-benzyl-4-carbamoyl-7-methoxy-1,2,3,4-tetrahydrocarbazol-5-yl]oxyacetic acid ethyl ester.

A solution of 700mg. (2.0 mmol) of the product from Part A in 70ml. of dimethylformamide and 15ml. of tetrahydrofuran was treated with 100mg. of sodium hydride (60% in mineral oil; 2.5 mmol) for 10 minutes and then with 0.3ml. (2.7 mmol) of ethyl bromoacetate for 3 hours. The mixture was diluted with ethyl acetate, washed with water, washed with brine, dried over sodium sulfate, and evaporated *in vacuo*. The residue was chromatographed on silica gel eluting with a gradient methylene chloride/1-2% methanol to give sub-titled product, 670mg., 77%, mp 167-169°C/ether.

| Elemental Analyses for $C_{25}H_{28}N_2O_5$: | | | |
|---|---|---|---|
| Calculated | C 68.79; | H 6.47; | N 6.42 |
| Found | C 69.57; | H 6.39; | N 5.77 |

C. Preparation of [9-benzyl-4-carbamoyl-7-methoxy-1,2,3,4-tetrahydrocarbazol-5-yl]oxyacetic acid.

A suspension of 650mg. of the product from Part B in 20ml. of tetrahydrofuran and 70ml. of ethanol was treated with 5ml. of 2N sodium hydroxide and the resulting solution was stirred for 15.5 hours. The solution was diluted with ethyl acetate and water and acidified with hydrochloric acid. The organic phase was washed with brine, dried over sodium sulfate, concentrated *in vacuo*, and filtered to give title product, 540mg., 87%, mp 251-254°C.

| Elemental Analyses for $C_{23}H_{24}N_2O_5$: | | | |
|---|---|---|---|
| Calculated | C 67.63; | H 5.92; | N 6.86 |
| Found | C 67.73; | H 5.74; | N 6.82 |

D. Preparation of [9-benzyl-4-carbamoyl-7-methoxy-1,2,3,4-tetrahydrocarbazol-5-yl]oxyacetic acid sodium salt. A

suspension of 120mg. of the product from Part C in 20mL of ethanol was treated with 0.15mL of 2.0 N sodium hydroxide and warmed until dissolved. The resulting solution was concentrated *in vacuo*, diluted with ethyl acetate and again concentrated *in vacuo* and left to stand overnight. The precipitate was filtered and air dried to give the title product as an amorphous solid, 80mg, 63%.

| Elemental Analyses for $C_{23}H_{23}NaN_2O_5 \cdot 0.4H_2O$: | | | |
|---|---|---|---|
| Calculated | C 63.18; | H 5.39; | N 6.40 |
| Found | C 63.31; | H 5.48; | N 6.25 |

Example 4

Preparation of [9-benzyl-4-carbamoyl-7-methoxycarbazol-5-yl]oxyacetic acid

A mixture of 430mg. of the product from Example 3 Part D, 2.0gm. of 5% Pd/C, and 20mL of carbitol was heated to reflux and refluxed for 21 hours, cooled, and filtered. The filtrate was diluted with water, acidified with hydrochloric acid, and extracted well with ethyl acetate. The organic phase was washed with brine, dried over sodium sulfate and evaporated *in vacuo*. The residue was triturated with dichloromethane and filtered to remove solid tetrahydrocarbazole. The filtrate was evaporated *in vacuo* to give the title product, 125mg, 31%.

| Elemental Analyses for $C_{23}H_{20}N_2O_5 \cdot 0.4H_2O$: | | | |
|---|---|---|---|
| Calculated | C 67.11; | H 5.09; | N 6.81 |
| Found | C 67.25; | H 5.19; | N 6.75 |

Example 5

Preparation of methyl [9-benzyl-4-carbamoyl-7-methoxycarbazol-5-yl]oxyacetic acid

A. Preparation of 9-benzyl-4-carbamoyl-5,7-dimethoxycarbazole

A mixture of 2.0gm. of the product from Example 2, 2gm. of 5% Pd/C, and 100mL of carbitol was refluxed for 17 hours, filtered while still hot, and the solid washed well with ethyl acetate. The combined filtrates were washed with water, washed with brine, dried over sodium sulfate, and evaporated *in vacuo* to give subtitled product, 1.4gm., 70%, mp 240-243°C.

| Elemental Analyses for $C_{22}H_{20}N_2O_3$: | | | |
|---|---|---|---|
| Calculated | C 73.32; | H 5.59; | N 7.77 |
| Found | C 74.26; | H 5.73; | N 8.04 |

B. Preparation of 9-benzyl-4-carbamoyl-5-hydroxy-7-methoxycarbazole.

A solution of 1.2gm. (3.3 mmol) of the product from Part A and 10 mmol of sodium ethanethiolate in 100mL of dimethylformamide was heated at 100°C for 42 hours, cooled, diluted with water, and the pH adjusted to 5-6 with hydrochloric acid. The mixture was extracted with ethyl acetate, the organic phase was washed with water, washed with brine, dried over sodium sulfate, and evaporated *in vacuo*. The residue was chromatographed on silica gel eluting with ethyl acetate to give product, 550mg., 48%, mp 234-236°C dec.

| Elemental Analyses for $C_{21}H_{18}N_2O_3$: | | | |
|---|---|---|---|
| Calculated | C 72.82; | H 5.24; | N 8.09 |
| Found | C 72.54; | H 5.19; | N 8.04 |

C. Preparation of methyl [9-benzyl-4-carbamoyl-7-methoxycarbazol-5-yl]oxyacetic acid.

A solution of 430mg. (1.2 mmol) of the product from Part B in 40mL of dimethylformamide and a few mLs of tetrahydrofuran was treated with 60mg. of sodium hydride (60% in mineral oil; 1.5 mmol) for 15 minutes and then with 0.13mL (1.4 mmol) of methylbromoacetate for 16 hours, diluted with ethyl acetate, washed with water, washed with brine, dried over sodium sulfate, and evaporated *in vacuo*. The residue was chromatographed on silica gel eluting with a gradient dichloromethane/ 1-3% methanol to give title compound, 320mg., 62%, mp 170-172°C.

| Elemental Analyses for $C_{24}H_{22}N_2O_5$: | | | |
|---|---|---|---|
| Calculated | C 68.89; | H 5.30; | N 6.69 |
| Found | C 68.64; | H 5.41; | N 6.57 |

D. Preparation of [9-benzyl-4-carbamoyl-7-methoxycarbazol-5-yl]oxyacetic acid sodium salt

To a suspension of 60mg (0.15mmol) of the product from Part C in 30mL of ethanol was added 0.075mL of 2.0 N sodium hydroxide. The mixture was heated until solution, cooled, concentrated *in vacuo*, diluted with ethyl acetate, concentrated *in vacuo*, cooled, and filtered to give product, amorphous solid, 50mg., 80%. MS (FAB+) 427.2 : MS (ion spray) +Q1 405.5, -Q1 403.5

Example 6

Preparation of 9-benzyl-7-methoxy-5-cyanomethyloxy-1,2,3,4-tetrahydrocarbazole-4-carboxamide

A solution of 1.47gram (4.19 mmol) of the product from Example 3, Part A in 146ml. of dimethylformamide and 31ml. tetrahydrofuran was treated with 210mg. of sodium hydride (60% in mineral oil; 5.24mmol) for 10 minutes and then with 0.39ml. (0.66 mmol) of bromoacetonitrile for 3.5 hours. The mixture was diluted with ethyl acetate, washed with water, washed with brine, dried over sodium sulfate, and evaporated *in vacuo*. The residue was chromatographed on silica gel eluting with a gradient of 0 to 4% methanol in methylene chloride to give the titled product, 1.34gram, 82%.

| Elemental analysis for $C_{23}H_{23}N_3O_3$: | | | |
|---|---|---|---|
| Calculated | C 70.93; | H 5.95; | N 10.79 |
| Theory | C 70.67; | H 6.06; | N 10.83 |

Example 7

Preparation of 9-benzyl-7-methoxy-5-(1H-tetrazol-5-yl-methyl)oxy)-1,2,3,4-tetrahydrocarbazole-4-carboxamide

A portion of the compound of Example 6, 0.45gram (1.16mmol) was heated with 5ml. tri-n-butyl in hydride at 95°C for 1 hour. The reaction was then added to a mixture of 125 ml. acetonitrile, 25ml. tetrahydrofuran, and 50ml. acetic acid and stirred for 2 hours. The mixture was extracted 4 times with hexane and the residue evaporated *in vacuo*. Crystallization from acetone and hexane afforded the titled compound, 0.30gram, 60%.

| Elemental analysis for $C_{23}H_{24}N_6O_3$: | | | |
|---|---|---|---|
| Calculated | C 63.88; | H 5.59; | N 19.43 |
| Theory | C 64.06; | H 5.64; | N 19.28 |

Preparation 1

Preparation of 5-Carbomethoxy-1,2-dihydro-2-methyl-9H-carbazol-4(3H)-one from 2-bromo-3-nitrobenzoic acid

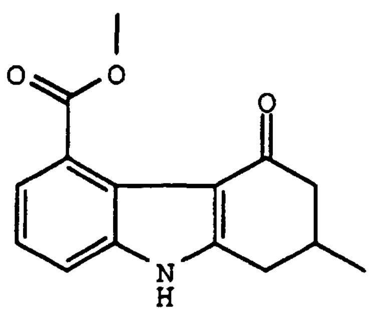

a) Methyl 2-bromo-3-nitrobenzoate

A solution of 2-bromo-3-nitrobenzoic acid (28.4 g, 115.0 mM), iodomethane (18.0 g, 127 mM), and potassium carbonate (19.0 g, 137.4 mM) in 100 mL dimethylformamide was stirred at room temperature for 72 hours. The mixture was poured into 1.5 liters of water. The resultant precipitate was collected by filtration and dried *in vacuo* to afford 28.79 g (96%) of methyl 2-bromo-3-nitrobenzoate as a white solid. $^1H$ NMR (DMSO-d6) δ 8.3 (dd, 1H, J=1 and 8 Hz), 7.9 (dd, 1H, J=1 and 8 Hz), 7.7 (t, 1H, J=8 Hz), and 3.9 (s, 3H). IR (KBr, $cm^{-1}$) 2950, 1738, 1541, 1435, 1364, 1298, and 1142. MS (FD) m/e 259, 261.

| Elemental Analyses for $C_8H_6NO_4Br$: | | | |
|---|---|---|---|
| Calculated | C, 36.95; | H, 2.33; | N, 5.39. |
| Found | C, 37.14; | H, 2.37; | N, 5.45. |

b) Methyl 2-bromo-3-aminobenzoate

Hydrogen gas was passed through a solution of methyl 2-bromo-3-nitrobenzoate (0.20 g, 0.77 mM) and 0.1 g of 3% sulfided platinum on carbon in 25 mL ethyl acetate for 24 hours at room temperature. The catalyst was removed by filtration through celite. Concentration of the filtrate afforded 0.175 g (99%) of methyl 2-bromo-3-aminobenzoate as a yellow oil. $^1H$ NMR ($CDCl_3$) δ 7.15 (t, 1H, J=8 Hz), 7.1 (dd, 1H, J=1 and 8 Hz), 6.8 (dd, 1H, J=1 and 8 Hz), and 3.95 (s, 3H). IR ($CHCl_3$, $cm^{-1}$) 3550, 3380, 2980, 2900, 1729, 1613, 1465, 1451, 1434, 1324, 1266, and 1025. MS (FD) m/e 230, 232.

| Elemental Analyses for $C_8H_8NO_2Br$: | | | |
|---|---|---|---|
| Calculated | C, 41.77; | H, 3.51; | N, 6.09. |

(continued)

| Elemental Analyses for $C_8H_8NO_2Br$: | | | |
|---|---|---|---|
| Found | C, 42.01; | H, 3.29; | N, 6.00. |

c) 3-(3-Carbomethoxy-2-bromoanilino)-5-methyl-cyclohex-2-en-1-one

A mixture of methyl 2-bromo-3-aminobenzoate (10.2 g, 44.3 mM) and 5-methyl-1,3-cyclohexanedione (6.15 g, 48.7 mM) was heated at 125 °C under a stream of nitrogen for 1.5 hours. The resultant solid was triturated with ethyl acetate to afford 9.98 g (67%) of 3-(3-carbomethoxy-2-bromoanilino)-5-methyl-cyclohex-2-en-1-one. $^1$H NMR ($CDCl_3$) δ 7.55 (m, 2H), 7.35 (dd, J=8 and 8 Hz, 1H), 6.4 (bs, 1H), 5.55 (s, 1H), 3.95 (s, 3H), 2.6-2.0 (m, 5H), 1.15 (d, J=7 Hz, 3H). MS (ES) m/e 338, 340.

d) 5-Carbomethoxy-1,2-dihydro-2-methyl-9H-carbazol-4(3H)-one

A suspension of 3-(3-carbomethoxy-2-bromoanilino)-5-methyl-cyclohex-2-en-1-one (9.98 g, 29.5 mM), palladium acetate (0.66 g, 2.95 mM), tri-*o*-tolylphosphine (1.8 g, 5.9 mM), and triethylamine (5.10 ml, 36.6 mM) in 75 mL acetonitrile was heated at reflux for 3 hours. The solvent was removed *in vacuo*. The residue was dissolved in methylene chloride, washed with 1 N HCl, then with saturated brine, dried over anhydrous sodium sulfate, filtered, and concentrated to afford 11 g of crude product. Purification by HPLC on silica gel (elution with gradient methylene chloride/ethyl acetate) afforded 5.7 g (75%) of 5-carbomethoxy-1,2-dihydro-2-methyl-9H-carbazol-4(3H)-one. $^1$H NMR ($CDCl_3$) δ 9.5 (bs, 1H), 7.4 (d, J=8 Hz, 1H), 7.35 (d, J=8 Hz, 1H), 7.2 (dd, J=8 and 8 Hz, 1H), 4.0 (s, 3H), 2.9 (dd, J=13 and 4 Hz, 1H), 2.55 (m, 2H), 2.4 (m, 1H), 2.25 (dd, J=15 and 9 Hz, 1H), 1.05 (d, J=7 Hz, 3H). MS (ES) m/e 226, 258.

EXAMPLE 8

Preparation of {9-[(phenyl)methyl]-5-carbamoyl-2-methylcarbazol-4-yl}oxyacetic acid

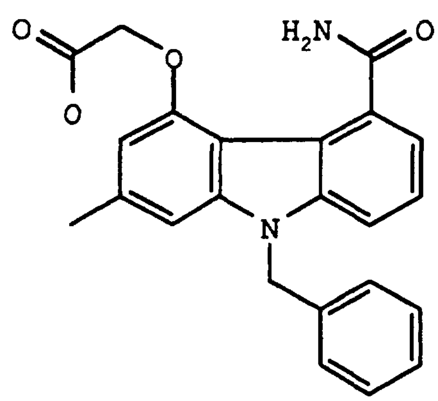

A. 9-[(Phenyl)methyl]-5-carbomethoxy-2-methyl-1,2-dihydrocarbazol-4(3H)-one

A suspension of 5-carbomethoxy-1,2-dihydro-2-methyl-9H-carbazol-4(3H)-one (2.0 g, 7.77 mM), benzyl bromide (0.94 ml, 7.93 mM), and potassium carbonate (2.15 g, 15.5 mM) in 39 mL DMF was stirred at room temperature for 22 hours. The mixture was diluted with ethyl acetate and 1N HCl. The layers were separated and the aqueous layer extracted with ethyl acetate. The combined ethyl acetate layers were extracted with 1N HCl twice, once with water and once with brine. After drying ($NaSO_4$), evaporation *in vacuo* afforded 2.61g (97%) of 9-[(phenyl)methyl]-5-carbomethoxy-2-methyl-1,2-dihydrocarbazol-4(3H)-one. $^1$H NMR ($CDCl_3$) δ 7.6-7.4 (m, 6H), 7.0 (m, 2H), 5.4 (s, 2H), 4.05 (s, 3H), 3.0 (m, 1H), 2.65-2.45 (m, 3H), 2.3 (dd, J=15 and 9 Hz, 1H), 1.1 (d, J=7 Hz, 3H). MS (ES) m/e 316, 348.

B. 9-[(Phenyl)methyl]-2-methyl-4-hydroxy-5-carbomethoxy carbazole

A solution of the 9-[(phenyl)methyl]-5-carbomethoxy-2-methyl-1,2-dihydrocarbazol-4(3H)-one (1.30 g, 3.74 mM) and 2,3-dichloro-5,6-dicyano-1,4-benzoquinone (0.93 g, 4.12 mM) in 37 mL of toluene was stirred between 80-90 °C for 5 hours. The mixture was purified by column chromatography on silica gel (elution with methylene chloride) to afford 270 mg (21%) of the 9-[(phenyl)methyl]-2-methyl-4-hydroxy-5-carbomethoxy carbazole. $^1$H NMR ($CDCl_3$) δ 10.45 (s, 1H), 8.0 (d, J=8 Hz, 1H), 7.55 (d, J=8 Hz, 1H), 7.4 (dd, J=8 and 8 Hz, 1H), 7.3 (m, 3H), 7.05 (m, 2H), 6.65 (s, 1H), 6.6 (s, 1H), 5.5 (s, 2H), 4.1 (s, 3H), 2.45 (s, 3H). MS (ES) m/e 314, 346.

C. 9-[(Phenyl)methyl]-2-methyl-4-hydroxy-5-carbamoyl carbazole

A solution of the 9-[(phenyl)methyl]-2-methyl-4-hydroxy-5-carbomethoxy carbazole (470 mg, 1.36 mM) in 20 ml THF and 80 mL concentrated aqueous ammonium hydroxide was sonicated for 6 hours at 30-40 °C. The precipitated solid was filtered and triturated with $Et_2O$ to afford 200 mg (44%) of 9-[(phenyl)methyl]-2-methyl-4-hydroxy-5-carbamoyl carbazole. $^1$H NMR (DMSO-d6) δ 10.5 (s, 1H), 8.8 (bs, 1H), 8.4 (bs, 1H), 7.75 (m, 1H), 7.4 (m, 2H), 7.25 (m, 3H), 7.1 (m, 2H), 6.95 (s, 1H) , 6.45 (s, 1H) , 5.65 (s, 2H), 2.4 (s, 3H). MS (ES) m/e 314, 331.

D. {9-[(Phenyl)methyl]-2-methyl-5-carbamoylcarbazol-4-yl}oxyacetic acid, methyl ester

60% Sodium hydride in mineral oil (30.4 mg, 0.76 mM) was added to a solution of 9-[(phenyl)methyl]-2-methyl-4-hydroxy-5-carbamoyl carbazole (202 mg, 0.61 mM) in 21 mL DMF and 4.6 ml THF. After 10 minutes, methyl bromoacetate (77 µl, 0.482 mM) was added and the resultant mixture stirred at room temperature for 1.25 hours. The mixture was diluted with ethyl acetate and washed with $H_2O$. The aqueous layer was extracted with ethyl acetate. The combined organic layers were extracted with saturated brine, dried over sodium sulfate, filtered, and concentrated. The residue was purified by column chromatography on silica gel (elution with ethyl acetate) to afford 184 mg (75%) of {9-[(phenyl)methyl]-2-methyl-5-carbamoylcarbazol-4-yl}oxyacetic acid, methyl ester. $^1$H NMR (DMSO-d6) δ 7.55 (d, 1H, J=8 Hz), 7.5 (bs, 1H), 7.4-7.15 (m, 9H), 6.45 (s, 1H) , 5.7 (s, 2H), 4.9 (s, 2H), 3.75 (s, 3H), 2.4 (s, 3H). MS (FD) m/e 386, 403.

| Elemental Analyses for $C_{24}H_{22}N_2O_4$: | | | |
|---|---|---|---|
| Calculated | C, 71.63; | H, 5.51; | N, 6.96. |
| Found | C, 71.74; | H, 5.81; | N, 6.69. |

E. {9-[(Phenyl)methyl]-2-methyl-5-carbamoylcarbazol-4-yl}oxyacetic acid

A solution of the {9-[(phenyl)methyl]-2-methyl-5-carbamoylcarbazol-4-yl}oxyacetic acid, methyl ester (83.5 mg, 0.207 mM) and 1.0 mL (2.0 mM) of 2 N NaOH in 10 mL of ethanol was stirred for 45 minutes at 25 °C. The resultant white precipitate was collected by filtration, washed with a small amount of EtOH, then dried *in vacuo* to afford 48 mg (56%) of the {9-[(phenyl)methyl]-2-methyl-5-carbamoylcarbazol-4-yl}oxyacetic acid sodium salt as a white powder. MS (ES) m/e 314, 372, 389, 411. The filtrate was acidified with 1N HCl to pH =1. After cooling to 5°C, the resultant white precipitate was collected by filtration, washed with water, then dried *in vacuo* to afford 24 mg (29%) {9-[(phenyl)methyl]-2-methyl-5-carbamoylcarbazol-4-yl}oxyacetic acid. $^1$H NMR (DMSO-d6) δ 11.2 (bs, 1H), 7.8 (bs, 1H), 7.6 (d, J=8 Hz, 1H), 7.45 (bs, 1H), 7.4-7.05 (m, 8H), 6.45 (s, 1H) , 5.65 (s, 2H), 4.9 (s, 2H), 2.4 (s, 3H). MS (ES) m/e 314, 372, 389.

| Elemental Analyses for $C_{23}H_{20}N_2O_4$: | | | |
|---|---|---|---|
| Calculated | C, 71.12; | H, 5.19; | N, 7.21. |
| Found | C, 71.33; | H, 5.47; | N, 7.19. |

EXAMPLE 9

Preparation of {9-[(3-fluorophenyl)methyl]-5-carbamoyl-2-methyl-carbazol-4-yl}oxyacetic acid

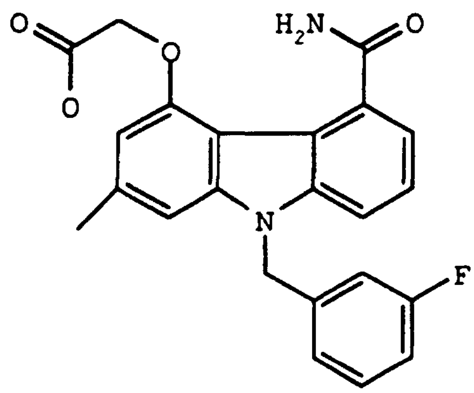

A. 9-[(3-Fluorophenyl)methyl]-5-carbomethoxy-2-methyl-1,2-dihydrocarbazol-4(3H)-one

A suspension of 5-carbomethoxy-1,2-dihydro-2-methyl-9H-carbazol-4(3H)-one (1.0 g, 3.89 mM), 3-fluorobenzyl bromide (0.48 ml, 3.97 mM), and potassium carbonate (1.07 g, 7.78 mM) in 20 mL DMF was stirred at room temperature for 22 hours. The mixture was diluted with EtOAc and 1N HCl. The layers were separated and the aqueous extracted with EtOAc. The combined EtOAc layers were extracted with 1N HCl, water, then brine. After drying ($Na_2SO_4$), evaporation *in vacuo* afforded 1.38g (97%) of the 9-[(3-fluorophenyl)methyl]-5-carbomethoxy-2-methyl-1,2-dihydrocarbazol-4(3H)-one.
$^1H$ NMR ($CDCl_3$) δ 7.4-7.2 (m, 5H) , 7.0 (m, 1H), 6.75 (m, 2H), 5.4 (s, 2H), 4.05 (s, 3H), 3.0 (m, 1H), 2.65-2.45 (m, 3H), 2.3 (dd, J=15 and 9 Hz, 1H), 1.1 (d, J=7 Hz, 3H). MS (ES) m/e 334, 366.

B. 9-[(3-Fluorophenyl)methyl]-2-methyl-4-hydroxy-5-carbomethoxy carbazole

A solution of the 9-[(3-fluorophenyl)methyl]-5-carbomethoxy-2-methyl-1,2-dihydrocarbazol-4(3H)-one (1.37 g, 3.75 mM) and 2,3-dichloro-5,6-dicyano-1,4-benzoquinone (0.94 g, 4.13 mM) in 38 mL of toluene was stirred between 80-90 °C for 3 hours. The mixture was purified by column chromatography on silica gel (elution with methylene chloride) to afford 0.33 g (24%) of 9-[(3-fluorophenyl)methyl]-2-methyl-4-hydroxy-5-carbomethoxy carbazole. $^1H$ NMR ($CDCl_3$) δ 10.45 (s, 1H), 8.0 (d, J=8 Hz, 1H), 7.55 (d, J=8 Hz, 1H), 7.4 (dd, J=8 and 8 Hz, 1H), 7.3 (m, 2H), 6.95 (m, 1H), 6.85 (d, J=8 Hz, 1H), 6.75 (m, 1H), 6.65 (s, 1H), 5.5 (s, 2H), 4.1 (s, 3H), 2.45 (s, 3H). MS (ES) m/e 332, 364.

C. 9-[(3-Fluorophenyl)methyl]-2-methyl-4-hydroxy-5-carbamoyl carbazole

A solution of the 9-[(3-fluorophenyl)methyl]-2-methyl-4-hydroxy-5-carbomethoxy carbazole (0.33 g, 0.91 mM) in 14 ml THF and 54 mL concentrated aqueous ammonium hydroxide was sonicated for 6.5 h at 30-40 °C. The precipitated solid was filtered, washed with water, and triturated with 35 ml $Et_2O$ to afford 182 mg (57%) of 9-[(3-fluorophenyl)methyl]-2-methyl-4-hydroxy-5-carbamoyl carbazole. $^1H$ NMR (DMSO-d6) δ 10.5 (s, 1H), 8.8 (bs, 1H), 8.4 (bs, 1H), 7.75 (m, 1H), 7.4 (m, 2H), 7.25 (m, 1H), 7.05 (m, 1H), 6.9 (m, 2H), 6.85 (d, J=8 Hz, 1H), 6.45 (s, 1H), 5.65 (s, 2H), 2.4 (s, 3H). MS (ES) m/e 332, 349.

D. {9-[(3-Fluorophenyl)methyl]-2-methyl-5-carbamoylcarbazol-4-yl}oxyacetic acid, methyl ester

60% Sodium hydride in mineral oil (25.9 mg, 0.65 mM) was added to a solution of 9-[(3-fluorophenyl)methyl]-2-methyl-4-hydroxy-5-carbamoyl carbazole (181 mg, 0.52 mM) in 18 mL DMF and 3.9 ml THF. After 10 minutes, methyl bromoacetate (66 µl, 0.70 mM) was added and the resultant mixture stirred at room temperature for 1.25 hours. The mixture was diluted with ethyl acetate and washed with $H_2O$. The aqueous layer was extracted with ethyl acetate. The combined organic layers were extracted with saturated brine, dried over sodium sulfate, filtered, and concentrated. The residue was purified by column chromatography on silica gel (elution with methylene chloride/acetone gradient) to afford 170 mg (78%) of the {9-[(3-fluorophenyl)methyl]-2-methyl-5-carbamoylcarbazol-4-yl}oxyacetic acid, methyl

ester. $^1$H NMR (DMSO-d6) δ 7.55 (d, 1H, J=8 Hz), 7.5 (bs, 1H), 7.4-7.25 (m, 2H), 7.2 (bs, 1H), 7.05 (m, 3H), 6.95 (d, J=8 Hz, 1H), 6.9 (d, J=8 Hz), 6.45 (s, 1H), 5.65 (s, 2H), 4.9 (s, 2H), 3.75 (s, 3H), 2.4 (s, 3H). MS (FD) m/e 404, 421.

| Elemental Analyses for $C_{24}H_{21}FN_2O_4$: | | | |
|---|---|---|---|
| Calculated | C, 68.56; | H, 5.03; | N, 6.66. |
| Found | C, 67.75; | H, 4.95; | N, 6.33. |

E. {9-[(3-Fluorophenyl)methyl]-2-methyl-5-carbamoylcarbazol-4-yl}oxyacetic acid

A solution of {9-[(3-fluorophenyl)methyl]-2-methyl-5-carbamoylcarbazol-4-yl}oxyacetic acid, methyl ester (68.3 mg, 0.162 mM) and 0.81 mL (1.6 mM) of 2 N NaOH in 8.1 mL of ethanol was stirred for 30 minutes at 25 °C. The resultant white precipitate was collected by filtration, washed with a small amount of EtOH, then dried *in vacuo* to afford 11 mg (16%) of {9-[(3-fluorophenyl)methyl]-2-methyl-5-carbamoylcarbazol-4-yl}oxyacetic acid, sodium salt as a white powder. The filtrate was acidified with 1N HCl to pH =2. After cooling to 5°C, the resultant white precipitate was collected by filtration, washed with water, then dried *in vacuo* to afford 31 mg (47%) {9-[(3-fluorophenyl)methyl]-2-methyl-5-carbamoylcarbazol-4-yl}oxyacetic acid. $^1$H NMR (DMSO-d6) δ 7.75 (bs, 1H), 7.6 (d, 1H, J=8 Hz), 7.45 (bs, 1H), 7.4-7.25 (m, 2H), 7.05 (m, 3H), 6.95 (d, J=8 Hz, 1H), 6.9 (d, J=8 Hz, 1H), 6.45 (s, 1H), 5.65 (s, 2H), 4.8 (s, 2H), 2.4 (s, 3H). MS (ES) m/e 390, 407. Recrystallization from acetone/hexane provided an analytical sample:

| Elemental Analyses for $C_{23}H_{19}FN_2O_4$: | | | |
|---|---|---|---|
| Calculated | C, 67.97; | H, 4.71; | N, 6.89. |
| Found | C, 68.21; | H, 4.93; | N, 7.16. |

EXAMPLE 10

Preparation of {9-[(3-methylphenyl)methyl]-5-carbamoyl-2-methyl-carbazol-4-yl}oxyacetic acid

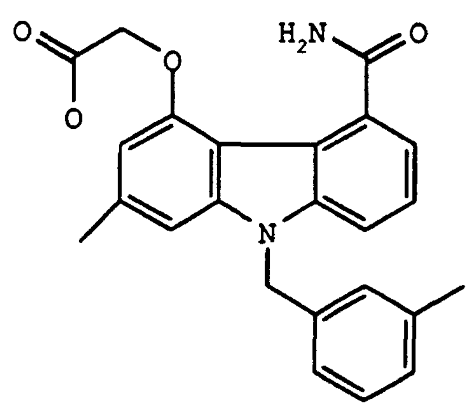

A. 9-[(3-Methylphenyl)methyl]-5-carbomethoxy-2-methyl-1,2-dihydrocarbazol-4(3H)-one

A suspension of 5-carbomethoxy-1,2-dihydro-2-methyl-9H-carbazol-4(3H)-one (1.0 g, 3.89 mM), 3-methylbenzyl bromide (0.54 ml, 3.97 mM), and potassium carbonate (1.07 g, 7.78 mM) in 20 mL DMF was stirred at room temperature for 19 hours. The mixture was diluted with EtOAc and 1N HCl. The layers were separated and the aqueous layer extracted with EtOAc. The combined EtOAc layers were extracted with 1N HCl, water, then brine. After drying ($NaSO_4$), evaporation *in vacuo* afforded 1.41g (100%) of 9-[(3-methylphenyl)methyl]-5-carbomethoxy-2-methyl-1,2-dihydrocarbazol-4(3H)-one. $^1$H NMR ($CDCl_3$) δ 7.4-7.05 (m, 6H), 6.8 (m, 1H), 5.3 (s, 2H), 4.05 (s, 3H), 3.0 (m, 1H), 2.7-2.3 (m, 4H), 2.3 (s, 1H), 1.2 (d, J=7 Hz, 3H). MS (ES) m/e 362.

B. 9-[(3-Methylphenyl)methyl]-2-methyl-4-hydroxy-5-carbomethoxy carbazole

To a solution of 9-[(3-methylphenyl)methyl]-5-carbomethoxy-2-methyl-1,2-dihydrocarbazol-4(3H)-one (1.41 g, 3.89 mM) in 13 ml dioxane was added 60% sodium hydride in mineral oil (0.36 g, 8.95 mM). The reaction was stirred 6 minutes, then methyl benzenesulfinate (0.81 ml, 6.22 mM) was added. The reaction was stirred an additional 6 hours, then diluted with 20 ml dioxane and 0.51 ml acetic acid. The mixture was refluxed 30 minutes, diluted with ethyl acetate, and extracted with saturated $NaHCO_3$, brine, then water. After drying ($NaSO_4$), evaporation *in vacuo* afforded 2.30g. The mixture was purified by column chromatography on silica gel (elution with toluene/methylene chloride) to afford 0.92 g (66%) of 9-[(3-methylphenyl)methyl]-2-methyl-4-hydroxy-5-carbomethoxy carbazole. $^1H$ NMR ($CDCl_3$) δ 10.45 (s, 1H), 8.0 (d, J=8 Hz, 1H), 7.55 (d, J=8 Hz, 1H), 7.4 (dd, J=8 and 8 Hz, 1H), 7.4 (dd, J=8 and 8 Hz, 1H), 7.05 (d, J=8 HZ, 1H), 6.9 (s, 1H), 6.85 (d, J=8 Hz, 1H), 6.75 (s, 1H), 6.7 (s, 1H), 5.45 (s, 2H), 4.1 (s, 3H), 2.4 (s, 3H), 2.25 (s, 3H). MS (ES) m/e 328, 360.

C. 9-[(3-Methylphenyl)methyl]-2-methyl-4-hydroxy-5-carbamoyl carbazole

A solution of 9-[(3-methylphenyl)methyl]-2-methyl-4-hydroxy-5-carbomethoxy carbazole (0.92 g, 2.56 mM) in 38 ml THF and 154 mL concentrated aqueous ammonium hydroxide was sonicated for 6 h at 30-40 °C. The precipitated solid was filtered, washed with water to afford 0.55g (63%) of 9-[(3-methylphenyl)methyl]-2-methyl-4-hydroxy-5-carbamoyl carbazole. $^1H$ NMR (DMSO-d6) δ 10.5 (s, 1H), 8.8 (bs, 1H), 8.4 (bs, 1H), 7.75 (m, 1H), 7.4 (m, 2H), 7.15 (dd, J=8 and 8 Hz, 1H), 7.05 (m, 1H), 7.0 (s, 1H), 6.9 (s, 1H), 6.8 (d, J=8 Hz, 1H), 6.45 (s, 1H), 5.65 (s, 2H), 2.4 (s, 3H), 2.2 (s, 3H). MS (ES) m/e 328, 345.

D. {9-[(3-Methylphenyl)methyl]-2-methyl-5-carbamoylcarbazol-4-yl}oxyacetic acid, methyl ester

60% Sodium hydride in mineral oil (79.8 mg, 2.0 mM) was added to a solution of 9-[(3-methylphenyl)methyl]-2-methyl-4-hydroxy-5-carbamoyl carbazole (0.55 g, 1.60 mM) in 56 mL DMF and 12 ml THF. After 10 minutes, methyl bromoacetate (0.20 ml, 2.16 mM) was added and the resultant mixture stirred at room temperature for 1 hour. The mixture was diluted with ethyl acetate and washed with $H_2O$. The aqueous layer was extracted with ethyl acetate. The combined organic layers were extracted with saturated brine, dried over sodium sulfate, filtered, and concentrated. The residue was purified by column chromatography on silica gel (elution with methylene chloride/acetone gradient) to afford 0.51 g (76%) of {9-[(3-methylphenyl)methyl]-2-methyl-5-carbamoylcarbazol-4-yl}oxyacetic acid, methyl ester. $^1H$ NMR (DMSO-d6) δ 7.5 (m, 2H), 7.35 (dd, J=8 and 8 Hz, 1H), 7.2-7.1 (m, 2H), 7.05-6.95 (m, 4H), 6.85 (d, J=8 Hz, 1H), 6.45 (s, 1H), 5.6 (s, 2H), 4.9 (s, 2H), 3.75 (s, 3H), 2.4 (s, 3H), 2.2 (s, 3H). MS (FD) m/e 400, 417.

| Elemental Analyses for $C_{25}H_{24}N_2O_4$: | | | |
|---|---|---|---|
| Calculated | C, 72.10; | H, 5.81; | N, 6.73. |
| Found | C, 71.94; | H, 5.71; | N, 6.96. |

E. {9-[(3-Methylphenyl)methyl]-2-methyl-5-carbamoylcarbazol-4-yl}oxyacetic acid

A solution of {9-[(3-methylphenyl)methyl]-2-methyl-5-carbamoylcarbazol-4-yl}oxyacetic acid, methyl ester (0.12 g, 0.288 mM) and 1.4 mL (2.8 mM) of 2 N NaOH in 14 mL of ethanol was stirred for 30 minutes at 25 °C. The reaction was acidified with 1N HCl to pH =2. After stirring 1 hour, the resultant white precipitate was collected by filtration, washed with water, then dried *in vacuo* to afford 114 mg (95%) {9-[(3-methylphenyl)methyl]-2-methyl-5-carbamoylcarbazol-4-yl}oxyacetic acid. $^1H$ NMR (DMSO-d6) δ 11.1 (bs, 1H), 7.75 (bs, 1H), 7.6 (d, J=8 Hz, 1H), 7.45 (bs, 1H), 7.35 (dd, J=8 and 8Hz, 1H), 7.2-7.0 (m, 5H), 6.85 (d, J=8Hz, 1H), 6.45 (s, 1H), 5.6 (s, 2H), 4.8 (s, 2H), 2.4 (s, 3H), 2.2 (s, 3H). MS (ES) m/e 386, 403. Recrystallization from acetone/hexane provided an analytical sample:

| Elemental Analyses for $C_{24}H_{22}N_2O_4$: | | | |
|---|---|---|---|
| Calculated | C, 71.63; | H, 5.51; | N, 6.96. |
| Found | C, 71.88; | H, 5.65; | N, 7.20. |

Preparation 2

Preparation of 5-Carbomethoxy-1,2-dihydro-2-(4-trifluoromethylphenyl)-9H-carbazol-4(3H)-one from 2-bromo-3-nitrobenzoic acid

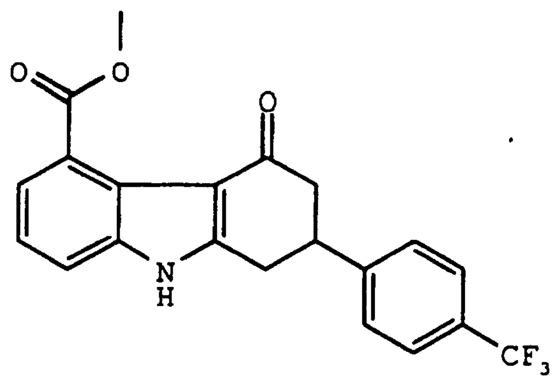

a) Methyl 2-bromo-3-nitrobenzoate

A solution of 2-bromo-3-nitrobenzoic acid (28.4 g, 115.0 mM), iodomethane (18.0 g, 127 mM), and potassium carbonate (19.0 g, 137.4 mM) in 100 mL DMF was stirred at room temperature for 72 hours. The mixture was poured into 1.5 liters of $H_2O$. The resultant precipitate was collected by filtration and dried *in vacuo* to afford 28.79 g (96%) of methyl 2-bromo-3-nitrobenzoate as a white solid. $^1H$ NMR (DMSO-d6) δ 8.3 (dd, 1H, J=1 and 8 Hz), 7.9 (dd, 1H, J=1 and 8 Hz), 7.7 (t, 1H, J=8 Hz), and 3.9 (s, 3H). IR (KBr, $cm^{-1}$) 2950, 1738, 1541, 1435, 1364, 1298, and 1142. MS (FD) m/e 259, 261.

| Elemental Analyses for $C_8H_6NO_4Br$: | | | |
|---|---|---|---|
| Calculated | C, 36.95; | H, 2.33; | N, 5.39. |
| Found | C, 37.14; | H, 2.37; | N, 5.45. |

b) Methyl 2-bromo-3-aminobenzoate

Hydrogen gas was passed through a solution of methyl 2-bromo-3-nitrobenzoate (0.20 g, 0.77 mM) and 0.1 g of 3% sulfided platinum on carbon in 25 mL ethyl acetate for 24 hours at room temperature. The catalyst was removed by filtration through celite. Concentration of the filtrate afforded 0.175 g (99%) of methyl 2-bromo-3-aminobenzoate as a yellow oil. $^1H$ NMR ($CDCl_3$) δ 7.15 (t, 1H, J=8 Hz), 7.1 (dd, 1H, J=1 and 8 Hz), 6.8 (dd, 1H, J=1 and 8 Hz), and 3.95 (s, 3H). IR ($CHCl_3$, $cm^{-1}$) 3550, 3380, 2980, 2900, 1729, 1613, 1465, 1451, 1434, 1324, 1266, and 1025. MS (FD) m/e 230, 232.

| Elemental Analyses for $C_8H_8NO_2Br$: | | | |
|---|---|---|---|
| Calculated | C, 41.77; | H, 3.51; | N, 6.09. |
| Found | C, 42.01; | H, 3.29; | N, 6.00. |

c) 3-(3-Carbomethoxy-2-bromoanilino)-5-(4-trifluoromethylphenyl-cyclohex-2-en-1-one

A mixture of methyl 2-bromo-3-aminobenzoate (10.2 g, 44.3 mM) and 5-(4-trifluoromethylphenyl)-1,3-cyclohexanedione (1.77 g, 6.93 mM) was heated at 150 °C under a stream of nitrogen for 20 minutes. The resultant solid was triturated with 4:1 EtOAc/$Et_2O$ to afford 2.18 g (74%) of 3-(3-carbomethoxy-2-bromoanilino)-5-(4-trifluoromethylphenyl)-cyclohex-2-en-1-one. $^1H$ NMR (DMSO-d6) δ 8.9 (s, 1H), 7.75-7.5 (m, 7H), 3.9 (s, 3H), 3.5 (m, 1H), 2.9 (dd, J=14 and 9 Hz, 1H), 2.7 (dd, J=14 and 4 Hz, 1H), 2.55 (dd, J=14 and 9 Hz, 1H), 2.35 (dd, J=14 and 4 Hz, 1H). MS (ES) m/e 368, 370.

d) 5-Carbomethoxy-1,2-dihydro-2-(4-trifluoromethylphenyl)-9H-carbazol-4(3H)-one

A suspension of 3-(3-carbomethoxy-2-bromoanilino)-5-(4-trifluoromethylphenyl)-cyclohex-2-en-1-one (2.18 g, 4.66 mM), palladium acetate (0.10 g, 0.47 mM), tri-*o*-tolylphosphine (0.28 g, 0.93 mM), and triethylamine (0.8 ml, 5.78 mM) in 12 mL acetonitrile was heated at reflux for 3 hours. The solvent was removed *in vacuo*. The residue was dissolved in methylene chloride, washed with 1 N HCl, then with saturated brine, dried over anhydrous sodium sulfate, filtered, and concentrated to afford 2.21 g. Purification by HPLC on silica gel (elution with gradient methylene chloride/ ethyl acetate) afforded 1.57 g (87%) of the 5-carbomethoxy-1,2-dihydro-2-(4-trifluoromethylphenyl)-9H-carbazol-4 (3H)-one. $^1$H NMR ($CDCl_3$) δ 9.2 (bs, 1H), 7.6 (d, J=8 Hz, 2H), 7.45-7.35 (m, 5H), 7.25 (d, J=8 Hz, 2H), 3.55 (m, 1H), 3.2-3.0 (m, 2H), 2.7 (m, 2H). MS (ES) m/e 356,388.

EXAMPLE 11

Preparation of {9-[(phenyl)methyl]-5-carbamoyl-2-(4-trifluoromethylphenyl)-carbazol-4-yl}oxyacetic acid

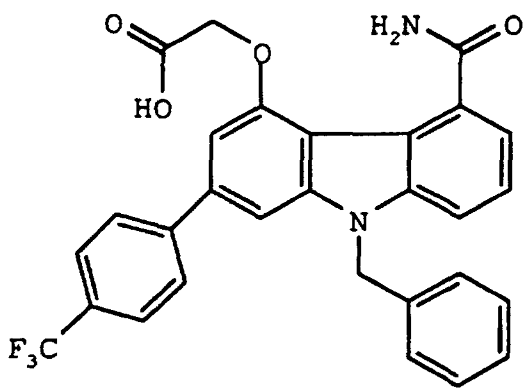

a) 9-[(Phenyl)methyl]-5-carbomethoxy-2-(4-trifluoromethylphenyl)-1,2-dihydrocarbazol-4(3H)-one

A suspension of 5-carbomethoxy-1,2-dihydro-2-(4-trifluoromethylphenyl)-9H-carbazol-4(3H)-one (1.57 g, 4.05 mM), benzyl bromide (0.49 ml, 4.13 mM), and potassium carbonate (1.12 g, 8.10 mM) in 20 mL DMF was stirred at room temperature for 22 hours. The mixture was diluted with EtOAc and 1N HCl. The layers were separated and the aqueous extracted with EtOAc. The combined EtOAc layers were extracted with 1N HCl, water, then brine. After drying ($NaSO_4$), evaporation *in vacuo* afforded 1.87g (96%) of the 9-[(phenyl)methyl]-5-carbomethoxy-2-(4-trifluoromethylphenyl)-1,2-dihydrocarbazol-4(3H)-one. $^1$H NMR ($CDCl_3$) δ 7.6 (d, J=8 Hz, 2H), 7.45-7.25 (m, 8H), 6.95 (m, 2H), 5.35 (s, 2H), 4.05 (s, 3H), 3.65 (m, 1H), 3.2 (dd, J=16 and 5 Hz, 1H), 3.0 (dd, J=16 and 10 Hz, 1H), 2.8 (m, 2H). MS (ES) m/e 478.

b) 9-[(Phenyl)methyl]-2-(4-trifluoromethylphenyl)-4-hydroxy-5-carbomethoxy carbazole

A solution of the 9-[(phenyl)methyl]-5-carbomethoxy-2-(4-trifluoromethylphenyl-1,2-dihydrocarbazol-4(3H)-one (1.87 g, 3.92 mM) and 2,3-dichloro-5,6-dicyano-1,4-benzoquinone (0.89 g, 3.92 mM) in 39 mL of toluene was refluxed for 25 minutes. The mixture was purified by column chromatography on silica gel (elution with toluene) to afford 1.10 g (59%) of the 9-[(phenyl)methyl]-2-(4-trifluoromethylphenyl)-4-hydroxy-5-carbomethoxy carbazole. $^1$H NMR ($CDCl_3$) δ 10.65 (s, 1H), 8.05 (d, J=8 Hz, 1H), 7.8 (m, 3H), 7.65 (m, 3H), 7.6 (d, J=8 Hz, 1H), 7.45 (dd, J=8 and 8 Hz, 1H), 7.3-7.1 (m, 2H), 5.6 (s, 2H), 4.1 (s, 3H). MS (ES) m/e 444. 476.

c) 9-[(Phenyl)methyl]-2-(4-trifluoromethylphenyl)-4-hydroxy-5-carbamoyl carbazole

A solution of the 9-[(phenyl)methyl]-2-(4-trifluoromethylphenyl)-4-hydroxy-5-carbomethoxy carbazole (1.10 g, 2.31 mM) in 35 ml THF and 140 mL concentrated aqueous ammonium hydroxide was sonicated for 6 hours at 30-40 °C. The precipitated solid was filtered and washed with water. Trituration with $Et_2O$, then with 2:1 $Et_2O/CH_2Cl_2$ afforded 0.20g (19%) of the 9-[(phenyl)methyl]-2-(4-trifluoromethylphenyl)-4-hydroxy-5-carbamoyl carbazole. $^1$H NMR (DMSO-d6) δ 10.8 (s, 1H) , 8.9 (bs, 1H), 8.45 (bs, 1H), 8.0 (d, J=8 Hz, 2H), 7.8 (d, J=8 Hz, 2H), 7.6 (s, 1H), 7.5 (m, 2H), 7.3-7.1

(m, 6H), 7.0 (s, 1H), 5.8 (s, 2H). MS (ES) m/e 444, 461.

d) {9-[(Phenyl)methyl]-2-(4-trifluoromethylphenyl)-5-carbamoylcarbazol-4-yl}oxyacetic acid, methyl ester

60% Sodium hydride in mineral oil (22 mg, 0.54 mM) was added to a solution of the 9-[(phenyl)methyl]-2-(4-trifluoromethylphenyl)-4-hydroxy-5-carbamoyl carbazole (0.20 g, 0.43 mM) in 15 mL DMF and 3 ml THF. After 7 minutes, methyl bromoacetate (56 µl, 0.59 mM) was added and the resultant mixture stirred at room temperature for 1 hour. The mixture was diluted with ethyl acetate and washed with $H_2O$. The aqueous layer was extracted with ethyl acetate. The combined organic layers were extracted with saturated brine, dried over sodium sulfate, filtered, and concentrated. The residue was purified by column chromatography on silica gel (elution with ethyl acetate) to afford 0.19 g (84%) of the {9-[(phenyl)methyl]-2-(4-trifluoromethylphenyl)-5-carbamoylcarbazol-4-yl}oxyacetic acid, methyl ester. $^1H$ NMR (DMSO-d6) δ 8.0 (d, J=8 Hz, 2H), 7.85 (d, J=8 Hz, 2H), 7.7 (s, 1H), 7.6 (d, J=8 Hz, 1H) , 7.6 (bs, 1H), 7.4 (dd, J=8 and 8 Hz, 1H), 7.3-7.1 (m, 6H), 7.1 (d, J=8 Hz, 1H), 7.0 (s, 1H), 5.8 (s, 2H), 5.1 (s, 2H), 3.7 (s, 3H). MS (FD) m/e 516,533.

| Elemental Analyses for $C_{30}H_{23}F_3N_2O_4$: | | | |
|---|---|---|---|
| Calculated | C, 67.66; | H, 4.36; | N, 5.26. |
| Found | C, 68.38; | H, 4.29 ; | N 5.67, |

e) {9-[(Phenyl)methyl]-2-(4-trifluoromethylphenyl)-5-carbamoylcarbazol-4-yl}oxyacetic acid

A solution of the {9-[(phenyl)methyl]-2-(4-trifluoromethylphenyl)-5-carbamoylcarbazol-4-yl}oxyacetic acid, methyl ester (101 mg, 0.19 mM) and 0.95 mL (1.9 mM) of 2 N NaOH in 9.5 mL of ethanol was stirred for 30 minutes at 25 °C. The reaction was acidified with 1N HCl to pH =2. After stirring 30 minutes, the resultant white precipitate was collected by filtration, washed with water, then dried *in vacuo* to afford 73 mg (75%) {9-[(phenyl)methyl]-2-(4-trifluoromethylphenyl)-5-carbamoylcarbazol-4-yl}oxyacetic acid. $^1H$ NMR (DMSO-d6) δ 11.0 (bs, 1H), 8.0 (d, J=8 Hz, 2H), 7.85 (d, J=8 Hz, 2H), 7.8 (bs, 1H), 7.7 (s, 1H), 7.65 (d, J=8 Hz, 1H), 7.4 (m, 2H), 7.3-7.1 (m, 6H), 7.0 (s, 1H), 5.8 (s, 2H), 5.0 (s, 2H). MS (ES) m/e 502,519.

| Elemental Analyses for $C_{29}H_{21}F_3N_2O_4$: | | | |
|---|---|---|---|
| Calculated | C, 67.17; | H, 4.09; | N, 5.40. |
| Found | C, 67.05; | H, 4.11; | N, 5.31. |

Example 12

Preparation of 9-benzyl-5-(2-methanesulfonamido)ethyloxy-7-methoxy-1,2,3,4-tetrahydrocarbazole-4-carboxamide

A. Preparation of 5-(2-amino)ethyloxy-9-benzyl-7-methoxy-1,2,3,4-tetrahydrocarbazole-4-carboxamide

To 1.93ml (1.93mmol) 1M lithium aluminum hydride/THF in 13ml THF at 0°C was added $H_2SO_4$ (53µl, 0.97mmol) dropwise over 5 min. The mixture was allowed to stir at room temperature 1 hour, then a solution of 9-benzyl-7-methoxy-5-cyanomethyloxy-1,2,3,4-tetrahydrocarbazole-4-carboxamide (0.50g, 1.29mmol) in 13ml THF was added dropwise at a rate which kept the temperature below 26°C. After an additional 45 minutes, the reaction was quenched with 0.5ml 1:1 THF/$H_2O$, 0.75ml 13% NaOH, and finally 80µl $H_2O$. The reaction was diluted with EtOAc and saturated $NaHCO_3$, and the layers separated. The organic layer was extracted with brine, dried over sodium sulfate, and evaporated *in vacuo*. The residue was chromatographed on silica gel eluting with 9:1:0.1 $CH_2Cl_2$/MeOH/$NH_4OH$ to give the subtitled product (0.28g, 55%). MS (ES+) 394

B. Preparation of 9-benzyl-5-(2-methanesulfonamido)ethyloxy-7-methoxy-1,2,3,4-tetrahydrocarbazole-4-carboxamide

To 0.20g (0.51mmol) of the product from Part A in 10ml THF at 0°C was added triethylamine (71µl, 0.51mmol) and methanesulfonylchloride (39µl, 0.51mmol). After 40 minutes at 0°C, the reaction was diluted with EtOAc and saturated $NaHCO_3$, and the layers separated. The organic layer was extracted with brine, dried over sodium sulfate, and evaporated *in vacuo*. The residue was chromatographed on silica gel eluting with 20:1 $CH_2Cl_2$/MeOH and crystallized from EtOAc/hexane to give the titled product (0.13g, 54%). MS (ES+) 472

| Elemental Analyses for $C_{24}H_{29}N_3O_5S$: | | | |
|---|---|---|---|
| Calculated | C 61.13; | H 6.20; | N 8.91 |
| Found | C 61.04; | H 6.16; | N 9.21 |

Example 13

Preparation of 9-benzyl-4-(2-methanesulfonamido)ethyloxy-2-methoxycarbazole-5-carboxamide

A. Preparation of 4-(2-amino)ethyloxy-9-benzyl-2-methoxycarbazole-5-carboxamide

To 1.25ml (1.25mmol) 1M lithium aluminum hydride/THF in 8.3ml THF at 0°C was added $H_2SO_4$ (34.5µl, 0.63mmol) dropwise over 5 min. The mixture was allowed to stir at room temperature 1 hour, then a suspension of 9-benzyl-4-cyanomethyloxy-2-methoxycarbazole-5-carboxamide (0.32g, 0.83mmol) in 8.3ml THF was added dropwise at a rate which kept the temperature below 26°C. After an additional 45 minutes, the reaction was quenched with 0.32ml 1:1 THF/$H_2O$, 0.48ml 13% NaOH, and finally 51µl $H_2O$. The reaction was diluted with EtOAc and saturated $NaHCO_3$, and the layers separated. The organic layer was extracted with brine, dried over sodium sulfate, and evaporated *in vacuo*. The residue was chromatographed on silica gel eluting with 9:1:0.1 $CH_2Cl_2$/MeOH/$NH_4OH$ to give the subtitled product (148mg, 46%). MS (ES+) 390

B. Preparation of 9-benzyl-4-(2-methanesulfonamido)ethyloxy-2-methoxycarbazole-5-carboxamide

To 107mg (0.27mmol) of the product from Part A in 11ml THF at 0°C was added triethylamine (38µl,0.27mmol) and methanesulfonylchloride (21µl, 0.27mmol). After 40 minutes at 0°C, the reaction was diluted with EtOAc and saturated $NaHCO_3$, and the layers separated. The organic layer was extracted with brine, dried over sodium sulfate, and evaporated *in vacuo*. The residue was chromatographed on silica gel eluting with 10:1 $CH_2Cl_2$/acetone and crystallized from EtOAc/hexane to give the titled product (28.6mg, 22%). MS (ES+) 468

| Elemental Analyses for $C_{24}H_{25}N_3O_5S$: | | | |
|---|---|---|---|
| Calculated | C 61.66; | H 5.39; | N 8.99 |
| Found | C 61.52; | H 5.31; | N 8.81 |

Example 14

Preparation of 9-benzyl-4-(2-trifluoromethanesulfonamido)ethyloxy-2-methoxycarbazole-5-carboxamide

To 31.2mg (0.08mmol) of the product from Example 13, Part A in 3.2ml THF at 0°C was added triethylamine (11.1µl, 0.08mmol) and trifluoromethanesulfonylchloride (8.5µl, 0.08mmol). After 40 min at 0°C, the reaction was diluted EtOAc and saturated $NaHCO_3$, and the layers separated. The organic layer was extracted with brine, dried over sodium sulfate, and evaporated *in vacuo*. The residue was chromatographed on silica gel eluting with a $CH_2Cl_2$/EtOAc gradient and triturated with ether to give the titled product (19.3mg, 46%). MS (ES+) 522

| Elemental Analyses for $C_{24}H_{22}F_3N_3O_5S$: | | | |
|---|---|---|---|
| Calculated | C 55.27; | H 4.25; | N 8.06 |
| Found | C 55.11; | H 4.40; | N 7.99 |

Example 15

Preparation of 9-benzyl-5-methanesulfonamidoylmethyloxy-7-methoxy-1,2,3,4-tetrahydrocarbazole-4-carboxamide

To 51mg (0.125mmol) of (9-benzyl-4-carbamoyl-7-methoxy-1,2,3,4-tetrahydrocarbazol-5-yl)oxyacetic acid in 25ml THF was added carbonyldiimidazole (20.2mg, 0.125mmol). The reaction was refluxed for 21 hours, then allowed to cool to room temperature . To this was added a mixture of methanesulfonamide (11.9mg, 0.125mmol) and diazabicycloundecene (18.7µl, 0.125mmol) in 2.5ml THF. After 3.5 hours, the reaction was diluted with EtOAc and extracted with 10% $NaHSO_3$, saturated $NaHCO_3$, 10% $NaHSO_3$, and brine, respectively. The organic layer was dried with sodium

sulfate and evaporated *in vacuo*. The residue was chromatographed on silica gel eluting with a $CH_2Cl_2$/EtOH gradient to give the titled product (8mg, 10%).

| High Resolution MS for $C_{24}H_{27}N_3O_6S$: | |
|---|---|
| Calculated | 485.1621 |
| Found | 485.1625 |

Example 16

Preparation of 9-benzyl-4-methanesulfonamidoylmethyloxycarbazole-5-carboxamide

To 48mg (0.13mmol) of (9-benzyl-5-carbamoyl-carbazol-4-yl)oxyacetic acid in 26ml THF was added carbonyldiimidazole (21mg, 0.13mmol). The reaction was refluxed for 25 hours, then allowed to cool to room temperature . To this was added a mixture of methanesulfonamide (12mg, 0.13mmol) and diazabicycloundecene (19µl, 0.13mmol) in 2.6ml THF. After 3 hours, the reaction was diluted with EtOAc and extracted with 10% $NaHSO_3$, then brine. The organic layer was dried with sodium sulfate and evaporated *in vacuo*. The residue was chromatographed on silica gel eluting with a $CH_2Cl_2$/EtOH gradient to give impure product. Extraction from EtOAc into saturated $NaHCO_3$ and reacidification gave the titled product (3.9mg, 6.7%).

| High Resolution MS for $C_{23}H_{21}N_3O_5S$: | |
|---|---|
| Calculated | 452.1280 |
| Found | 452.1284 |

Example 17

[5-carbamoyl-2-pentyl-9-(phenylmethyl)carbazol-4-yl]oxyacetic acid

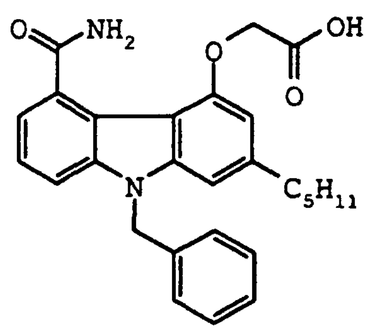

A. Preparation of a mixture of 5-pentylcyclohexa-1,3-dione and its enol isomer

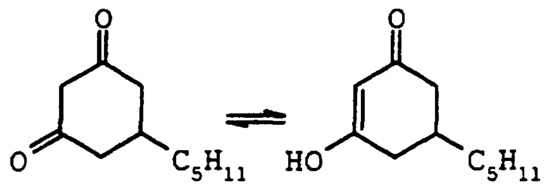

Sodium hydroxide (1.98 g, 49.5 mmol) was added to a stirred suspension of olivetol (7.20 g, 39.9 mmol) in THF (20 mL)/$H_2O$ (20 mL) at ambient temperature under nitrogen atmosphere. The solution was stirred until it became

a clear solution. Stir bar was removed before 5% $Rh/Al_2O_3$ (500 mg) was added to the solution. The mixture was then subject to hydrogenation condition under a 60 pounds per square inch hydrogen atmosphere in a Parr shaker for 17 hours. After filtration through celite, the filtrate was cooled to 0 °C, then treated with 5 N HCl (10.9 mL). The mixture was evaporated *in vacuo* at 40 °C and the residue was chromatographed on silica (gradient 40-100% ethyl acetate in hexane, then 0-15% methanol in ethyl acetate) to give sub-titled compound (4.80 g, 66%) as a white solid mixture of keto/enol isomers in a 3:2 ratio. mp 68.5-69.5 °C; IR (KBr) 3200-2400 (br), 1610, 1542 $cm^{-1}$; $^1H$ NMR ($CDCl_3$) δ 0.88 (br t, $J$ = 6.6 Hz, 3H, $-CH_3$), 3.38 (s, 2H, $-CH_2-$ of keto isomer), 4.13 (s, 1H, =CH- of enol isomer), 8.90 (br s, 1H, -OH); ESIMS *m*/*e* 183 ($M^+$+1);

| Elemental Analyses for $C_{11}H_{18}O_2$: | | |
|---|---|---|
| Calculated | C, 72.49; | H, 9.95. |
| Found | C, 72.72; | H, 9.95. |

B. Preparation of a mixture of 5-(hydroxymethyl)clohexa-1,3-dione and its enol isomer

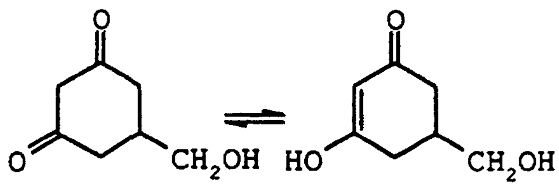

Following the experimental procedure as described in part A, above synthesis of subtitled compound was obtained in a 75% yield. IR (KBr) 3547, 3453 (br), 1633, 1580 $cm^{-1}$; $^1H$ NMR (DMSO-$d_6$) δ 1.90-2.30 (m, 5H), 3.30 (br s, 2H, $-CH_2O-$), 4.61 (br s, 1H, -OH), 5.13 (s, 1H), 10.94 (s, 1H, -OH); ESIMS *m*/*e* 143 ($M^+$+1);

| Elemental Analyses for $C_7H_{10}O_3$: | | |
|---|---|---|
| Calculated | C, 59.14; | H, 7.09. |
| Found | C, 59.44; | H, 7.08. |

C. Preparation of 3-(2-bromo-3-carbomethoxyanilino)-5-pentylcyclohex-2-en-1-one

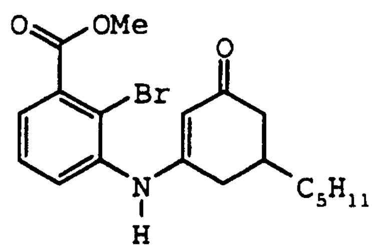

A stirred mixture of methyl-3-amino-2-bromobenzoate prepared as described in Preparation 4 (5.12 g, 22.3 mmol) and the compound of Part A (4.06 g, 22.3 mmol) was heated in an oil bath at 150 °C for 1.4 hours under a positive nitrogen pressure to continuously remove the water vapor. At ambient temperature, the mixture was chromatographed on silica (gradient 30-100% ethyl acetate in hexane) to provide subtitled compound (6.06 g, 69%) as a white solid. mp 132.0-134.0 °C; IR (KBr) 3220 (br), 1726, 1580 $cm^{-1}$; $^1H$ NMR ($CDCl_3$) δ 0.90 (br t, $J$ = 6.6 Hz, 3H, $-CH_3$), 1.25-1.45 (m, 8H), 2.05-2.27 (m, 2H), 2.35-2.57 (m, 3H), 3.94 (s, 3H, $-OCH_3$), 5.57 (s, 1H, =CH-), 6.44 (br s, 1H, -NH), 7.35 (t, $J$ = 6.8 Hz, 1H), 7.53 (d, $J$ = 6.8 Hz, 2H); ESIMS *m*/*e* 394 ($M^+$+1, $^{79}Br$), 396 ($M^+$+1, $^{81}Br$).

D. Preparation of 5-carbomethoxy-1,2-dihydro-2-pentyl-9H-carbazol-4(3H)-one

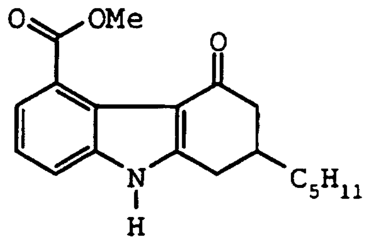

Triethylamine (2.09 mL, 15.0 mmol) was added to a stirred suspension of the compound of part C, above (3.94 g, 10.0 mmol), $Pd(OAc)_2$ (338 mg, 1.50 mmol), and tri-*o*-tolylphosphine (914 mg, 3.00 mmol) in acetonitrile (40 mL) at ambient temperature under nitrogen atmosphere. The resultant mixture was then heated in an oil bath at 85 °C for 1 h. The mixture was evaporated *in vacuo* at 35 °C and the residue was chromatographed on silica (gradient 20-100% ethyl acetate in hexane) to give subtitled compound (2.45 g, 78%) as a white solid. mp 116.0-117.5 °C; IR (KBr) 3379 (br), 3180 (Br), 1725, 1627 $cm^{-1}$; $^1H$ NMR ($CDCl_3$) δ 0.89 (br t, *J* = 6.6 Hz, 3H, $-CH_3$), 1.20-1.47 (m, 8H), 2.20-2.32 (m, 2H), 2.50-2.67 (m, 2H), 2.92-3.05 (m, 1H), 4.02 (s, 3H, $-OCH_3$), 7.18-7.26 (m, 1H), 7.35-7.43 (m, 2H), 9.20-9.42 (br s, 1H, -NH); ESIMS *m/e* 314 ($M^+$+1);

| Elemental Analyses for $C_{19}H_{23}NO_3$: | | | |
|---|---|---|---|
| Calculated | C, 72.82; | H, 7.40; | N, 4.47. |
| Found | C, 72.59; | H, 7.43; | N, 4.51. |

E. Preparation of 5-carbomethoxy-1,2-dihydro-2-pentyl-9-(phenylmethyl)carbazol-4(3H)-one

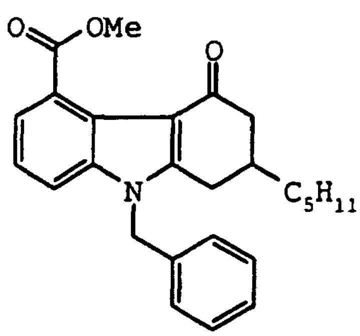

Benzylbromide (1.25 mL, 10.5 mmol) was added to a stirred suspension of the compound of example 17D (3.00 g, 9.57 mmol) and potassium carbonate (1.98 g, 14.4 mmol) in anhydrous DMF (30 mL) under nitrogen atmosphere. The resultant mixture was stirred for 5 hours. The mixture was evaporated *in vacuo* at 40 °C and the residue was chromatographed on silica (gradient 10-60% ethyl acetate in hexane) to give subtitled compound (3.28 g, 85%) as a white solid. mp 119.0-120.5 °C; IR (KBr) 1723, 1650 $cm^{-1}$; $^1H$ NMR ($CDCl_3$) δ 0.87 (br t, *J* = 6.6 Hz, 3H, $-CH_3$), 1.23-1.52 (m, 8H), 2.25-2.40 (m, 2H), 2.47-2.57 (m, 1H), 2.69 (d, *J* = 12.8 Hz, 1H), 2.99 (dd, *J* = 16.6, 3.6 Hz, 1H), 4.05 (s, 3H, $-OCH_3$), 5.36 (s, 2H), 6.98-7.02 (m, 2H), 7.20-7.40 (m, 6H); ESIMS *m/e* 404 ($M^+$+1).

F. Preparation of 5-carbomethoxy-4-hydroxy-2-pentyl-9-(phenylmethyl)carbazole

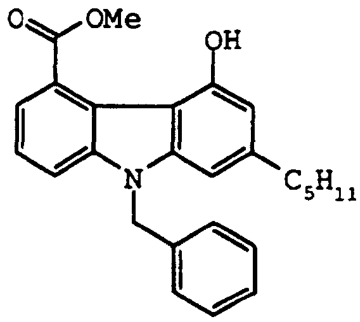

(a) from DDQ oxidation: DDQ (563 mg, 2.48 mmol) was added to a stirred suspension of the compound of part E, above (1.00 g, 2.48 mmol) in anhydrous toluene (30 mL) under nitrogen atmosphere. The resultant mixture was heated to reflux for 25 min. At ambient temperature, the mixture was subject to chromatographic purification on silica (gradient 0-30% ethyl acetate in toluene) to give subtitled compound (290 mg, 29%) as a yellow solid (310 mg, 31%).

(b) from benzenesulfinate elimination: Sodium hydride (60% in oil, 192 mg, 4.80 mmol) was added to a stirred solution of the compound of part E, above (807 mg, 2.00 mmol) and methyl benzenesulfinate (375 mg, 2.40 mmol) in anhydrous 1,4-dioxane (10 mL) under nitrogen atmosphere. The mixture was heated in an oil bath at 50 °C for 2h 15 min. After dilution with additional 15 mL 1,4-dioxane, the mixture was treated with acetic acid (0.343 mL, 6.00 mmol) and the resultant suspension was heated to reflux for 40 min. The mixture was evaporated *in vacuo* and the residue was chromatographed on silica (gradient 0-5% ethyl acetate in toluene) to afford subtitled compund (690 mg, 86%) as a yellow solid. mp 130.0-132.0 °C; IR (KBr) 3200 (br), 1686 $cm^{-1}$; $^1H$ NMR ($CDCl_3$) δ 0.87 (br t, *J* = 6.6 Hz, 3H, $-CH_3$), 1.25-1.38 (m, 4H), 1.60-1.75 (m, 2H), 2.69 (t, *J* = 7.7 Hz, 2H), 4.10 (s, 3H, $-OCH_3$), 5.52 (s, 2H), 6.71 (s, 1H), 6.76 (s, 1H), 7.09-7.11 (m, 2H), 7.20-7.30 (m, 3H), 7.37 (t, *J* = 8.0 Hz, 1H), 7.55 (d, *J* = 8.0 Hz, 1H) , 7.97 (d, *J* = 8.0 Hz, 1H), 10.43 (s, 1H, -OH); ESIMS *m/e* 402 ($M^+$+1);

| Elemental Analyses for $C_{26}H_{27}NO_3$•0.2($C_7H_8$) : | | | |
|---|---|---|---|
| Calculated | C, 78.37; | H, 6.86; | N, 3.34. |
| Found | C, 78.48; | H, 6.68; | N, 3.53. |

G. Preparation of 5-carbamoyl-4-hydroxy-2-pentyl-9-(phenylmethyl)carbazole

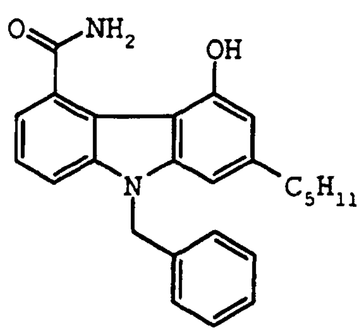

Ammonia was bubbled through a stirred suspension of the compund of part F, above (590 mg, 1.47 mmol) in ammonia water (50 mL)/THF (10 mL) at -25 °C for 5 minutes in a pressure bottle. The bottle was screw-capped before

the mixture was allowed to stir at ambient temperature for 3 days. After cooling to -25 °C, the screw cap was removed and the mixture was allowed to stir at ambient temperature for 10 minutes. After concentration, the residue was subject to chromatographic purification on silica (gradient 0-40% tetrahydrofuran in toluene) to recover the compound of part F (160 mg, 27%) and obtain the desired subtitled product (397 mg, 70%) as a yellowish solid. IR (KBr) 3437, 3200 (br), 1633, 1601 $cm^{-1}$; $^1H$ NMR ($CDCl_3$) δ 0.86 (br t, *J* = 6.6 Hz, 3H, $-CH_3$), 1.22-1.38 (m, 4H), 1.60-1.75 (m, 2H), 2.69 (t, *J* = 7.7 Hz, 2H), 5.52 (s, 2H) , 6.16 (s, 1H, -NH), 6.53 (s, 1H, -NH), 6.72 (s, 1H), 6.76 (s, 1H), 7.07-7.11 (m, 2H), 7.23-7.30 (m, 3H), 7.35 (t, *J* = 7.7 Hz, 1H), 7.43 (d, *J* = 7.7 Hz, 1H), 7.48 (d, *J* = 7.7 Hz, 1H), 9.80 (s, 1H, -OH); ESIMS *m*/*e* 387 ($M^+$+1);

| Elemental Analyses for $C_{25}H_{26}N_2O_2$: | | | |
|---|---|---|---|
| Calculated | C, 77.69; | H, 6.78; | N, 7.25. |
| Found | C, 77.69; | H, 6.63; | N, 7.15. |

H. Preparation of [5-carbamoyl-2-pentyl-9-(phenylmethyl)carbazol-4-yl]oxyacetic acid, methyl ester

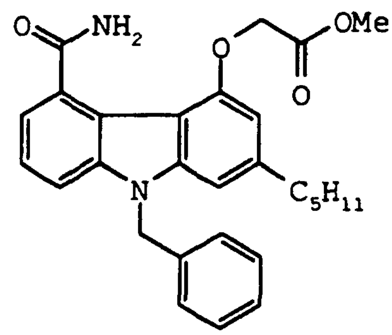

Methyl bromoacetate (48.0 mg, 0.314 mmol) was added to a stirred suspension of the compound of example 17G, above (110 mg, 0.285 mmol) and cesium carbonate (186 mg, 0.570 mmol) in anhydrous DMF (2 mL) at ambient temperature under nitrogen atmosphere. The resultant mixture was stirred for 1 hour. After concentration *in vacuo* at 40 °C, the residue was chromatographed on silica (gradient 10-60% tetrahydrofuran in toluene) to give subtitled product (115 mg, 88%) as a white solid. mp 195.0-196.0 °C; IR (KBr) 3365 (br), 3157 (br), 1758, 1640 $cm^{-1}$; $^1H$ NMR ($CDCl_3$) δ 0.87 (br t, *J* = 6.6 Hz, 3H, $-CH_3$), 1.22-1.35 (m, 4H), 1.58-1.70 (m, 2H), 2.69 (t, *J* = 7.6 Hz, 2H), 3.84 (s, 3H, $-OCH_3$), 4.89 (s, 2H, $-OCH_2-$), 5.50 (s, 2H, $-NCH_2-$), 5.95 (br s, 1H, -NH), 6.08 (br s, 1H, -NH), 6.41 (s, 1H), 6.85 (s, 1H), 7.07-7.11 (m, 2H), 7.23-7.40 (m, 6H); ESIMS *m*/*e* 459 ($M^+$+1);

| Elemental Analyses for $C_{28}H_{30}N_2O_4$: | | | |
|---|---|---|---|
| Calculated | C, 73.34; | H, 6.59; | N, 6.11. |
| Found | C, 73.56; | H, 6.43; | N, 6.25. |

I. Preparation of [5-carbamoyl-2-pentyl-9-(phenylmethyl)carbazol-4-yl]oxyacetic acid

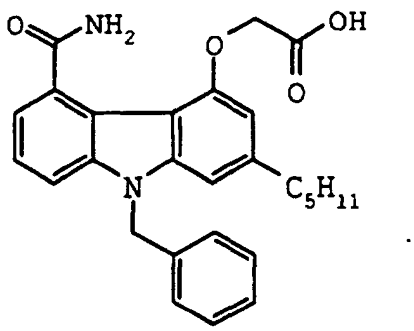

Lithium hydroxide (4.17 N, 86.3 mL, 0.360 mmol) was added to a stirred suspension of the compound of Example 17H, above (110 mg, 0.240 mmol) in THF (2 mL)/$CH_3OH$ (0.3 mL)/$H_2O$ (0.3 mL). The resultant mixture was stirred in an oil bath at 55 °C for 30 minutes to form a white suspension. Five milliliter of THF was added to the suspension before it was treated with 5 N HCl (96.0 mL, 0.480 mmol) to become a clear solution. After concentration, the white solid was resuspended in THF (0.5 mL)/$H_2O$ (5 mL), sonicated, filtered, and dried to give the subtitled compound (106 mg, 99%) as a white solid. IR (KBr) 3458 (br), 3500-3100 (br), 1656, 1620 $cm^{-1}$; $^1H$ NMR (DMSO-$d_6$) δ 0.80 (br t, *J* = 6.6 Hz, 3H, -$CH_3$), 1.18-1.30 (m, 4H), 1.50-1.62 (m, 2H), 2.61 (br t, *J* = 7.3 Hz, 2H), 4.55 (s, 2H, -$OCH_2$-), 5.60 (s, 2H, -$NCH_2$-), 6.40 (s, 1H), 6.95-7.32 (m, 9H), 7.51 (d, *J* = 8.0 Hz, 1H), 7.70 (br s, 1H, -NH); ESIMS *m/e* 445 ($M^+$+1).

Example 18

[5-carbamoyl-2-(1-methylethyl)-9-(phenylmethyl)carbazol-4-yl]oxyacetic acid

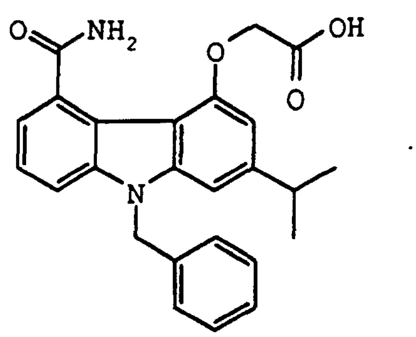

A. Preparation of 3-(2-bromo-3-carbomethoxyanilino)-5-[(1-methyl)ethyl]cyclohex-2-en-1-one

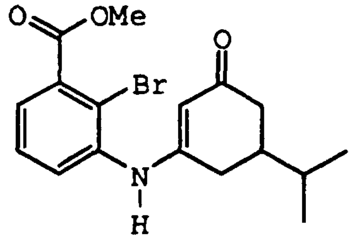

Prepared in 70% yield by the method of Example 17 part C. mp 129.0-130.0 °C; $^1$H NMR ($CDCl_3$) δ 0.98 (t, *J*=5.5 Hz, 6H), 1.66 (m, 1H), 2.00 (m, 1H), 2.14 (t, *J* = 14.8 Hz, 1H), 2.46 (m, 3H), 4.00 (s, 3H), 5.57 (s, 1H), 6.40 (br s, 1H), 7.35 (t, *J* = 7.9 Hz, 1H), 7.35 (d, *J* = 7.8 Hz, 2H); ESIMS *m/e* 366 ($M^+$+1, $^{79}$Br), 368 ($M^+$+1, $^{81}$Br).

B Preparation of 5-carbomethoxy-1,2-dihydro-2-(1-methylethyl)-9H-carbazol-4(3H)-one

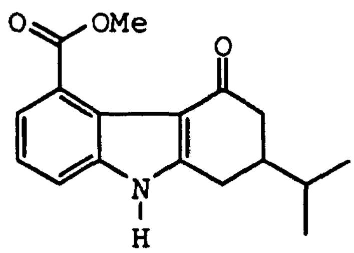

Prepared in 65% yield by the procedure of Example 17D. mp 175.0-177.0 °C; $^1$H NMR ($CDCl_3$) δ 0.95 (t, *J*=6.7 Hz, 6H), 1.62 (m, 1H), 2.05 (m, 1H), 2.27 (dd, *J* = 16.1, 12.6 Hz, 1H), 2.60 (m, 2H), 2.89 (dd, *J* = 16.4, 4.3 Hz, 1H), 4.02 (s, 3H), 7.23 (m, 1H), 7.36 (m, 2H), 9.28 (br s, 1H); ESIMS *m/e* 286 ($M^+$+1);

| Elemental Analyses for $C_{17}H_{19}NO_3$: | | | |
|---|---|---|---|
| Calculated | C,71.56; | H,6.71; | N,4.91. |
| Found | C, 71.43; | H, 6.62; | N, 4.74. |

C. Preparation of 5-carbomethoxy-1,2-dihydro-2-(1-methylethyl)-9-(phenylmethyl)carbazol-4(3H)-one

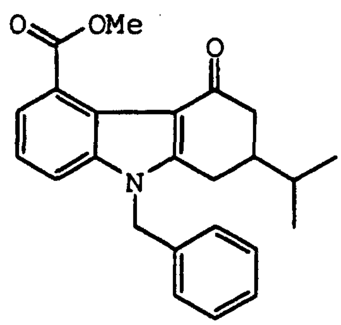

Prepared in 37% yield by the method of Example 17E. mp: 155.0-156.0 °C; $^1$H NMR ($CDCl_3$) δ 1.28 (d, *J* = 8.0 Hz, 6H), 3.00 (m, 1H), 4.10 (s, 3H), 5.23 (s, 2H), 6.78 (d, *J* = 9.5 Hz, 2H), 7.11 (m, 2H), 7.28 (m, 3H), 7.37 (t, *J* = 7.9 Hz, 1H), 7.54 (d, *J* = 7.9 Hz, 1H), 7.98 (d, *J* = 7.9 Hz, 1H), 10.46 (s, 1H); ESIMS *m/e* 374 ($M^+$+1);

| Elemental Analyses for $C_{24}H_{23}NO_3$: | | | |
|---|---|---|---|
| Calculated | C, 77.19; | H, 6.21; | N, 3.75. |
| Found | C, 76.96; | H, 6.33; | N, 3.77. |

D. Preparation of 5-carbomethoxy-4-hydroxy-2-(1-methylethyl)-9-(phenylmethyl)carbazole

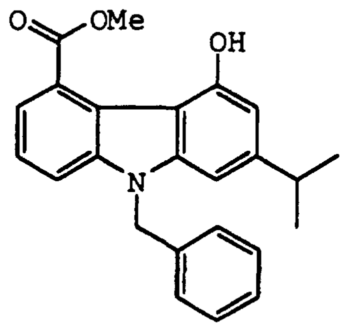

DDQ (1.15 g, 5.07 mmol) was added to a stirred suspension of the compound of Example 18C (1.90 g, 5.07 mmol) in anhydrous toluene (30 mL) under argon atmosphere. The resultant mixture was heated under reflux for 25 min. After cooling to room temperature, the mixture was subjected to chromatography on silica gel eluting with a gradient of hexanes/toluene(1:1) to toluene/EtOAc (97:3). The desired product was obtained a mixture with the corresponding isopropylidene compound. The mixture was dissolved in EtOAc (30 mL) under nitrogen atmosphere, and 0.1 g of $PtO_2$ was added. The mixture was stirred at room temperature under hydrogen at balloon pressure for 25 min. Filtration through celite, followed by recrystallization from $Et_2O$/hexanes gave the subtitled compound as a pale yellow crystalline solid (0.705 g; 37% yield). mp: 155.0-156.0 °C; $^1$H NMR ($CDCl_3$) δ 1.28 (d, *J* = 8.0 Hz, 6H), 3.00 (m, 1H), 4.10 (s, 3H), 5.23 (s, 2H), 6.78 (d, *J* = 9.5 Hz, 2H), 7.11 (m, 2H), 7.28 (m, 3H), 7.37 (t, *J* = 7.9 Hz, 1H), 7.54 (d, *J* = 8.0 Hz, 1H), 7.98 (d, *J* = 7.9 Hz, 1H), 10.46 (s, 1H); ESIMS *m/e* 374 ($M^+$+1);

| Elemental Analyses for $C_{24}H_{23}NO_3$: | | | |
|---|---|---|---|
| Calculated | C, 77.19; | H, 6.21; | N, 3.75. |
| Found | C, 76.96; | H, 6.33; | N, 3.77. |

E. Preparation of 5-carbamoyl-4-hydroxy-2-(1-methylethyl)-9-(phenylmethyl)carbazole

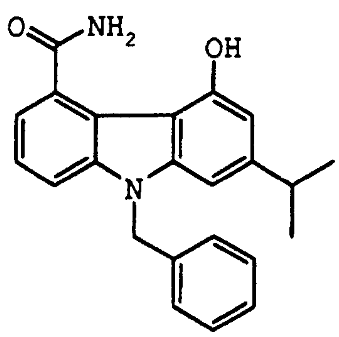

Prepared in 50% yield by the procedure of example 17G. mp 216.0-218.0 °C; $^1$H NMR ($CDCl_3$) δ 1.29 (d, *J* = 6.9, 6H), 3.00 (m, 1H), 5.53(s, 2H), 6.16 (br s, 1H), 6.52 (br s, 1H), 6.78 (d, *J* = 8.6 Hz, 2H), 7.11 (m, 2H), 7.28 (m, 3H), 7.32 (m, 1H), 7.4 (m, 2H), 9.8 (br s, 1H); ESIMS *m/e* 359.3 ($M^+$+1);

| Elemental Analyses for $C_{23}H_{22}N_2O_2$: | | | |
|---|---|---|---|
| Calculated | C, 77.07; | H, 6.19; | N, 7.82. |
| Found | C, 77.10; | H, 6.35; | N, 7.74. |

F. Preparation of [5-carbamoyl-2-(1-methylethyl)-9-(phenylmethyl)carbazol-4-yl]oxyacetic acid, methyl ester

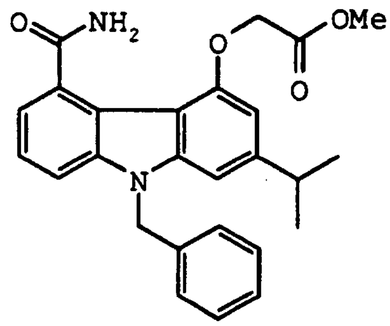

Prepared by the procedure of example 17H in 54% yield. mp 189.0-191.0 °C; $^1$H NMR ($CDCl_3$) δ 1.27 (d, *J* = 6.9 Hz, 6H), 2.98 (m, 1H), 3.84 (s, 3H), 4.90 (s, 2H), 5.51(s, 2H), 5.8-6.2 (m, 2H), 6.47 (s, 1H), 6.90 (s, 1H), 7.11 (m, 2H), 7.2-7.4 (m, 6H); ESIMS *m*/*e* 431 ($M^+$+1);

| Elemental Analyses for $C_{26}H_{26}N_2O_4$: | | | |
|---|---|---|---|
| Calculated | C, 72.54; | H, 6.09; | N, 6.51. |
| Found | C, 72.58; | H, 6.24; | N, 6.43. |

G. Preparation of [5-carbamoyl-2-(1-methylethyl)-9-(phenylmethyl)carbazol-4-yl]oxyacetic acid

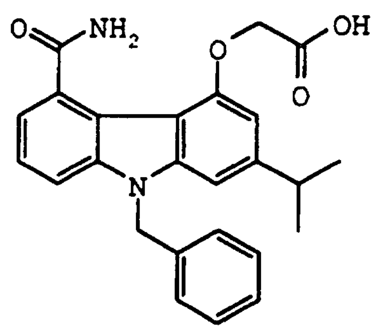

Prepared by the procedure of example Example 17I in 82% yield. $^1$H NMR ($CDCl_3$) δ 1.20 (d, *J* = 6.7, 6H), 2.94 (m, 1H), 4.79 (s, 2H), 5.63 (s, 2H), 6.49(s, 1H), 7.00-7.40 (m, 9H), 7.51 (m, 1H), 7.70 (br s, 1H), 12.94 (br s, 1H); ESIMS *m*/*e* 417 ($M^+$+1);

| Elemental Analyses for $C_{25}H_{24}N_2O_4$: | | | |
|---|---|---|---|
| Calculated | C, 72.10; | H, 5.81; | N, 6.73. |
| Found | C, 72.11; | H, 5.62; | N, 6.49. |

Example 19

[5-carbamoyl-9-(phenylmethyl)-2-[(tri(-1-methylethyl)silyl)oxymethyl]carbazol-4-yl]oxyacetic acid

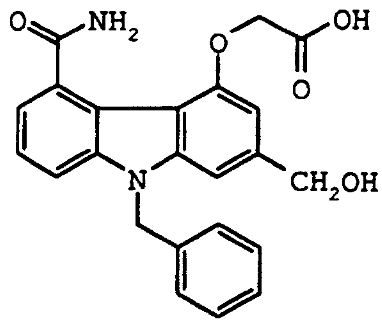

A. Preparation of 3-(2-bromo-3-carbomethoxyanilino)-5-(hydroxymethyl)cyclohex-2-en-1-one

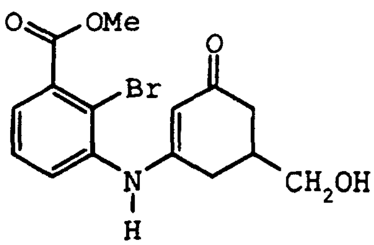

Following the experimental procedure as described in the synthesis of Example 17 part C, subtitled compound was obtained as a white solid in a 68% yield. IR (KBr) 3407 (br), 3364 (br), 3222 (br), 1738, 1600, 1566 $cm^{-1}$; $^1H$ NMR (DMSO-$d_6$) $\delta$ 1.94 (dd, *J* = 16.5, 12.5 Hz, 1H), 2.02-2.15 (m, 2H), 2.32 (dd, *J* = 16.5, 9.9 Hz, 1H), 2.50-2.58 (m, 1H), 3.27-3.42 (m, 2H), 3.83 (s, 3H, -$OCH_3$), 4.55 (s, 1H, =CH-), 4.64 (t, *J* = 5.1 Hz, 1H, -OH), 7.42-7.58 (m, 3H), 8.76 (s, 1H, -NH); ESIMS *m*/*e* 354 ($M^+$+1, $^{79}Br$), 356 ($M^+$+1, $^{81}Br$);

| Elemental Analyses for $C_{15}H_{16}BrNO_4$: | | | |
|---|---|---|---|
| Calculated | C, 50.87; | H, 4.55; | N, 3.95. |
| Found | C, 51.07; | H, 4.60; | N, 3.93. |

B. Preparation of 5-carbomethoxy-1,2-dihydro-2-(hydroxymethyl)-9H-carbazol-4(3H)-one

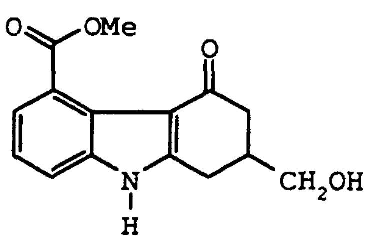

Following the experimental procedure as described in the synthesis of Example 17D, subtitled compound was obtained as a white solid in a 66% yield. IR (KBr) 3350 (br), 1720, 1624 $cm^{-1}$; $^1H$ NMR ($CDCl_3$) δ 1.92-2.20 (m, 2H), 2.33 (dd, *J* = 16.0, 3.0 Hz, 1H) , 2.44 (dd, *J* = 16.8, 9.9 Hz, 1H), 2.67 (dd, *J* = 16.8, 4.0 Hz, 1H), 3.33-3.48 (m, 2H), 4.05 (s, 3H, $-OCH_3$), 7.20-7.26 (m, 1H) , 7.33 (d, *J* = 7.3 Hz, 1H), 7.43 (d, *J* = 7.9 Hz, 1H), 10.25 (s, 1H, -NH); ESIMS *m/e* 274 ($M^{+}$+1);

| Elemental Analyses for $C_{15}H_{15}NO_4$: | | | |
|---|---|---|---|
| Calculated | C, 65.93; | H, 5.53; | N, 5.13. |
| Found | C, 65.68; | H, 5.78; | N, 5.08. |

C. Preparation of 5-carbomethoxy-1,2-dihydro-2-(hydroxymethyl)-9-(phenylmethyl)carbazol-4(3H)-one

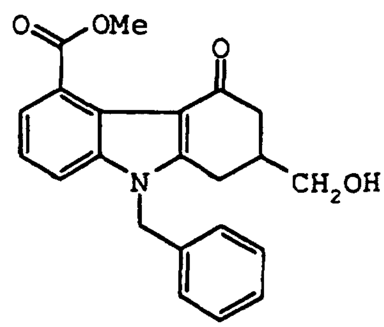

Following the experimental procedure as described in the synthesis of Example 17E, the subtitled compound was obtained as a white solid in a 88% yield. IR (KBr) 3366 (br), 1728, 1632 $cm^{-1}$; $^1H$ NMR ($CDCl_3$) δ 1.98 (s, 1H, -OH), 2.30-2.62 (m, 3H), 2.72 (dd, *J* = 16.8, 9.7 Hz, 1H), 3.06 (dd, *J* = 17.0, 3.6 Hz, 1H), 3.58-3.75 (m, 2H), 4.04 (s, 3H, $-OCH_3$), 5.25-5.40 (m, 2H, $-NCH_2-$), 6.98-7.05 (m, 2H), 7.20-7.40 (m, 6H); ESIMS *m/e* 364 ($M^{+}$+1).

D. Preparation of [5-carbamoyl-9-(phenylmethyl)-2-[(tri(-1-methylethyl)silyl)oxymethyl]carbazol-4-yl]oxyacetic acid, methyl ester

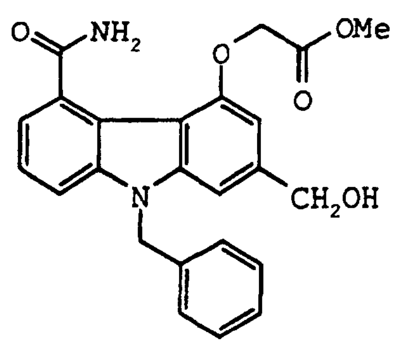

etrabutylammonium fluoride (1 N in THF, 0.626 mL) was added to a stirred solution of the compound of Example 23D (300 mg, 0.522 mmol) in THF (5 mL). The mixture was stirred at ambient temperature for 1 hour. After conentration *in vacuo* at 35 °C, the residue was subject to chromatographic purification (gradient 50-100% tetrahydrofuran in toluene, then 5% methanol in tetrahydrofuran) to give subtitled compound (122 mg, 56%) as a white solid. IR (KBr) 3380 (br), 3205 (br), 1733, 1641, 1628 $cm^{-1}$; $^1H$ NMR (DMSO-$d_6$) δ 3.69 (s, 3H, $-OCH_3$), 4.55 (s, 2H, $-OCH_2-$), 4.86 (s, 2H, $-OCH_2-$), 5.22 (br s, 1H, -OH), 5.62 (s, 2H, $-NCH_2-$), 6.53 (s, 1H), 7.00-7.25 (m, 8H), 7.32 (br t, *J* = 7.7 Hz, 1H), 7.50 (br d, *J* = 7.7 Hz, 1H), 7.53 (br d, *J* = 7.7 Hz, 1H); ESIMS *m/e* 419 ($M^{+}$+1);

| Elemental Analyses for $C_{24}H_{22}N_2O_5$: | | | |
|---|---|---|---|
| Calculated | C, 68.89; | H, 5.30; | N, 6.69. |
| Found | C, 68.80; | H, 5.17; | N, 6.72. |

E. Preparation of [5-carbamoyl-9-(phenylmethyl)-2-[(tri(-1-methylethyl)silyl)oxymethyl]carbazol-4-yl]oxyacetic acid

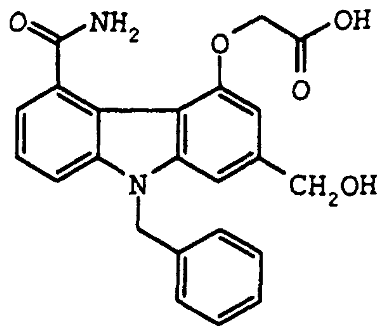

Following the experimental procedure as described in the synthesis of Example 17I, subtitiled compound was obtained as a white solid in a 99% yield. IR (KBr) 3427, 3331 (br), 1732, 1682, 1636 $cm^{-1}$; $^1H$ NMR (DMSO-$d_6$) δ 4.55 (d, *J* = 4.6 Hz, 2H, -OC$\underline{H_2}$OH), 4.78 (s, 2H, -$OCH_2$-), 5.25 (br t, *J* = 4.6 Hz, 1H, -OH), 5.62 (s, 2H, -$NCH_2$-), 6.57 (s, 1H), 7.00-7.25 (m, 7H), 7.33 (br t, *J* = 7.8 Hz, 1H), 7.39 (s, 1H, - NH), 7.55 (d, *J* = 7.8 Hz, 1H), 7.72 (s, 1H, -NH), 12.93 (s, 1H, -$CO_2H$); ESIMS *m/e* 405 ($M^+$+1);

| Elemental Analyses for $C_{23}H_{20}N_2O_5$•$0.3H_2O$: | | | |
|---|---|---|---|
| Calculated | C, 67.41; | H, 5.07; | N, 6.84. |
| Found | C, 67.34; | H, 5.13; | N, 6.98. |

Example 20

Preparation of [5-carbamoyl-2-phenyl-9-(phenylmethyl)carbazol-4-yl]oxyacetic acid

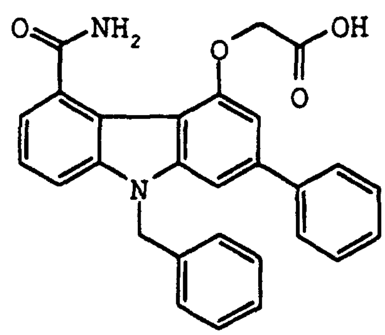

A. Preparation of 3-(2-bromo-3-carbomethoxyanilino)-5-phenylcyclohex-2-en-1-one

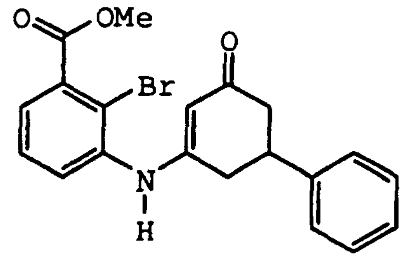

Prepared in 61% yield by the method of Example 17, part A. IR (KBr) 3180 (br), 1734, 1592 $cm^{-1}$; $^1H$ NMR ($CDCl_3$) $\delta$ 2.64-2.71 (m, 2H), 2.84 (dd, $J$ = 11.7, 16.2 Hz, 1H), 3.45-3.49 (m, 1H), 3.95 (s, 3H) 5.67 (s, 1H), 6.29 (br s, 1H), 7.29-7.40 (m, 6H), 7.54-7.59 (m, 2H); ESIMS *m/e* 400 ($M^+$+1, $^{79}Br$), 402 ($M^+$+1, $^{81}Br$);

| Elemental Analyses for $C_{20}H_{18}BrNO_3$: | | | |
|---|---|---|---|
| Calculated | C, 60.01; | H, 4.53; | N, 3.50. |
| Found | C, 60.23; | H, 4.80; | N, 3.47. |

B. Preparation of 5-carbomethoxy-1,2-dihydro-2-phenyl-9H-carbazol-4(3H)-one

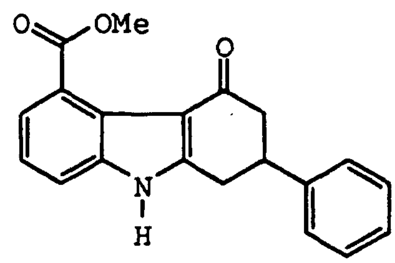

Prepared by the method of Example 17D in 70% yield. IR (KBr) 3180, 1736, 1628 $cm^{-1}$; $^1H$ NMR ($CDCl_3$) $\delta$ 2.70 (d, $J$ = 4 Hz, 1H), 2.72 (s, 1H), 2.97-3.03 (m, 2H), 4.03 (s, 3H), 7.18-7.39 (m, 8H), 9.52 (br s, 1H); ESIMS *m/e* 320 ($M^+$+1).

C. Preparation of 5-carbomethoxy-1,2-dihydro-2-phenyl-9-(phenylmethyl)carbazol-4(3H)-one

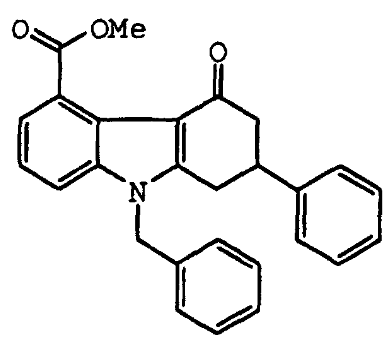

Prepared in 85% yield by the method of Example 17E. IR (KBr) 1723, 1652 $cm^{-1}$; ESIMS *m/e* 410 ($M^{+}$+1).

D. Preparation of 5-carbomethoxy-4-hydroxy-2-phenyl-9-(phenylmethyl)carbazole

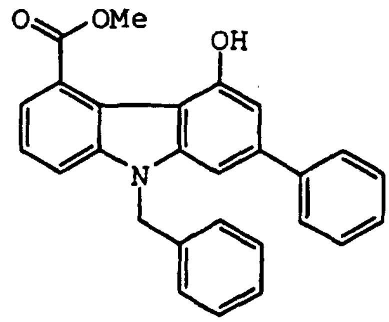

Prepared in 50% yield by the method (a) of Example 17F. IR (KBr) 3326, 1711 $cm^{-1}$; $^{1}H$ NMR ($CDCl_3$) δ 4.10 (s, 3H), 5.52 (s, 2H), 7.08-7.10 (m, 4H), 7.24-7.56 (m, 7H), 7.38 (d, *J* = 8.1 Hz, 1H), 7.55 (d, *J* = 8.0 Hz, 2H), 7.97 (d, *J* = 8.1 Hz, 1H) 10.43 (br s, 1H); ESIMS *m/e* 408 ($M^{+}$+1);

| Elemental Analyses for $C_{27}H_{21}NO_3$•0.1 $C_7H_8$: | | | |
|---|---|---|---|
| Calculated | C, 79.85; | H, 5.27; | N, 3.36. |
| Found | C, 80.19; | H, 5.32; | N, 3.49. |

E. Preparation of 5-carbamoyl-4-hydroxy-2-phenyl-9-(phenylmethyl)carbazole

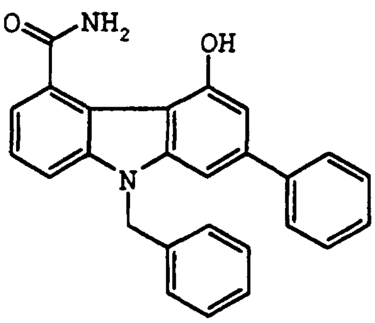

Prepared in 40% yield by the method of Example 17G. $^{1}H$ NMR ($CDCl_3$) δ 5.58 (s, 2H), 6.25 (s, 1H), 6.59 (s, 1H), 7.11-7.16 (m, 4H), 7.26-7.48 (m, 8H), 7.52 (d, *J* = 8.1 Hz, 1H), 7.68 (d, *J* = 7.2 Hz, 2H), 9.99 (br s, 1H); ESIMS *m/e* 393 ($M^{+}$+1).

F. Preparation of [5-carbamoyl-2-phenyl-9-(phenylmethyl)carbazol-4-yl]oxyacetic acid, methyl ester

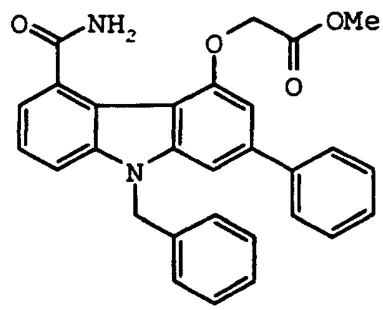

Prepared in 58% yield by the method of Example 17H. IR (KBr) 3359, 1755, 1634 $cm^{-1}$; $^1H$ NMR ($CDCl_3$) δ 3.85 (s, 3H), 4.96 (s, 2H), 5.58 (s, 2H), 5.92 (br s, 2H), 7.11 (s, 1H), 7.13-7.24 (m, 2H), 7.26-7.30 (m, 3H), 7.34-7.47 (m, 7H), 7.59 (d, *J* = 7.3 Hz, 2H); ESIMS *m*/*e* 465 ($M^+$+1);

| Elemental Analyses for $C_{29}H_{24}N_2O_4$: | | | |
|---|---|---|---|
| Calculated | C, 74.98; | H, 5.21; | N, 6.03. |
| Found | C, 74.97; | H, 5.22; | N, 5.80. |

G. Preparation of [5-carbamoyl-2-phenyl-9-(phenylmethyl)carbazol-4-yl]oxyacetic acid

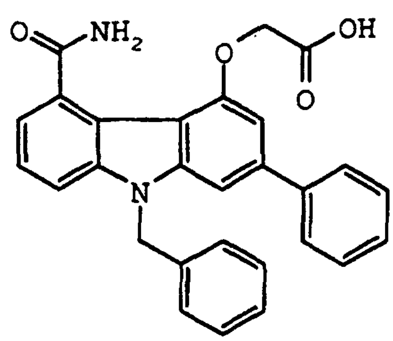

Prepared in 86% yield by the method of Example 17I. IR (KBr) 3426, 3332, 2625-2100 (br),1734, 1636 $cm^{-1}$; $^1H$ NMR (DMSO-$d_6$) δ 4.94 (s, 2H), 5.57 (s, 2H),6.96 (s, 1H), 7.06-7.31 (m, 6H), 7.33-7.47 (m, 5H), 7.55-7.60 (m, 2H), 7.71-7.73 (m, 3H), 12.94 (br s, 1H); ESIMS *m*/*e* 451 ($M^+$+1);

| Elemental Analyses for $C_{28}H_{22}N_2O_4$: | | | |
|---|---|---|---|
| Calculated | C, 74.65; | H, 4.92; | N, 6.22. |
| Found | C, 74.87; | H, 5.15; | N, 6.11. |

Example 21 [5-carbamoyl-2-(4-chlorophenyl)-9-(phenylmethyl)carbazol-4-yl]oxyacetic acid

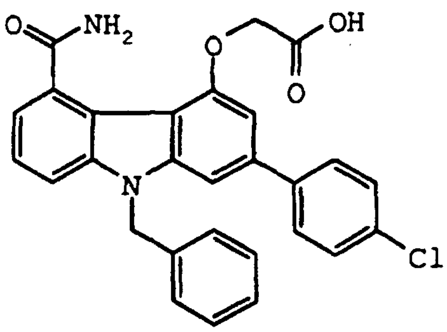

A. Preparation of 3-(2-bromo-3-carbomethoxyanilino)-5-(4-chlorophenyl)cyclohex-2-en-1-one

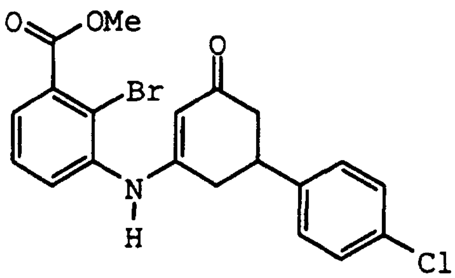

Prepared by the method of Example 17, part C in 80% yield. $^1H$ NMR ($CDCl_3$) $\delta$ 2.66 (m, 3H), 2.86 (m, 1H), 3.44 (m, 1H), 3.95 (s, 3H), 5.75 (s, 1H), 7.24 (m, 3H), 7.32 (m, 3H), 7.57 (t, *J* = 7.1 Hz, 2H) ; ESIMS *m*/*e* 434 ($M^+$+1, $^{79}Br^{35}Cl$), 436 ($M^+$+1, $^{81}Br^{35}Cl$, $^{79}Br^{37}Cl$), 438 ($M^+$+1, $^{81}Br^{37}Cl$);

| Elemental Analyses for $C_{20}H_{17}BrClNO_3$: | | | |
|---|---|---|---|
| Calculated | C, 55.26; | H, 3.94; | N, 3.22. |
| Found | C, 55.55; | H, 3.91; | N, 3.21. |

B. Preparation of 5-carbomethoxy-1,2-dihydro-2-(4-chlorophenyl)-9H-carbazol-4(3H)-one

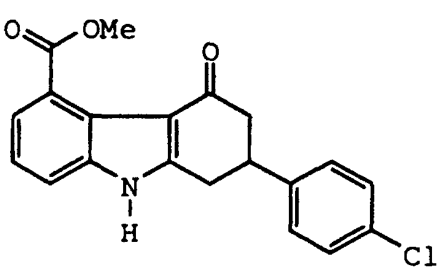

Prepared by the method of Example 17D in 64% yield. $^1H$ NMR ($CDCl_3$) $\delta$ 2.72 (m, 2H), 2.99 (dd, *J* = 16.7, 16.5 Hz, 1H), 3.12 (dd, *J* = 16.7, 4.7 Hz, 1H), 3.45 (m, 1H), 4.04 (s, 3H), 7.17 (d, *J* = 8.5 Hz, 2H), 7.23 (d, *J* = 7.9 Hz, 1H), 7.30 (d, *J* = 8.4 Hz, 2H), 7.43 (t, *J* = 7.9 Hz, 2H), 9.61 (br s, 1H); ESIMS *m*/*e* 354 ($M^+$+1, $^{35}Cl$), 356 ($M^+$+1, $^{37}Cl$);

| Elemental Analyses for $C_{20}H_{16}ClNO_3$: | | | |
|---|---|---|---|
| Calculated | C, 67.90; | H, 4.56; | N, 3.96. |
| Found | C, 68.14; | H, 4.51; | N, 3.90. |

C. Preparation of 5-carbomethoxy-1,2-dihydro-2-(4-chlorophenyl)- 9-(phenylmethyl)carbazol-4(3H)-one

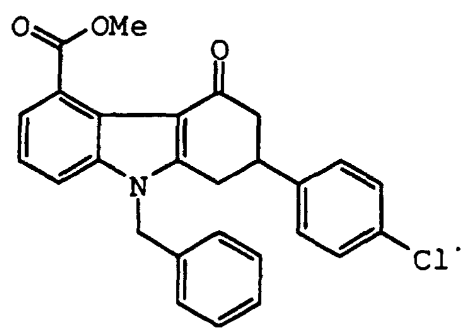

Prepared by the procedure of Example 17E in 90% yield. $^1H$ NMR ($CDCl_3$) δ 2.79 (d, *J* = 3.7 Hz, 1H), 2.82 (s, 1H), 2.97 (dd, *J* = 16.7, 11.5 Hz, 1H), 3.19 (dd, *J* = 16.7, 4.7 Hz, 1H), 3.59 (m, 1H), 4.06 (s, 3H), 5.35 (s, 2H), 6.96 (t, *J* = 3.6 Hz, 2H), 7.21 (m, 2H), 7.30 (m, 6H), 7.36 (t, *J* = 7.5 Hz, 2H); ESIMS *m/e* 444 ($M^+$+1, $^{35}Cl$), 446 ($M^+$+1, $^{37}Cl$);

| Elemental Analyses for $C_{27}H_{22}ClNO_3$: | | | |
|---|---|---|---|
| Calculated | C, 73.05; | H, 5.00; | N, 3.16. |
| Found | C, 73.23; | H, 5.15; | N, 3.36. |

D. Preparation of 5-carbomethoxy-2-(4-chlorophenyl)-4-hydroxy-9-(phenylmethyl)carbazole

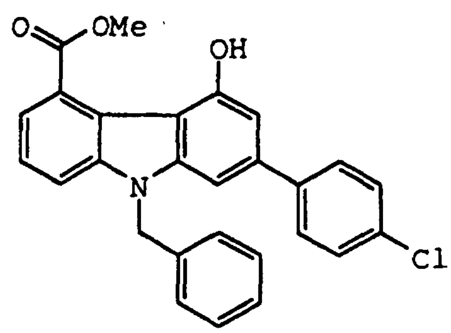

Prepared by method of example 17(b) in 66% yield. $^1H$ NMR ($CDCl_3$) δ 4.12 (s, 3H), 5.60 (s, 2H), 7.10 (t, *J* = 4.5 Hz, 4H), 7.32 (m, 3H), 7.41 (m, 3H), 7.60 (d, *J* = 8.5 Hz, 3H), 8.04 (d, *J* = 7.2 Hz, 1H).

E. Preparation of 5-carbamoyl-2-(4-chlorophenyl)-4-hydroxy-9-(phenylmethyl)carbazole

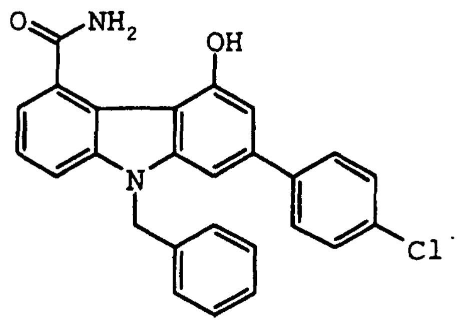

Prepared by the procedure of example Example 17G in 43% yield. $^1$H NMR ($CDCl_3$) δ 5.77 (s, 2H), 6.89 (s, 1H), 7.07 (d, *J* = 7.0 Hz, 2H), 7.23 (m, 3H), 7.45 (m, 5H), 7.76 (d, *J* = 8.4 Hz, 3H), 8.40 (s, 1H), 8.85 (s, 1H); ESIMS *m*/*e* 427 ($M^+$+1, $^{35}$Cl), 429 ($M^+$+1, $^{37}$Cl);

| Elemental Analyses for $C_{26}H_{19}ClN_2O_2$: | | | |
|---|---|---|---|
| calculated | C, 73.15; | H, 4.49; | N, 6.56. |
| Found | C, 72.92; | H, 4.57; | N, 6.46. |

F. Preparation of [5-carbamoyl-2-(4-chlorophenyl)-9-(phenylmethyl)carbazol-4-yl]oxyacetic acid, methyl ester

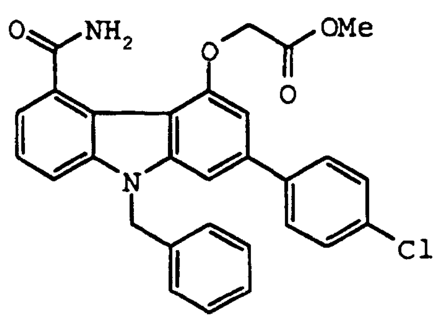

Prepared by the procedure of example Example 17H in 79% yield. $^1$H NMR ($CDCl_3$) δ 3.84 (s, 3H), 4.97 (s, 2H), 5.57 (s, 2H), 6.08 (br s, 1H), 6.14 (br s, 1H), 6.74 (s, 1H), 7.12 (m, 2H), 7.18 (s, 1H), 7.22 (m, 2H), 7.41 (m, 6H), 7.51 (d, *J* = 8.5 Hz, 2H); ESIMS *m*/*e* 499 ($M^+$+1, $^{35}$Cl), 501 ($M^+$+1, $^{37}$Cl).

G. Preparation of [5-carbamoyl-2-(4-chlorophenyl)-9-(phenylmethyl)carbazol-4-yl]oxyacetic acid

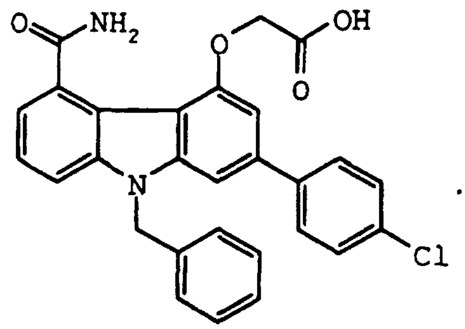

Prepared by the procedure of example Example 17I in 100% yield. $^1H$ NMR ($CDCl_3$) δ 4.95 (s, 2H), 5.75 (s, 2H), 6.88 (s, 1H), 7.20 (m, 4H), 7.52 (m, 6H), 7.76 (m, 3H), 12.92 (s, 1H); ESIMS *m/e* 485 ($M^+$+1, $^{35}Cl$), 487 ($M^+$+1, $^{37}Cl$).

Example 22

[5-carbamoyl-2-(2-furyl)-9-(phenylmethyl)carbazol-4-yl]oxyacetic acid

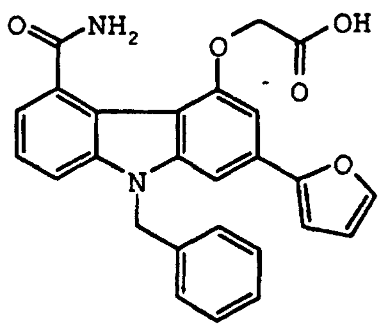

A. Preparation of 3-(2-bromo-3-carbomethoxyanilino)-5-(2-furyl)cyclohex-2-en-1-one

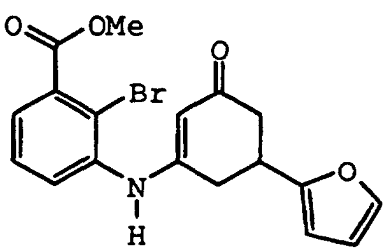

Prepared in 55% yield by the method of Example 17, part C. IR (KBr) 3201, 1735, 1593, 1575 $cm^{-1}$; $^1H$ NMR ($CDCl_3$) δ 2.56-2.94 (m, 4H), 3.53-3.60 (m, 1H), 3.94 (s, 1H), 5.60 (s, 1H), 6.11 (d, *J* = 2.8Hz, 1H), 6.22 (br s, 1H), 6.32-6.34 (m, 1H), 7.34-7.39 (m, 1H), 7.37 (br s, 1H), 7.55 (d, *J* = 7.9 Hz, 2H); *m/e* 390 ($M^+$+1, $^{79}Br$), 392 ($M^+$+1, $^{81}Br$);

| Elemental Analyses for $C_{18}H_{16}BrNO_4$: | | | |
|---|---|---|---|
| Calculated | C, 55.40; | H, 4.13; | N, 3.59. |
| Found | C, 55.62; | H, 4.27; | N, 3.71. |

B. Preparation of 5-carbomethoxy-1,2-dihydro-2-(2-furyl)-9H-carbazol-4(3H)-one

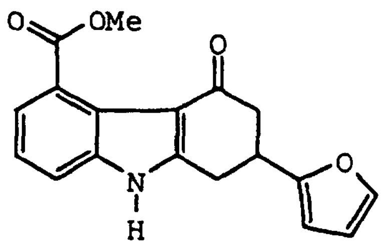

Prepared by the method of Example 17D in 47% yield. IR (KBr) 1736, 1633 $cm^{-1}$; $^1H$ NMR (THF-$d_8$): δ 2.59-2.77 (m, 2H) , 3.14 (dd, *J* = 16.5, 10.5 Hz, 1H), 3.30 (dd, *J* = 16.5, 10.3 Hz, 1H), 3.56-3.69 (m, 1H), 3.81 (s, 3H), 6.13-6.14 (m, 1H), 6.27-6.29 (d, *J* = 2.5 Hz, 1H), 7.11-7.21 (m, 2H), 7.37-7.39 (m, 1H), 7.39 (s, 1H), 11.03 (br s, 1H); ESIMS *m/e* 310 ($M^+$+1);

| Elemental Analyses for $C_{18}H_{15}NO_4$•0.1$C_7H_8$: | | | |
|---|---|---|---|
| Calculated | C, 70.51; | H, 4.50; | N, 4.40. |
| Found | C, 70.75; | H, 4.85; | N, 4.61. |

C. Preparation of 5-carbomethoxy-1,2-dihydro-2-(2-furyl)-9-(phenylmethyl)carbazol-4(3H)-one

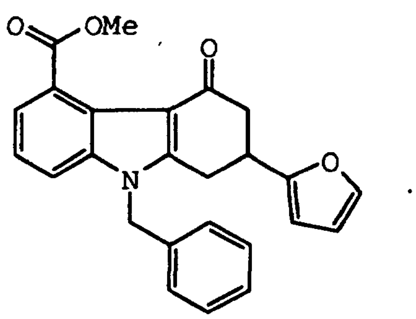

Prepared in 91% yield by the method of Example 17E. IR (KBr) 3500 (br), 1722, 1650 $cm^{-1}$; $^1H$ NMR ($CDCl_3$) δ 2.79 (dd, *J* = 16.6, 10.8 Hz, 1H), 2.94 (dd, *J* = 16.6, 4.2 Hz, 1H), 3.06 (dd, *J* = 16.8, 9.8 Hz, 1H), 3.31 (dd, *J* = 18.8, 4.7 Hz, 1H), 3.68-3.74 (m, 1H), 4.06 (s, 3H), 5.37 (s, 2H), 6.08 (d, *J* = 2.7 Hz, 1H), 6.28 (m, 1H), 6.96 (m, 2H), 7.22-7.40 (m, 7H); ESIMS *m/e* 400 ($M^+$+1);

| Elemental Analysis for $C_{25}H_{21}NO_4$: | | | |
|---|---|---|---|
| Calculated | C, 75.17; | H, 5.30; | N, 3.51. |
| Found | C, 75.46; | H, 5.32; | N, 3.67. |

D. Preparation of 5-carbomethoxy-2-(2-furyl)-4-hydroxy-9-(phenylmethyl)carbazole

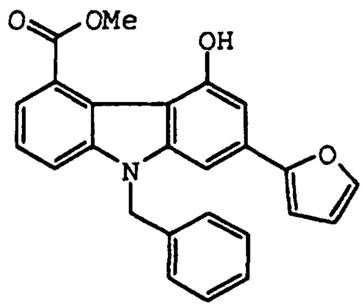

Prepared in 72% yield by the method (b) of Example 17F. IR (KBr) 3500 (br), 1674 $cm^{-1}$; $^1H$ NMR ($CDCl_3$) $\delta$ 4.11 (s, 3H), 5.58 (s, 2H), 6.48-6.50 (m, 1H), 6.75 (d, *J* = 3.2 Hz, 1H), 7.09-7.12 (m, 2H), 7.16 (s, 1H), 7.26-7.30 (m, 4H), 7.36-7.44 (t, *J* = 8.0 Hz, 1H), 7.48 (s, 1H) , 7.56 (d, *J* = 8.1 Hz, 1H), 8.01 (d, *J* = 7.5 Hz, 1H), 10.50 (br s, 1H); ESIMS *m*/*e* 398 ($M^{+}$+1).

E. Preparation of 5-carbamoyl-2-(2-furyl)-4-hydroxy-9-(phenylmethyl)carbazole

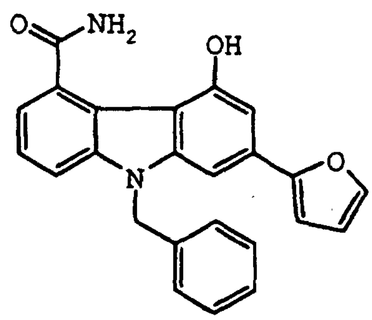

Prepared in 60% yield by the method of Example 17G. IR (KBr) 3425 (br), 3325 (br), 1642, 1628 $cm^{-1}$; $^1H$ NMR (DMSO-$d_6$) $\delta$ 5.72 (s, 2H), 6.56-6.57 (m, 1H), 6.95 (s, 1H), 6.98 (d, *J* = 3.0 Hz, 1H), 7.07 (d, *J* = 7.2 Hz, 2H), 7.17-7.26 (m, 3H), 7.42-7.43 (m, 3H), 7.71-7.76 (m, 2H), 8.38 (s, 1H), 8.83 (s, 1H), 10.70 (s, 1H); ESIMS *m*/*e* 381 ($M^{-}$-1);

| Elemental Analyses for $C_{24}H_{18}N_2O_3$: | | | |
|---|---|---|---|
| Calculated | C, 75.38; | H, 4.74; | N, 7.33. |
| Found | C, 75.35; | H, 4.95; | N, 7.29. |

F. Preparation of [5-carbamoyl-2-(2-furyl)-9-(phenylmethyl)carbazol-4-yl]oxyacetic acid, methyl ester

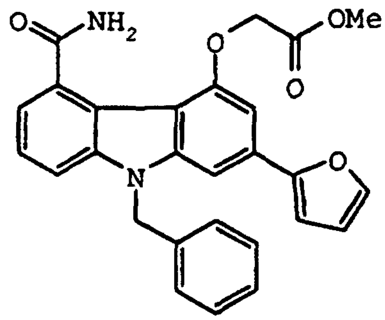

Prepared in 80% yield by the method of Example 17H. IR (KBr) 3358, 1756, 1643 $cm^{-1}$; $^1H$ NMR (DMSO-$d_6$) δ 3.70 (s, 3H), 4.98 (s, 2H), 5.70 (s, 2H), 6.58 (d, *J* = 1.5 Hz, 1H), 6.93 (s, 1H), 7.01-7.30 (m, 8H), 7.35 (t, *J* = 7.7 Hz, 1H), 7.51-7.57 (m, 3H), 7.72 (s, 1H); ESIMS *m/e* 455 ($M^+$+1);

| Elemental Analyses for $C_{27}H_{22}N_2O_5$: | | | |
|---|---|---|---|
| Calculated | C, 71.36; | H, 4.88; | N, 6.16. |
| Found | C, 76.46; | H, 4.91; | N, 6.24. |

G. Preparation of [5-carbamoyl-2-(2-furyl)-9-(phenylmethyl)carbazol-4-yl]oxyacetic acid

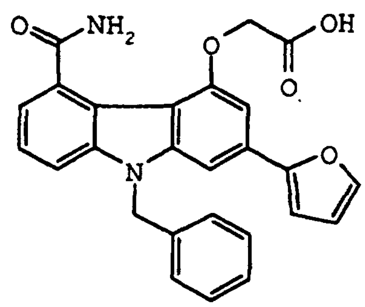

Prepared in 88% yield by the method of Example 17I. $^1H$ NMR (DMSO-$d_6$) δ 4.89 (s, 2H) 5.71 (s, 2H), 6.58 (s, 1H), 6.94 (s, 1H), 7.00-7.38 (m, 9H), 7.59 (d, *J* = 9.2 Hz, 1H), 7.58 (s, 1H), 7.72 (br s, 2H), 12.98 (br s, 1H); ESIMS *m/e* 441 ($M^+$+1);

| Elemental Analyses for $C_{26}H_{20}N_2O_5$: | | | |
|---|---|---|---|
| Calculated | C, 70.90; | H, 4.58; | N, 6.36. |
| Found | C, 71.20; | H, 4.67; | N, 6.28. |

Example 23

[5-carbamoyl-9-(phenylmethyl)-2-[(tri(-1-methylethyl)silyl)oxymethyl]carbazol-4-yl]oxyacetic acid, lithium salt

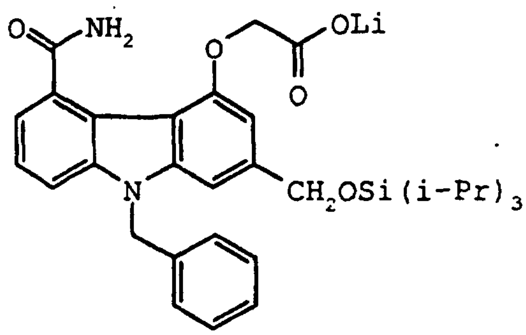

A. Preparation of 5-carbomethoxy-1,2-dihydro-9-(phenylmethyl)-2-[(tri(-1-methylethyl)silyl)oxymethyl]carbazol-4(3H)-one

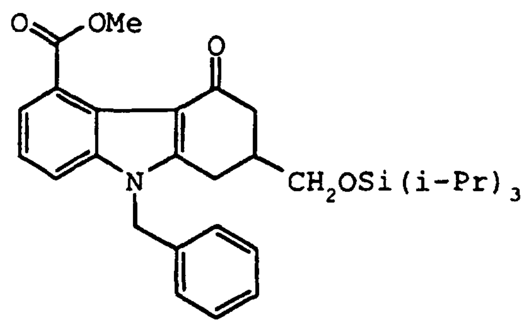

Triisopropylsilyl trifluoromethanesulfonate (2.46 mL, 9.15 mmol) was added to a stirred suspension of the compound of Example 19C (2.89 g, 7.95 mmol) and anhydrous pyridine (0.964 mL, 11.9 mmol) in anhydrous methylene chloride (29 mL) at 0 °C under a nitrogen atmosphere. The mixture was stirred at 0 °C for 1 hour. Methanol (0.5 mL) was added to the mixture and stirring was continued for 1 minute. After dilution with toluene (10 mL), the mixture was concentrated and the residue was subject to chromatographic purification on silica (gradient 10-50% ethyl acetate in hexane) to give subtitled compound (4.06 g, 98%) as a white solid. IR (KBr) 1725, 1645 $cm^{-1}$; $^1H$ NMR ($CDCl_3$) $\delta$ 0.90-1.15 (m, 21H, $-CH(CH_3)_2$), 2.50-2.63 (m, 3H), 2.74-2.86 (m, 1H), 3.02 (br d, $J$ = 7.7 Hz, 1H), 3.67-3.81 (m, 2H, $-CH_2O-$), 4.05 (s, 3H, $-OCH_3$), 5.37 (s, 2H, $-NCH_2-$), 7.00-7.04 (m, 2H), 7.22-7.44 (m, 6H); ESIMS *m/e* 520 ($M^+$+1);

| Elemental Analyses for $C_{31}H_{41}NO_4Si$: | | | |
|---|---|---|---|
| Calculated | C, 71.64; | H, 7.95; | N, 2.69. |
| Found | C, 71.75; | H, 7.91; | N, 2.82. |

B. Preparation of 5-carbomethoxy-4-hydroxy-9-(phenylmethyl)-2-[(tri(-1-methylethyl)silyl)oxymethyl]carbazole

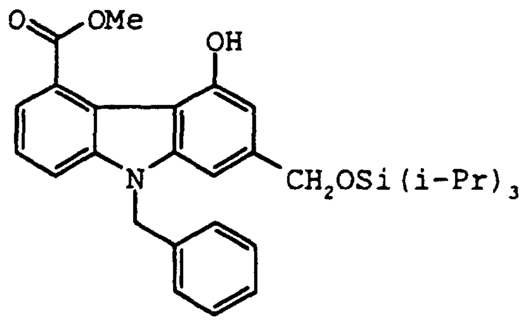

Following the experimental procedure (b) as described in the synthesis of Example 17F, subtitled compound was obtained as a yellowish solid in a 93% yield. IR (KBr) 3165 (br), 1671, 1629 $cm^{-1}$; $^1H$ NMR ($CDCl_3$) δ 1.00-1.22 (m, 21H, $-CH(CH_3)_2$), 4.10 (s, 3H, $-OCH_3$), 4.97 (s, 2H, $-OCH_2-$), 5.51 (s, 2H, $-NCH_2-$), 6.79 (s, 1H), 7.05-7.14 (m, 3H), 7.20-7.30 (m, 3H), 7.39 (t, *J* = 8.0 Hz, 1H), 7.59 (d, *J* = 8.0 Hz, 1H), 7.98 (d, *J* = 8.0 Hz, 1H), 10.50 (s, 1H, -OH); ESIMS *m*/*e* 518 ($M^+$+1);

| Elemental Analyses for $C_{31}H_{39}NO_4Si$: | | | |
|---|---|---|---|
| Calculated | C, 71.92; | H, 7.59; | N, 2.71. |
| Found | C, 72.19; | H, 7.21; | N, 2.76. |

C. Preparation of 5-carbamoyl-4-hydroxy-9-(phenylmethyl)-2-[(tri(-1-methylethyl)silyl)oxymethyl]carbazole

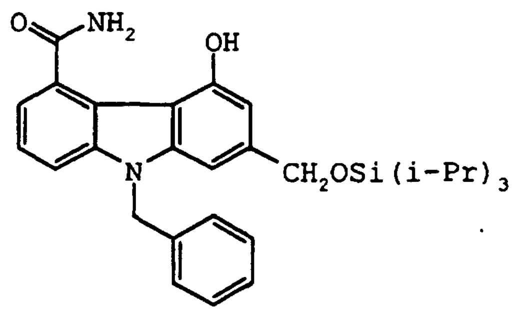

Following the experimental procedure as described in the synthesis of Example 17F(b), subtitled compound was obtained as a yellowish solid in a 80% yield. IR (KBr) 3348 (br), 3200, 1660, 1628 $cm^{-1}$; $^1H$ NMR ($CDCl_3$) δ 1.00-1.20 (m, 21H, $-CH(CH_3)_2$), 4.94 (s, 2H, $-OCH_2-$), 5.52 (s, 2H, $-NCH_2-$), 6.22 (s, 1H, -NH), 6.56 (s, 1H, -NH), 6.75 (s, 1H), 7.05-7.10 (m, 3H), 7.20-7.28 (m, 3H), 7.36 (t, *J* = 7.6 Hz, 1H), 7.43 (d, *J* = 7.6 Hz, 1H), 7.53 (d, *J* = 7.6 Hz, 1H), 9.75 (br s, 1H, -OH); ESIMS *m*/*e* 503 ($M^+$+1).

D. Preparation of [5-carbamoyl-9-(phenylmethyl)-2-[(tri(-1-methylethyl)silyl)oxymethyl]carbazol-4-yl]oxyacetic acid, methyl ester

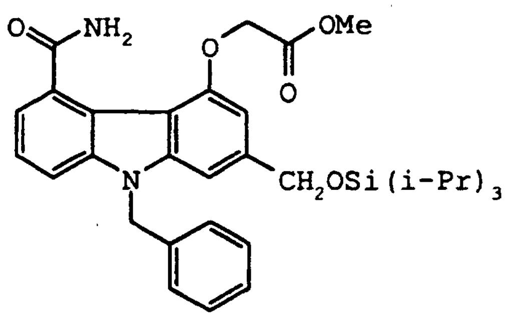

Following the experimental procedure as described in the synthesis of Example 17H, subtitled produce was obtained as a white solid in a 94% yield. IR (KBr) 3484, 3180 (br), 1764, 1675 $cm^{-1}$; $^1H$ NMR ($CDCl_3$) δ 1.00-1.20 (m, 21H, $-CH(CH_3)_2$), 3.82 (s, 3H, $-OCH_3$), 4.89 (s, 2H, $-OCH_2-$), 4.93 (s, 2H, $-OCH_2-$), 5.50 (s, 2H, $-NCH_2-$), 6.00 (br s, 2H, $-NH_2$), 6.60 (s, 1H), 7.05-7.12 (m, 3H), 7.22-7.28 (m, 3H), 7.32-7.38 (m, 1H), 7.39-7.41 (m, 2H); ESIMS *m/e* 575 ($M^+$+1);

| Elemental Analyses for $C_{33}H_{42}N_2O_5Si$: | | | |
|---|---|---|---|
| Calculated | C, 68.96; | H, 7.37; | N, 4.87. |
| Found | C, 69.14; | H, 7.20; | N, 4.95. |

E. Preparation of [5-carbamoyl-9-(phenylmethyl)-2-[(tri(-1-methylethyl)silyl)oxymethyl]carbazol-4-yl]oxyacetic acid, lithium salt

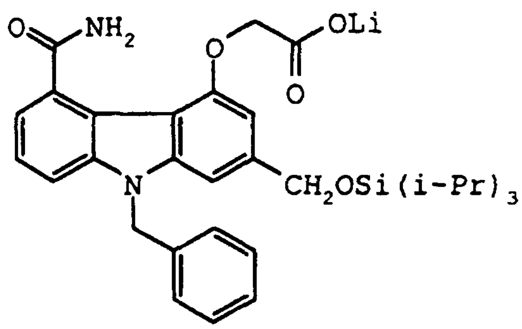

Lithium hydroxide (4.17 N, 42.5 mL, 0.177 mmol) was added to a stirred suspension of part D above (50.9 mg, 0.0886 mmol) in THF (1 mL)/$CH_3OH$ (0.3 mL)/$H_2O$ (0.3 mL). The resultant mixture was stirred in an oil bath at 55 °C for 1 hour to form a white suspension. At ambient temperature, the white suspension was diluted with water (5 mL) and THF was evaporated *in vacuo*. After filtration and washing with water, the white solid was dried under vacuum to give 11 (40.0 mg, 80%) of the title product. IR (KBr) 3470, 3315, 1652, 1621 $cm^{-1}$; $^1H$ NMR (DMSO-$d_6$) δ 0.90-1.15 (m, 21H, $-CH(CH_3)_2$), 4.29 (s, 2H, $-OCH_2-$), 4.84 (s, 2H, $-OCH_2-$), 5.57 (s, 2H, $-NCH_2-$), 6.49 (s, 1H), 7.00-7.25 (m, 7H), 7.31 (br t, *J* = 7.9 Hz, 1H), 7.43 (br s, 1H, -NH), 7.59 (br d, *J* = 7.9 Hz, 1H), 7.72 (br s, 1H, -NH); ESIMS *m/e* 567 ($M^+$+1).

Preparation 3

Preparation of 5-Carbomethoxy-1,2-dihydro-9H-carbazol-4(3H)-one from 2-chloro-3-nitrobenzoic acid

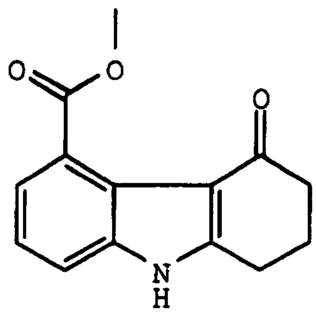

a) Methyl 2-chloro-3-nitrobenzoate

A solution of 2-chloro-3-nitrobenzoic acid (20.16 g, 100.0 mM), iodomethane (15.6 g, 110 mM), and potassium carbonate (15.0 g, 108.5 mM) in 100 mL DMF was stirred at room temperature for 48 hours. The mixture was poured into 1.5 liters of $H_2O$. The resultant precipitate was collected by 20.0 g (93%) of methyl 2-chloro-3-nitrobenzoate as a white solid. $^1H$ NMR ($CDCl_3$) δ 8.42 (dd, 1H, J=1 and 8 Hz), 8.18 (dd, 1H, J=1 and 8 Hz), 7.43 (t, 1H, J=8 Hz), and 3.9 (s, 3H). IR (KBr, $cm^{-1}$)1743, 1719, 1595, 1540, 1532, 1433, 1357, 1300, and 730. MS (FD) m/e 215, 216.

| Elemental Analyses for $C_8H_6NO_4Cl$: | | | |
|---|---|---|---|
| Calculated | C, 44.57; | H, 3.81; | N, 6.50. |
| Found | C, 44.19; | H, 3.45; | N, 6.19. |

b) Methyl 2-chloro-3-aminobenzoate

Hydrogen gas was passed through a solution of methyl 2-chloro-3-nitrobenzoate (10.0 g, 46.4 mM) and 1.0 g of 3% sulfided platinum on carbon in 150 mL ethyl acetate for 48 hours at room temperature. The catalyst was removed by filtration through celite. Concentration of the filtrate afforded 8.6 g (100%) of methyl 2-chloro-3-aminobenzoate as a yellow oil. $^1H$ NMR ($CDCl_3$) δ 7.25 (dd, 1H, J=1 and 8 Hz), 7.2 (t, 1H, J=8 Hz), 6.95 (dd, 1H, J=1 and 8 Hz), and 3.9 (s, 3H). IR ($CHCl_3$, $cm^{-1}$) 3450, 3380, 2980, 2900, 1729, 1615, 1456, 1434, 1322, 1290, and 1268. MS (ES) m/e 186,188.

| Elemental Analyses for $C_8H_8NO_2Cl$: | | | |
|---|---|---|---|
| Calculated | C, 51.77; | H, 4.34; | N, 7.55. |
| Found | C, 51.52; | H, 4.17; | N, 7.54. |

b) Methyl 2-chloro-3-aminobenzoate

A solution of stannous chloride (27.0 g, 137.0 mM) in 55 mL of concentrated hydrochloric acid was slowly added to a solution of methyl 2-chloro-3-nitrobenzoate (6.0 g, 27.9 mM) in 75 mL ethanol at 15-20 °C over 1 hour. The mixture was then heated at 50-60 °C for 15 minutes. The mixture was cooled to room temperature and made alkaline by slow addition of solid sodium hydroxide maintaining a temperature of 30-35 °C. The resultant mixture was extracted three times with chloroform. The extracts were washed with brine, dried over sodium sulfate, filtered and concentrated to afford 2.6 g (50%) of methyl 2-chloro-3-aminobenzoate as a yellow oil, identical in all respects to the material derived via catalytic hydrogenation described above.

b) Methyl 2-chloro-3-aminobenzoate

A solution of sodium dithionite (14.0 g, 20.0 mM) and sodium carbonate (6.7 g) in 200 mL of water was slowly

added to a solution of methyl 2-chloro-3-nitrobenzoate (6.0 g, 27.9 mM) in 40 mL methanol and 40 mL of tetrahydrofuran at 25 °C over 30 minutes. The mixture was stirred at room temperature for an additional 30 minutes, then extracted with ethyl acetate. The extracts were washed with brine, dried over sodium sulfate, filtered and concentrated to afford 1.2 g (33%) of methyl 2-chloro-3-aminobenzoate as a yellow oil, identical in all respects to the material derived via catalytic hydrogenation described above.

c) 3-(3-Carbomethoxy-2-chloroanilino)cyclohex-2-en-1-one

A mixture of methyl 2-chloro-3-aminobenzoate (11.11 g, 59.86 mM) and 1,3-cyclohexanedione (9.05 g, 80.8 mM) was heated at 120 °C under a stream of nitrogen for 4 hours. The resultant solid was triturated with hot ethyl acetate, then dried *in vacuo* to afford 14.05 g (84%) of 3-(3-carbomethoxy-2-chloroanilino)cyclohex-2-en-1-one as a yellow orange solid. $^1H$ NMR ($CDCl_3$) δ 7.6 (dt, 1H, J=1 and 8 Hz), 7.3 (t, 1H, J=8 Hz), 6.6 (br s, 1H), 5.62 (s, 1H), 3.95 (s, 3H), 2.6 (t, 2H, J=6 Hz), 2.4 (t, 2H, J=6 Hz), and 2.1 (m, 2H). IR ($CHCl_3$, $cm^{-1}$) 3050, 2950, 1729, 1536, 1351, 1299, 1290, 1267, and 1135. MS (ES) m/e 278, 280, 282.

| Elemental Analyses for $C_{14}H_{14}NO_3Cl$: | | | |
|---|---|---|---|
| Calculated | C, 60.11; | H, 5.04; | N, 5.01. |
| Found | C, 57.51; | H, 4.99; | N, 4.68. |

d) 5-Carbomethoxy-1,2-dihydro-9H-carbazol-4(3H)-one

A suspension of 3-(3-carbomethoxy-2-chloroanilino)cyclohex-2-en-1-one (10.22 g, 36.67 mM), palladium acetate (0.82 g, 3.66 mM), tricyclohexylphosphine (4.10 g, 14.62 mM), and triethylamine (30.0 mL, 21.78 g, 215.2 mM) in 100 mL acetonitrile was heated at 130 °C in a sealed vessel for 14 days. The mixture was diluted with ethyl acetate, washed twice with 1 N HCl, twice with $H_2O$, once with saturated brine, dried over anhydrous magnesium sulfate, filtered, and concentrated to afford 9.9 g of a light brown gum. Purification by HPLC on silica gel (elution with gradient methylene chloride/ethyl acetate) afforded 4.68 g (52%) of the 5-carbomethoxy-1,2-dihydro-9H-carbazol-4(3H)-one as a yellow solid. $^1H$ NMR ($CDCl_3$) δ 9.15 (br s, 1H), 7.4 (dd, 1H, J=1 and 8 Hz), 7.35 (dd, 1H, J=1 and 8 Hz), 7.25 (t, 1H, J=8 Hz), 4.05 (s, 3H), 2.95 (t, 2H, J=6 Hz), 2.55 (t, 2H, J=6 Hz), and 2.2 (m , 2H). IR ($CHCl_3$, $cm^{-1}$) 3400, 3200 (br), 3000, 2950, 1721, 1646, 1466, 1439, 1427, 1299, 1284, 1165, and 1135. MS (ES) m/e 242, 244.

| Elemental Analyses for $C_{14}H_{13}NO_3$: | | | |
|---|---|---|---|
| Calculated | C, 69.12; | H, 5.39; | N, 5.76. |
| Found | C, 68.82; | H, 5.67; | N, 5.60. |

Preparation 4

Preparation of 5-Carbomethoxy-1,2-dihydro-9H-carbazol-4(3H)-one from 2-bromo-3-nitrobenzoic acid

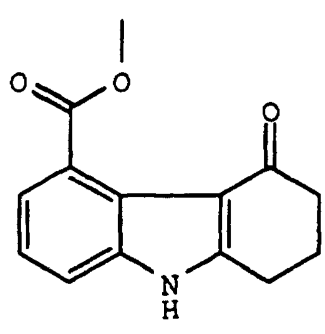

a) Methyl 2-bromo-3-nitrobenzoate

A solution of 2-bromo-3-nitrobenzoic acid (28.4 g, 115.0 mM), iodomethane (18.0 g, 127 mM), and potassium carbonate (19.0 g, 137.4 mM) in 100 mL DMF was stirred at room temperature for 72 hours. The mixture was poured into 1.5 liters of $H_2O$. The resultant precipitate was collected by filtration and dried *in vacuo* to afford 28.79 g (96%) of methyl 2-bromo-3-nitrobenzoate as a white solid. $^1H$ NMR (DMSO-d6) δ 8.3 (dd, 1H, J=1 and 8 Hz), 7.9 (dd, 1H, J=1 and 8 Hz), 7.7 (t, 1H, J=8 Hz), and 3.9 (s, 3H). IR (KBr, $cm^{-1}$) 2950, 1738, 1541, 1435, 1364, 1298, and 1142. MS (FD) m/e 259, 261.

| Elemental Analyses for $C_8H_6NO_4Br$: | | | |
|---|---|---|---|
| Calculated | C, 36.95; | H, 2.33; | N, 5.39. |
| Found | C, 37.14; | H, 2.37; | N, 5.45. |

b) Methyl 2-bromo-3-aminobenzoate

Hydrogen gas was passed through a solution of methyl 2-bromo-3-nitrobenzoate (0.20 g, 0.77 mM) and 0.1 g of 3% sulfided platinum on carbon in 25 mL ethyl acetate for 24 hours at room temperature. The catalyst was removed by filtration through celite. Concentration of the filtrate afforded 0.175 g (99%) of methyl 2-bromo-3-aminobenzoate as a yellow oil. $^1H$ NMR ($CDCl_3$) δ 7.15 (t, 1H, J=8 Hz), 7.1 (dd, 1H, J=1 and 8 Hz), 6.8 (dd, 1H, J=1 and 8 Hz), and 3.95 (s, 3H). IR ($CHCl_3$, $cm^{-1}$) 3550, 3380, 2980, 2900, 1729, 1613, 1465, 1451, 1434, 1324, 1266, and 1025. MS (FD) m/e 230, 232.

| Elemental Analyses for $C_8H_8NO_2Br$: | | | |
|---|---|---|---|
| Calculated | C, 41.77; | H, 3.51; | N, 6.09. |
| Found | C, 42.01; | H, 3.29; | N, 6.00. |

b) Methyl 2-bromo-3-aminobenzoate

A solution of stannous chloride (15.0 g, 76.1 mM) in 30 mL of concentrated hydrochloric acid was slowly added to a solution of methyl 2-bromo-3-nitrobenzoate (4.0 g, 15.4 mM) in 90 mL ethanol at 15-30 °C over 1 hour. The mixture was then heated at 50-60 °C for 15 minutes. The mixture was cooled to room temperature and made alkaline by slow addition of solid sodium hydroxide maintaining a temperature of 30-35 °C. The resultant mixture was extracted three times with chloroform. The extracts were washed with brine, dried over sodium sulfate, filtered and concentrated to afford 3.51 g (99%) of methyl 2-bromo-3-aminobenzoate as a yellow oil, identical in all respects to the material derived via catalytic hydrogenation described above.

c) 3-(3-Carbomethoxy-2-bromoanilino)cyclohex-2-en-1-one

A mixture of methyl 2-bromo-3-aminobenzoate (13.2 g, 60.0 mM) and 1,3-cyclohexanedione (8.4 g, 75 mM) was heated at 125 °C under a stream of nitrogen for 4 h. The resultant solid was purified by HPLC on silica gel (elution with methylene chloride/ethyl acetate) to afford 17.2 g (88%) of 3-(3-carbomethoxy-2-bromoanilino)cyclohex-2-en-1-one as a tan foam. $^1H$ NMR (DMSO-d6) δ 8.75 (s, 1H), 7.6-7.4 (m, 3H), 4.65 (s, 1H), 3.85 (s, 3H), 2.6 (t, 2H, J=6 Hz), 2.15 (t, 2H, J=6 Hz), and 1.9 (m, 2H). IR ($CHCl_3$, $cm^{-1}$) 3400, 3004, 2954, 1732, 1607, 1588, 1573, 1513, 1464, 1436, 1412, 1308, 1249, 1177, and 1144. MS (ES) m/e 322, 324, 326.

| Elemental Analyses for $C_{14}H_{14}NO_3Br$: | | | |
|---|---|---|---|
| Calculated | C, 51.85; | H, 4.32; | N, 4.32. |
| Found | C, 53.60; | H, 4.73; | N, 4.09. |

d) 5-Carbomethoxy-1,2-dihydro-9H-carbazol-4(3H)-one

A suspension of 3-(3-carbomethoxy-2-bromoanilino)cyclohex-2-en-1-one (15.8 g, 48.8 mM), palladium acetate (1.12 g, 5.0 mM), tri-o-tolylphosphine (3.1 g, 10.0 mM), and triethylamine (6.3 g, 62.0 mM) in 120 mL acetonitrile was heated at reflux for 8 hours. The solvent was removed *in vacuo*. The residue was dissolved in methylene chloride, washed twice with 1 N HCl, twice with $H_2O$, once with saturated brine, dried over anhydrous magnesium sulfate, filtered,

and concentrated to afford 17 g of a light brown foam. Purification by HPLC on silica gel (elution with gradient methylene chloride/ethyl acetate) afforded 9.2 g (78%) of the 5-carbomethoxy-1,2-dihydro-9H-carbazol-4(3H)-one as a yellow solid, identical with the material derived from 3-(3-carbomethoxy-2-chloroanilino)cyclohex-2-en-1-one, described above. $^1H$ NMR (DMSO-d6) δ 7.5 (d, 1H, J=8 Hz), 7.25-7.1 (m, 2H), 5.7 (s, 1H), 3.8 (s, 3H), 2.95 (t, 2H, J=6 Hz), 2.4 (t, 2H, J=6 Hz), and 2.1 (m , 2H). MS (ES) m/e 242, 244.

EXAMPLE 24

Preparation of {9-[(phenyl)methyl]-5-carbamoylcarbazol-4-yl}oxyacetic acid, sodium salt

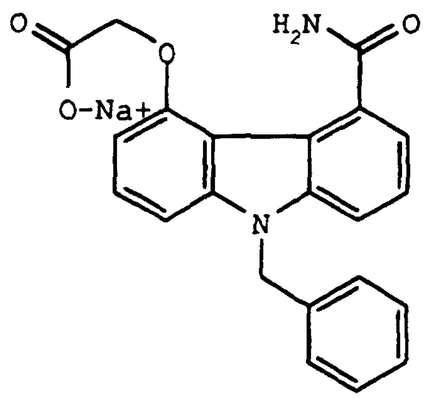

A. 9-[(Phenyl)methyl]-5-carbomethoxy-1,2-dihydrocarbazol-4(3H)-one

A suspension of 5-carbomethoxy-1,2-dihydro-9H-carbazol-4(3H)-one (300 mg, 1.23 mM), benzyl bromide (210 mg, 1.23 mM), and potassium carbonate (170 mg, 1.23 mM) in 15 mL DMF was stirred at room temperature for 6 hours. The mixture was diluted with 80 mL $H_2O$ and chilled in the refrigerator. The resultant white precipitate was collected by filtration, washed with $H_2O$, and dried *in vacuo* to afford 325 mg (79%) of the 9-[(phenyl)methyl]-5-carbomethoxy-1,2-dihydrocarbazol-4(3H)-one as a white solid. $^1H$ NMR (DMSO-d6) δ 7.7 (dd, 1H, J=1 and 8 Hz), 7.45-7.0 (m, 7H), 5.6 (s, 2H), 3.8 (s, 3H), 3.05 (t, 2H, J=6 Hz), 2.5 (t, 2H, J=6 Hz), and 2.2 (m , 2H). IR (KBr, $cm^{-1}$) 3421, 1726, 1676, 1636, 1473, 1450, 1435, 1288, 1122, 764, 745, and 706. MS (ES) m/e 334.

| Elemental Analyses for $C_{21}H_{19}NO_3$: | | | |
|---|---|---|---|
| Calculated | C, 75.68; | H, 5.71; | N, 4.20. |
| Found | C, 70.85; | H, 5.53; | N, 4.49. |

B. 9-[(Phenyl)methyl]-4-hydroxy-5-carbomethoxy carbazole

(a) A solution of the 9-[(phenyl)methyl]-5-carbomethoxy-1,2-dihydrocarbazol-4(3H)-one (1.5 g, 4.5 mM) and 2,3-dichloro-5,6-dicyano-1,4-benzoquinone (1.12 g, 5.0 mM) in 25 mL of toluene was stirred between 80-90 °C for 6 h. The mixture was purified directly by column chromatography on silica gel (elution with methylene chloride/ethyl acetate) to afford 420 mg (28%) of the 9-[(phenyl)methyl]-4-hydroxy-5-carbomethoxy carbazole as a yellow solid. $^1H$ NMR (DMSO-d6) δ 10.25 (s, 1H), 7.7 (d, 1H, J=8 Hz), 7.4 (t, 1H, J=8 Hz), 7.4-7.0 (m, 8H), 6.6 (d, 1H, J=8 Hz), 5.6 (s, 2H), and 3.8 (s, 3H). IR ($CHCl_3$, $cm^{-1}$) 1723, 1685, 1621, 1597, 1568, 1496, 1453, 1442, 1392, 1286, 1267, 1156, and 1138. MS (ES) m/e 330, 332.

| Elemental Analyses for $C_{21}H_{17}NO_3$: | | | |
|---|---|---|---|
| Calculated | C, 76.13; | H, 5.14; | N, 4.23. |
| Found | C, 75.90; | H, 5.20; | N, 4.46. |

(b) To a solution of the 9-[(phenyl)methyl]-5-carbomethoxy-1,2-dihydrocarbazol-4(3H)-one (2.87 g, 8.61 mM) in 29 ml dioxane was added 60% sodium hydride in mineral oil (0.79 g, 19.8 mM). The reaction was stirred 8 minutes, then methyl benzenesulfinate (1.80 ml, 13.8 mM) was added. The reaction was stirred an additional 1.5 h, then diluted with 43 ml dioxane and 1.13 ml acetic acid. The mixture was refluxed 1 h, diluted with ethyl acetate, and extracted with sat'd $NaHCO_3$ two times, then with brine. After drying ($NaSO_4$), evaporation in vacuo afforded 4.90 g. The mixture was purified by column chromatography on silica gel (elution with toluene/methylene chloride) to afford 2.31 g (81%) of the 9-[(phenyl)methyl]-4-hydroxy-5-carbomethoxy carbazole. $^1$H NMR (DMSO-d6) δ 10.25 (s, 1H), 7.7 (d, 1H, J=8 Hz), 7.4 (t, 1H, J=8 Hz), 7.4-7.0 (m, 8H), 6.6 (d, 1H, J=8 Hz), 5.6 (s, 2H), and 3.8 (s, 3H). IR ($CHCl_3$, $cm^{-1}$) 1723, 1685, 1621, 1597, 1568, 1496, 1453, 1442, 1392, 1286, 1267, 1156, and 1138. MS (ES) m/e 330, 332.

| Elemental Analyses for $C_{21}H_{17}NO_3$: | | | |
|---|---|---|---|
| Calculated | C, 76.13; | H, 5.14; | N, 4.23. |
| Found | C, 75.90; | H, 5.20; | N, 4.46. |

C. 9-[(Phenyl)methyl]-4-hydroxy-5-carbamoyl carbazole

A solution of the 9-[(phenyl)methyl]-4-hydroxy-5-carbomethoxy carbazole (200 mg, 0.6 mM) in 4 mL MeOH and 40 mL concentrated aqueous ammonium hydroxide was sonicated for 30 h at 40-50 °C. The mixture was diluted with ethyl acetate and acidified to pH 1 with 5 N HCl. The aqueous layer was extracted three times with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 50 mg (26%) of the 9-[(phenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.85 (dd, 1H, J=1 and 8 Hz), 7.5-7.1 (m, 9H), 6.6 (d, 1H, J=8 Hz), and 5.8 (s, 2H). IR (KBr, $cm^{-1}$) 3428, 3198, 3063, 1631, 1599, 1579, 1562, 1496, 1442, 1330, 1261, 1215, 775, and 697. MS (ES) m/e 315, 317.

| Elemental Analyses for $C_{20}H_{16}N_2O_2$: | | | |
|---|---|---|---|
| Calculated | C, 75.95; | H, 5.06; | N, 8.86. |
| Found | C, 74.88; | H, 5.40; | N, 7.78. |

D. {9-[(Phenyl)methyl]-5-carbamoylcarbazol-4-yl}oxyacetic acid, methyl ester

40% Methanolic Triton B (0.11 mL, 0.24 mM) was added to a solution of the 9-[(phenyl)methyl]-4-hydroxy-5-carbamoyl carbazole (70 mg, 0.22 mM) in 20 mL DMF at 0 °C. After 15 minutes, methyl bromoacetate (70 mg, 0.44 mM) was added and the resultant mixture stirred at room temperature for 5 h. The mixture was diluted with ethyl acetate, washed with 1 N HCl, $H_2O$, and saturated brine, dried over magnesium sulfate, filtered, and concentrated. The residue was combined with the crude material derived from a similar run utilizing 45 mg (0.14 mM [0.36 mM total]) of 9-[(phenyl)methyl]-4-hydroxy-5-carbamoyl carbazole. The combined residues were purified by column chromatography on silica gel (elution with ethyl acetate) to afford 76 mg (54%) of the {9-[(phenyl)methyl]-5-carbamoylcarbazol-4-yl}oxyacetic acid, methyl ester as a white solid. $^1$H NMR (DMSO-d6) δ 7.65 (d, 1H, J=8 Hz), 7.5 (br s, 1H), 7.4-7.15 (m, 9H), 7.1 (d, 1H, J=8 Hz), 6.6 (d, 1H, J=8 Hz), 5.7 (s, 2H), 4.9 (s, 2H), and 3.75 (s, 3H). IR (KBr, $cm^{-1}$) 3367, 3200, 1760, 1643, 1579, 1496, 1452, 1427, 1216, 1157, 772, and 716. MS (FD) m/e 388.

| Elemental Analyses for $C_{23}H_{20}N_2O_4$: | | | |
|---|---|---|---|
| Calculated | C, 71.13; | H, 5.15; | N, 7.22. |
| Found | C, 70.77; | H, 5.49; | N, 6.79. |

E. {9-[(Phenyl)methyl]-5-carbamoylcarbazol-4-yl}oxyacetic acid, sodium salt.

A solution of the {9-[(phenyl)methyl]-5-carbamoylcarbazol-4-yl}oxyacetic acid, methyl ester (10.1 mg, 0.025 mM) and 0.025 mL (0.025 mM) of 1 N NaOH in 3 mL of ethanol was stirred for 16 h at 25 °C. The resultant white precipitate was collected by filtration, washed with a small amount of EtOH, then dried *in vacuo* to afford 7.1 mg (70%) of the {9-[(phenyl)methyl]-5-carbamoylcarbazol-4-yl}oxyacetic acid, sodium salt as a white powder. $^1$H NMR (DMSO-d6) δ 7.6 (d, 1H, J=8 Hz), 7.5-7.05 (m, 11H), 6.55 (d, 1H, J=8 Hz), 5.75 (s, 2H), and 4.3 (s, 2H). IR (KBr, $cm^{-1}$) 3471,

1657, 1615, 1591, 1496, 1453, 1412, 1330, 1272, and 1151. MS (ES) m/e 373, 375, 397. Elemental Analyses for $C_{22}H_{17}N_2O_4Na$: C, 66.67; H, 4.29; N, 7.07. Found C, 66.75; H, 4.55; N, 6.83.

EXAMPLE 25

Preparation of {9-[(3-fluorophenyl)methyl]-5-carbamoylcarbazol-4-yl}oxyacetic acid

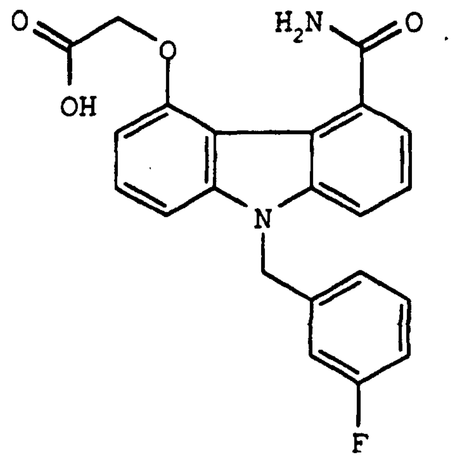

A. 9-[(3-Fluorophenyl)methyl]-5-carbomethoxy-1,2-dihydrocarbazol-4(3H)-one

40% Methanolic Triton B (2.06 mL, 4.53 mM) was slowly added dropwise to a solution of 5-carbomethoxy-1,2-dihydro-9H-carbazol-4(3H)-one (930.0 mg, 3.82 mM) in 5 mL of DMF at 0 °C. After 5 minutes, 3-fluorobenzyl chloride (664.0 mg, 4.59 mM) was added and the resultant mixture stirred at 0 °C for 3 h, then at room temperature for 20 hours. The mixture was diluted with ethyl acetate, washed three times with 1 N HCl, three times with $H_2O$, once with saturated brine, dried over anhydrous magnesium sulfate, filtered, and concentrated. The residue was purified by column chromatography on silica gel (elution with gradient methylene chloride/ethyl acetate) to afford 502.3 mg (37%) of the 9-[(3-fluorophenyl)methyl]-5-carbomethoxy-1,2-dihydrocarbazol-4(3H)-one as a yellow foam. $^1H$ NMR ($CDCl_3$) δ 7.4-7.2 (m, 4H), 6.9 (m, 1H), 6.7 (m, 2H), 5.35 (s, 2H), 4.05 (s, 3H), 2.9 (t, 2H, J=6 Hz), 2.65 (t, 2H, J=6 Hz), and 2.3 (m , 2H). IR ($CHCl_3$, $cm^{-1}$) 3050, 2950, 1725, 1654, 1464, 1451, 1440, 1288 and 1119. MS (ES) m/e 350, 352.

| Elemental Analyses for $C_{21}H_{18}NO_3F$: | | | |
|---|---|---|---|
| Calculated | C, 71.78; | H, 5.16; | N, 3.99. |
| Found | C, 72.00; | H, 4.95; | N, 4.11. |

B. 9-[(3-Fluorophenyl)methyl]-4-hydroxy-5-carbomethoxy carbazole

A solution of the 9-[(3-fluorophenyl)methyl]-5-carbomethoxy-1,2-dihydrocarbazol-4(3H)-one (434.0 mg, 1.23 mM) and 2,3-dichloro-5,6-dicyano-1,4-benzoquinone (324.0 mg, 1.42 mM) in 20 mL of toluene was stirred between 70-80 °C for 5 h. The mixture was purified directly by column chromatography on silica gel (elution with methylene chloride) to afford 137.0 mg (32%) of the 9-[(3-fluorophenyl)methyl]-4-hydroxy-5-carbomethoxy carbazole as a yellow foam. $^1H$ NMR (DMSO-d6) δ 10.2 (s, 1H), 7.7 (d, 1H, J=8 Hz), 7.4 (t, 1H, J=8 Hz), 7.3 (m, 2H), 7.2 (d, 1H, J=8 Hz), 7.1 (d, 1H, J=8 Hz), 7.05-6.85 (m, 3H), 6.6 (d, 1H, J=8 Hz), 5.65 (s, 2H), and 3.85 (s, 3H). IR ($CHCl_3$, $cm^{-1}$) 3200 (br), 1687, 1597, 1452, 1442, 1285, and 1267. MS (ES) m/e 348, 350.

| Elemental Analyses for $C_{21}H_{16}NO_3F$: | | | |
|---|---|---|---|
| Calculated | C, 72.20; | H, 4.62; | N, 4.01. |
| Found | C, 72.30; | H, 4.66; | N, 4.04. |

C. 9-[(3-Fluorophenyl)methyl]-4-hydroxy-5-carbamoyl carbazole

A solution of the 9-[(3-fluorophenyl)methyl]-4-hydroxy-5-carbomethoxy carbazole (130.8 mg, 0.37 mM) in 5 mL THF and 20 mL concentrated aqueous ammonium hydroxide was sonicated for 5 h at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 57.4 mg (45%) of the 9-[(3-fluorophenyl)methyl]-4-hydroxy-5-carbamoyl carbazole as a white solid. $^1H$ NMR (DMSO-d6) δ 10.5 (s, 1H), 8.8 (br s, 1H), 8.4 (br s, 1H), 7.8 (dd, 1H, J=1 and 8 Hz), 7.5 (m, 2H), 7.3 (m, 2H) , 7.15-7.0 (m, 2H), 6.95 (d, 1H, J=8 Hz), 6.85 (d, 1H, J=8 Hz), 6.6 (d, 1H, J=8 Hz), and 5.7 (s, 2H). IR ($CHCl_3$, $cm^{-1}$) 3431, 3200 (br), 1628, 1614, 1600, 1580, 1546, 1488, 1448, 1329, 1261, and 776. MS (ES) m/e 333, 335.

| Elemental Analyses for $C_{20}H_{15}N_2O_2F$: | | | |
|---|---|---|---|
| Calculated | C, 71.85; | H, 4.52; | N, 8.38. |
| Found | C, 74.45; | H, 6.01; | N, 8.48. |

D. {9-[(3-Fluorophenyl)methyl]-5-carbamoylcarbazol-4-yl}oxyacetic acid, tert-butyl ester

40% Methanolic Triton B (0.086 mL, 0.19 mM) was added to a solution of the 9-[(3-fluorophenyl)methyl]-4-hydroxy-5-carbamoyl carbazole (51.9 mg, 0.155 mM) in 3 mL DMF at room temperature. After 3 minutes, t-butyl bromoacetate (87.8 mg, 0.44 mM) was added and the resultant mixture stirred at room temperature for 5 hours. The mixture was diluted with ethyl acetate, washed four times with $H_2O$, and saturated brine, dried over magnesium sulfate, filtered, and concentrated. The residue was purified by column chromatography on silica gel (elution with gradient methylene chloride/ethyl acetate) to afford 44.0 mg (63%) of the {9-[(3-fluorophenyl)methyl]-5-carbamoylcarbazol-4-yl} oxyacetic acid, tert-butyl ester as a white solid. $^1H$ NMR (DMSO-d6) δ 7.6 (d, 1H, J=8 Hz), 7.5-6.8 (m, 10H), 6.55 (d, 1H, J=8 Hz), 5.7 (s, 2H), 4.8 (s, 2H), and 1.45 (s, 9H). IR ($CHCl_3$, $cm^{-1}$) 3450, 3400, 1746, 1674, 1592, 1457, 1369, and 1151. MS (FD) m/e 448.

| Elemental Analyses for $C_{26}H_{25}N_2O_4F$: | | | |
|---|---|---|---|
| Calculated | C, 69.63; | H, 5.62; | N, 6.25. |
| Found | C, 69.35; | H, 5.44; | N, 6.23. |

E. {9-[(3-Fluorophenyl)methyl]-5-carbamoylcarbazol-4-yl}oxyacetic acid

A solution of the {9-[(3-fluorophenyl)methyl]-5-carbamoylcarbazol-4-yl}oxyacetic acid, tert-butyl ester (40.0 mg, 0.089 mM) in 2 mL of trifluoroacetic acid was stirred at room temperature for 5 hours. The solvent was removed *in vacuo*. The residue was triturated with ethyl ether, then dried *in vacuo* to afford 35.0 mg (100%) of the {9-[(3-fluorophenyl)methyl]-5-carbamoylcarbazol-4-yl}oxyacetic acid as a white powder. $^1H$ NMR (DMSO-d6) δ 13.0 (br s, 1H), 7.75 (s, 1H), 7.6 (d, 1H, J=8 Hz), 7.5-7.25 (m, 5H), 7.2-6.8 (m, 4H), 6.6 (d, 1H, J=8 Hz), 5.7 (s, 2H), and 4.8 (s, 2H). IR (KBr, $cm^{-1}$) 3423, 3400, 1736, 1637, 1615, 1589, 1499, 1487, 1450, 1436, 1331, 1250, and 1156. MS (ES) m/e 391, 393.

| Elemental Analyses for $C_{22}H_{17}N_2O_4F$: | | | |
|---|---|---|---|
| Calculated | C, 67.34; | H, 4.37; | N, 7.14. |
| Found | C, 67.63; | H, 4.22; | N, 7.35. |

EXAMPLE 26

Preparation of {9-[(3-Chlorophenyl)methyl]-5-carbamoylcarbazol-4-yl}oxyacetic acid

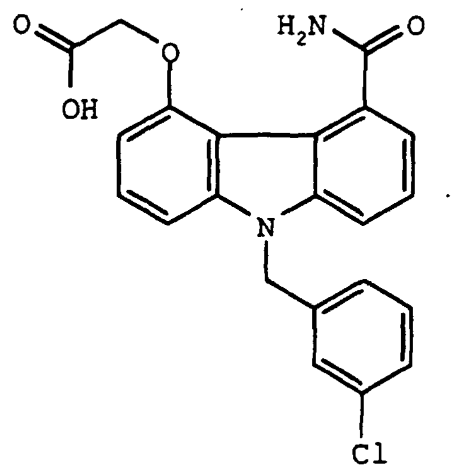

A. 9-[(3-Chlorophenyl)methyl]-5-carbomethoxy-1,2-dihydrocarbazol-4(3H)-one

A suspension of 5-carbomethoxy-1,2-dihydro-9H-carbazol-4(3H)-one (527.0 mg, 2.17 mM), 3-chlorobenzyl bromide (802.2 mg, 3.90 mM), a catalytic amount of sodium iodide (ca. 1 mg), and potassium carbonate (500.0 mg, 3.62 mM) was stirred at room temperature for 150 hours. The mixture was diluted with ethyl acetate, washed five times with $H_2O$, once with saturated brine, dried over anhydrous magnesium sulfate, filtered, and concentrated. The residue was purified by column chromatography on silica gel (elution with gradient methylene chloride/ethyl acetate) to afford 537.1 mg (67%) of the 9-[(3-chlorophenyl)methyl]-5-carbomethoxy-1,2-dihydrocarbazol-4(3H)-one as a yellow foam. $^1H$ NMR ($CDCl_3$) δ 7.5-7.2 (m, 5H), 7.1 (s, 1H), 6.85 (m, 1H), 5.35 (s, 2H), 4.05 (s, 3H), 2.9 (t, 2H, J=6 Hz), 2.65 (t, 2H, J=6 Hz), and 2.3 (m , 2H). IR ($CHCl_3$, $cm^{-1}$) 3050, 2950, 1725, 1654, 1464, 1444, 1432, 1288 and 1120. MS (ES) m/e 366, 368, 370.

| Elemental Analyses for $C_{21}H_{18}NO_3Cl$: | | | |
|---|---|---|---|
| Calculated | C, 68.57; | H, 4.93; | N, 3.81. |
| Found | C, 68.61; | H, 4.92; | N, 3.70. |

B. 9-[(3-Chlorophenyl)methyl]-4-hydroxy-5-carbomethoxy carbazole

A solution of the 9-[(3-chlorophenyl)methyl]-5-carbomethoxy-1,2-dihydrocarbazol-4(3H)-one (480.5 mg, 1.31 mM) and 2,3-dichloro-5,6-dicyano-1,4-benzoquinone (325.7 mg, 1.43 mM) in 50 mL of toluene was stirred between 70-80 °C for 3 hours. The mixture was purified directly by column chromatography on silica gel (elution with methylene chloride) to afford 172.6 mg (36%) of the 9-[(3-chlorophenyl)methyl]-4-hydroxy-5-carbomethoxy carbazole as a yellow foam. $^1H$ NMR ($CDCl_3$) δ 10.4 (s, 1H), 8.05 (d, 1H, J=8 Hz), 7.6 (d, 1H, J=8 Hz), 7.4 (m, 2H), 7.3-7.1 (m, 3H), 6.9-6.7 (m, 3H), 5.55 (s, 2H), and 4.15 (s, 3H). IR ($CHCl_3$, $cm^{-1}$) 3200 (br) , 1684, 1598, 1442, 1428, 1331, 1285, and 1267. MS (ES) m/e 364, 366, 368.

| Elemental Analyses for $C_{21}H_{16}NO_3Cl$: | | | |
|---|---|---|---|
| Calculated | C, 68.95; | H, 4.41; | N, 3.83. |
| Found | C, 69.23; | H, 4.52; | N, 3.88. |

C. 9-[(3-Chlorophenyl)methyl]-4-hydroxy-5-carbamoyl carbazole

A solution of the 9-[(3-chlorophenyl)methyl]-4-hydroxy-5-carbomethoxy carbazole (156.2 mg, 0.43 mM) in 5

mL THF and 20 mL concentrated aqueous ammonium hydroxide was sonicated for 5 hours at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 69.7 mg (47%) of the 9-[(3-chlorophenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.8 (dd, 1H, J=1 and 8 Hz) , 7.45 (m, 2H) , 7.3 (m, 3H) , 7.2 (s, 1H), 7.1 (d, 1H, J=8 Hz), 6.95 (s, 1H), 6.6 (d, 1H, J=8 Hz), and 5.7 (s, 2H). IR ($CHCl_3$, $cm^{-1}$) 3433, 3202 (br), 1630, 1600, 1580, 1564, 1433, 1330, 1261, and 776. MS (ES) m/e 349, 351, 353.

| Elemental Analyses for $C_{20}H_{15}N_2O_2Cl$: | | | |
|---|---|---|---|
| Calculated | C, 68.48; | H, 4.31; | N, 7.99. |
| Found | C, 68.64; | H, 4.55; | N, 7.93. |

D. {9-[(3-Chlorophenyl)methyl]-5-carbamoylcarbazol-4-yl}oxyacetic acid, tert-butyl ester

40% Methanolic Triton B (0.053 mL, 0.12 mM) was added to a solution of the 9-[(3-chlorophenyl)methyl]-4-hydroxy-5-carbamoyl carbazole (33.2 mg, 0.12 mM) in 2 mL DMF at room temperature. After 3 minutes, t-butyl bromoacetate (53.8 mg, 0.27 mM) was added and the resultant mixture stirred at room temperature for 20 h. The mixture was diluted with ethyl acetate, washed four times with $H_2O$, once with saturated brine, dried over magnesium sulfate, filtered, and concentrated. The residue was purified by column chromatography on silica gel (elution with gradient methylene chloride/ethyl acetate) to afford 42.1 mg (95%) of the {9-[(3-chlorophenyl)methyl]-5-carbamoylcarbazol-4-yl}oxyacetic acid, tert-butyl ester as a white solid. $^1$H NMR (DMSO-d6) δ 7.6 (d, 1H, J=8 Hz), 7.5-6.8 (m, 10H), 6.55 (d, 1H, J=8 Hz), 5.7 (s, 2H), 4.8 (s, 2H), and 1.45 (s, 9H). IR ($CHCl_3$, $cm^{-1}$) 3450, 3400, 1744, 1676, 1591, 1457, 1369, and 1150. MS (FD) m/e 464, 466.

| Elemental Analyses for $C_{26}H_{25}N_2O_4Cl$: | | | |
|---|---|---|---|
| Calculated | C, 67.17; | H, 5.42; | N, 6.03. |
| Found | C, 67.17; | H, 5.65; | N, 5.97. |

E. {9-[(3-Chlorophenyl)methyl]-5-carbamoylcarbazol-4-yl}oxyacetic acid

A solution of the {9-[(3-chlorophenyl)methyl]-5-carbamoylcarbazol-4-yl}oxyacetic acid, tert-butyl ester (35.6 mg, 0.077 mM) in 2 mL of trifluoroacetic acid was stirred at room temperature for 6 hours. The solvent was removed *in vacuo*. The residue was triturated with ethyl acetate, then dried *in vacuo* to afford 31.4 mg (100%) of the {9-[(3-chlorophenyl)methyl]-5-carbamoylcarbazol-4-yl}oxyacetic acid as a white powder. $^1$H NMR (DMSO-d6) δ 13.0 (br s, 1H), 7.75 (s, 1H), 7.6 (d, 1H, J=8 Hz), 7.4-7.25 (m, 7H), 7.2 (d, 1H, J=8 Hz), 7.0 (br t, 1H), 6.6 (d, 1H, J=8 Hz), 5.7 (s, 2H), and 4.8 (s, 2H). IR (KBr, $cm^{-1}$) 3456, 3416, 3335, 1735, 1638, 1617, 1580, 1499, 1452, 1431, 1431, 1329, 1255, 1157, 772, 764, and 717. MS (ES) m/e 407, 409, 411.

| Elemental Analyses for $C_{22}H_{17}N_2O_4Cl$: | | | |
|---|---|---|---|
| Calculated | C, 64.63; | H, 4.19; | N, 6.85. |
| Found | C, 64.55; | H, 4.12; | N, 6.74. |

EXAMPLE 27

Preparation of {9-[(3-phenoxyphenyl)methyl]-5-carbamoylcarbazol-4-yl}oxyacetic acid

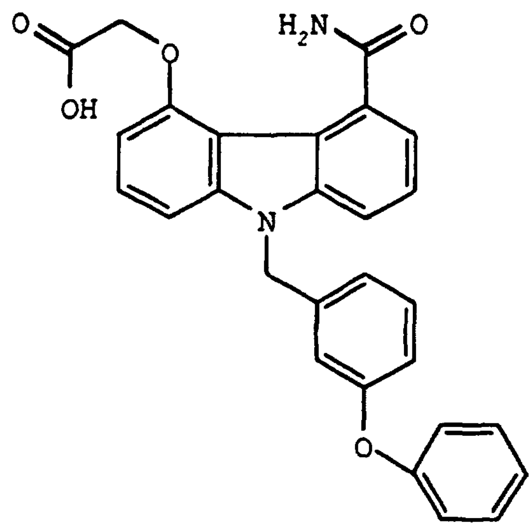

A. 9-[(3-Phenoxyphenyl)methyl]-5-carbomethoxy-1,2-dihydrocarbazol-4(3H)-one

40% Methanolic Triton B (1.53 mL, 3.4 mM) was slowly added dropwise to a solution of 5-carbomethoxy-1,2-dihydro-9H-carbazol-4(3H)-one (554.6 mg, 2.28 mM) in 5 mL of DMF at 25 °C. After 5 minutes, 3-phenoxybenzyl chloride (748.0 mg, 3.42 mM) was added and the resultant mixture stirred at room temperature for 24 hours. The mixture was diluted with ethyl acetate, washed three times with 1N HCl, three times with $H_2O$, once with saturated brine, dried over anhydrous magnesium sulfate, filtered, and concentrated. The residue was purified by column chromatography on silica gel (elution with gradient methylene chloride/ethyl acetate) to afford 563.6 mg (58%) of 9-[(3-phenoxyphenyl)methyl]-5-carbomethoxy-1,2-dihydrocarbazol-4(3H)-one as a thick yellow oil. $^1H$ NMR (DMSO-d6) δ 7.7 (dd, 1H, J=1 and 8 Hz), 7.4-7.2 (m, 6H), 7.1 (t, 1H, J=8 Hz), 6.95 (m, 2H), 6.8-6.7 (m, 2H), 5.55 (s, 2H), 3.75 (s, 3H), 3.0 (t, 2H, J=6 Hz), 2.45 (t, 2H, J=6 Hz), and 2.1 (m , 2H). IR ($CHCl_3$, $cm^{-1}$) 3050, 2950, 1725, 1653, 1585, 1487, 1465, 1288, 1252, and 1119. MS (ES) m/e 426.

| Elemental Analyses for $C_{27}H_{23}NO_4$: | | | |
|---|---|---|---|
| Calculated | C, 76.22; | H, 5.45; | N, 3.29. |
| Found | C, 76.21; | H, 5.35; | N, 3.36. |

B. 9-[(3-Phenoxyphenyl)methyl]-4-hydroxy-5-carbomethoxy carbazole

A solution of the 9-[(3-phenoxyphenyl)methyl]-5-carbomethoxy-1,2-dihydrocarbazol-4(3H)-one (544.5 mg, 1.28 mM) and 2,3-dichloro-5,6-dicyano-1,4-benzoquinone (337.5 mg, 1.48 mM) in 20 mL of toluene was stirred between 70-80 °C for 4 hours. The mixture was purified directly by column chromatography on silica gel (elution with methylene chloride) to afford 107.0 mg (20%) of 9-[(3-phenoxyphenyl)methyl]-4-hydroxy-5-carbomethyoxy carbazole as a yellow powder. $^1H$ NMR (DMSO-d6) δ 10.4 (s, 1H), 7.7 (d, 1H, J=8 Hz), 7.4-6.7 (m, 13H), 6.55 (d, 1H, J=8 Hz), 5.65 (s, 2H), and 3.85 (s, 3H). IR ($CHCl_3$, $cm^{-1}$) 3200 (br), 1687, 1597, 1584, 1487, 1441, 1332, 1284, 1267, and 1252. MS (ES) m/e 422, 424.

| Elemental Analyses for $C_{27}H_{21}NO_4$: | | | |
|---|---|---|---|
| Calculated | C, 76.58; | H, 5.00; | N, 3.31. |
| Found | C, 76.68; | H, 5.20; | N, 3.40. |

c) 9-[(3-Phenoxyphenyl)methyl]-4-hydroxy-5-carbamoyl carbazole

A solution of the 9-[(3-phenoxyphenyl)methyl]-4-hydroxy-5-carbomethoxy carbazole (100.0 mg, 0.24 mM) in 5 mL THF and 20 mL concentrated aqueous ammonium hydroxide was sonicated for 24 hours at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 41.0 mg (43%) of the 9-[(3-phenoxyphenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.8 (dd, 1H, J=1 and 8 Hz), 7.5-6.7 (m, 13H), 6.6 (d, 1H, J=8 Hz), and 5.7 (s, 2H). MS (ES) m/e 407, 409.

| Elemental Analyses for $C_{26}H_{20}N_2O_3$: | | | |
|---|---|---|---|
| Calculated | C, 76.46; | H, 4.94; | N, 6.86. |
| Found | C, 75.66; | H, 5.29; | N, 6.58. |

D. {9-[(3-Phenoxyphenyl)methyl]-5-carbamoylcarbazol-4-yl}oxyacetic acid, tert-butyl ester

40% Methanolic Triton B (0.054 mL, 0.12 mM) was added to a solution of the 9-[(3-phenoxyphenyl)methyl]-4-hydroxy-5-carbamoyl carbazole (39.5 mg, 0.10 mM) in 3 mL DMF at room temperature. After 3 minutes, t-butyl bromoacetate (54.8 mg, 0.27 mM) was added and the resultant mixture stirred at room temperature for 5 hours. The mixture was diluted with ethyl acetate, washed four times with $H_2O$, once with saturated brine, dried over magnesium sulfate, filtered, and concentrated. The residue was purified by column chromatography on silica gel (elution with gradient methylene chloride/ethyl acetate) to afford 33.0 mg (65%) of the {9-[(3-phenoxyphenyl)methyl]-5-carbamoylcarbazol-4-yl}oxyacetic acid, tert-butyl ester as a white solid. $^1$H NMR (DMSO-d6) δ 7.6 (d, 1H, J=8 Hz), 7.5-6.8 (m, 15H), 6.55 (d, 1H, J=8 Hz), 5.7 (s, 2H), 4.8 (s, 2H), and 1.45 (s, 9H). IR (KBr, $cm^{-1}$) 3450, 1748, 1670, 1582, 1486, 1246, 1225, and 1151. MS (ES) m/e 523.

| Elemental Analyses for $C_{32}H_{30}N_2O_5$: | | | |
|---|---|---|---|
| Calculated | C, 73.55; | H, 5.79; | N, 5.36. |
| Found | C, 73.84; | H, 5.83; | N, 5.30. |

E. {9-[(3-Phenoxyphenyl)methyl]-5-carbamoylcarbazol-4-yl} oxyacetic acid

A solution of the {9-[(3-phenoxyphenyl)methyl]-5-carbamoylcarbazol-4-yl}oxyacetic acid, tert-butyl ester (30.0 mg, 0.063 mM) in 2 mL of trifluoroacetic acid was stirred at room temperature for 6 hours. The solvent was removed *in vacuo*. The residue was dried *in vacuo* to afford 30.0 mg (100%) of the {9-[(3-phenoxyphenyl)methyl]-5-carbamoylcarbazol-4-yl}oxyacetic acid as a white powder. $^1$H NMR (DMSO-d6) δ 13.0 (br s, 1H), 7.75 (s, 1H), 7.6 (d, 1H, J=8 Hz), 7.5-6.8 (m, 14H), 6.6 (d, 1H, J=8 Hz), 5.7 (s, 2H), and 4.8 (s, 2H). IR (KBr, $cm^{-1}$) 3450, 3400, 1740, 1651, 1592, 1585, 1487, 1457, 1441, 1329, 1250, and 1158. MS (ES) m/e 465, 467.

| Elemental Analyses for $C_{28}H_{22}N_2O_5$: | | | |
|---|---|---|---|
| Calculated | C, 72.09; | H, 4.75; | N, 6.00. |
| Found | C, 67.65; | H, 4.64; | N, 6.02. |

EXAMPLE 28

Preparation of {9-[(2-Fluorophenyl)methyl]-5-carbamoylcarbazol-4-yl}oxyacetic acid

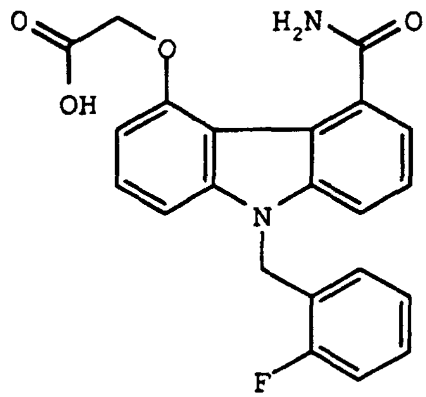

A. 9-[(2-Fluorophenyl)methyl]-5-carbomethoxy-1,2-dihydrocarbazol-4(3H)-one

40% Methanolic Triton B (2.82 mL, 6.2 mM) was slowly added dropwise to a solution of 5-carbomethoxy-1,2-dihydro-9H-carbazol-4(3H)-one (1.27 g, 5.22 mM) in 10 mL of DMF at 25 °C. After 5 minutes, 2-fluorobenzyl bromide (1.19 g, 6.2 mM) was added and the resultant mixture stirred at room temperature for 17 days. The mixture was diluted with ethyl acetate, washed five times with $H_2O$, once with saturated brine, dried over anhydrous magnesium sulfate, filtered, and concentrated. The residue was purified by column chromatography on silica gel (elution with gradient methylene chloride/ethyl acetate) to afford 1.00 g (55%) of the 9-[(2-fluorophenyl)methyl]-5-carbomethoxy-1,2-dihydrocarbazol-4(3H)-one as a tan foam. $^1$H NMR (DMSO-d6) δ 7.7 (dd, 1H, J=1 and 8 Hz), 7.4-7.2 (m, 4H), 7.1 (t, 1H, J=8 Hz) , 6.7 (t, 1H, J=8 Hz), 5.65 (s, 2H) , 3.8 (s, 3H), 3.0 (t, 2H, J=6 Hz), 2.45 (t, 2H, J=6 Hz), and 2.1 (m, 2H). IR ($CHCl_3$, $cm^{-1}$) 3050, 2950, 1725, 1652, 1464, 1441, 1288 and 1120. MS (ES) m/e 350, 352.

| Elemental Analyses for $C_{21}H_{18}NO_3F$: | | | |
|---|---|---|---|
| Calculated | C, 71.78; | H, 5.16; | N, 3.99. |
| Found | C, 71.51; | H, 5.08; | N, 3.85. |

B. 9-[(2-Fluorophenyl)methyl]-4-hydroxy-5-carbomethoxy carbazole

A solution of the 9-[(2-fluorophenyl)methyl]-5-carbomethoxy-1,2-dihydrocarbazol-4(3H)-one (1.00 g, 2.85 mM) and 2,3-dichloro-5,6-dicyano-1,4-benzoquinone (800.0 mg, 3.51 mM) in 50 mL of toluene was stirred between 70-80 °C for 6 h. The mixture was purified directly by column chromatography on silica gel (elution with methylene chloride) to afford 250.0 mg (25%) of the 9-[(2-fluorophenyl)methyl]-4-hydroxy-5-carbomethyoxy carbazole as a dark solid. $^1$H NMR (DMSO-d6) δ 10.2 (s, 1H), 7.7 (d, 1H, J=8 Hz), 7.4 (t, 1H, J=8 Hz), 7.3-6.85 (m, 6H), 6.75 (dt, 1H, J=. 5 and 8 Hz), 6.6 (d, 1H, J=8 Hz), 5.7 (s, 2H), and 3.85 (s, 3H). IR ($CHCl_3$, $cm^{-1}$) 3200 (br), 1686, 1598, 1490, 1442, 1285, 1268, 1230, and 1139. MS (ES) m/e 348, 350.

| Elemental Analyses for $C_{21}H_{16}NO_3F$: | | | |
|---|---|---|---|
| Calculated | C, 72.20; | H, 4.62; | N, 4.01. |
| Found | C, 71.32; | H, 4.75; | N, 4.11. |

C. 9-[(2-Fluorophenyl)methyl]-4-hydroxy-5-carbamoyl carbazole

A solution of the 9-[(2-fluorophenyl)methyl]-4-hydroxy-5-carbomethoxy carbazole (237.5 mg, 0.68 mM) in 10 mL THF and 40 mL concentrated aqueous ammonium hydroxide was sonicated for 20 h at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 89.7 mg (40%) of the 9-[(2-fluorophenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.8 (dd, 1H, J=1 and 8 Hz), 7.5-6.9 (m, 7H), 6.65 (m, 2H), and 5.75 (s, 2H). IR (KBr, cm$^{-1}$) 3395, 3192 (br), 1621, 1599, 1580, 1564, 1491, 1455, 1334, 1261, and 774. MS (ES) m/e 333, 335.

| Elemental Analyses for $C_{20}H_{15}N_2O_2F$: | | | |
|---|---|---|---|
| Calculated | C, 71.85; | H, 4.52; | N, 8.38. |
| Found | C, 72.57; | H, 4.88; | N, 7.84. |

D. {9-[(2-Fluorophenyl)methyl]-5-carbamoylcarbazol-4-yl}oxyacetic acid, methyl ester

40% Methanolic Triton B (0.14 mL, 0.31 mM) was added to a solution of the 9-[(2-fluorophenyl)methyl]-4-hydroxy-5-carbamoyl carbazole (51.9 mg, 0.155 mM) in 5 mL DMF at room temperature. After 3 minutes, methyl bromoacetate (110.5 mg, 0.72 mM) was added and the resultant mixture stirred at room temperature for 20 hours. The mixture was diluted with ethyl acetate, washed four times with $H_2O$, once with saturated brine, dried over magnesium sulfate, filtered, and concentrated. The residue was purified by column chromatography on silica gel (elution with gradient methylene chloride/ethyl acetate) to afford 72.8 mg (71%) of the {9-[(2-fluorophenyl)methyl]-5-carbamoylcarbazol-4-yl}oxyacetic acid, methyl ester as a white solid. $^1$H NMR (DMSO-d6) δ 7.65 (d, 1H, J=8 Hz), 7.5 (s, 1H), 7.4-7.2 (m, 5H), 7.15 (s, 1H), 7.1 (d, 1H, J=8 Hz), 7.05 (t, 1H, J=8 Hz), 6.7 (t, 1H, J=8 Hz) , 6.55 (d, 1H, J=8 Hz) , 5.7 (s, 2H), 4.85 (s, 2H), and 3.7 (s, 3H). IR ($CHCl_3$, cm$^{-1}$) 3436, 1763, 1675,1457, 1327, 1208, 1198, 1150, 1102, 772, 756, and 719. MS (FD) m/e 407.

| Elemental Analyses for $C_{23}H_{19}N_2O_4F$: | | | |
|---|---|---|---|
| Calculated | C, 67.97; | H, 4.71; | N, 6.89. |
| Found | C, 68.00; | H, 4.92; | N, 6.75. |

E. {9-[(2-Fluorophenyl)methyl]-5-carbamoylcarbazol-4-yl}oxyacetic acid

A solution of the {9-[(2-fluorophenyl)methyl]-5-carbamoylcarbazol-4-yl}oxyacetic acid, methyl ester (47.9 mg, 0.118 mM) and 0.28 mL (0.28 mM) of 1 N NaOH in 10 mL of methanol was sonicated for 6 hours at 50-60 °C, then stirred at room temperature for 16 hours. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated to afford 42.8 mg (92%) of the {9-[(2-fluorophenyl) methyl]-5-carbamoylcarbazol-4-yl}oxyacetic acid as a white powder. $^1$H NMR (DMSO-d6) δ 7.75 (s, 1H), 7.6 (d, 1H, J=8 Hz), 7.5-7.25 (m, 6H), 7.15 (d, 1H, J=8 Hz), 7.05 (dt, 1H, J=1 and 8 Hz), 6.75 (dt, 1H, J=1 and 8 Hz), , 6.65 (d, 1H, J=8 Hz), 5.7 (s, 2H), and 4.8 (s, 2H). IR (KBr, cm$^{-1}$) 3428, 3400, 1737, 1635, 1617, 1583, 1572, 1500, 1491, 1453, 1434, 1330, 1248, 1158, 1098, 760, and 714. MS (FD) m/e 392.

| Elemental Analyses for $C_{22}H_{17}N_2O_4F$: | | | |
|---|---|---|---|
| Calculated | C, 67.34; | H, 4.37; | N, 7.14. |
| Found | C, 66.65; | H, 4.55; | N, 6.92. |

EXAMPLE 29

Preparation of {9-[(2-trifluoromethylphenyl)methyl]-5-carbamoylcarbazol-4-yl}oxyacetic acid

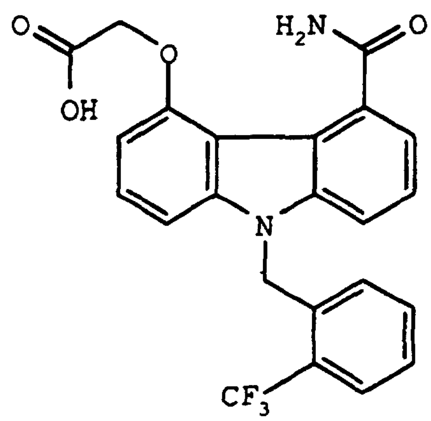

A. 9-[(2-Trifluoromethylphenyl)methyl]-5-carbomethoxy-1,2-dihydrocarbazol-4(3H)-one

40% Methanolic Triton B (2.18 mL, 4.8 mM) was slowly added dropwise to a solution of 5-carbomethoxy-1,2-dihydro-9H-carbazol-4(3H)-one (973 mg, 4.0 mM) in 10 mL of DMF at -10 °C. After 30 minutes, 2-(trifluoromethyl) benzyl bromide (1.3 g, 5.2 mM) was added and the resultant mixture stirred at room temperature for 23 hours. The mixture was diluted with ethyl acetate, washed five times with $H_2O$, once with saturated brine, dried over anhydrous magnesium sulfate, filtered, concentrated, and dried *in vacuo* to afford 1.34 g (83%) of the 9-[(2-trifluoromethylphenyl) methyl]-5-carbomethoxy-1,2-dihydrocarbazol-4(3H)-one as a tan solid. $^1H$ NMR ($CDCl_3$) δ 7.7 (d, 1H, J=8 Hz), 7.4-7.1 (m, 5H), 6.4 (d, 1H, J=8 Hz), 5.5 (s, 2H), 4.05 (s, 3H), 2.8 (t, 2H, J=6 Hz), 2.6 (t, 2H, J=6 Hz), and 2.2 (m, 2H). IR (KBr, $cm^{-1}$) 1729 and 1656. MS (ES) m/e 402.

| Elemental Analyses for $C_{22}H_{18}NO_3F_3$: | | | | |
|---|---|---|---|---|
| Calculated | C, 65.83; | H, 4.52; | N, 3.49; | F, 14.20. |
| Found | C, 66.07; | H, 4.59; | N, 3.20; | F, 13.95. |

B. 9-[(2-Trifluoromethylphenyl)methyl]-4-hydroxy-5-carbomethoxy carbazole

A solution of the 9-[(2-trifluoromethylphenyl)methyl]-5-carbomethoxy-1,2-dihydrocarbazol-4(3H)-one (1.21 g, 3.00 mM) and 2,3-dichloro-5,6-dicyano-1,4-benzoquinone (764 mg, 3.3 mM) in 25 mL of toluene was stirred between 80-90 °C for 7 h. The mixture was purified directly by column chromatography on silica gel (elution with methylene chloride) to afford 340.0 mg (28%) of the 9-[(2-trifluoromethylphenyl)methyl]-4-hydroxy-5-carbomethyoxy carbazole as a brown powder. $^1H$ NMR ($CDCl_3$) δ 10.4 (s, 1H), 8.0 (d, 1H, J=8 Hz), 7.7 (d, 1H, J=8 Hz), 7.5-7.2 (m, 5H), 6.85 (m, 2H), 6.45 (d, 1H, J=8 Hz), 5.7 (s, 2H), and 4.1 (s, 3H). IR ($CHCl_3$, $cm^{-1}$) 3200 (br) and 1677. MS (ES) m/e 398, 400.

| Elemental Analyses for $C_{22}H_{16}NO_3F_3$: | | | | |
|---|---|---|---|---|
| Calculated | C, 66.17; | H, 4.04; | N, 3.51; | F, 14.27. |
| Found | C, 66.93; | H, 4.06; | N, 3.54; | F, 14.00. |

C. 9-[(2-Trifluoromethylphenyl)methyl]-4-hydroxy-5-carbamoyl carbazole

A solution of the 9-[(2-trifluoromethylphenyl)methyl]-4-hydroxy-5-carbomethoxy carbazole (310 mg, 0.77 mM) in 5 mL THF and 20 mL concentrated aqueous ammonium hydroxide was sonicated for 25 hours at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered,

and concentrated. The residue was purified by column chromatography on silica gel (elution with gradient methylene chloride/ethyl acetate) to afford 145 mg (49%) of the 9-[(2-trifluoromethylphenyl)methyl]-4-hydroxy-5-carbamoyl carbazole as a white solid. $^1H$ NMR (DMSO-d6) δ 10.5 (s, 1H), 8.8 (br s, 1H), 8.4 (br s, 1H), 7.8 (d, 1H, J=8 Hz), 7.6-7.2 (m, 6H), 6.85 (d, 1H, J=8 Hz), 6.6 (d, 1H, J=8 Hz), 6.25 (d, 1H, J=8 Hz), and 5.8 (s, 2H). IR (KBr, $cm^{-1}$) 3460, 3360, and 1589. MS (ES) m/e 383, 385.

| Elemental Analyses for $C_{21}H_{15}N_2O_2F_3$: | | | | |
|---|---|---|---|---|
| Calculated | C, 65.62; | H, 3.93; | N, 7.29; | F, 14.83. |
| Found | C, 65.65; | H, 3.94; | N, 7.51; | F, 14.94. |

D. {9-[(2-Trifluoromethylphenyl)methyl]-5-carbamoylcarbazol-4-yl}oxyacetic acid, methyl ester

40% Methanolic Triton B (0.18 mL, 0.4 mM) was added to a solution of the 9-[(2-trifluoromethylphenyl)methyl]-4-hydroxy-5-carbamoyl carbazole (120 mg, 0.31 mM) in 5 mL DMF at room temperature. After 15 minutes, methyl bromoacetate (98.5 mg, 0.62 mM) was added and the resultant mixture stirred at room temperature for 4.5 hours. The mixture was diluted with ethyl acetate, washed four times with $H_2O$, 1 N HCl, $H_2O$, sat. $NaHCO_3$, and saturated brine, dried over magnesium sulfate, filtered, and concentrated. The residue was purified by column chromatography on silica gel (elution with gradient methylene chloride/ethyl acetate/THF) to afford 95 mg (67%) of the {9-[(2-trifluoromethylphenyl)methyl]-5-carbamoylcarbazol-4-yl}oxyacetic acid, methyl ester as a white solid. $^1H$ NMR ($CDCl_3$) δ 7.7 (d, 1H, J=8 Hz), 7.5-7.2 (m, 6H), 6.95 (d, 1H, J=8 Hz), 6.6 (d, 1H, J=8 Hz), 6.45 (d, 1H, J=8 Hz), 6.3 (br s, 1H), 6.1 (br s, 1H), 5.7 (s, 2H), 4.9 (s, 2H), and 3.9 (s, 3H). IR ($CHCl_3$, $cm^{-1}$) 1763 and 1674. MS (ES) m/e 457.

| Elemental Analyses for $C_{24}H_{19}N_2O_4F_3$: | | | |
|---|---|---|---|
| Calculated | C, 63.16; | H, 4.20; | N, 6.14. |
| Found | C, 61.82; | H, 4.31; | N, 5.86. |

E. {9-[(2-Trifluoromethylphenyl)methyl]-5-carbamoylcarbazol-4-yl}oxyacetic acid

A solution of the {9-[(2-trifluoromethylphenyl)methyl]-5-carbamoylcarbazol-4-yl}oxyacetic acid, methyl ester (70 mg, 0.153 mM) and 0.21 mL (0.21 mM) of 1 N NaOH in 5 mL of methanol was sonicated for 23 hours at 50-60 °C. The methanol was removed *in vacuo* and the mixture acidified to pH 1.6 with 1 N HCl. The resultant white precipitate was collected by filtration, washed with $H_2O$, small amounts of MeOH and diethyl ether, then dried *in vacuo* to afford 59 mg (88%) of the {9-[(2-trifluoromethylphenyl)methyl]-5-carbamoylcarbazol-4-yl}oxyacetic acid as a white powder. $^1H$ NMR (DMSO-d6) δ 13.0 (br s, 1H), 7.8 (d, 1H, J=8 Hz), 7.75 (s, 1H), 7.5-7.3 (m, 6H), 7.1 (d, 1H, J=8 Hz), 7.05 (d, 1H, J=8 Hz), 6.6 (d, 1H, J=8 Hz), , 6.3 (d, 1H, J=8 Hz), 5.8 (s, 2H), and 4.8 (s, 2H). IR (KBr, $cm^{-1}$) 1737 and 1635. MS (ES) m/e 441, 443.

| Elemental Analyses for $C_{23}H_{17}N_2O_4F_3$: | | | | |
|---|---|---|---|---|
| Calculated | C, 62.45; | H, 3.87; | N, 6.33; | F, 12.88. |
| Found | C, 60.86; | H, 3.89; | N, 6.08; | F, 12.59. |

EXAMPLE 30

Preparation of {9-[(2-benzylphenyl)methyl]-5-carbamoylcarbazol-4-yl}oxyacetic acid, sodium salt

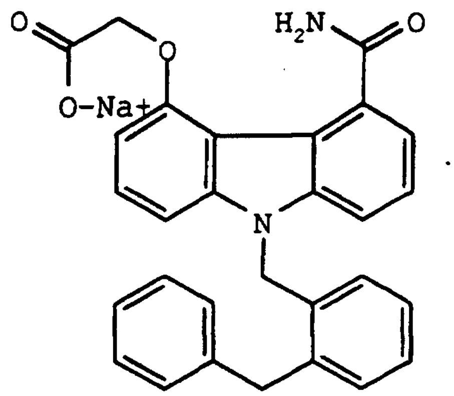

A. 2-Benzylbenzyl bromide

A solution of phosphorus tribromide (2.1 mL, 6.0 g, 22.1 mM) in 30 mL of carbon tetrachloride was slowly added dropwise to solution of 2-benzylbenzyl alcohol (1.98 g, 10 mM) in 70 mL of carbon tetrachloride at 0 °C. The mixture was stirred at 0 °C for 2 hours, then at room temperature for 2 hours. The solvent was removed *in vacuo* and the residue diluted with ethyl acetate and saturated aqueous sodium bicarbonate. The organic layer was washed with brine, dried over magnesium sulfate, filtered, and concentrated to afford 2.6 g (99%)of 2-benzylbenzyl bromide as a yellow solid. $^1H$ NMR (DMSO-d6) δ 7.5-7.0 (m, 9H), 4.7 (s, 2H), and 4.15 (s, 2H). IR ($CHCl_3$, $cm^{-1}$) 3065, 1601, 1495,and 1453. MS (FD) m/e 260, 262.

| Elemental Analyses for $C_{14}H_{13}Br$: | | | |
|---|---|---|---|
| Calculated | C, 64.37; | H, 4.98; | N, 0.00. |
| Found | C, 65.26; | H, 5.26; | N, 0.00. |

B. 9-[(2-Benzylphenyl)methyl]-5-carbomethoxy-1,2-dihydrocarbazol-4(3H)-one

40% Methanolic Triton B (0.95 mL, 2.1 mM) was slowly added dropwise to a solution of 5-carbomethoxy-1,2-dihydro-9H-carbazol-4(3H)-one (510 mg, 2.1 mM) in 30 mL of DMF at -10 °C. After 3 minutes, 2-benzylbenzyl bromide (548 mg, 2.1 mM) was added and the resultant mixture stirred at room temperature for 6 hours. The mixture was diluted with ethyl acetate and 1 N HCl, washed twice with $H_2O$, once with saturated brine, dried over anhydrous magnesium sulfate, filtered, concentrated, and dried *in vacuo*. The reside was purified by column chromatography on silica gel (elution with ethyl acetate) to afford 324 mg (36%) of the 9-[(2-benzylphenyl)methyl]-5-carbomethoxy-1,2-dihydrocarbazol-4(3H)-one as a tan solid. $^1H$ NMR ($CDCl_3$) δ 7.45-7.0 (m, 10H), 6.9 (d, 1H, J=8 Hz), 6.3 (d, 1H, J=8 Hz), 5.2 (s, 2H), 4.15 (s, 2H), 4.05 (s, 3H), 2.5 (m, 4H), and 2.1 (m , 2H). IR (KBr, $cm^{-1}$) 1726, 1653, 1466, 1443, 1411, 1283, 1200, 1119, and 749. MS (ES) m/e 422, 424.

| Elemental Analyses for $C_{28}H_{25}NO_3$: | | | |
|---|---|---|---|
| Calculated | C, 79.43; | H, 5.91; | N, 3.31. |
| Found | C, 79.58; | H, 5.94; | N, 3.32. |

C. 9-[(2-Benzylphenyl)methyl]-4-hydroxy-5-carbomethoxy carbazole

A solution of the 9-[(2-benzylphenyl)methyl]-5-carbomethoxy-1,2-dihydrocarbazol-4(3H)-one (480 mg, 1.14 mM) and 2,3-dichloro-5,6-dicyano-1,4-benzoquinone (250 mg, 5.0 mM) in 30 mL of toluene was stirred between 80-90 °C for 5 hours. The mixture was purified directly by column chromatography on silica gel (elution with methylene chloride/ethyl acetate) to afford 166 mg (35%) of the 9-[(2-benzylphenyl)methyl]-4-hydroxy-5-carbomethyoxy carbazole as a yellow solid. $^1H$ NMR ($CDCl_3$) δ 10.4 (s, 1H), 8.0 (d, 1H, J=8 Hz), 7.4-7.0 (m, 11H), 6.8 (d, 1H, J=8 Hz), 6.6 (d, 1H, J=8 Hz), 6.4 (d, 1H, J=8 Hz), 5.4 (s, 2H), 4.25 (s, 2H), and 4.1 (s, 3H). IR ($CHCl_3$, $cm^{-1}$) 1684, 1597, 1495, 1452, 1442, 1333, 1284, 1269, and 1140. MS (ES) m/e 420, 422.

D. 9-[(2-8enzylphenyl)methyl]-4-hydroxy-5-carbamoyl carbazole

A solution of the 9-[(2-benzylphenyl)methyl]-4-hydroxy-5-carbomethoxy carbazole (166 mg, 0.39 mM) in 8 mL THF and 30 mL concentrated aqueous ammonium hydroxide was sonicated for 30 hours at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with ethyl acetate) to afford 70 mg (44%) of the 9-[(2-benzylphenyl)methyl]-4-hydroxy-5-carbamoyl carbazole as a white solid. $^1H$ NMR ($CDCl_3$) δ 8.0 (d, 1H, J=8 Hz), 7.4-7.0 (m, 12H), 6.8 (d, 1H, J=8 Hz), 6.6 (d, 1H, J=8 Hz), 6.5 (m, 1H), 6.4 (m, 1H), 5.8 (s, 1H), 5.4 (s, 2H), and 4.2 (s, 2H). MS (ES) m/e 405, 407.

E. {9-[(2-Benzylphenyl)methyl]-5-carbamoylcarbazol-4-yl}oxyacetic acid, methyl ester

40% Methanolic Triton B (0.12 mL, 0.26 mM) was added to a solution of the 9-[(2-benzylphenyl)methyl]-4-hydroxy-5-carbamoyl carbazole (70 mg, 0.17 mM) in 10 mL DMF at 25 °C. After 3 minutes, methyl bromoacetate (55 mg, 0.34 mM) was added and the resultant mixture stirred at room temperature for 25 hours. The mixture was diluted with ethyl acetate, washed with 1 N HCl, $H_2O$, and saturated brine, dried over magnesium sulfate, filtered, and concentrated. The residue was purified by column chromatography on silica gel (elution with ethyl acetate) to afford 60 mg (73%) of the {9-[(2-benzylphenyl)methyl]-5-carbamoylcarbazol-4-yl}oxyacetic acid, methyl ester as a white solid. $^1H$ NMR ($CDCl_3$) δ 7.4-7.00 (m, 14H), 6.65 (d, 1H, J=8 Hz), 6.55 (d, 1H, J=8 Hz), 6.4 (d, 1H, J=8 Hz), 5.4 (s, 2H), 4.95 (s, 2H), 4.2 (s, 2H), and 3.80 (s, 3H). IR (KBr, $cm^{-1}$) 3414, 3186, 1759, 1625, 1583, 1500, 1452, 1424, 1340, 1325, 1213, 1199, and 1108. MS (ES) m/e 477, 479.

| Elemental Analyses for $C_{30}H_{26}N_2O_4$: | | | |
|---|---|---|---|
| Calculated | C, 75.31; | H, 5.44; | N, 5.86. |
| Found | C, 75.08; | H, 5.61; | N, 5.70. |

F. {9-[(2-Benzylphenyl)methyl]-5-carbamoylcarbazol-4-yl}oxyacetic acid, sodium salt.

A solution of the {9-[(2-benzylphenyl)methyl]-5-carbamoylcarbazol-4-yl}oxyacetic acid, methyl ester (16.2 mg, 0.034 mM) and 0.034 mL (0.034 mM) of 1 N NaOH in 3 mL of ethanol was stirred for 16 hours at 25 °C. The resultant white precipitate was collected by filtration, washed with a small amount of EtOH, then dried *in vacuo* to afford 7.1 mg (70%) of the {9-[(2-benzylphenyl)methyl]-5-carbamoylcarbazol-4-yl}oxyacetic acid, sodium salt as a white powder. $^1H$ NMR (DMSO-d6) δ 7.5-6.8 (m, 14H), 6.65 (d, 1H, J=8 Hz), 6.55 (d, 1H, J=8 Hz), 6.05 (d, 1H, J=8 Hz), 5.55 (s, 2H), 4.35 (s, 2H), and 4.3 (s, 2H). IR ($CHCl_3$, $cm^{-1}$) 1666, 1616, 1495, 1452, and 1422. MS (ES) m/e 463, 465.

| Elemental Analyses for $C_{29}H_{23}N_2O_4Na$: | | | |
|---|---|---|---|
| Calculated | C, 71.60; | H, 4.73; | N, 5.76. |
| Found | C, 64.68; | H, 4.79; | N, 5.08. |

EXAMPLE 31

Preparation of {9-[(3-trifluoromethylphenyl)methyl]-5-carbamoylcarbazol-4-yl}oxyacetic acid, sodium salt

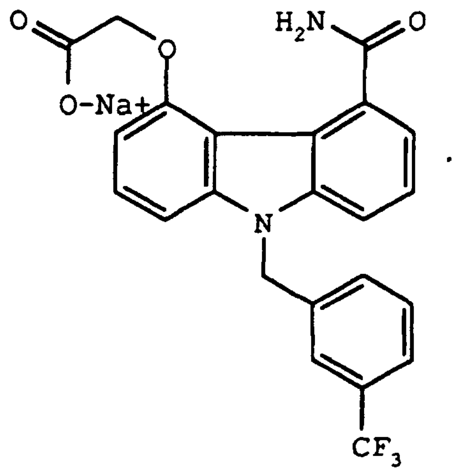

A. 9-[(3-Trifluoromethylphenyl)methyl]-5-carbomethoxy-1,2-dihydrocarbazol-4(3H)-one

40% Methanolic Triton B (2.18 mL, 4.8 mM) was slowly added dropwise to a solution of 5-carbomethoxy-1,2-dihydro-9H-carbazol-4(3H)-one (973 mg, 4.0 mM) in 10 mL of DMF at -10 °C. After 30 minutes, 3-(trifluoromethyl) benzyl chloride (1.53 g, 6.0 mM) and sodium iodide (900 mg, 6.0 mM) were added and the resultant mixture stirred at room temperature for 25 hours. The mixture was diluted with ethyl acetate, washed five times with $H_2O$, 1 N HCl, $H_2O$, sat $NaHCO_3$, and saturated brine, dried over anhydrous magnesium sulfate, filtered, concentrated, and dried *in vacuo*. The residue was purified by column chromatography on silica gel (elution with gradient methylene chloride/ethyl acetate) to afford 1.02 g (63%) of the 9-[(3-trifluoromethylphenyl)methyl]-5-carbomethoxy -1,2-dihydrocarbazol-4(3H)-one as a tan solid. $^1H$ NMR ($CDCl_3$) δ 7.6 (d, 1H, J=8 Hz), 7.45-7.2 (m, 5H) , 7.0 (d, 1H, J=8 Hz), 5.4 (s, 2H), 4.05 (s, 3H), 2.85 (t, 2H, J=6 Hz), 2.6 (t, 2H, J=6 Hz), and 2.2 (m, 2H). IR (KBr, $cm^{-1}$) 1727 and 1652. MS (ES) m/e 400, 402.

| Elemental Analyses for $C_{22}H_{18}NO_3F_3$: | | | | |
|---|---|---|---|---|
| Calculated | C, 65.83; | H, 4.52; | N, 3.49; | F, 14.20. |
| Found | C, 65.63; | H, 4.58; | N, 3.39; | F, 14.14. |

B. 9-[(3-Trifluoromethylphenyl)methyl]-4-hydroxy-5-carbomethoxy carbazole

A solution of the 9-[(3-trifluoromethylphenyl)methyl]-5-carbomethoxy-1,2-dihydrocarbazol-4(3H)-one (1.21 g, 3.00 mM) and 2,3-dichloro-5,6-dicyano-1,4-benzoquinone (764 mg, 3.3 mM) in 25 mL of toluene was stirred between 80-90 °C for 7 hours. The mixture was purified directly by column chromatography on silica gel (elution with methylene chloride) to afford 340.0 mg (28%) of the 9-[(3-trifluoromethylphenyl)methyl]-4-hydroxy-5-carbomethyoxy carbazole as a yellow solid. $^1H$ NMR ($CDCl_3$) δ 10.35 (s, 1H), 8.0 (d, 1H, J=8 Hz), 7.6-7.3 (m, 6H), 7.05 (d, 1H, J=8 Hz), 6.85 (m, 2H), 5.6 (s, 2H), and 4.1 (s, 3H). IR ($CHCl_3$, $cm^{-1}$) 3378 and 1712. MS (ES) m/e 398, 400.

| Elemental Analyses for $C_{22}H_{16}NO_3F_3$: | | | |
|---|---|---|---|
| Calculated | C, 66.17; | H, 4.04; | N, 3.51. |
| Found | C, 66.99; | H, 4.12; | N, 3.53; F. |

C. 9-[(3-Trifluoromethylphenyl)methyl]-4-hydroxy-5-carbamoyl carbazole

A solution of the 9-[(3-trifluoromethylphenyl)methyl]-4-hydroxy-5-carbomethoxy carbazole (250 mg, 0.625

mM) in 5 mL THF and 20 mL concentrated aqueous ammonium hydroxide was sonicated for 30 h at 40-50 °C. The mixture was diluted with ethyl acetate and acidified to pH 1 with 5 N HCl. The aqueous layer was extracted three times with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 120 mg (50%) of the 9-[(3-trifluoromethylphenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.8 (d, 1H, J=8 Hz), 7.6-7.5 (m, 5H), 7.3 (t, 1H, J=8 Hz), 7.15 (d, 1H, J=8 Hz), 7.1 (d, 1H, J=8 Hz), 6.6 (d, 1H, J=8 Hz), and 5.8 (s, 2H). IR (KBr, cm$^{-1}$) 3429, 3206, and 1630. MS (ES) m/e 383, 385.

| Elemental Analyses for $C_{21}H_{15}N_2O_2F_3$: | | | |
|---|---|---|---|
| Calculated | C, 65.62; | H, 3.93; | N, 7.29. |
| Found | C, 67.50; | H, 4.00; | N, 7.19. |

D. {9-[(3-Trifluoromethylphenyl)methyl]-5-carbamoylcarbazol-4-yl}oxyacetic acid, methyl ester

40% Methanolic Triton B (0.18 mL, 0.4 mM) was added to a solution of the 9-[(3-trifluoromethylphenyl)methyl]-4-hydroxy-5-carbamoyl carbazole (115 mg, 0.3 mM) in 5 mL DMF at room temperature. After 15 minutes, methyl bromoacetate (95 mg, 0.6 mM) was added and the resultant mixture stirred at room temperature for 22 hours. The mixture was diluted with ethyl acetate, washed four times with $H_2O$, 1 N HCl, $H_2O$, sat. $NaHCO_3$, and saturated brine, dried over magnesium sulfate, filtered, and concentrated. The residue was purified by column chromatography on silica gel (elution with ethyl acetate) to afford 120 mg (88%) of the {9-[(3-trifluoromethylphenyl)methyl]-5-carbamoylcarbazol-4-yl}oxyacetic acid, methyl ester as a white solid. $^1$H NMR ($CDCl_3$) δ 7.5-7.2 (m, 7H), 7.1 (d, 1H, J=8 Hz), 7.0 (d, 1H, J=8 Hz), 6.6 (d, 1H, J=8 Hz), 6.4 (br s, 1H), 6.0 (br s, 1H), 5.55 (s, 2H), 4.9 (s, 2H), and 3.9 (s, 3H). IR (KBr, cm$^{-1}$) 1763 and 1673. MS (ES) m/e 457.

| Elemental Analyses for $C_{24}H_{19}N_2O_4F_3$: | | | |
|---|---|---|---|
| Calculated | C, 63.16; | H, 4.20; | N, 6.14. |
| Found | C, 61.37; | H, 4.19; | N, 5.77. |

E. {9-[(3-Trifluoromethylphenyl)methyl]-5-carbamoylcarbazol-4-yl}oxyacetic acid, sodium salt.

A solution of the {9-[(3-trifluoromethylphenyl)methyl]-5-carbamoylcarbazol-4-yl}oxyacetic acid, methyl ester (91 mg, 0.153 mM) and 0.22 mL (0.22 mM) of 1 N NaOH in 8 mL of ethanol was stirred for 17 h at 25 °C. The ethanol was removed *in vacuo*. The resultant white precipitate was collected by filtration, washed with small amounts of EtOH and diethyl ether, then dried *in vacuo* to afford 75 mg (81%) of the {9-[(3-trifluoromethylphenyl)methyl]-5-carbamoylcarbazol-4-yl}oxyacetic acid, sodium salt as a white powder. $^1$H NMR (DMSO-d6) δ 7.65 (s, 1H), 7.6 (m, 4H), 7.45 (t, 1H, J=8 Hz), 7.35 (t, 1H, J=8 Hz), 7.3 (t, 1H, J=8 Hz), 7.2 (d, 1H, J=8 Hz), 7.1 (d, 1H, J=8 Hz), 7.05 (d, 1H, J=8 Hz), 6.5 (d, 1H, J=8 Hz), 5.75 (s, 2H), and 4.3 (s, 2H). IR (KBr, cm$^{-1}$) 1665 and 1618. MS (ES) m/e 441, 443.

| Elemental Analyses for $C_{23}H_{16}N_2O_4F_3Na$: | | | |
|---|---|---|---|
| Calculated | C, 59.49; | H, 3.47; | N, 6.03. |
| Found | C, 60.69; | H, 3.78; | N, 5.75. |

EXAMPLE 32

Preparation of {9-[(1-naphthyl)methyl]-5-carbamoylcarbazol-4-yl}oxyacetic acid, sodium salt

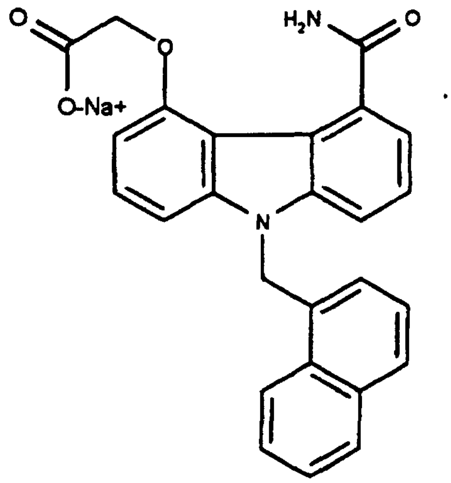

A. 9-[(1-Naphthyl)methyl]-5-carbomethoxy-1,2-dihydrocarbazol-4(3H)-one

40% Methanolic Triton B (1.6 mL, 3.6 mM) was slowly added dropwise to a solution of 5-carbomethoxy-1,2-dihydro-9H-carbazol-4(3H)-one (870 mg, 3.6 mM) in 30 mL of DMF at 25 °C. After 5 minutes, 1-chloromethyl naphthylene (642 mg, 3.6 mM) and sodium iodide (450 mg, 3.0 mM) were added and the resultant mixture stirred at room temperature for 25 hours. The mixture was diluted with ethyl acetate, washed five times with $H_2O$, 1 N HCl, $H_2O$, sat $NaHCO_3$, and saturated brine, dried over anhydrous magnesium sulfate, filtered, concentrated, and dried *in vacuo*. The residue was purified by column chromatography on silica gel (elution with ethyl acetate) to afford 560 mg (41%) of the 9-[(1-naphthyl)methyl]-5-carbomethoxy-1,2-dihydrocarbazol-4(3H)-one as a yellow solid. $^1$H NMR ($CDCl_3$) δ 8.0 (d, 1H, J=8 Hz), 7.9 (d, 1H, J=8 Hz), 7.7 (d, 1H, J=8 Hz), 7.6 (t, 1H, J=8 Hz), 7.55 (t, 1H, J=8 Hz), 7.35 (d, 1H, J=8 Hz), 7.15-7.05 (m, 3H), 6.4 (d, 1H, J=8 Hz), 5.8 (s, 2H), 4.05 (s, 3H), 2.8 (t, 2H, J=6 Hz), 2.55 (t, 2H, J=6 Hz), and 2.2 (m , 2H). IR (KBr, $cm^{-1}$) 1721, 1646, 1464, 1448, 1438, 1285, 1122, 796, and 761. MS (ES) m/e 382, 384.

| Elemental Analyses for $C_{25}H_{21}NO_3$: | | | |
|---|---|---|---|
| Calculated | C, 78.33; | H, 5.48; | N, 3.66 |
| Found | C, 76.28; | H, 5.46; | N, 3.93. |

B. 9-[(1-Naphthyl)methyl]-4-hydroxy-5-carbomethoxy carbazole

A solution of the 9-[(1-naphthyl)methyl]-5-carbomethoxy-1,2-dihydrocarbazol-4(3H)-one (540 mg, 1.4 mM) and 2,3-dichloro-5,6-dicyano-1,4-benzoquinone (304 mg, 1.33 mM) in 30 mL of toluene was stirred between 80-90 °C for 5 hours. The mixture was purified directly by column chromatography on silica gel (elution with ethyl acetate) to afford 240.0 mg (45%) of the 9-[(1-naphthyl)methyl]-4-hydroxy-5-carbomethyoxy carbazole as a yellow solid. $^1$H NMR (DMSO-d6) δ 10.25 (s, 1H), 8.35 (d, 1H, J=8 Hz), 8.0 (d, 1H, J=8 Hz), 7.8 (d, 1H, J=8 Hz), 7.6-7.1 (m, 7H), 6.9 (d, 1H, J=8 Hz), 6.6 (d, 1H, J=8 Hz), 6.3 (d, 1H, J=8 Hz), 6.15 (s, 2H), and 3.8 (s, 3H). IR ($CHCl_3$, $cm^{-1}$) 1685, 1598, 1442, 1269, and 1140. MS (ES) m/e 380, 382.

| Elemental Analyses for $C_{25}H_{19}NO_3$: | | | |
|---|---|---|---|
| Calculated | C, 78.74; | H, 4.99; | N, 3.67. |
| Found | C, 78.67; | H, 5.14; | N, 3.54. |

C. 9-[(1-Naphthyl)methyl]-4-hydroxy-5-carbamoyl carbazole

A solution of the 9-[(1-naphthyl)methyl]-4-hydroxy-5-carbomethoxy carbazole (210 mg, 0.55 mM) in 10 mL THF and 30 mL concentrated aqueous ammonium hydroxide was sonicated for 20 hours at 40-50 °C. The mixture was diluted with ethyl acetate and acidified to pH 1 with 5 N HCl. The aqueous layer was extracted three times with ethyl acetate. The combined organic extracts were washed with saturated brine, dried over magnesium sulfate, filtered, and concentrated. The residue was purified by column chromatography on silica gel (elution with gradient hexanes/ethyl acetate) to afford 80 mg (40%) of the 9-[(1-naphthyl)methyl]-4-hydroxy-5-carbamoyl carbazole as a white solid. $^1$H NMR (DMSO-d6) δ 10.55 (s, 1H), 8.8 (br s, 1H), 8.4 (br s, 1H), 8.35 (d, 1H, J=8 Hz), 7.95 (d, 1H, J=8 Hz), 7.8 (d, 1H, J=8 Hz), 7.65 (m, 4H), 7.45 (m, 2H), 7.25 (t, 1H, J=8 Hz), 7.15 (t, 1H, J=8 Hz), 6.9 (d, 1H, J=8 Hz), 6.6 (d, 1H, J=8 Hz), and 6.2 (s, 2H). MS (ES) m/e 365, 367.

D. {9-[(1-Naphthyl)methyl]-5-carbamoylcarbazol-4-yl}oxyacetic acid, methyl ester

40% Methanolic Triton B (0.2 mL, 0.26 mM) was added to a solution of the 9-[(1-naphthyl)methyl]-4-hydroxy-5-carbamoyl carbazole (80 mg, 0.22 mM) in 7 mL DMF at room temperature. After 15 minutes, methyl bromoacetate (40 mg, 0.3 mM) was added and the resultant mixture stirred at room temperature for 3 hours. The mixture was diluted with ethyl acetate, washed twice with $H_2O$, and once with saturated brine, dried over magnesium sulfate, filtered, and concentrated. The residue was purified by column chromatography on silica gel (elution with ethyl acetate) to afford 81 mg (85%) of the {9-[(1-naphthyl)methyl]-5-carbamoylcarbazol-4-yl}oxyacetic acid, methyl ester as a white solid. $^1$H NMR ($CDCl_3$) δ 8.2 (d, 1H, J=8 Hz), 8.05 (d, 1H, J=8 Hz), 7.85-7.0 (m, 11H), 6.65 (d, 1H, J=8 Hz), 6.3 (d, 1H, J=8 Hz), 6.2 (s, 2H), 4.95 (s, 2H), and 3.8 (s, 3H). IR (KBr, $cm^{-1}$) 3364, 1739, 1630, 1582, 1500, 1455, 1285, 1232, 1153, and 774. MS (FD) m/e 438.

| Elemental Analyses for $C_{27}H_{22}N_2O_4$: | | | |
|---|---|---|---|
| Calculated | C, 73.97; | H, 5.02; | N, 6.39. |
| Found | C, 71.66; | H, 5.14; | N, 5.96. |

E. {9-[(1-Naphthyl)methyl]-5-carbamoylcarbazol-4-yl}oxyacetic acid, sodium salt.

A solution of the {9-[(1-naphthyl)methyl]-5-carbamoylcarbazol-4-yl}oxyacetic acid, methyl ester (21 mg, 0.048 mM) and 0.05 mL (0.05 mM) of 1 N NaOH in 5 mL of ethanol was stirred for 20 hours at 25 °C. The resultant white precipitate was collected by filtration, washed with a small amount of EtOH, then dried *in vacuo* to afford 17 mg (80%) of the {9-[(1-naphthyl)methyl]-5-carbamoylcarbazol-4-yl}oxyacetic acid, sodium salt as a white powder. $^1$H NMR (DMSO-d6) δ 8.4 (d, 1H, J=8 Hz), 8.05 (d, 1H, J=8 Hz), 7.8 (d, 1H, J=8 Hz), 7.75-7.2 (m, 8H), 7.1 (d, 1H, J=8 Hz), 6.95 (d, 1H, J=8 Hz), 6.55 (d, 1H, J=8 Hz), 6.3 (d, 1H, J=8 Hz), 6.15 (s, 2H), and 4.4 (s, 2H). IR (KBr, $cm^{-1}$) 1664, 1615, 1595, 1455, 1408, 1324, 1275, and 775. MS (ES) m/e 423, 425.

| Elemental Analyses for $C_{26}H_{19}N_2O_4Na$: | | | |
|---|---|---|---|
| Calculated | C, 69.96; | H, 4.26; | N, 6.28. |
| Found | C, 67.91; | H, 4.24; | N, 5.76. |

EXAMPLE 33

Preparation of {9-[(2-cyanophenyl)methyl]-5-carbamoylcarbazol-4-yl}oxyacetic acid, sodium salt

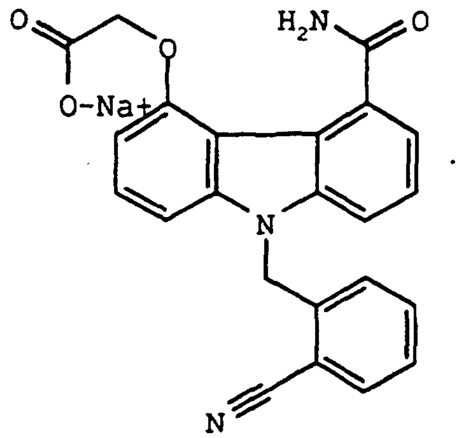

A. 9-[(2-Cyanophenyl)methyl]-5-carbomethoxy-1,2-dihydrocarbazol-4(3H)-one

40% Methanolic Triton B (2.18 mL, 4.8 mM) was slowly added dropwise to a solution of 5-carbomethoxy-1,2-dihydro-9H-carbazol-4(3H)-one (973 mg, 4.0 mM) in 10 mL of DMF at 25 °C. After 10 minutes, a-bromo-*o*-tolunitrile (1.0 g, 5.0 mM) was added and the resultant mixture stirred at room temperature for 30 hours. The mixture was diluted with ethyl acetate, washed five times with $H_2O$, 1 N HCl, $H_2O$, sat $NaHCO_3$, $H_2O$, and saturated brine, dried over anhydrous magnesium sulfate, filtered, concentrated. The residue was triturated with diethyl ether and methylene chloride, then dried *in vacuo* to afford 1.31 g (91%) of the 9-[(2-cyanophenyl)methyl]-5-carbomethoxy-1,2-dihydrocarbazol-4(3H)-one as a tan solid. $^1H$ NMR ($CDCl_3$) δ 7.75 (dd, 1H, J=1 and 8 Hz), 7.5-7.2 (m, 5H), 6.6 (d, 1H, J=8 Hz), 5.55 (s, 2H), 4.05 (s, 3H), 2.85 (t, 2H, J=6 Hz), 2.6 (t, 2H, J=6 Hz), and 2.25 (m , 2H). IR (KBr, $cm^{-1}$) 2222, 1711, and 1650. MS (ES) m/e 357, 359.

| Elemental Analyses for $C_{22}H_{18}N_2O_3$: | | | |
|---|---|---|---|
| Calculated | C, 73.73; | H, 5.06; | N, 7.82. |
| Found | C, 73.62; | H, 5.34; | N, 7.59. |

B. 9-[(2-Cyanophenyl)methyl]-4-hydroxy-5-carbomethoxy carbazole

A solution of the 9-[(2-cyanophenyl)methyl]-5-carbomethoxy-1,2-dihydrocarbazol-4(3H)-one (1.27 g, 3.5 mM) and 2,3-dichloro-5,6-dicyano-1,4-benzoquinone (892 mg, 3.85 mM) in 25 mL of toluene was stirred at reflux for 7 hours. The mixture was purified directly by column chromatography on silica gel (elution with methylene chloride) to afford 305 mg (24%) of the 9-[(2-cyanophenyl)methyl]-4-hydroxy-5-carbomethyoxy carbazole as a yellow solid. $^1H$ NMR ($CDCl_3$) δ 10.35 (s, 1H), 8.0 (d, 1H, J=8 Hz), 7.75 (d, 1H, J=8 Hz), 7.5-7.2 (m, 5H), 6.85 (m, 2H), 6.6 (d, 1H, J=8 Hz), 5.75 (s, 2H), and 4.1 (s, 3H). IR ($CHCl_3$, $cm^{-1}$) 3025, 2223, and 1686. MS (ES) m/e 355, 357.

| Elemental Analyses for $C_{22}H_{16}N_2O_3$: | | | |
|---|---|---|---|
| Calculated | C, 74.15; | H, 4.53; | N, 7.86. |
| Found | C, 72.99; | H, 4.41; | N, 7.65. |

C. 9-[(2-Cyanophenyl)methyl]-4-hydroxy-5-carbamoyl carbazole

A solution of the 9-[(2-cyanophenyl)methyl]-4-hydroxy-5-carbomethoxy carbazole (295 mg, 0.83 mM) in 5 mL THF and 20 mL concentrated aqueous ammonium hydroxide was sonicated for 22 hours at 40-50 °C. The mixture was diluted with ethyl acetate and acidified to pH 1 with 5 N HCl. The aqueous layer was extracted three times with ethyl

acetate. The combined organic extracts were washed with $H_2O$ and saturated brine, dried over magnesium sulfate, filtered, and concentrated. The residue was purified by column chromatography on silica gel (elution with gradient hexanes/ethyl acetate) to afford 140 mg (49%) of the 9-[(2-cyanophenyl)methyl]-4-hydroxy-5-carbamoyl carbazole as a tan solid. $^1H$ NMR (DMSO-d6) δ 10.5 (s, 1H), 8.8 (br s, 1H), 8.4 (br s, 1H), 7.9 (d, 1H, J=8 Hz), 7.75 (d, 1H, J=8 Hz), 7.5-7.4 (m, 4H), 7.25 (t, 1H, J=8 Hz), 7.0 (d, 1H, J=8 Hz), 6.6 (d, 1H, J=8 Hz), 6.4 (m, 1H), and 5.85 (s, 2H). IR (KBr, $cm^{-1}$) 3448, 3356, 2225, 1628, and 1600. MS .(ES) m/e 340, 342.

| Elemental Analyses for $C_{21}H_{15}N_3O_2$: | | | |
|---|---|---|---|
| Calculated | C, 73.89; | H, 4.43; | N, 12.31. |
| Found | C, 73.39; | H, 4.56; | N, 13.32. |

D. {9-[(2-Cyanophenyl)methyl]-5-carbamoylcarbazol-4-yl}oxyacetic acid, methyl ester

40% Methanolic Triton B (0.24 mL, 0.53 mM) was added to a solution of the 9-[(2-cyanophenyl)methyl]-4-hydroxy-5-carbamoyl carbazole (140 mg, 0.41 mM) in 5 mL DMF at room temperature. After 15 minutes, methyl bromoacetate (130 mg, 0.82 mM) was added and the resultant mixture stirred at room temperature for 24 hours. The mixture was diluted with ethyl acetate, washed four times with $H_2O$, 1 N HCl, $H_2O$, sat. $NaHCO_3$, and saturated brine, dried over magnesium sulfate, filtered, and concentrated. The residue was purified by column chromatography on silica gel (elution with gradient methylene chloride/ethyl acetate/THF) to afford 116 mg (68%) of the {9-[(2-cyanophenyl)methyl]-5-carbamoylcarbazol-4-yl}oxyacetic acid, methyl ester as a white solid. $^1H$ NMR ($CDCl_3$) δ 7.75 (d, 1H, J=8 Hz), 7.5-7.2 (m, 6H), 6.95 (d, 1H, J=8 Hz), 6.6 (d, 2H, J=8 Hz),), 6.3 (br s, 1H), 6.1 (br s, 1H), 5.75 (s, 2H), 4.9 (s, 2H), and 3.8 (s, 3H). IR (KBr, $cm^{-1}$) 2228, 1732, and 1675. MS (ES) m/e 412, 414.

| Elemental Analyses for $C_{24}H_{19}N_3O_4$: | | | |
|---|---|---|---|
| Calculated | C, 69.72; | H, 4.63; | N, 10.16. |
| Found | C, 70.00; | H, 4.69; | N, 10.32. |

E. {9-[(2-Cyanophenyl)methyl]-5-carbamoylcarbazol-4-yl}oxyacetic acid, sodium salt

A suspension of the {9-[(2-cyanophenyl)methyl]-5-carbamoylcarbazol-4-yl}oxyacetic acid, methyl ester (110 mg, 0.266 mM) and 0.29 mL (0.29 mM) of 1 N NaOH in 5 mL of ethanol was sonicated for 2 hours at 25 °C. The resultant white precipitate was collected by filtration, washed with small amounts of EtOH, diethyl ether, and hexanes, then dried *in vacuo* to afford 107 mg (95%) of the {9-[(2-cyanophenyl)methyl]-5-carbamoylcarbazol-4-yl}oxyacetic acid, sodium salt as a white powder. $^1H$ NMR (DMSO-d6) δ 7.9 (d, 1H, J=8 Hz), 7.6 ( br s, 1H), 7.5 (d, 1H, J=8 Hz), 7.45-7.4 (m, 3H), 7.35 (t, 1H, J=8 Hz), 7.25 (t, 1H, J=8 Hz), 7.1 (d, 1H, J=8 Hz), 7.05 (d, 1H, J=8 Hz), 6.55 (d, 1H, J=8 Hz), 6.4 (d, 1H, J=8 Hz), 5.8 (s, 2H), and 4.3 (s, 2H). IR (KBr, $cm^{-1}$) 2220, 1652, and 1613. MS (ES) m/e 398, 400.

| Elemental Analyses for $C_{23}H_{16}N_3O_4Na$: | | | |
|---|---|---|---|
| Calculated | C, 65.56; | H, 3.83; | N, 9.97. |
| Found | C, 65.61; | H, 3.71; | N, 9.89. |

EXAMPLE 34

Preparation of {9-[(3-cyanophenyl)methyl]-5-carbamoylcarbazol-4-yl}oxyacetic acid

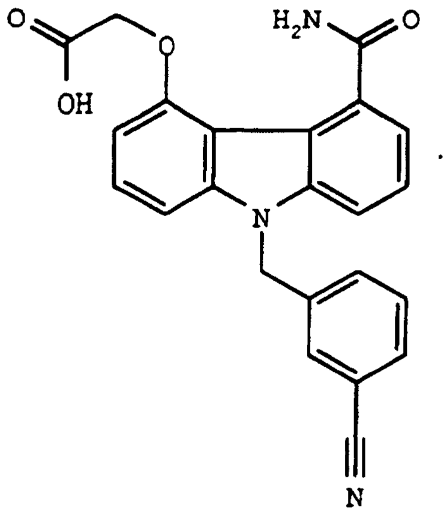

A. 9-[(3-Cyanophenyl)methyl]-5-carbomethoxy-1,2-dihydrocarbazol-4(3H)-one

A suspension of 5-carbomethoxy-1,2-dihydro-9H-carbazol-4(3H)-one (973 mg, 4.0 mM), a-bromo-*m*-tolunitrile (1.0 g, 4.9 mM), and potassium carbonate (553 mg, 4.0 mM) in 10 mL of DMF was stirred at 25 °C for 24 hours. The mixture was diluted with ethyl acetate, washed five times with $H_2O$, 1 N HCl, $H_2O$, sat $NaHCO_3$, $H_2O$, and saturated brine, dried over anhydrous magnesium sulfate, filtered, concentrated. The residue was dried *in vacuo* to afford 1.18 g (82%) of the 9-[(3-cyanophenyl)methyl]-5-carbomethoxy -1,2-dihydrocarbazol-4(3H)-one as a tan solid. $^1H$ NMR ($CDCl_3$) δ 7.65-7.2 (m, 6H), 7.15 (d, 1H, J=8 Hz), 5.4 (s, 2H), 4.05 (s, 3H), 2.85 (t, 2H, J=6 Hz), 2.6 (t, 2H, J=6 Hz), and 2.25 (m, 2H). IR (KBr, $cm^{-1}$) 2226, 1729, and 1646. MS (ES) m/e 357, 359.

| Elemental Analyses for $C_{22}H_{18}N_2O_3$: | | | |
|---|---|---|---|
| Calculated | C, 73.73; | H, 5.06; | N, 7.82. |
| Found | C, 70.18; | H, 4.97; | N, 7.07. |

B. 9-[(3-Cyanophenyl)methyl]-4-hydroxy-5-carbomethoxy carbazole

A solution of the 9-[(3-cyanophenyl)methyl]-5-carbomethoxy-1,2-dihydrocarbazol-4(3H)-one (1.15 g, 3.2 mM) and 2,3-dichloro-5,6-dicyano-1,4-benzoquinone (815 mg, 3.52 mM) in 25 mL of toluene was stirred at reflux for 2 hours. The mixture was purified directly by column chromatography on silica gel (elution with methylene chloride) to afford 120 mg (10%) of the 9-[(3-cyanophenyl)methyl]-4-hydroxy-5-carbomethyoxy carbazole as a yellow solid. $^1H$ NMR ($CDCl_3$) δ 10.35 (s, 1H), 8.0 (d, 1H, J=8 Hz), 7.6-7.2 (m, 7H), 6.85 (m, 2H), 5.55 (s, 2H), and 4.1 (s, 3H). IR (KBr, $cm^{-1}$) 3063, 3025, 2234, and 1685. MS (ES) m/e 355, 357.

| Elemental Analyses for $C_{22}H_{16}N_2O_3$: | | | |
|---|---|---|---|
| Calculated | C, 74.15; | H, 4.53; | N, 7.86. |
| Found | C, 73.36; | H, 4.51; | N, 8.06. |

C. 9-[(3-Cyanophenyl)methyl]-9-hydroxy-5-carbamoyl carbazole

A solution of the 9-[(3-cyanophenyl)methyl]-4-hydroxy-5-carbomethoxy carbazole (114 mg, 0.32 mM) in 5 mL THF and 20 mL concentrated aqueous ammonium hydroxide was sonicated for 7 hours at 40-50 °C. The mixture was

diluted with ethyl acetate and acidified to pH 1 with 5 N HCl. The aqueous layer was extracted three times with ethyl acetate. The combined organic extracts were washed with $H_2O$ and saturated brine, dried over magnesium sulfate, filtered, and concentrated. The residue was purified by column chromatography on silica gel (elution with gradient hexanes/ethyl acetate) to afford 40 mg (49%) of the 9-[(3-cyanophenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.8 (d, 1H, J=8 Hz), 7.7 (d, 1H, J=8 Hz), 7.6 (s, 1H), 7.5-7.4 (m, 3H), 7.3 (t, 1H, J=8 Hz), 7.25 (d, 1H, J=8 Hz), 7.1 (d, 1H, J=8 Hz), 6.6 (d, 1H, J=8 Hz), and 5.75 (s, 2H). IR (KBr, $cm^{-1}$) 3430, 3347, 2231, 1628, and 1601. MS (ES) m/e 340, 342.

| Elemental Analyses for $C_{21}H_{15}N_3O_2$: | | | |
|---|---|---|---|
| Calculated | C, 73.89; | H, 4.43; | N, 12.31. |
| Found | C, 75.20; | H, 4.80; | N, 12.15. |

D. {9-[(3-Cyanophenyl)methyl]-5-carbamoylcarbazol-4-yl}oxyacetic acid, tert-butyl ester

40% Methanolic Triton B (0.06 mL, 0.13 mM) was added to a solution of the 9-[(3-cyanophenyl)methyl]-4-hydroxy-5-carbamoyl carbazole (34.1 mg, 0.1 mM) in 5 mL DMF at room temperature. After 1 minute, tert-butyl bromoacetate (40 mg, 0.2 mM) was added and the resultant mixture stirred at room temperature for 24 h. The mixture was diluted with ethyl acetate, washed four times with $H_2O$, 1 N HCl, $H_2O$, sat. $NaHCO_3$, and saturated brine, dried over magnesium sulfate, filtered, and concentrated. The residue was triturated with hexane to afford 51 mg (100%) of the {9-[(3-cyanophenyl)methyl]-5-carbamoylcarbazol-4-yl}oxyacetic acid, tert-butyl ester as a white solid. $^1$H NMR ($CDCl_3$) δ 7.55 (d, 1H, J=8 Hz), 7.5-7.2 (m, 7H), 6.95 (d, 1H, J=8 Hz), 6.6 (d, 1H, J=8 Hz), 6.3 (br s, 1H), 6.1 (br s, 1H), 5.5 (s, 2H), 4.8 (s, 2H), and 1.5 (s, 9H). IR (KBr, $cm^{-1}$) 2228, 1748, and 1669. MS (ES) m/e 455, 456.

| Elemental Analyses for $C_{27}H_{25}N_3O_4$: | | | |
|---|---|---|---|
| Calculated | C, 71.19; | H, 5.53; | N, 9.22. |
| Found | C, 70.24; | H, 5.68; | N, 8.96. |

E. {9-[(3-Cyanophenyl)methyl]-5-carbamoylcarbazol-4-yl}oxyacetic acid

A solution of the {9-[(3-cyanophenyl)methyl]-5-carbamoylcarbazol-4-yl}oxyacetic acid, tert-butyl ester (45 mg, 0.1 mM) ) in 3 mL of trifluoroacetic acid was stirred at room temperature for 2 hours. The solvent was removed *in vacuo*. The residue was triturated with ethyl ether-hexanes, then dried *in vacuo* to afford 41 mg (100%) of the {9-[(2-cyanophenyl)methyl]-5-carbamoylcarbazol-4-yl}oxyacetic acid as a tan powder. $^1$H NMR (DMSO-d6) δ 13.0 (br s, 1H), 7.6-7.3 (m, 10H), 7.1 (d, 1H, J=8 Hz), 6.65 (d, 1H, J=8 Hz), 5.8 (s, 2H), and 4.8 (s, 2H). IR (KBr, $cm^{-1}$) 2226, 1733, and 1640. MS (ES) m/e 398, 400.

| Elemental Analyses for $C_{23}H_{17}N_3O_4$: | | | |
|---|---|---|---|
| Calculated | C, 69.17; | H, 4.29; | N, 10.52. |
| Found | C, 66.96; | H, 4.37; | N, 10.03. |

EXAMPLE 35

Preparation of {9-[(2-methylphenyl)methyl]-5-carbamoylcarbazol-4-yl}oxyacetic acid, sodium salt

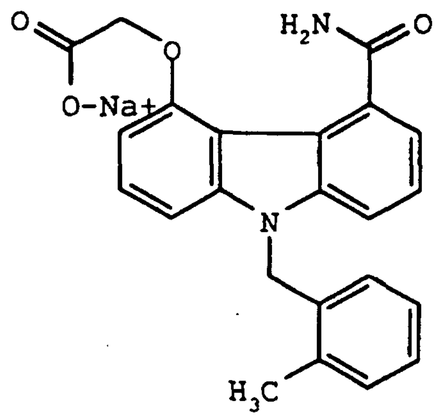

A. 9-[(2-Methylphenyl)methyl]-5-carbomethoxy-1,2-dihydrocarbazol-4(3H)-one

A suspension of 5-carbomethoxy-1,2-dihydro-9H-carbazol-4(3H)-one (870 mg, 3.58 mM), a-bromo-*o*-xylene (662 mg, 3.58 mM), and potassium carbonate (500 mg, 3.61 mM) in 20 mL DMF was stirred at room temperature for 20 hours. The mixture was diluted with ethyl acetate, washed with $H_2O$ and saturated brine, dried over anhydrous magnesium sulfate, filtered, concentrated to afford 1.21 g (98%) of the 9-[(2-methylphenyl)methyl]-5-carbomethoxy-1,2-dihydrocarbazol-4(3H)-one as a dark oil. $^1$H NMR (DMSO-d6) δ 7.5-7.2 (m, 4H), 7.15 (t, 1H, J=8 Hz), 7.0 (t, 1H, J=8 Hz), 6.15 (d, 1H, J=8 Hz), 5.55 (s, 2H), 3.85 (s, 3H), 2.6 (m, 2H), 2.4 (m, 2H), 2.4 (s, 3H), and 2.1 (m, 2H). IR ($CHCl_3$, $cm^{-1}$) 3010, 2952, 1724, 1671, 1653, 1604, 1460, 1444, 1290, 1174, and 1122. MS (ES) m/e 348.5.

| Elemental Analyses for $C_{22}H_{21}NO_3$: | | | |
|---|---|---|---|
| Calculated | C, 76.08; | H, 6.05; | N, 4.03. |
| Found | C, 73.33; | H, 6.36; | N, 4.30. |

B. 9-[(2-Methylphenyl)methyl]-4-hydroxy-5-carbomethoxy carbazole

A solution of the 9-[(2-methylphenyl)methyl]-5-carbomethoxy-1,2-dihydrocarbazol-4(3H)-one (1.2 g, 3.5 mM) and 2,3-dichloro-5,6-dicyano-1,4-benzoquinone (800 mg, 3.6 mM) in 70 mL of toluene was stirred at 80-90 °C for 5 hours. The mixture was purified directly by column chromatography on silica gel (elution with methylene chloride) to afford 260 mg (22%) of the 9-[(2-methylphenyl)methyl]-4-hydroxy-5-carbomethyoxy carbazole as a yellow solid. $^1$H NMR (DMSO-d6) δ 10.25 (s, 1H), 7.5 (d, 1H, J=8 Hz), 7.4 (t, 1H, J=8 Hz), 7.3-7.1 (m, 4H), 6.9 (m, 2H), 6.6 (d, 1H, J=8 Hz), 6.1 (d, 1H, J=8 Hz), 5.65 (s, 2H), 3.8 (s, 3H), and 2.5 (s, 3H). IR (KBr, $cm^{-1}$) 3200, 1672, 1440, 1426, 1332, 1302, 1265, 1216, 1141, 761, 749, and 718. MS (ES) m/e 344, 346.

| Elemental Analyses for $C_{22}H_{19}NO_3$: | | | |
|---|---|---|---|
| Calculated | C, 76.52; | H, 5.51; | N, 4.06. |
| Found | C, 76.44; | H, 5.66; | N, 3.94. |

C. 9-[(2-Methylphenyl)methyl]-4-hydroxy-5-carbamoyl carbazole

A solution of the 9-[(2-methylphenyl)methyl]-4-hydroxy-5-carbomethoxy carbazole (260 mg, 0.75 mM) in 10 mL THF and 30 mL concentrated aqueous ammonium hydroxide was sonicated for 5 hours at 40-50 °C. The mixture was diluted with ethyl acetate and acidified to pH 1 with 5 N HCl. The aqueous layer was extracted three times with ethyl acetate. The combined organic extracts were washed with $H_2O$ and saturated brine, dried over magnesium sulfate,

filtered, and concentrated. The residue was purified by column chromatography on silica gel (elution with gradient hexanes/ethyl acetate) to afford 90 mg (36%) of the 9-[(2-methylphenyl)methyl]-4-hydroxy-5-carbamoyl carbazole as a tan solid. $^1$H NMR (DMSO-d6) δ 10.5 (s, 1H), 8.8 (br s, 1H), 8.4 (br s, 1H), 7.7 (m, 1H), 7.5 (m, 2H), 7.3 (m, 2H), 7.1 (t, 1H, J=8 Hz), 6.95 (d, 1H, J=8 Hz), 6.85 (t, 1H, J=8 Hz), 6.6 (d, 1H, J=8 Hz), 5.95 (d, 1H, J=8 Hz), 5.7 (s, 2H), and 2.5 (s, 3H). IR (KBr, $cm^{-1}$) 3451, 3191, 1627, 1600, 1584, 1562, 1435, 1329, 1322, 1263, and 774. MS (ES) m/e 329, 331.

| Elemental Analyses for $C_{21}H_{18}N_2O_2$: | | | |
|---|---|---|---|
| Calculated | C, 76.36; | H, 5.45; | N, 8.48. |
| Found | C, 75.66; | H, 5.79; | N, 8.07. |

D. {9-[(2-Methylphenyl)methyl]-5-carbamoylcarbazol-4-yl}oxyacetic acid, methyl ester

40% Methanolic Triton B (0.45 mL, 0.99 mM) was added to a solution of the 9-[(2-methylphenyl)methyl]-4-hydroxy-5-carbamoyl carbazole (80 mg, 0.24 mM) in 8 mL DMF at room temperature. After 3 minutes, methyl bromoacetate (115 mg, 0.72 mM) was added and the resultant mixture stirred at room temperature for 48 hours. The mixture was diluted with ethyl acetate, washed with $H_2O$, 1 N HCl, $H_2O$, and saturated brine, dried over magnesium sulfate, filtered, and concentrated. The residue was purified by column chromatography on silica gel (elution with ethyl acetate) to afford 80 mg (82%) of the {9-[(2-methylphenyl)methyl]-5-carbamoylcarbazol-4-yl}oxyacetic acid, methyl ester as a white solid. $^1$H NMR (DMSO-d6) δ 7.56 (br s, 1H), 7.5-7.1 (m, 9H), 6.9 (t, 1H, J=8 Hz), 6.6 (d, 1H, J=8 Hz), 5.65 (s, 2H), 4.9 (s, 2H), 3.8 (s, 3H), and 2.5 (s, 3H). IR (KBr, $cm^{-1}$) 3367, 3153, 1760, 1740, 1672, 1644, 1619, 1591, 1578, 1498, 1456, 1425, 1327, 1200, 1153, 1109, 1100, and 777. MS (FD) m/e 402.

| Elemental Analyses for $C_{24}H_{22}N_2O_4$: | | | |
|---|---|---|---|
| Calculated | C, 71.64; | H, 5.47; | N, 6.96. |
| Found | C, 71.51; | H, 5.56; | N, 6.67. |

E. {9-[(2-Methylphenyl)methyl]-5-carbamoylcarbazol-4-yl}oxyacetic acid, sodium salt

A suspension of the {9-[(2-methylphenyl)methyl]-5-carbamoylcarbazol-4-yl}oxyacetic acid, methyl ester (15.5 mg, 0.039 mM) and 0.04 mL (0.04 mM) of 1 N NaOH in 5 mL of ethanol was stirred for 24 hours at 25 °C. The resultant white precipitate was collected by filtration, washed with a small amount of EtOH, then dried *in vacuo* to afford 10 mg (63%) of the {9-[(2-methylphenyl)methyl]-5-carbamoylcarbazol-4-yl}oxyacetic acid, sodium salt as a white powder. $^1$H NMR (DMSO-d6) δ 7.55 (br s, 1H), 7.5-7.0 (m, 7H), 6.9 (d, 1H, J=8 Hz), 6.85 (t, 1H, J=8 Hz), 6.6 (d, 1H, J=8 Hz), 6.2 (d, 1H, J=8 Hz), 5.6 (s, 2H), 4.35 (s, 2H), and 2.5 (s, 3H). IR (KBr, $cm^{-1}$) 3390, 1656, 1613, 1595, 1573, 1498, 1455, 1408, 1325, 1332, and 719. MS (ES) m/e 387, 389.

| Elemental Analyses for $C_{23}H_{19}N_2O_4$: | | | |
|---|---|---|---|
| Calculated | C, 67.32; | H, 4.63; | N, 6.83. |
| Found | C, 64.72; | H, 4.44; | N, 6.40. |

EXAMPLE 36

Preparation of {9-[(3-methylphenyl)methyl]-5-carbamoylcarbazol-4-yl}oxyacetic acid, sodium salt

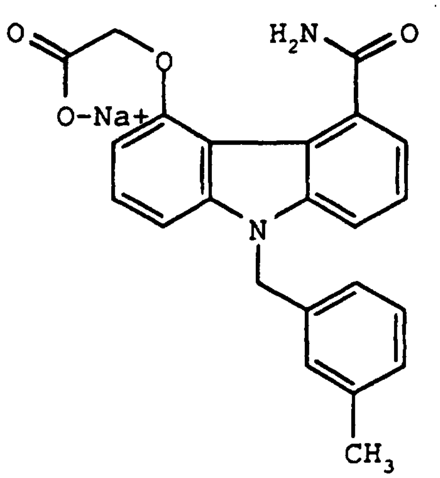

A. 9-[(3-Methylphenyl)methyl]-5-carbomethoxy-1,2-dihydrocarbazol-4(3H)-one

A suspension of 5-carbomethoxy-1,2-dihydro-9H-carbazol-4(3H)-one (870 mg, 3.58 mM), a-bromo-*m*-xylene (662 mg, 3.58 mM), and potassium carbonate (500 mg, 3.61 mM) in 20 mL DMF was stirred at room temperature for 16 hours. The mixture was diluted with ethyl acetate, washed with $H_2O$ and saturated brine, dried over anhydrous magnesium sulfate, filtered, concentrated to afford 1.18 g (95%) of the 9-[(3-methylphenyl)methyl]-5-carbomethoxy-1,2-dihydrocarbazol-4(3H)-one as a dark oil. $^1H$ NMR (DMSO-d6) $\delta$ 7.65 (dd, 1H, J=1 and 8 Hz), 7.3-7.1 (m, 3H), 7.05 (d, 1H, J=8 Hz), 7.0 (s, 1H) , 6.85 (d, 1H, J=8 Hz), 5.5 (s, 2H), 3.8 (s, 3H), 3.0 (m, 2H), 2.45 (m, 2H), 2.3 (s, 3H), and 2.1 (m, 2H). IR ($CHCl_3$, $cm^{-1}$) 3010, 2953, 1724, 1652, 1605, 1465, 1442, 1288, 1174, and 1119. MS (ES) m/e 348.5.

| Elemental Analyses for $C_{22}H_{21}NO_3$: | | | |
|---|---|---|---|
| Calculated | C, 76.08; | H, 6.05; | N, 4.03. |
| Found | C, 74.53; | H, 6.03; | N, 3.68. |

B. 9-[(3-Methylphenyl)methyl]-4-hydroxy-5-carbomethoxy carbazole

A solution of the 9-[(3-methylphenyl)methyl]-5-carbomethoxy-1,2-dihydrocarbazol-4(3H)-one (1.18 g, 3.4 mM) and 2,3-dichloro-5,6-dicyano-1,4-benzoquinone (800 mg, 3.6 mM) in 70 mL of toluene was stirred at 80-90 °C for 6 hours. The mixture was purified directly by column chromatography on silica gel (elution with methylene chloride) to afford 300 mg (26%) of the 9-[(3-methylphenyl)methyl]-4-hydroxy-5-carbomethyoxy carbazole as a yellow solid. $^1H$ NMR (DMSO-d6) $\delta$ 10.2 (s, 1H), 7.65 (d, 1H, J=8 Hz), 7.35 (t, 1H, J=8 Hz), 7.25 (t, 1H, J=8 Hz), 7.2-7.0 (m, 4H), 6.9 (m, 2H), 6.6 (d, 1H, J=8 Hz), 5.6 (s, 2H), 3.85 (s, 3H), and 2.2 (s, 3H). IR (KBr, $cm^{-1}$) 3200, 1673, 1596, 1440, 1426, 1394, 1265, 1216, 1152, 750, 711, and 694. MS (ES) m/e 344, 346.

| Elemental Analyses for $C_{22}H_{19}NO_3$: | | | |
|---|---|---|---|
| Calculated | C, 76.52; | H, 5.51; | N, 4.06. |
| Found | C, 76.22; | H, 5.55; | N, 3.97. |

C. 9-[(3-Methylphenyl)methyl]-4-hydroxy-5-carbamoyl carbazole

A solution of the 9-[(3-methylphenyl)methyl]-4-hydroxy-5-carbomethoxy carbazole (300 mg, 0.87 mM) in 10 mL THF and 30 mL concentrated aqueous ammonium hydroxide was sonicated for 5 hours at 40-50 °C. The mixture was diluted with ethyl acetate and acidified to pH 1 with 5 N HCl. The aqueous layer was extracted three times with

ethyl acetate. The combined organic extracts were washed with $H_2O$ and saturated brine, dried over magnesium sulfate, filtered, and concentrated. The residue was purified by column chromatography on silica gel (elution with gradient hexanes/ethyl acetate) to afford 114 mg (40%) of the 9-[(3-methylphenyl)methyl]-4-hydroxy-5-carbamoyl carbazole as an off-white solid. $^1H$ NMR (DMSO-d6) δ 10.5 (s, 1H), 8.8 (br s, 1H), 8.4 (br s, 1H), 7.8 (dd, 1H, J=1 and 8 Hz), 7.4 (m, 2H), 7.3 (t, 1H, J=8 Hz), 7.15-7.0 (m, 3H), 6.85 (d, 1H, J=8 Hz), 6.6 (d, 1H, J=8 Hz), 5.95 (d, 1H, J=8 Hz), 5.65 (s, 2H), and 2.25 (s, 3H). IR (KBr, $cm^{-1}$) 3434, 3203, 1629, 1599, 1579, 1552, 1443, 1330, 1262, 1214, and 776. MS (ES) m/e 329, 331.

| Elemental Analyses for $C_{21}H_{18}N_2O_2$: | | | |
|---|---|---|---|
| Calculated | C, 76.36; | H, 5.45; | N, 8.48. |
| Found | C, 77.56; | H, 5.67; | N, 8.26. |

D. {9-[(3-Methylphenyl)methyl]-5-carbamoylcarbazol-4-yl}oxyacetic acid, methyl ester

40% Methanolic Triton B (0.45 mL, 0.99 mM) was added to a solution of the 9-[(3-methylphenyl)methyl]-4-hydroxy-5-carbamoyl carbazole (100 mg, 0.30 mM) in 8 mL DMF at room temperature. After 3 minutes, methyl bromoacetate (115 mg, 0.72 mM) was added and the resultant mixture stirred at room temperature for 24 hours. The mixture was diluted with ethyl acetate, washed with $H_2O$, and saturated brine, dried over magnesium sulfate, filtered, and concentrated. The residue was purified by column chromatography on silica gel (elution with ethyl acetate) to afford 80 mg (66%) of the {9-[(3-methylphenyl)methyl]-5-carbamoylcarbazol-4-yl}oxyacetic acid, methyl ester as a white solid. $^1H$ NMR (DMSO-d6) δ 7.6 (d, 1H, J=8 Hz), 7.55 (br s, 1H), 7.45-7.0 (m, 8H), 6.9 (d, 1H, J=8 Hz), 6.6 (d, 1H, J=8 Hz), 5.65 (s, 2H), 4.9 (s, 2H), 3.75 (s, 3H), and 2.2 (s, 3H). IR (KBr, $cm^{-1}$) 3367, 3157, 1760, 1642, 1589, 1499, 1455, 1424, 1328, 1216, 1151, 1102, 772, and 714. MS (FD) m/e 402.

| Elemental Analyses for $C_{24}H_{22}N_2O_4$: | | | |
|---|---|---|---|
| Calculated | C, 71.64; | H, 5.47; | N, 6.96. |
| Found | C, 71.01; | H, 5.60; | N, 6.66. |

E. {9-[(3-Methylphenyl)methyl]-5-carbamoylcarbazol-4-yl}oxyacetic acid, sodium salt

A suspension of the {9-[(3-methylphenyl)methyl]-5-carbamoylcarbazol-4-yl}oxyacetic acid, methyl ester (15.8 mg, 0.039 mM) and 0.04 mL (0.04 mM) of 1 N NaOH in 5 mL of ethanol was stirred for 24 h at 25 °C. The resultant white precipitate was collected by filtration, washed with a small amount of EtOH, then dried *in vacuo* to afford 10 mg (62%) of the {9-[(3-methylphenyl)methyl]-5-carbamoylcarbazol-4-yl}oxyacetic acid, sodium salt as a white powder. $^1H$ NMR (DMSO-d6) δ 7.55 (d, 1H, J=8 Hz), 7.5-7.0 (m, 9H), 6.85 (d, 1H, J=8 Hz), 6.55 (d, 1H, J=8 Hz), 5.6 (s, 2H), 4.35 (s, 2H), and 2.2 (s, 3H). IR (KBr, $cm^{-1}$) 3390, 1656, 1613, 1595, 1573, 1498, 1455, 1408, 1325, 1332, and 719. MS (ES) m/e 387, 389.

| Elemental Analyses for $C_{23}H_{19}N_2O_4Na$: | | | |
|---|---|---|---|
| Calculated | C, 67.32; | H, 4.63; | N, 6.83. |
| Found | C, 61.20; | H, 4.64; | N, 6.06. |

EXAMPLE 37

Preparation of {9-[(3,5-dimethylphenyl)methyl]-5-carbamoylcarbazol-4-yl}oxyacetic acid, sodium salt

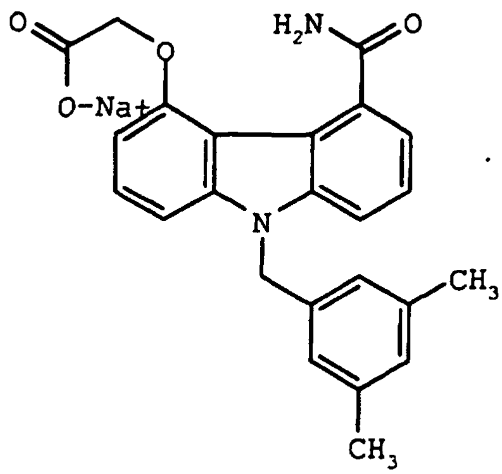

A. 9-[(3,5-Dimethylphenyl)methyl]-5-carbomethoxy-1,2-dihydrocarbazol-4(3H)-one

A suspension of 5-carbomethoxy-1,2-dihydro-9H-carbazol-4(3H)-one (850 mg, 3.5 mM), 3,5-dimethylbenzyl bromide (765 mg, 3.8 mM), and potassium carbonate (500 mg, 3.61 mM) in 25 mL DMF was stirred at room temperature for 19 hours. The mixture was diluted with ethyl acetate, washed with $H_2O$ and saturated brine, dried over anhydrous magnesium sulfate, filtered, concentrated. The residue was purified by column chromatography on silica gel (elution with ethyl acetate) to afford 0.84 g (67%) of the 9-[(3,5-dimethylphenyl)methyl]-5-carbomethoxy-1,2-dihydrocarbazol-4(3H)-one as a foam. $^1H$ NMR (DMSO-d6) δ 7.7 (dd, 1H, J=1 and 8 Hz), 7.3-7.2 (m, 2H), 6.9 (s, 1H), 6.75 (s, 2H), 5.45 (s, 2H), 3.8 (s, 3H), 3.0 (m, 2H), 2.45 (m, 2H), 2.2 (s, 6H), and 2.1 (m, 2H). IR (KBr, $cm^{-1}$) 1726, 1653, 1602, 1465, 1442, 1282, 1172, and 1116. MS (ES) m/e 362.

| Elemental Analyses for $C_{23}H_{23}NO_3$: | | | |
|---|---|---|---|
| Calculated | C, 76.45; | H, 6.37; | N, 3.88. |
| Found | C, 76.82; | H, 6.54; | N, 3.91. |

B. 9-[(3,5-Dimethylphenyl)methyl]-4-hydroxy-5-carbomethoxy carbazole

A solution of the 9-[(3,5-dimethylphenyl)methyl]-5-carbomethoxy-1,2-dihydrocarbazol-9(3H)-one (0.8 g, 2.2 mM) and 2,3-dichloro-5,6-dicyano-1,9-benzoquinone (550 mg, 2.43 mM) in 70 mL of toluene was stirred at 80-90 °C for 5 hours. The mixture was purified directly by column chromatography on silica gel (elution with methylene chloride) to afford 234 mg (29%) of the 9-[(3,5-dimethylphenyl)methyl]-4-hydroxy-5-carbomethyoxy carbazole as a yellow solid. $^1H$ NMR (DMSO-d6) δ 10.2 (s, 1H), 7.65 (d, 1H, J=8 Hz), 7.35 (t, 1H, J=8 Hz), 7.25 (t, 1H, J=8 Hz), 7.15 (d, 1H, J=8 Hz), 7.05 (d, 1H, J=8 Hz), 6.9 (s, 1H), 6.7 (s, 2H), 6.6 (d, 1H, J=8 Hz), 5.6 (s, 2H), 3.85 (s, 3H), and 2.2 (s, 6H). IR (KBr, $cm^{-1}$) 3016, 1675, 1598, 1441, 1426, 1394, 1288, 1270, 1221, 1152, 754, and 713. MS (ES) m/e 358, 360.

| Elemental Analyses for $C_{23}H_{21}NO_3$: | | | |
|---|---|---|---|
| Calculated | C, 76.88; | H, 5.85; | N, 3.90. |
| Found | C, 76.94; | H, 6.00; | N, 3.93. |

C. 9-[(3,5-Dimethylphenyl)methyl]-4-hydroxy-5-carbamoyl carbazole

A solution of the 9-[(3,5-dimethylphenyl)methyl]-4-hydroxy-5-carbomethoxy carbazole (200 mg, 0.55 mM) in

10 mL THF and 30 mL concentrated aqueous ammonium hydroxide was sonicated for 4 days at 40-50 °C. The mixture was diluted with ethyl acetate and acidified to pH 2.5 with 5 N HCl. The aqueous layer was extracted three times with ethyl acetate. The combined organic extracts were washed with saturated brine, dried over magnesium sulfate, filtered, and concentrated. The residue was purified by column chromatography on silica gel (elution with gradient hexanes/ethyl acetate) to afford 90 mg (47%) of the 9-[(3,5-dimethylphenyl)methyl]-4-hydroxy-5-carbamoyl carbazole as an off-white solid. $^1$H NMR (DMSO-d6) δ 10.5 (s, 1H), 8.6 (s, 1H), 8.4 (s, 1H), 7.75 (d, 1H, J=8 Hz), 7.45 (m, 2H), 7.3 (t, 1H, J=8 Hz), 7.1 (d, 1H, J=8 Hz), 6.85 (s, 1H), 6.7 (s, 2H), 6.6 (d, 1H, J=8 Hz), 5.6 (s, 2H), and 2.2 (s, 6H). IR (KBr, $cm^{-1}$) 3417, 3198, 3113, 3063, 1631, 1601, 1562, 1438, 1332, 1263, 1217, 781, and 773. MS (ES) m/e 343, 345.

| Elemental Analyses for $C_{22}H_{20}N_2O_2$: | | | |
|---|---|---|---|
| Calculated | C, 76.74; | H, 5.81; | N, 8.14. |
| Found | C, 76.97; | H, 5.94; | N, 7.95. |

D. {9-[(3,5-Dimethylphenyl)methyl]-5-carbamoylcarbazol-4-yl}oxyacetic acid, methyl ester

40% Methanolic Triton B (0.13 mL, 0.28 mM) was added to a solution of the 9-[(3,5-dimethylphenyl)methyl]-4-hydroxy-5-carbamoyl carbazole (80 mg, 0.23 mM) in 8 mL DMF at room temperature. After 3 minutes, methyl bromoacetate (43 mg, 0.28 mM) was added and the resultant mixture stirred at room temperature for 17 hours. The mixture was diluted with ethyl acetate, washed with $H_2O$, and saturated brine, dried over magnesium sulfate, filtered, and concentrated. The residue was purified by column chromatography on silica gel (elution with ethyl acetate) to afford 70 mg (72%) of the {9-[(3,5-dimethylphenyl)methyl]-5-carbamoylcarbazol-4-yl}oxyacetic acid, methyl ester as a white solid. $^1$H NMR (DMSO-d6) δ 7.6 (d, 1H, J=8 Hz), 7.55 (br s, 1H), 7.45-7.3 (m, 2H), 7.25 (d, 1H, J=8 Hz), 7.2 (br s, 1H), 7.1 (d, 1H, J=8 Hz), 6.9 (s, 1H), 6.8 (s, 2H), 6.6 (d, 1H, J=8 Hz), 5.65 (s, 2H), 4.9 (s, 2H), 3.75 (s, 3H), and 2.2 (s, 6H). IR (KBr, $cm^{-1}$) 3362, 3173, 1758, 1638, 1583, 1500, 1454, 1434, 1330, 1215, 1151, 1106, 772, 715, and 706. MS (FD) m/e 417.

| Elemental Analyses for $C_{25}H_{24}N_2O_4$: | | | |
|---|---|---|---|
| Calculated | C, 72.12; | H, 5.76; | N, 6.73. |
| Found | C, 71.80; | H, 5.60; | N, 6.73. |

E. {9-[(3,5-Dimethylphenyl)methyl]-5-carbamoylcarbazol-4-yl}oxyacetic acid, sodium salt

A suspension of the {9-[(3,5-dimethylphenyl)methyl]-5-carbamoylcarbazol-4-yl}oxyacetic acid, methyl ester (18 mg, 0.043 mM) and 0.043 mL (0.043 mM) of 1 N NaOH in 5 mL of ethanol was stirred for 42 hours at 25 °C. The resultant white precipitate was collected by filtration, washed with a small amount of EtOH, then dried *in vacuo* to afford 12 mg (67%) of the {9-[(3,5-dimethylphenyl)methyl]-5-carbamoylcarbazol-4-yl}oxyacetic acid, sodium salt as a white powder. $^1$H NMR (DMSO-d6) δ 7.6 (d, 1H, J=8 Hz), 7.5-7.3 (m, 4H), 7.1 (m, 2H), 6.9 (s, 1H), 6.8 (s, 2H), 6.6 (d, 1H, J=8 Hz), 5.65 (s, 2H), 4.35 (s, 2H), and 2.2 (s, 6H). IR (KBr, $cm^{-1}$) 3385, 1663, 1616, 1575, 1498, 1456, 1412, and 1330. MS (ES) m/e 401, 403.

| Elemental Analyses for $C_{24}H_{21}N_2O_4Na$: | | | |
|---|---|---|---|
| Calculated | C, 67.92; | H, 4.95; | N, 6.60. |
| Found | C, 66.53; | H, 5.06; | N, 6.37. |

EXAMPLE 38

Preparation of {9-[(3-iodophenyl)methyl]-5-carbamoylcarbazol-4-yl}oxyacetic acid, sodium salt

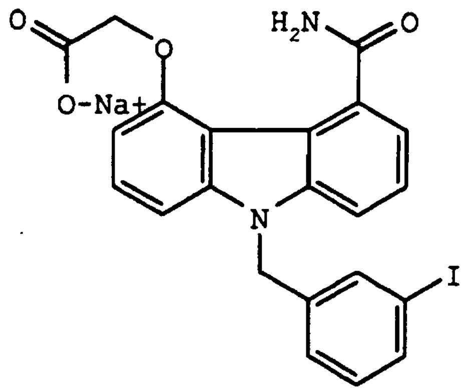

A. 9-[(3-Iodophenyl)methyl]-5-carbomethoxy-1,2-dihydrocarbazol-4(3H)-one

A suspension of 5-carbomethoxy-1,2-dihydro-9H-carbazol-4(3H)-one (680 mg, 3.5 mM), 3-iodobenzyl bromide (1.2 g, 4.7 mM), and potassium carbonate (500 mg, 3.61 mM) in 20 mL DMF was stirred at room temperature for 18 hours. The mixture was diluted with ethyl acetate, washed with $H_2O$ and saturated brine, dried over anhydrous magnesium sulfate, filtered, concentrated. The residue was purified trituration with methylene chloride-diethyl ether) to afford 0.70 g (55%) of the 9-[(3-iodophenyl)methyl]-5-carbomethoxy-1,2-dihydrocarbazol-4(3H)-one as a white solid. $^1$H NMR (DMSO-d6) δ 7.7-7.6 (m, 3H), 7.2 (m, 2H), 7.1 (t, 1H, J=8 Hz), 6.95 (d, 1H, J=8Hz), 5.6 (s, 2H), 3.8 (s, 3H), 3.0 (t, 2H, J=6 Hz), 2.5 (t, 2H, J=6 Hz), and 2.2 (m , 2H). IR (KBr, $cm^{-1}$) 1732, 1639, 1441, 1421, 1273, 1117, and 763. MS (ES) m/e 458, 460.

| Elemental Analyses for $C_{21}H_{18}NO_3I$: | | | |
|---|---|---|---|
| Calculated | C, 54.90; | H, 3.92; | N, 3.05. |
| Found | C, 54.92; | H, 3.98; | N, 2.97. |

B. 9-[(3-Iodophenyl)methyl]-4-hydroxy-5-carbomethoxy carbazole

A solution of the 9-[(3-iodophenyl)methyl]-5-carbomethoxy-1,2-dihydrocarbazol-4(3H)-one (700 mg, 1.52 mM) and 2,3-dichloro-5,6-dicyano-1,4-benzoquinone (380 mg, 1.67 mM) in 70 mL of toluene was stirred between 70-80 °C for 5 hours. The mixture was purified directly by column chromatography on silica gel (elution with methylene chloride) to afford 220 mg (31%) of the 9-[(3-iodophenyl)methyl]-9-hydroxy-5-carbomethyoxy carbazole as a yellow foam. $^1$H NMR (DMSO-d6) δ 10.25 (s, 1H), 7.7 (d, 1H, J=8 Hz), 7.6 (m, 2H), 7.4 (t, 1H, J=8 Hz), 7.25 (t, 1H, J=8 Hz), 7.2 (d, 1H, J=8 Hz), 7.1 (m, 3H), 6.65 (d, 1H, J=8 Hz), 5.65 (s, 2H), and 3.8 (s, 3H). IR (KBr, $cm^{-1}$) 3377 (br), 3028, 1711, 1672, 1621, 1580, 1565, 1495, 1459, 1439, 1423, 1332, 1287, 1267, 1135, 773, 752, 712, and 688. MS (ES) m/e 456, 458.

| Elemental Analyses for $C_{21}H_{16}NO_3I$: | | | |
|---|---|---|---|
| Calculated | C, 55.14; | H, 3.50; | N, 3.06. |
| Found | C, 56.18; | H, 3.87; | N, 3.32. |

C. 9-[(3-Iodophenyl)methyl]-4-hydroxy-5-carbamoyl carbazole

A solution of the 9-[(3-iodophenyl)methyl]-4-hydroxy-5-carbomethoxy carbazole (170 mg, 0.37 mM) in 10 mL THF and 30 mL concentrated aqueous ammonium hydroxide was stirred vigorously at room temperature for 120 hours. The mixture was diluted with ethyl acetate and acidified to pH 2 with 5 N HCl. The aqueous layer was extracted twice

with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 61 mg (37%) of the 9-[(3-iodophenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.8 (dd, 1H, J=1 and 8 Hz), 7.6-7.4 (m, 4H), 7.3 (t, 1H, J=8 Hz), 7.1-6.9 (m, 3H), 6.6 (d, 1H, J=8 Hz), and 5.7 (s, 2H). IR ($CHCl_3$, $cm^{-1}$) 3423, 3201 (br), 1630, 1600, 1579, 1564, 1445, 1330, 1261, and 775. MS (ES) m/e 441, 443.

| Elemental Analyses for $C_{20}H_{15}N_2O_2I$: | | | |
|---|---|---|---|
| Calculated | C, 54.30; | H, 3.39; | N, 6.33. |
| Found | C, 54.92; | H, 3.81; | N, 6.08. |

D. {9-[(3-Iodophenyl)methyl]-5-carbamoylcarbazol-4-yl}oxyacetic acid, methyl ester

40% Methanolic Triton B (0.07 mL, 0.15 mM) was added to a solution of the 9-[(3-iodophenyl)methyl]-4-hydroxy-5-carbamoyl carbazole (60 mg, 0.13 mM) in 8 mL DMF at room temperature. After 3 minutes, methyl bromoacetate (30 mg, 0.19 mM) was added and the resultant mixture stirred at room temperature for 17 hours. The mixture was diluted with ethyl acetate, washed with $H_2O$ and saturated brine, dried over magnesium sulfate, filtered, and concentrated. The residue was purified by column chromatography on silica gel (elution with ethyl acetate) to afford 60 mg (86%) of the {9-[(3-iodophenyl)methyl]-5-carbamoylcarbazol-4-yl}oxyacetic acid, methyl ester as a white solid. $^1$H NMR (DMSO-d6) δ 7.6-7.0 (m, 11H), 6.6 (d, 1H, J=8 Hz), 5.7 (s, 2H), 4.9 (s, 2H), and 3.75 (s, 3H). IR (KBr, $cm^{-1}$) 3500, 3350, 1727, 1642, 1291, 1236, and 772. MS (ES) m/e 515.

| Elemental Analyses for $C_{23}H_{19}N_2O_4I$: | | | |
|---|---|---|---|
| Calculated | C, 53.70; | H, 3.70; | N, 5.45. |
| Found | C, 53.92; | H, 3.72; | N, 5.32. |

E. {9-[(3-Iodophenyl)methyl]-5-carbamoylcarbazol-4-yl}oxyacetic acid

A suspension of the {9-[(3-iodophenyl)methyl]-5-carbamoylcarbazol-4-yl}oxyacetic acid, methyl ester (15 mg, 0.03 mM) and 0.03 mL (0.03 mM) of 1 N NaOH in 5 mL of ethanol was stirred for 43 hours at 25 °C, then cooled in an ice-bath. The resultant white precipitate was collected by filtration, washed with a small amount of EtOH, then dried *in vacuo* to afford 6.5 mg (43%) of the {9-[(3-iodophenyl)methyl]-5-carbamoylcarbazol-4-yl}oxyacetic acid, sodium salt as a white powder. $^1$H NMR (DMSO-d6) δ 7.6-7.0 (m, 11H), 6.6 (d, 1H, J=8 Hz), 5.7 (s, 2H), and 4.35 (s, 2H). IR (KBr, $cm^{-1}$) 3456, 3416, 3335, 1735, 1638, 1617, 1580, 1499, 1452, 1431, 1431, 1329, 1255, 1157, 772, 764, and 717. MS (ES) m/e 407, 409, 411.

| Elemental Analyses for $C_{22}H_{16}N_2O_4INa$, | | | |
|---|---|---|---|
| Calculated | C 50.57; | H, 3.07; | N, 5.36. |
| Found | C, 49.57; | H, 2.93; | N, 5.06. |

EXAMPLE 39

Preparation of {9-[(2-Chlorophenyl)methyl]-5-carbamoylcarbazol-4-yl}oxyacetic acid

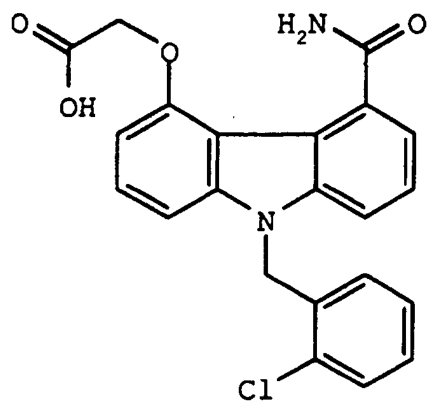

A. 9-[(2-Chlorophenyl)methyl]-5-carbomethoxy-1,2-dihydrocarbazol-4(3H)-one

40% Methanolic Triton B (2.42 mL, 5.3 mM) was slowly added dropwise to a solution of 5-carbomethoxy-1,2-dihydro-9H-carbazol-4(3H)-one (873.7 mg, 3.59 mM) in 10 mL of DMF at 25 °C. After 5 minutes, 2-chlorobenzyl bromide (1.11 g, 5.39 mM) was added and the resultant mixture stirred at room temperature for 72 hours. The mixture was diluted with ethyl acetate, washed five times with $H_2O$, once with saturated brine, dried over anhydrous magnesium sulfate, filtered, and concentrated. The residue was purified by crystallization from ethyl acetate to afford 706.3 mg (53%) of the 9-[(2-chlorophenyl)methyl]-5-carbomethoxy-1,2-dihydrocarbazol-4(3H)-one as a yellow solid. $^1H$ NMR (DMSO-d6) δ 7.6 (m, 2H), 7.4-7.1 (m, 4H), 6.5 (d, 1H, J=8 Hz), 5.6 (s, 2H), 3.8 (s, 3H), 2.9 (t, 2H, J=6 Hz), 2.4 (t, 2H, J=6 Hz), and 2.1 (m , 2H). IR ($CHCl_3$, $cm^{-1}$) 3050, 2950, 1725, 1655, 1462, 1446, 1435, 1288 and 1120. MS (ES) m/e 368, 370.

| Elemental Analyses for $C_{21}H_{18}NO_3Cl$: | | | |
|---|---|---|---|
| Calculated | C, 68.57; | H, 4.93; | N, 3.81. |
| Found | C, 68.52; | H, 5.18; | N, 3.67. |

B. 9-[(2-Chlorophenyl)methyl]-4-hydroxy-5-carbomethoxy carbazole

A solution of the 9-[(2-chlorophenyl)methyl]-5-carbomethoxy-1,2-dihydrocarbazol-4(3H)-one (692.2 mg, 1.88 mM) and 2,3-dichloro-5,6-dicyano-1,4-benzoquinone (529 mg, 2.32 mM) in 35 mL of toluene was stirred between 70-80 °C for 6 hours. The mixture was purified directly by column chromatography on silica gel (elution with methylene chloride) to afford 245 mg (35%) of the 9-[(2-chlorophenyl)methyl]-4-hydroxy-5-carbomethyoxy carbazole as a greenish solid. $^1H$ NMR (DMSO-d6) δ 10.3 (s, 1H), 7.6 (t, 2H, J=8 Hz), 7.4 (t, 1H, J=8 Hz), 7.3-7.1 (m, 4H), 6.9 (d, 1H, J=8 Hz), 6.6 (d, 1H, J=8 Hz), 6.3 (d, 1H, J=8 Hz), 5.65 (s, 2H), and 3.85 (s, 3H). IR ($CHCl_3$, $cm^{-1}$) 3200 (br), 1686, 1598, 1442, 1428, 1332, 1285, 1267, and 1141. MS (ES) m/e 364, 366, 368.

| Elemental Analyses for $C_{21}H_{16}NO_3Cl$: | | | |
|---|---|---|---|
| Calculated | C, 68.95; | H, 4.41; | N, 3.83. |
| Found | C, 67.88; | H, 4.29; | N, 3.67. |

C. 9-[(2-Chlorophenyl)methyl]-4-hydroxy-5-carbamoyl carbazole

A solution of the 9-[(2-chlorophenyl)methyl]-4-hydroxy-5-carbomethoxy carbazole (238 mg, 0.43 mM) in 20 mL THF and 25 mL concentrated aqueous ammonium hydroxide was sonicated for 20 hours at 40-50 °C. The mixture was diluted with ethyl acetate and acidified to pH 1 with 5 N HCl. The aqueous layer was extracted twice with ethyl

acetate. The combined organic extracts were washed with saturated brine, dried over magnesium sulfate, filtered, and concentrated. The residue was purified by column chromatography on silica gel (elution with gradient methylene chloride/ethyl acetate) to afford 86.9 mg (38%) of the 9-[(2-chlorophenyl)methyl]-4-hydroxy-5-carbamoyl carbazole as a white solid. $^1$H NMR (DMSO-d6) δ 10.5 (s, 1H), 8.8 (br s, 1H), 8.4 (br s, 1H), 7.7 (m, 1H), 7.55 (d, 1H, J=8 Hz), 7.45 (m, 2H), 7.3 (m, 2H), 7.15 (t, 1H, J=8 Hz), 6.95 (d, 1H, J=8 Hz), 6.6 (d, 1H, J=8 Hz), 6.2 (d, 1H, J=8 Hz), 5.75 (s, 2H). IR ($CHCl_3$, $cm^{-1}$) 3500, 3400, 3200 (br), 1649, 1597, 1585, 1446, 1431, 1331, and 1269. MS (ES) m/e 349, 351, 353.

| Elemental Analyses for $C_{20}H_{15}N_2O_2Cl$: | | | |
|---|---|---|---|
| Calculated | C, 68.48; | H, 4.31; | N, 7.99. |
| Found | C, 68.05; | H, 4.33; | N, 7.19 |

D. {9-[(2-Chlorophenyl)methyl]-5-carbamoylcarbazol-4-yl}oxyacetic acid, tert-butyl ester

40% Methanolic Triton B (0.15 mL, 0.34 mM) was added to a solution of the 9-[(2-chlorophenyl)methyl]-4-hydroxy-5-carbamoyl carbazole (80 mg, 0.23 mM) in 5 mL DMF at room temperature. After 3 minutes, t-butyl bromoacetate (182 mg, 0.91 mM) was added and the resultant mixture stirred at room temperature for 72 hours. The mixture was diluted with ethyl acetate, washed five times with $H_2O$, and saturated brine, dried over magnesium sulfate, filtered, and concentrated. The residue was purified by column chromatography on silica gel (elution with ethyl acetate) to afford 57 mg (53%) of the {9-[(2-chlorophenyl)methyl]-5-carbamoylcarbazol-4-yl}oxyacetic acid, tert-butyl ester as a white solid. $^1$H NMR (DMSO-d6) δ 7.6 (d, 1H, J=8 Hz), 7.5-6.9 (m, 9H), 6.55 (d, 1H, J=8 Hz), 6.35 (d, 1H, J=8 Hz), 5.7 (s, 2H), 4.75 (s, 2H), and 1.45 (s, 9H). IR (KBr, $cm^{-1}$) 1753 and 1678. MS (FD) m/e 464.

| Elemental Analyses for $C_{26}H_{25}N_2O_4Cl$: | | | |
|---|---|---|---|
| Calculated | C, 67.17; | H, 5.42; | N, 6.03. |
| Found | C, 64.02; | H, 5.33; | N, 5.77. |

E. {9-[(2-Chlorophenyl)methyl]-5-carbamoylcarbazol-4-yl}oxyacetic acid

A solution of the {9-[(2-chlorophenyl)methyl]-5-carbamoylcarbazol-4-yl}oxyacetic acid, tert-butyl ester (46 mg, 0.1 mM) in 3 mL of trifluoroacetic acid was stirred at room temperature for 2 hours. The solvent was removed *in vacuo*. The residue was triturated with diethyl ether/hexanes, then dried *in vacuo* to afford 40 mg (98%) of the {9-[(2-chlorophenyl)methyl]-5-carbamoylcarbazol-4-yl}oxyacetic acid as a white powder. $^1$H NMR (DMSO-d6) δ 12.9 (br s, 1H), 7.55 (s, 1H), 7.5 (d, 1H, J=8 Hz), 7.45 (s, 1H), 7.4-7.3 (m, 3H), 7.25 (t, 1H, J=8 Hz), 7.1-7.0 (m, 3H), 6.6 (d, 1H, J=8 Hz), 6.3 (d, 1H, J=8 Hz), 5.7 (s, 2H), and 4.8 (s, 2H). IR (KBr, $cm^{-1}$) 3430, 1735, and 1635. MS (ES) m/e 407, 409.

| Elemental Analyses for $C_{22}H_{17}N_2O_4Cl$: | | | |
|---|---|---|---|
| Calculated | C, 64.63; | H, 4.19; | N, 6.85. |
| Found | C, 64.60; | H, 4.08; | N, 6.70. |

EXAMPLE 40

Preparation of {9-[(2,3-difluorophenyl)methyl]-5-carbamoylcarbazol-4-yl}oxyacetic acid, sodium salt

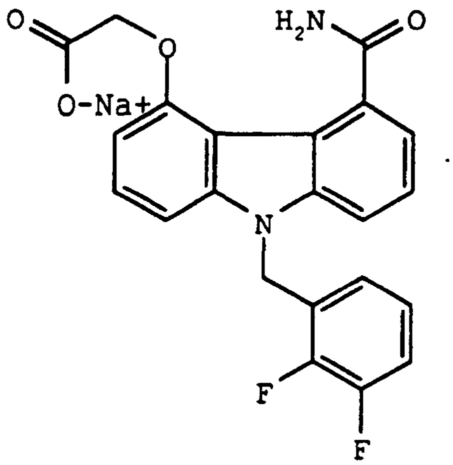

A. 9-[(2,3-Difluorophenyl)methyl]-5-carbomethoxy-1,2-dihydrocarbazol-4(3H)-one

A suspension of 5-carbomethoxy-1,2-dihydro-9H-carbazol-4(3H)-one (973 mg, 4.0 mM), a-bromo-2,3-difluorotoluene (1.01 g, 4.8 mM), and potassium carbonate (553 mg, 4.0 mM) in 10 mL DMF was stirred at room temperature for 73 hours. The mixture was diluted with ethyl acetate, washed with $H_2O$, 1 N HCl, $H_2O$, saturated $NaHCO_3$, $H_2O$, and saturated brine, dried over anhydrous magnesium sulfate, filtered, concentrated. The residue was purified by column chromatography on silica gel (elution with methylene chloride/ethyl acetate) to afford 1.04 g (70%) of the 9-[(2,3-difluorophenyl)methyl]-5-carbomethoxy-1,2-dihydrocarbazol-4(3H)-one as a tan solid. $^1H$ NMR ($CDCl_3$) δ 7.4 (d, 1H, J=8 Hz), 7.35 (d, 1H, J=8 Hz), 7.15-6.9 (m, 5H), 6.35 (t, 1H, J=8 Hz), 5.4 (s, 2H), 4.05 (s, 3H), 2.9 (t, 2H, J=6 Hz), 2.6 (t, 2H, J=6 Hz), and 2.25 (m , 2H). IR (KBr, $cm^{-1}$) 1719 and 1650. MS (ES) m/e 368, 370.

| Elemental Analyses for $C_{21}H_{17}NO_3F_2$: | | | |
|---|---|---|---|
| Calculated | C, 68.29; | H, 4.64; | N, 3.79. |
| Found | C, 68.50; | H, 4.62; | N, 3.94. |

B. 9-[(2,3-Difluorophenyl)methyl]-4-hydroxy-5-carbomethoxy carbazole

A solution of the 9-[(2,3-difluorophenyl)methyl]-5-carbomethoxy-1,2-dihydrocarbazol-4(3H)-one (490 mg, 1.32 mM) and 2,3-dichloro-5,6-dicyano-1,4-benzoquinone (336 mg, 1.45 mM) in 70 mL of toluene was stirred at 80-90 °C for 2.25 hours. The mixture was purified directly by column chromatography on silica gel (elution with methylene chloride) to afford 165 mg (34%) of the 9-[(2,3-difluorophenyl)methyl]-4-hydroxy-5-carbomethyoxy carbazole as a yellow solid. $^1H$ NMR ($CDCl_3$) δ 10.25 (s, 1H), 8.0 (d, 1H, J=8 Hz), 7.6 (d, 1H, J=8 Hz), 7.5-7.4 (m, 2H), 7.05 (m, 1H), 6.9 (d, 1H, J=8 Hz), 6.85 (d, 1H, J=8 Hz), 6.8 (m, 1H), 6.35 (t, 1H, J=8 Hz), 5.6 (s, 2H), and 4.1 (s, 3H). IR (KBr, $cm^{-1}$) 3025 and 1684. MS (ES) m/e 366, 368.

| Elemental Analyses for $C_{21}H_{15}NO_3F_2$: | | | |
|---|---|---|---|
| Calculated | C, 68.66; | H, 4.12; | N, 3.81. |
| Found | C, 69.54; | H, 4.44; | N, 3.81. |

C. 9-[(2,3-Difluorophenyl)methyl]-4-hydroxy-5-carbamoyl carbazole

A solution of the 9-[(2,3-difluorophenyl)methyl]-4-hydroxy-5-carbomethoxy carbazole (514 mg, 1.4 mM) in 5 mL THF and 20 mL concentrated aqueous ammonium hydroxide was stirred at room temperature for 94 hours. The

mixture was diluted with ethyl acetate and acidified to pH 1 with 5 N HCl. The aqueous layer was extracted three times with ethyl acetate. The combined organic extracts were washed with $H_2O$ and saturated brine, dried over magnesium sulfate, filtered, and concentrated. The residue was purified by column chromatography on silica gel (elution with gradient hexanes/ethyl acetate) to afford 320 mg (65%) of the 9-[(2,3-difluorophenyl)methyl]-4-hydroxy-5-carbamoyl carbazole as a yellow solid. $^1H$ NMR (DMSO-d6) δ 10.45 (s, 1H), 8.8 (br s, 1H), 8.4 (br s, 1H), 7.8 (d, 1H, J=8 Hz), 7.5-7.2 (m, 4H), 7.05 (d, 1H, J=8 Hz), 6.95 (m, 1H), 6.6 (d, 1H, J=8 Hz), 6.35 (t, 1H, J=8 Hz), and 5.9 (s, 2H). IR (KBr, $cm^{-1}$) 3350, 3125, 1628, 1598, and 1583. MS (ES) m/e 351, 353.

| Elemental Analyses for $C_{20}H_{14}N_2O_2F_2$: | | | |
|---|---|---|---|
| Calculated | C, 68.18; | H, 4.01; | N, 7.95. |
| Found | C, 68.15; | H, 4.23; | N, 8.01. |

D. {9-[(2,3-Difluorophenyl)methyl]-5-carbamoylcarbazol-4-yl}oxyacetic acid, methyl ester

40% Methanolic Triton B (0.51 mL, 1.12 mM) was added to a solution of the 9-[(2,3-difluorophenyl)methyl]-4-hydroxy-5-carbamoyl carbazole (303 mg, 0.86 mM) in 5 mL DMF at room temperature. After 15 minutes, methyl bromoacetate (270 mg, 1.72 mM) was added and the resultant mixture stirred at room temperature for 18 hours. The mixture was diluted with ethyl acetate, washed with $H_2O$, 1 N HCl, $H_2O$, and saturated brine, dried over magnesium sulfate, filtered, and concentrated. The residue was purified by column chromatography on silica gel (elution with ethyl acetate) to afford 295 mg (80%) of the {9-[(2,3-difluorophenyl)methyl]-5-carbamoylcarbazol-4-yl}oxyacetic acid, methyl ester as a white solid. $^1H$ NMR ($CDCl_3$) δ 7.5-7.3 (m, 5H), 7.05 (d, 1H, J=8 Hz), 6.8 (m, 1H), 6.6 (d, 1H, J=8 Hz), 6.4 (t, 1H, J=8 Hz), 6.2 (br s, 1H), 6.0 (br s, 1H), 5.6 (s, 2H), 4.9 (s, 2H), and 3.8 (s, 3H). IR (KBr, $cm^{-1}$) 3432, 3180, 1774, 1766,and 1674. MS (ES) m/e 425.

| Elemental Analyses for $C_{23}H_{18}N_2O_4F_2$: | | | |
|---|---|---|---|
| Calculated | C, 65.09; | H, 4.28; | N, 6.60. |
| Found | C, 64.11; | H, 4.12; | N, 6.32. |

E. {9-[(2,3-Difluorophenyl)methyl]-5-carbamoylcarbazol-4-yl}oxyacetic acid, sodium salt

A suspension of the {9-[(2,3-difluorophenyl)methyl]-5-carbamoylcarbazol-4-yl}oxyacetic acid, methyl ester (85 mg, 0.2 mM) and 0.22 mL (0.22 mM) of 1 N NaOH in 5 mL of ethanol was stirred for 18 hours at 25 °C. A small volume of diethyl ether/hexanes was added, then cooled in the refrigerator. The resultant white precipitate was collected by filtration, washed with a small amount of EtOH/diethyl ether/hexanes, then dried *in vacuo* to afford 77 mg (89%) of the {9-[(2,3-difluorophenyl)methyl]-5-carbamoylcarbazol-4-yl}oxyacetic acid, sodium salt as a white powder. $^1H$ NMR (DMSO-d6) δ 7.7-7.2 (m, 6H), 7.2-7.0 (m, 3H), 6.6 (d, 1H, J=8 Hz), 6.45 (t, 1H, J=8 Hz), 5.7 (s, 2H), and 4.35 (s, 2H). IR (KBr, $cm^{-1}$) 3467, 3390, 1662, and 1616. MS (ES) m/e 409, 411, 433.

| Elemental Analyses for $C_{22}H_{15}N_2O_4F_2Na$: | | | |
|---|---|---|---|
| Calculated | C, 61.12; | H, 3.50; | N, 6.48. |
| Found | C, 61.34; | H, 3.38; | N, 6.41. |

EXAMPLE 41

Preparation of {9-[(2,6-difluorophenyl)methyl]-5-carbamoylcarbazol-4-yl}oxyacetic acid, sodium salt

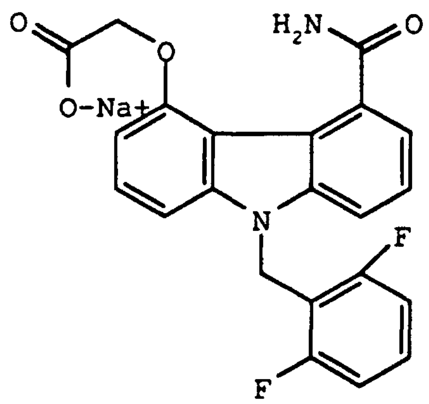

A. 9-[(2,6-Difluorophenyl)methyl]-5-carbomethoxy-1,2-dihydrocarbazol-4(3H)-one

A suspension of 5-carbomethoxy-1,2-dihydro-9H-carbazol-4(3H)-one (973 mg, 4.0 mM), a-bromo-2,6-difluorotoluene (1.01 g, 4.8 mM), and potassium carbonate (553 mg, 4.0 mM) in 10 mL DMF was stirred at room temperature for 74 hours. The mixture was diluted with ethyl acetate, washed with $H_2O$, 1 N HCl, $H_2O$, saturated $NaHCO_3$, $H_2O$, and saturated brine, dried over anhydrous magnesium sulfate, filtered, concentrated. The residue was purified by column chromatography on silica gel (elution with methylene chloride/ethyl acetate) to afford 1.04 g (70%) of the 9-[(2,6-difluorophenyl)methyl]-5-carbomethoxy-1,2-dihydrocarbazol-4(3H)-one as a tan solid. $^1$H NMR ($CDCl_3$) $\delta$ 7.5 (d, 1H, J=8 Hz), 7.35-7.2 (m, 3H), 6.95 (t, 2H, J=8 Hz), 5.4 (s, 2H), 4.0 (s, 3H), 3.05 (t, 2H, J=6 Hz), 2.6 (t, 2H, J=6 Hz), and 2.25 (m, 2H). IR (KBr, $cm^{-1}$) 1728 and 1655. MS (ES) m/e 370.

| Elemental Analyses for $C_{21}H_{17}NO_3F_2$: | | | |
|---|---|---|---|
| Calculated | C, 68.29; | H, 4.64; | N, 3.79. |
| Found | C, 68.51; | H, 4.82; | N, 3.78. |

B. 9-[(2,6-Difluorophenyl)methyl]-4-hydroxy-5-carbomethoxy carbazole

A 60% oil dispersion of sodium hydride (257 mg, 6.42 mM) was added to a solution of the 9-[(2,6-difluorophenyl)methyl]-5-carbomethoxy-1,2-dihydrocarbazol-4(3H)-one (1.03 g, 2.79 mM) in 7 mL of dioxane at room temperature. After 5 minutes methyl benzenesulfinate (0.6 mL, 4.46 mM) was added and the mixture stirred at room temperature for 1.75 hours. The mixture was diluted with 10 mL dioxane, then glacial acetic acid (0.37 mL, 6.42 mM) was added. The resultant mixture was refluxed for 45 min, cooled to room temperature, diluted with ethyl acetate, washed three times with saturated $NaHCO_3$, and saturated brine, dried over anhydrous magnesium sulfate, filtered and concentrated. The residue was purified by column chromatography on silica gel (elution with toluene) to afford 480 mg (47%) of the 9-[(2,6-difluorophenyl)methyl]-4-hydroxy-5-carbomethyoxy carbazole as a yellow solid. $^1$H NMR ($CDCl_3$) $\delta$ 10.15 (s, 1H), 7.95 (d, 1H, J=8 Hz), 7.5 (d, 1H, J=8 Hz), 7.5-7.0 (m, 4H), 6.9-6.8 (m, 3H), 5.6 (s, 2H), and 4.1 (s, 3H). IR (KBr, $cm^{-1}$) 3040 and 1682. MS (ES) m/e 366, 368.

| Elemental Analyses for $C_{21}H_{15}NO_3F_2$: | | | |
|---|---|---|---|
| Calculated | C, 68.66; | H, 4.12; | N, 3.81. |
| Found | C, 69.48; | H, 4.07; | N, 4.11. |

C. 9-[(2,6-Difluorophenyl)methyl]-4-hydroxy-5-carbamoyl carbazole

A solution of the 9-[(2,6-difluorophenyl)methyl]-4-hydroxy-5-carbomethoxy carbazole (514 mg, 1.4 mM) in 5

mL THF and 20 mL concentrated aqueous ammonium hydroxide was stirred at room temperature for 64 hours. The pH was adjusted to 10.5 with 5 N HCl. The resultant precipitate was collected by filtration, resuspended in $H_2O$, adjusted the pH to 11.7 with concentrated ammonium hydroxide. The resultant precipitate was collected by filtration. The precipitate was dissolved in ethyl acetate, washed three times with 5 N NaOH, $H_2O$, and saturated brine, dried over anhydrous magnesium sulfate, filtered, concentrated to afford 310 mg (70%) of the 9-[(2,6-difluorophenyl)methyl]-9-hydroxy-5-carbamoyl carbazole as a yellow solid. $^1H$ NMR (DMSO-d6) δ 10.4 (s, 1H), 8.8 (br s, 1H), 8.4 (br s, 1H), 7.8 (d, 1H, J=8 Hz), 7.6-7.0 (m, 7H), 6.6 (d, 1H, J=8 Hz), and 5.7 (s, 2H). IR (KBr, $cm^{-1}$) 3404, 3113, 1626, and 1587. MS (ES) m/e 351, 353.

| Elemental Analyses for $C_{20}H_{14}N_2O_2F_2$: | | | |
|---|---|---|---|
| Calculated | C, 68.18; | H, 4.01; | N, 7.95. |
| Found | C, 68.45; | H, 4.01; | N, 7.87. |

D. {9-[(2,6-Difluorophenyl)methyl]-5-carbamoylcarbazol-4-yl}oxyacetic acid, methyl ester

40% Methanolic Triton B (0.49 mL, 1.07 mM) was added to a solution of the 9-[(2,6-difluorophenyl)methyl]-4-hydroxy-5-carbamoyl carbazole (290 mg, 0.82 mM) in 5 mL DMF at room temperature. After 15 minutes, methyl bromoacetate (259 mg, 1.65 mM) was added and the resultant mixture stirred at room temperature for 24 hours. The mixture was diluted with $H_2O$ and the resultant white precipitate collected by filtration, triturated with diethyl ether/hexanes, and dried *in vacuo* to afford 228 mg (65%) of the {9-[(2,6-difluorophenyl)methyl]-5-carbamoylcarbazol-4-yl}oxyacetic acid, methyl ester as a white solid. $^1H$ NMR ($CDCl_3$) δ 7.65 (d, 1H, J=8 Hz), 7.45-7.2 (m, 5H), 6.85 (t, 2H, J=8 Hz), 6.55 (d, 1H, J=8 Hz), 6.3 (br s, 1H), 6.0 (br s, 1H), 5.5 (s, 2H), 4.9 (s, 2H), and 3.8 (s, 3H). IR (KBr, $cm^{-1}$) 3432, 3170, 1762, and 1675. MS (ES) m/e 425.

| Elemental Analyses for $C_{23}H_{18}N_2O_4F_2$: | | | |
|---|---|---|---|
| Calculated | C, 65.09; | H, 4.28; | N, 6.60. |
| Found | C, 65.05; | H, 4.40; | N, 6.53. |

E. {9-[(2,6-Difluorophenyl)methyl]-5-carbamoylcarbazol-4-yl}oxyacetic acid, sodium salt

A suspension of the {9-[(2,6-difluorophenyl)methyl]-5-carbamoylcarbazol-4-yl}oxyacetic acid, methyl ester (85 mg, 0.2 mM) and 0.22 mL (0.22 mM) of 1 N NaOH in 5 mL of ethanol was stirred for 18 hours at 25 °C. A small volume of diethyl ether/hexanes was added, then cooled in the refrigerator. The resultant white precipitate was collected by filtration, washed with a small amount of EtOH/diethyl ether/hexanes, then dried *in vacuo* to afford 82 mg (95%) of the {9-[(2,6-difluorophenyl)methyl]-5-carbamoylcarbazol-4-yl}oxyacetic acid, sodium salt as a white powder. $^1H$ NMR (DMSO-d6) δ 7.6 (d, 1H, J=8 Hz), 7.55 (br s, 1H), 7.45-7.3 (m, 3H), 7.25 (t, 1H, J=8 Hz), 7.1-7.0 (m, 4H), 6.5 (d, 1H, J=8 Hz), 5.65 (s, 2H), and 4.3 (s, 2H). IR (KBr, $cm^{-1}$) 3470, 3360, 1658, and 1606. MS (ES) m/e 409, 411, 433.

| Elemental Analyses for $C_{22}H_{15}N_2O_4F_2Na$: | | | |
|---|---|---|---|
| Calculated | C, 61.12; | H, 3.50; | N, 6.48. |
| Found | C, 59.18; | H, 3.70; | N, 6.19. |

EXAMPLE 42

Preparation of {9-[(2,6-dichlorophenyl)methyl]-5-carbamoylcarbazol-4-yl}oxyacetic acid, sodium salt

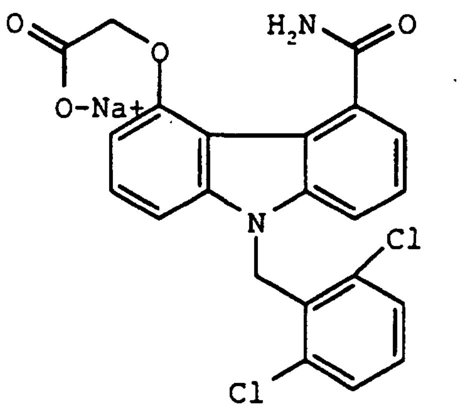

A. 9-[(2,6-Dichlorophenyl)methyl]-5-carbomethoxy-1,2-dihydrocarbazol-4(3H)-one

A suspension of 5-carbomethoxy-1,2-dihydro-9H-carbazol-4(3H)-one (973 mg, 4.0 mM), a-bromo-2,6-dichlorotoluene (1.19 g, 4.8 mM), and potassium carbonate (553 mg, 4.0 mM) in 10 mL DMF was stirred at room temperature for 24 hours. The mixture was diluted with ethyl acetate, washed with $H_2O$, 1 N HCl, $H_2O$, saturated $NaHCO_3$, $H_2O$, and saturated brine, dried over anhydrous magnesium sulfate, filtered, concentrated. The residue was purified by column chromatography on silica gel (elution with methylene chloride/ethyl acetate) to afford 900 mg (56%) of the 9-[(2,6-dichlorophenyl)methyl]-5-carbomethoxy-1,2-dihydrocarbazol-4(3H)-one as a white foam. $^1H$ NMR ($CDCl_3$) δ 7.4-7.2 (m, 6H), 5.6 (s, 2H), 4.0 (s, 3H), 2.9 (t, 2H, J=6 Hz), 2.55 (t, 2H, J=6 Hz), and 2.2 (m, 2H). IR (KBr, $cm^{-1}$) 1725 and 1652. MS (ES) m/e 400, 402, 404.

| Elemental Analyses for $C_{21}H_{17}NO_3Cl_2$: | | | |
|---|---|---|---|
| Calculated | C, 62.70; | H, 4.26; | N, 3.48. |
| Found | C, 62.98; | H, 4.35; | N, 3.35. |

B. 9-[(2,6-Dichlorophenyl)methyl]-4-hydroxy-5-carbomethoxy carbazole

A solution of the 9-[(2,6-dichlorophenyl)methyl]-5-carbomethoxy-1,2-dihydrocarbazol-4(3H)-one (861 mg, 2.14 mM) and 2,3-dichloro-5,6-dicyano-1,4-benzoquinone (595 mg, 2.57 mM) in 60 mL of toluene was stirred at reflux for 3.5 hours. The mixture was purified directly by column chromatography on silica gel (elution with methylene chloride) to afford 255 mg (29%) of the 9-[(2,6-dichlorophenyl)methyl]-4-hydroxy-5-carbomethyoxy carbazole as a yellow solid. $^1H$ NMR ($CDCl_3$) δ 10.05 (s, 1H), 7.95 (d, 1H, J=8 Hz), 7.6 (d, 1H, J=8 Hz), 7.45-7.2 (m, 5H), 6.95 (d, 1H, J=8 Hz), 6.8 (d, 1H, J=8 Hz), 5.75 (s, 2H), and 4.1 (s, 3H). IR (KBr, $cm^{-1}$) 3430 and 1668. MS (ES) m/e 409, 411.

| Elemental Analyses for $C_{21}H_{15}NO_3Cl_2$: | | | |
|---|---|---|---|
| Calculated | C, 63.02; | H, 3.78; | N, 3.50. |
| Found | C, 63.78; | H, 3.82; | N, 3.59. |

C. 9-[(2,6-Dichlorophenyl)methyl]-4-hydroxy-5-carbamoyl carbazole

A solution of the 9-[(2,6-dichlorophenyl)methyl]-4-hydroxy-5-carbomethoxy carbazole (240 mg, 0.6 mM) in 5 mL THF and 20 mL concentrated aqueous ammonium hydroxide was stirred at room temperature for 22 hours. The resultant precipitate was collected by filtration and dried *in vacuo* to afford 151 mg (65%) of the 9-[(2,6-dichlorophenyl) methyl]-4-hydroxy-5-carbamoyl carbazole as a yellow solid. $^1H$ NMR (DMSO-d6) δ 10.35 (s, 1H), 8.8 (br s, 1H)-, 8.4 (br s, 1H), 7.7 (d, 1H, J=8 Hz), 7.6-7.3 (m, 5H), 7.25 (t, 1H, J=8 Hz), 6.85 (d, 1H, J=8 Hz), 6.55 (d, 1H, J=8 Hz), and

5.85 (s, 2H). IR (KBr, $cm^{-1}$) 3429, 1631, and 1597. MS (ES) m/e 385, 387.

| Elemental Analyses for $C_{20}H_{14}N_2O_2Cl_2$: | | | |
|---|---|---|---|
| Calculated | C, 62.35; | H, 3.66; | N, 7.27. |
| Found | C, 62.87; | H, 3.99; | N, 6.00. |

D. {9-[(2,6-Dichlorophenyl)methyl]-5-carbamoylcarbazol-4-yl}oxyacetic acid, methyl ester

40% Methanolic Triton B (0.23 mL, 0.49 mM) was added to a solution of the 9-[(2,6-dichlorophenyl)methyl]-4-hydroxy-5-carbamoyl carbazole (146 mg, 0.38 mM) in 5 mL DMF at room temperature. After 15 minutes, methyl bromoacetate (119 mg, 0.76 mM) was added and the resultant mixture stirred at room temperature for 17 hours. The mixture was diluted with ethyl acetate, washed with $H_2O$, 1 N HCl, $H_2O$, sat. $NaHCO_3$, and saturated brine, dried over magnesium sulfate, filtered, and concentrated. The residue was purified by column chromatography on silica gel (elution with ethyl acetate) to afford 83 mg (48%) of the {9-[(2,6-dichlorophenyl)methyl]-5-carbamoylcarbazol-4-yl}oxyacetic acid, methyl ester as a tan solid. $^1H$ NMR (DMSO-d6) δ 7.5-7.0 (m, 10H), 6.55 (d, 1H, J=8 Hz), 5.8 (s, 2H), 4.9 (s, 2H), and 3.75 (s, 3H). IR (KBr, $cm^{-1}$) . MS (ES) m/e 457, 459.

E. {9-[(2,6-Dichlorophenyl)methyl]-5-carbamoylcarbazol-4-yl}oxyacetic acid, sodium salt

A suspension of the {9-[(2,6-dichlorophenyl)methyl]-5-carbamoylcarbazol-4-yl}oxyacetic acid, methyl ester (45.7 mg, 0.1 mM) and 0.11 mL (0.11 mM) of 1 N NaOH in 5 mL of ethanol was stirred for 22 hours at 25 °C. A small volume of diethyl ether/hexanes was added, then cooled in the refrigerator. The resultant white precipitate was collected by filtration, washed with a small amount of EtOH/diethyl ether/hexanes, then dried *in vacuo* to afford 40 mg (86%) of the {9-[(2,6-dichlorophenyl)methyl]-5-carbamoylcarbazol-4-yl}oxyacetic acid, sodium salt as a white powder. $^1H$ NMR (DMSO-d6) δ 7.6-7.4 (m, 6H), 7.3 (t, 1H, J=8 Hz), 7.2 (t, 1H, J=8 Hz), 7.0 (d, 1H, J=8 Hz), 6.9 (t, 1H, J=8 Hz), 6.5 (d, 1H, J=8 Hz), 5.8 (s, 2H), and 4.25 (s, 2H). MS (ES) m/e 441, 443, 445.

EXAMPLE 43

Preparation of {9-[(3-trifluoromethoxyphenyl)methyl]-5-carbamoylcarbazol-4-yl}oxyacetic acid, sodium salt

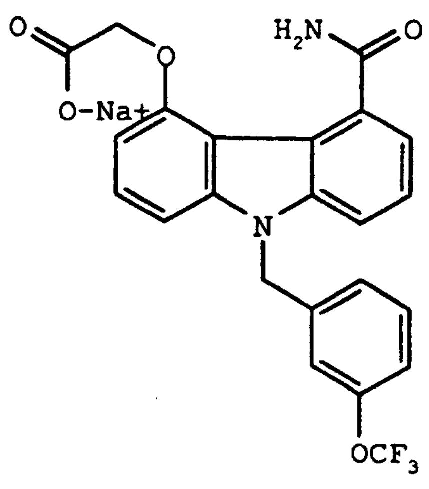

A. 9-[(3-Trifluoromethoxyphenyl)methyl]-5-carbomethoxy-1,2-dihydrocarbazol-4(3H)-one

A suspension of 5-carbomethoxy-1,2-dihydro-9H-carbazol-4(3H)-one (935 mg, 3.85 mM), 3-trifluoromethoxybenzyl bromide (1.0 g, 3.93 mM), and potassium carbonate (531 mg, 3.85 mM) in 20 mL DMF was stirred at room temperature for 17 hours. The mixture was diluted with ethyl acetate, washed with $H_2O$ and saturated brine, dried over anhydrous magnesium sulfate, filtered, concentrated to afford 1.6 g (100%) of the 9-[(3-trifluoromethoxyphenyl)methyl]-5-carbomethoxy-1,2-dihydrocarbazol-4(3H)-one as a foam. $^1$H NMR (DMSO-d6) δ 7.7 (dd, 1H, J=1 and 8 Hz), 7.45 (t, 1H, J=8 Hz), 7.3-7.1 (m, 4H), 7.05 (d, 1H, J=8 Hz), 5.6 (s, 2H), 3.8 (s, 3H), 3.0 (m, 2H), 2.45 (m, 2H), and 2.1 (m, 2H). IR ($CHCl_3$, $cm^{-1}$) 1729, 1647, 1439, 1259, 1176, and 1116. MS (ES) m/e 418.

| Elemental Analyses for $C_{22}H_{18}NO_4F_3$: | | | |
|---|---|---|---|
| Calculated | C, 63.31; | H, 4.32; | N, 3.36. |
| Found | C, 63.12; | H, 4.35; | N, 3.31. |

B. 9-[(3-Trifluoromethoxyphenyl)methyl]-4-hydroxy-5-carbomethoxy carbazole

A solution of the 9-[(3-trifluoromethoxyphenyl)methyl]-5-carbomethoxy-1,2-dihydrocarbazol-4(3H)-one (0.75 g, 1.8 mM) and 2,3-dichloro-5,6-dicyano-1,4-benzoquinone (490 mg, 2.16 mM) in 70 mL of toluene was stirred at reflux for 6 hours. The mixture was purified directly by column chromatography on silica gel (elution with methylene chloride) to afford 300 mg (40%) of the 9-[(3-trifluoromethoxyphenyl)methyl]-4-hydroxy-5-carbomethyoxy carbazole as a yellow solid. $^1$H NMR (DMSO-d6) δ 10.25 (s, 1H), 7.7 (d, 1H, J=8 Hz), 7.5-7.0 (m, 8H), 6.6 (d, 1H, J=8 Hz), 5.7 (s, 2H), and 3.85 (s, 3H). IR (KBr, $cm^{-1}$) 3200, 1673, 1441, 1268, 1217, 1173, and 753. MS (ES) m/e 414, 416.

| Elemental Analyses for $C_{22}H_{16}NO_3F_3$ | | | |
|---|---|---|---|
| Calculated | C, 63.61; | H, 3.86; | N, 3.37. |
| Found | C, 63.40; | H, 3.99; | N, 3.43. |

C. 9-[(3-Trifluoromethoxyphenyl)methyl]-4-hydroxy-5-carbamoyl carbazole

A solution of the 9-[(3-trifluoromethoxyphenyl)methyl]-4-hydroxy-5-carbomethoxy carbazole (260 mg, 0.62 mM) in 10 mL THF and 30 mL concentrated aqueous ammonium hydroxide was stirred vigorously for 132 hours. The mixture was diluted with ethyl acetate and acidified to pH 1 with 5 N HCl. The aqueous layer was extracted three times with ethyl acetate. The combined organic extracts were washed with $H_2O$ and saturated brine, dried over magnesium sulfate, filtered, and concentrated. The residue was purified by column chromatography on silica gel (elution with gradient hexanes/ethyl acetate) to afford 150 mg (60%) of the 9-[(3-trifluoromethoxyphenyl)methyl]-4-hydroxy-5-carbamoyl carbazole as an off-white solid. $^1$H NMR (DMSO-d6) δ 10.5 (s, 1H), 8.8 (br s, 1H), 8.4 (br s, 1H), 7.85 (dd, 1H, J=1 and 8 Hz), 7.5-7.15 (m, 5H), 7.1 (d, 1H, J=8 Hz), 7.0 (d, 1H, J=8 Hz), 6.6 (d, 1H, J=8 Hz), 5.95 (d, 1H, J=8 Hz), and 5.65 (s, 2H). IR (KBr, $cm^{-1}$) 3431, 3203, 1629, 1601, 1580, 1548, 1446, 1330, 1261, 1215, and 777. MS (ES) m/e 399, 401.

| Elemental Analyses for $C_{21}H_{15}N_2O_2F_3$: | | | |
|---|---|---|---|
| Calculated | C, 63.00; | H, 3.75; | N, 7.0. |
| Found | C, 63.15; | H, 4.07; | N, 6.84. |

D. {9-[(3-Trifluoromethoxyphenyl)methyl]-5-carbamoylcarbazol-4-yl}oxyacetic acid, methyl ester

40% Methanolic Triton B (0.15 mL, 0.34 mM) was added to a solution of the 9-[(3-trifluoromethoxyphenyl) methyl]-4-hydroxy-5-carbamoyl carbazole (115 mg, 0.28 mM) in 8 mL DMF at room temperature. After 3 minutes, methyl bromoacetate (65 mg, 0.41 mM) was added and the resultant mixture stirred at room temperature for 23 hours. The mixture was diluted with ethyl acetate, washed with $H_2O$, and saturated brine, dried over magnesium sulfate, filtered, and concentrated. The residue was purified by column chromatography on silica gel (elution with ethyl acetate) to afford 112 mg (83%) of the {9-[(3-trifluoromethoxyphenyl)methyl]-5-carbamoylcarbazol-4-yl}oxyacetic acid, methyl ester as a white solid. $^1$H NMR (DMSO-d6) δ 7.6 (d, 1H, J=8 Hz), 7.55 (br s, 1H), 7.5-7.0 (m, 9H), 6.6 (d, 1H, J=8 Hz), 5.7 (s, 2H), 4.9 (s, 2H), and 3.75 (s, 3H). IR (KBr, $cm^{-1}$) 3488, 3141, 1763, 1674, 1501, 1444, 1269, 1215, 1178, 1102, 772, and 714. MS (FD) m/e 472.

| Elemental Analyses for $C_{24}H_{19}N_2O_5F_3$: | | | |
|---|---|---|---|
| Calculated | C, 61.02; | H, 4.03; | N, 5.93. |
| Found | C, 61.05; | H, 4.17; | N, 5.81. |

E. {9-[(3-Trifluoromethoxyphenyl)methyl]-5-carbamoylcarbazol-4-yl}oxyacetic acid, sodium salt

A suspension of the {9-[(3-trifluoromethoxyphenyl)methyl]-5-carbamoylcarbazol-4-yl}oxyacetic acid, methyl ester (22.4 mg, 0.047 mM) and 0.065 mL (0.065 mM) of 1 N NaOH in 5 mL of ethanol was stirred for 24 hours at 25 °C. The solvent was removed *in vacuo* and the residue suspended in EtOH. The resultant white precipitate was collected by filtration, washed with a small amount of EtOH, then dried *in vacuo* to afford 9 mg (41%) of the {9-[(3-trifluoromethoxyphenyl)methyl]-5-carbamoylcarbazol-4-yl}oxyacetic acid, sodium salt as a white powder. MS (ES) m/e 457, 459.

EXAMPLE 44 Preparation of {9-[(2-biphenyl)methyl]-5-carbamoylcarbazol-4-yl}oxyacetic acid

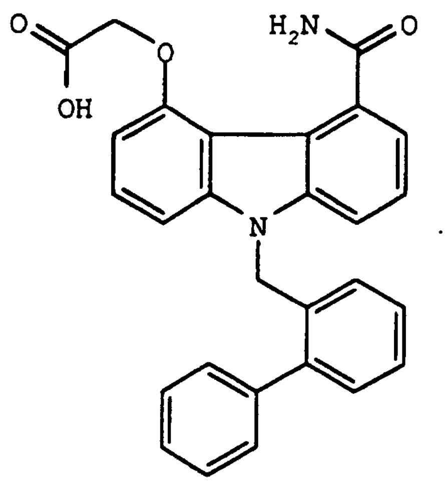

A. 2-Carbomethoxy-6-nitro-2'-methoxy-biphenyl

A solution of methyl 2-chloro-3-nitrobenzoate (2.16 g, 10.0 mM), 2-methoxybenzeneboronic acid (1.64g, 10.5 mM), tetrakis(triphenylphosphine)palladium (0) (584 mg, 0.5 mM), and 2 M aqueous sodium carbonate (10.5 mL, 21.0 mM) in 50 mL of THF was wrapped in aluminum foil and stirred at reflux for 27 hours. The THF was removed *in vacuo* and the residue dissolved in ethyl acetate. The mixture was washed with with $H_2O$, 1 N HCl, $H_2O$, sat. $NaHCO_3$, and saturated brine, dried over magnesium sulfate, filtered, and concentrated. The resultant light brown oil was purified by column chromatography on silica (elution with gradient toluene/ethyl acetate) to afford 2.0 g (69%) of 2-carbomethoxy-6-nitro-2'-methoxy-biphenyl as a yellow-orange solid. $^1H$ NMR ($CDCl_3$) δ 8.05 (d, 1H, J=8 Hz), 7.95 (d, 1H, J=8 Hz), 7.55 (t, 1H, J=8 Hz), 7.35 (t, 1H, J=8 Hz), 7.05 (d, 1H, J=8 Hz), 7.0 (t, 1H, J=8 Hz), 6.9 (d, 1H, J=8 Hz), 3.7 (s, 3H), and 3.6 (s, 3H). IR (KBr, $cm^{-1}$) 1730, 1538, 1499, 1366, 1298, 1271, 1130, 774, 765, 759, 752, and 707. MS (ES) m/e 288.

| Elemental Analyses for $C_{15}H_{13}NO_5$: | | | |
|---|---|---|---|
| Calculated | C, 62.72; | H, 4.56; | N, 4.88. |
| Found | C, 62.65; | H, 4.61; | N, 4.72. |

B. 9H-4-methoxy-5-carbomethoxy carbazole

A solution of the 2-carbomethoxy-6-nitro-2'-methoxybiphenyl (144 mg, 0.5 mM) in triethylphosphite (339 mg, 0.35 mL, 2.0 mM) was heated at 150-160 °C in a sealed tube for 4 h then at room temperature for 15 hours. The mixture was dried *in vacuo* with toluene, then purified by preparative TLC on silica gel (elution with 4:1 toluene/ethyl acetate to afford 39.0 mg (30%) of the 9H-4-methoxy-5-carbomethoxy carbazole as a tan solid. $^1H$ NMR ($CDCl_3$) δ 8.25 (s, 1H), 7.4-7.2 (m, 4H), 7.05 (d, 1H, J=8 Hz), 6.65 (d, 1H, J=8 Hz), and 4.05 (s, 6H). IR ($CHCl_3$, $cm^{-1}$) 3274 (br) , 1706, 1602, 1583, 1456, 1431, 1351, 1333, 1294, 1239, 1198, 1175, 1144, 1103, 781, and 724. MS (ES) m/e 256.

| Elemental Analyses for $C_{15}H_{13}NO_3$: | | | |
|---|---|---|---|
| Calculated | C, 70.58; | H, 5.13; | N, 5.49. |
| Found | C, 70.85; | H, 5.29; | N, 5.29. |

C. 9-[(2-Biphenyl)methyl]-4-methoxy-5-carbomethoxy carbazole

A solution of the 9H-4-methoxy-5-carbomethoxy carbazole (727 mg, 2.85 mM) in 15 mL DMF was added to 60% NaH mineral oil dispersion (342 mg, 8.56 mM, washed twice with hexane) at room temperature. Following cessation of gas evolution, 2-(bromomethyl)biphenyl (0.79 mL, 4.19 mM) was added and the mixture stirred at room temperature for 19 hours. The mixture was diluted with ethyl acetate and $H_2O$. The ethyl acetate layer was washed with with $H_2O$, 1 N HCl, $H_2O$, sat. $NaHCO_3$, and saturated brine, dried over magnesium sulfate, filtered, and concentrated to afford 1.2 g (100%) of the 9-[(2-biphenyl)methyl]-4-methoxy-5-carbomethoxy carbazole as a yellow solid. $^1H$ NMR ($CDCl_3$) δ 7.6-7.2 (m, 11H), 7.05 (t, 1H, J=8 Hz), 6.8 (d, 1H, J=8 Hz), 6.65 (d, 1H, J=8 Hz), 6.6 (d, 1H, J=8 Hz), 5.4 (s, 2H) and 4.0 (s, 6H). IR (KBr, $cm^{-1}$) 1727. MS (ES) m/e 422.

| Elemental Analyses for $C_{28}H_{23}NO_3$: | | | |
|---|---|---|---|
| Calculated | C, 79.79; | H, 5.50; | N, 3.32. |
| Found | C, 79.53; | H, 5.61; | N, 3.15. |

D. 9-[(2-Biphenyl)methyl]-4-hydroxy-5-carbomethoxy carbazole

Boron tribromide (1.0 M in methylene chloride, 1.69 mL, 1.69 mM) was slowly added to a solution of 9-[(2-biphenyl)methyl]-4-methoxy-5-carbomethoxy carbazole (547 mg, 1.3 mM) in 5 mL methylene chloride at -10 °C. After 2 hours, the mixture was quenched with methanol (1.31 mL, 32.5 mM) and stirred at room temperature for 5 hours. The mixture was diluted with ethyl acetate, washed with $H_2O$, 1 N HCl, $H_2O$, sat. $NaHCO_3$, and saturated brine, dried over magnesium sulfate, filtered, and concentrated. The residue was purified by column chromatography on silica gel (elution with methylene chloride) to afford 445 mg (84%) of the 9-[(2-biphenyl)methyl]-4-hydroxy-5-carbomethoxy carbazole as a yellow foam. $^1H$ NMR ($CDCl_3$) δ 10.35 (s, 1H), 7.95 (d, 1H, J=8 Hz), 7.6-7.2 (m, 10fH), 7.05 (t, 1H, J=8 Hz), 6.8 (m, 2H), 6.6 (d, 1H, J=8 Hz), 5.4 (s, 2H), and 4.1 (s, 3H). IR (KBr, $cm^{-1}$) 3200 (br), 1680, 1596, 1451, 1439, 1427, 1333, 1262, 1217, 1137, 752, 713, 1763 and 703. MS (ES) m/e 406, 408.

| Elemental Analyses for $C_{27}H_{21}NO_3$: | | | |
|---|---|---|---|
| Calculated | C, 79.59; | H, 5.19; | N, 3.44. |
| Found | C, 80.62; | H, 5.73; | N, 3.44. |

E. 9-[(2-Biphenyl)methyl]-4-hydroxy-5-carbamoyl carbazole

A solution of the 9-[(2-biphenyl)methyl]-4-hydroxy-5-carbomethoxy carbazole (407.5 mg, 1.0 mM) in 5 mL THF and 20 mL concentrated aqueous ammonium hydroxide was sonicated for 28.5 hours at 40-50 °C. The mixture was diluted with ethyl acetate and acidified to pH 1 with 5 N HCl. The aqueous layer was extracted three times with ethylacetate. The combined organic extracts were washed with $H_2O$ and saturated brine, dried over magnesium sulfate, filtered, and concentrated. The residue was purified by column chromatography on silica gel (elution with gradient methylene chloride/ethyl acetate) to afford 165 mg (42%) of the 9-[(2-biphenyl)methyl]-4-hydroxy-5-carbamoyl carbazole as a yellow solid. $^1H$ NMR (DMSO-d6) δ 10.45 (s, 1H), 8.8 (br s, 1H), 8.4 (br s, 1H), 7.6-7.2 (m, 11H), 7.05 (t, 1H, J=8 Hz), 6.75 (d, 1H, J=8 Hz), 6.55 (d, 1H, J=8 Hz), 6.35 (d, 1H, J=8 Hz), and 5.55 (s, 2H). IR (KBr, $cm^{-1}$) 3451, 3331, and 1639. MS (ES) m/e 391, 393.

| Elemental Analyses for $C_{26}H_{20}N_2O_2$: | | | |
|---|---|---|---|
| Calculated | C, 79.57; | H, 5.14; | N, 7.14. |
| Found | C, 79.60; | H, 5.37; | N, 6.90. |

F. {9-[(2-Biphenyl)methyl]-5-carbamoylcarbazol-4-yl}oxyacetic acid, tert-butyl ester

40% Methanolic Triton B (0.2 mL, 0.44 mM) was added to a solution of the 9-[(2-biphenyl)methyl]-4-hydroxy-5-carbamoyl carbazole (141 mg, 0.36 mM) in 5 mL DMF at room temperature. After 5 minutes, t-butyl bromoacetate (107 mg, 0.54 mM) was added and the resultant mixture stirred at room temperature for 6.5 hours. The mixture was diluted with ethyl acetate, washed with $H_2O$, 1 N HCl, $H_2O$, sat. $NaHCO_3$, and saturated brine, dried over magnesium sulfate, filtered, and concentrated. The residue was purified by column chromatography on silica gel (elution with gradient methylene chloride/ethyl acetate) to afford 140 mg (76%) of the {9-[(2-biphenyl)methyl]-5-carbamoylcarbazol-4-yl}oxyacetic acid, tert-butyl ester as a white foam. $^1H$ NMR ($CDCl_3$) $\delta$ 7.6-7.2 (m, 13H), 7.05 (t, 1H, J=8 Hz), 6.85 (d, 1H, J=8 Hz), 6.6 (d, 1H, J=8 Hz), 6.55 (d, 1H, J=8 Hz), 5.4 (s, 2H), 4.8 (s, 2H), and 1.45 (s, 9H). IR ($CHCl_3$, $cm^{-1}$) 1753 and 1674. MS (ES) m/e 507.

| Elemental Analyses for $C_{32}H_{30}N_2O_4$: | | | |
|---|---|---|---|
| Calculated | C, 75.87; | H, 5.97; | N, 5.53. |
| Found | C, 76.10; | H, 6.12; | N, 5.37. |

G. {9-[(2-Biphenyl)methyl]-5-carbamoylcarbazol-4-yl}oxyacetic acid

A solution of the {9-[(2-biphenyl)methyl]-5-carbamoylcarbazol-4-yl}oxyacetic acid, tert-butyl ester (116 mg, 0.23 mM) in 3 mL of trifluoroacetic acid was stirred at room temperature for 2 hours. The solvent was removed *in vacuo*. The residue was triturated with ethyl ether/hexanes, then dried *in vacuo* to afford 103 mg (100%) of the {9-[(2-biphenyl) methyl]-5-carbamoylcarbazol-4-yl}oxyacetic acid as a white powder. $^1H$ NMR (DMSO-d6) $\delta$ 12.95 (br s, 1H), 7.75 (s, 1H), 7.65 (d, 2H, J=8 Hz), 7.55 (t, 2H, J=8 Hz), 7.4 (s, 1H), 7.35-7.2 (m, 6H), 7.05 (m, 2H), 6.9 (d, 1H, J=8 Hz), 6.6 (d, 1H, J=8 Hz), 6.4 (d, 1H, J=8 Hz), 5.55 (s, 2H), and 4.8 (s, 2H). IR (KBr, $cm^{-1}$) 3400, 3200, 1736, 1636, 1618, 1583, 1499, 1455, 1433, 1329, 1249, 1155, 753, and 713. MS (ES) m/e 449, 451.

| Elemental Analyses for $C_{28}H_{22}N_2O_4$: | | | |
|---|---|---|---|
| Calculated | C, 74.65; | H, 4.92; | N, 6.22. |
| Found | C, 75.47; | H, 4.77; | N, 6.24. |

EXAMPLE 45

Esterification of {9-[(2-Biphenyl)methyl]-5-carbamoylcarbazol-4-yl}oxyacetic acid to the {9-[(2-Biphenyl)methyl]-5-carbamoylcarbazol-4-yl}oxyacetic acid, methyl ester

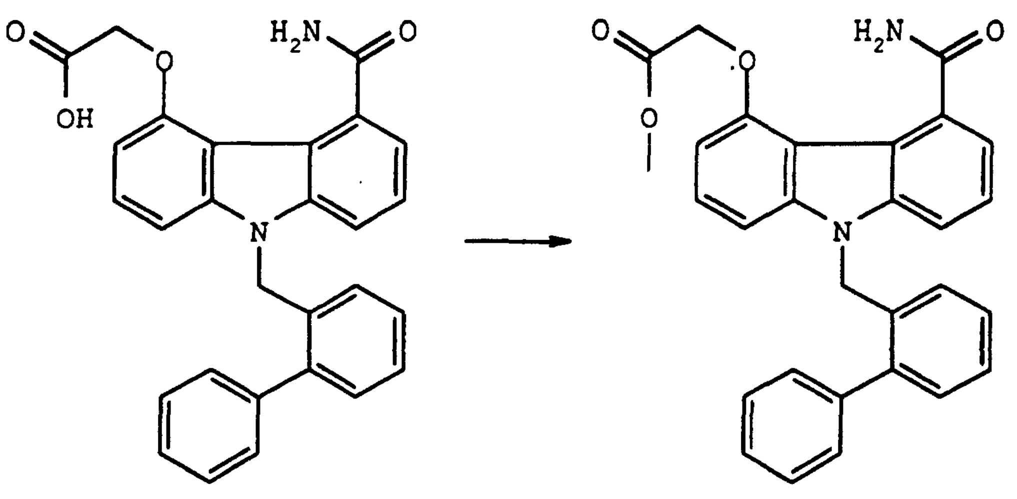

A suspension of the {9-[(2-biphenyl)methyl]-5-carbamoylcarbazol-4-yl}oxyacetic acid (35 mg, 0.08 mM), iodomethane (12 mg, 0.09 mM), and potassium carbonate (13 mg, 0.09 mM) in 2 mL DMF at room temperature for 4.5 hours. The mixture was diluted with ethyl acetate, washed with $H_2O$, sat. $NaHCO_3$, $H_2O$, and saturated brine, dried over magnesium sulfate, filtered, and concentrated to afford 36 mg (100%) of the {9-[(2-biphenyl)methyl]-5-carbamoyl-carbazol-4-yl}oxyacetic acid, methyl ester as a white solid. $^1H$ NMR ($CDCl_3$) δ 7.6-7.2 (m, 11H), 7.1 (t, 1H, J=8 Hz), 6.85 (d, 1H, J=8 Hz), 6.6 (d, 1H, J=8 Hz), 6.55 (d, 1H, J=8 Hz), 5.8 (br s, 2H), 5.4 (s, 2H), 4.8 (s, 2H), and 3.75 (s, 3H). IR (KBr, $cm^{-1}$) 1750 and 1666. MS (ES) m/e 465.

| Elemental Analyses for $C_{29}H_{24}N_2O_4$: | | | |
|---|---|---|---|
| Calculated | C, 74.98; | H, 5.21; | N, 6.03. |
| Found | C, 75.09; | H, 5.57; | N, 5.63. |

Example 46

Preparation of [9-Benzyl-4-carbamoyl-1,2,3,4-tetrahydrocarbazole-5-yl]oxyacetic acid

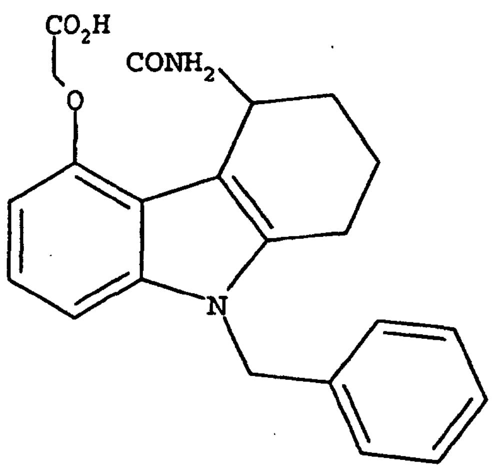

A. 9-Benzyl-4-carboxy-5-methoxy-1,2,3,4-tetrahydrocarbazole, Ethyl Ester.

A solution of 1.50 g (4.02 mmol) of 9-Benzyl-4-carboxy-8-chloro-5-methoxy-1,2,3,4-tetrahydrocarbazole, ethyl ester and 0.45 g (4.40 mmol) of $Et_3N$ in 25 mL of EtOH was treated with 0.24 g of 5% Pd-C and the mixture hydrogenated at 60 pounds per square inch for 16 hours. The reaction was filtered and concentrated *in vacuo* to give 1.40 g of a tan solid. $^1H$ NMR ($CDCl_3$) δ 7.30-7.19 (m, 3H), 7.03-6.95 (m, 3H), 6.80 (d, 1H, J=8.1 Hz), 6.44 (d, 1H, J=7.7 Hz), 5.22 (d, 2H, J=5.9 Hz), 4.22-4.11 (m, 3H), 3.82 (s, 3H), 2.75-2.64 (m, 1H), 2.59-2.48 (m, 1H), 2.11-1.64 (m, 4H), and 1.25 (t, 3H, J=7.0 Hz). IR ($CHCl_3$) 2959, 1725, 1499, 1453, 1260, 1178, 1128 cm-1;

| Elemental Analyses for $C_{23}H_{25}NO_3$: | |
|---|---|
| Calculated | 363.1836 |
| Found | 363.1834. |

B. 9-Benzyl-4-carbamoyl-5-hydroxy-1,2,3,4-tetrahydrocarbazole.

A 0 °C solution of 1.00 g (2.80 mmol) 9-benzyl-4-carboxy-5-methoxy-1,2,3,4-tetrahydrocarbazole, ethyl ester in 15 mL of $CH_2Cl_2$ was treated with 22.40 mL (22.40 mmol; 1M in $CH_2Cl_2$) of $BBr_3$. The cold bath was removed and the reaction stirred until tlc analysis (10% EtOAc in hexanes) indicated complete consumption of starting material (1.5 hours). The reaction was cooled to 0 °C and was quenched with 5.0 mL of MeOH. The mixture was stirred at ambient temperature for 18 hours and was concentrated *in vacuo*. The black oil was taken up in 200 mL of $CH_2Cl_2$ and the solution washed with H20 (100 mL) and saturated aqueous $NaHCO_3$ (100mL). Evaporation of the solvent *in vacuo*

afforded 700 mg of a black oil. Purification by radial chromatography (10 % EtOAc in hexanes) afforded 400 mg of 9-benzyl-4-carboxy-5-hydroxy-1,2,3,4-tetrahydrocarbazole, ethyl ester which was taken on directly to the next reaction

The phenol was taken up in 40 ml of THF and the solution treated with 10 mL of $NH_4OH$. The reaction vessel was capped and the mixture stirred vigorously for 13 days. The reaction was poured into $H_2O$ and the mixture extracted with EtOAc (3 x 150 mL). The combined organic layers were dried ($Na_2SO_4$), filtered and concentrated *in vacuo* to give 300 mg of a brown foam. Radial chromatography (3% MeOH in $CH_2Cl_2$) afforded 50 mg of starting phenol and 80 mg (0.03 mmol; 22 %) of 9-benzyl-4-carbamoyl-5-hydroxy-1,2,3,4-tetrahydrocarbazole. $^1H$ NMR ($CDCl_3$) δ 7.33-7.24 (m, 3H), 7.06-6.97 (m, 3H), 6.81 (d, 1H, J=8.1 Hz), 6.56 (d, 1H, J=7.5 Hz), 5.22 (d, 2H, J=2.2 Hz), 4.20-4.15 (m, 1H), 2.78-2.67 (m, 1H), 2.63-2.51 (m, 1H), 2.35-2.27 (m, 1H), and 2.09-1.91 (m, 3H), no phenol proton detected. IR (CHCl3) 3007, 1667, 1586, 1567, 1496, 1266 cm-1;

| Elemental Analyses for $C_{20}H_{21}N_2O_2$: | |
|---|---|
| Calculated | 321.1603. |
| Found | 321.1607. |

C. [9-Benzyl-4-carbamoyl-1,2,3,4-tetrahydrocarbazole-5-yl]oxyacetic acid, Methyl Ester.

A solution of 80 mg (0.25 mmol) of 9-benzyl-4-carbamoyl-5-hydroxy-1,2,3,4-tetrahydrocarbazole in 2.5 mL of DMF was treated with 61 mg (0.30 mmol) of $Cs_2CO_3$ followed by 26 mg (0.30 mmol) of methyl bromoacetate. The mixture was stirred at room temperature until tlc indicated complete consumption of starting material (2 hours). The reaction was diluted with $H_2O$ (10 mL) and was extracted with EtOAc (3x10 mL). The combined organic layers were washed with $H_2O$ (3 x2 0 mL), dried over $Na_2SO_4$, filtered, and concentrated *in vacuo*. The residue was purified by radial chromatography ($SiO_2$; 2.5% MeOH in $CH_2Cl_2$) to afford 50 mg (0.13 mmol; 51%) of [9-benzyl-4-carbamoyl-1,2,3,4-tetrahydrocarbazole-5-yl]oxyacetic acid, methyl ester as an oil. $^1H$ NMR ($CDCl_3$) δ 7.33-7.21 (m, 3H), 7.05-6.98 (m, 3H), 6.98 (d, 1H, J=7.4 Hz), 6.46 (br s, 1H), 6.37 (d, 1H, J=7.7 Hz), 5.52 (br s, 1H), 5.23 (d, 1H, J=4.9 Hz) 4.79-4.70 (m, 2H), 4.20-4.15 (m, 1H), 3.81 (s, 3H), 2.79-2.69 (m, 1H), 2.63-2.49 (m, 1H), 2.43-2.35 (m, 1H), 2.25-2.09 (m, 1H), and 1.99-1.78 (m, 2H). IR ($CHCl_3$, $cm^{-1}$) 1759, 1670, 1497, 1453, 1440, and 1132. MS (ES) m/e 393 (M+1).

| Elemental Analyses for $C_{23}H_{24}N_2O_4$: | | | |
|---|---|---|---|
| Calculated | C, 70.39; | H, 6.16; | N, 7.14. |
| Found | C, 70.29; | H, 6.31; | N, 7.08. |

D. [9-Benzyl-4-carbamoyl-1,2,3,4-tetrahydrocarbazole-5-yl]oxyacetic acid

A solution of 30 mg (0.076 mmol) of [9-benzyl-4-carbamoyl-1,2,3,4-tetrahydrocarbazole-5-yl]oxyacetic acid, methyl ester in 1.0 mL of THF and 1.0 mL of MeOH was treated with 0.2 mL of 1 N aqueous LiOH (0.2 mmol). The mixture was stirred for 18 hours. An additional 0.2 mL of 1 N aqueous LiOH (0.2 mmol) was added and stirring continued. After 1 hour, the mixture was concentrated *in vacuo*. The residue was dissolved in 2.0 mL of $H_2O$ and the solution acidified with 0.2 N aqueous HCl. The solid was filtered and dried to afford 25 mg of [9-benzyl-4-carbamoyl-1,2,3,9-tetrahydrocarbazole-5-yl]oxyacetic acid as a white solid. $^1H$ NMR (DMSO-d6) δ 7.36-7.12 (m, 5H), 7.05-6.83 (m, 5H), 6.71 (br s, 1H), 6.35 (d, 1H, J=7.6 Hz), 5.27 (s, 2H), 4.64 (s, 2H), 3.93-3.84 (m, 2H), 2.75-2.64 (m, 1H), 2.16-1.95 (m, 2H), 1.81-1.64 (m, 2H) and 1 proton masked by $H_2O$ peak between 2.58-2.40. IR (KBr, $cm^{-1}$) 3435, 2936, 1722, 1644, 1586, 1566, 1495, 1451, 1354, 1227, 1134, 730, 716, and 698. MS (ES) m/e 377 (M-1) and 379 (M+1).

| Elemental Analyses for $C_{22}H_{22}N_2O_4$: | | | |
|---|---|---|---|
| Calculated | C, 69.83; | H, 5.86; | N, 7.40. |
| Found | C, 70.11; | H, 5.76; | N, 7.12. |

EXAMPLE 47

Preparation of {9-[(2-Pyridyl)methyl]-5-carbamoylcarbazol-4-yl}oxyacetic acid

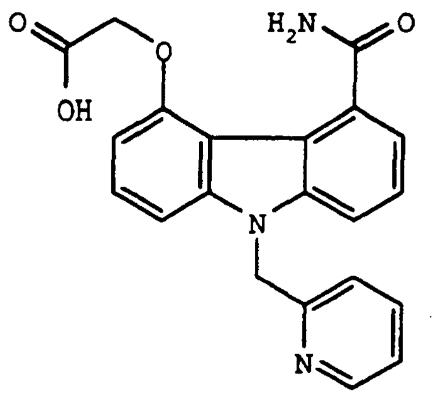

A. 9-[(2-Pyridyl)methyl]-5-carbomethoxy-1,2-dihydrocarbazol-4(3H)-one

A 0 °C suspension of 5-carbornethoxy-1,2-dihydro-9H-carbazol-4(3H)-one (1.50 g, 6.17 mmol), potassium carbonate (2.60 g, 18.8 mmol), and catalytic amount of sodium iodide (ca. 10 mg), was treated with 2-picolyl chloride hydrochloride (1.10 g, 6.70 mmol). The cold bath was removed and the reaction stirred at ambient temperature 72 hours. The reaction was poured into $H_2O$ (100 mL) and the mixture extracted four times with ethyl acetate. The combined organic layers were washed four times with $H_2O$, once with saturated brine, dried over anhydrous sodium sulfate, filtered, and concentrated. The residue was purified by flash chromatography on silica gel (elution with 70% then 80% then 85% EtOAc in hexanes) to afford 1.70 g (82%) of the 9-[(2-pyridyl)methyl]-5-carbomethoxy-1,2-dihydrocarbazol-4(3H)-one as an oil which solidified on standing. $^1H$ NMR ($CDCl_3$) δ 8.52 (br s, 1H), 7.54-7.47 (m, 1H), 7.34-7.26 (m, 2H), 7.18-7.11 (m, 2H), 6.67 (d, J=7.8 Hz, 1H) 5.34 (s, 2H), 3.99 (s, 3H), 2.87 (t, 2H, J=6.0 Hz), 2.50 (t, 2H, J=6.3 Hz), and 2.20-2.13 (m , 2H). IR ($CHCl_3$, $cm^{-1}$) 3010, 2953, 1725, 1654, 1463, 1446, 1288 and 1121. MS (ES) m/e 335 (M+1).

| Elemental Analyses for $C_{20}H_{18}N_2O_3$: | | | |
|---|---|---|---|
| Calculated | C, 71.84; | H, 5.43; | N, 8.38. |
| Found | C, 71.70; | H, 5.49; | N, 8.37. |

B. 9-[(2-Pyridyl)methyl]-4-hydroxy-5-carbomethoxy carbazole

A solution of the 9-[(2-pyridyl)methyl]-5-carbomethoxy-1,2-dihydrocarbazol-4(3H)-one (500 mg, 1.50 mmol) in 2 mL of dioxane was treated with sodium hydride (140 mg; 3.50 mmol; 60% dispersion in mineral oil) and the mixture stirred for 15 minutes. Methyl benzenesulfinate (0.32 mL; 2.45 mmol) was added dropwise and the reaction stirred at room temperature. After 0.5 hours, gas evolution began and the reaction turned dark brown. The mixture was stirred until tlc indicated complete consumption of starting material (1 hour) at which time glacial acetic acid (0.20 mL; 3.50 mmol) was added. An additional 2 mL of dioxane was added to assist stirring and the mixture was heated to mild reflux for 1 hour. The reaction was cooled and diluted with EtOAc (50 mL). The organic layer was separated, washed once with saturated aqueous. $NaHCO_3$ and once with saturated brine, dried over anhydrous sodium sulfate, filtered, and concentrated. The residue was purified by radial chromatography on silica gel (elution with 20% ethyl acetate in hexanes) to afford 470.0 mg (94%) of the 9-[(2-pyridyl)methyl]-4-hydroxy-5-carbomethyoxy carbazole as a solid. $^1H$ NMR ($CDCl_3$) δ 10.37 (s, 1H), 8.61 (d, 1H, J=3.7 Hz), 8.01 (d, 1H, J=7.8 Hz), 7.63 (d, 1H, J=8.3 Hz), 7.47-7.39 (m, 3H), 7.19-7.14 (m, 1H), 6.94 (d, 1H, J=8.3 Hz), 6.84 (d, 1H, J=8.3 Hz), 6.59 (d, 1H, J=7.8 Hz), 5.66 (s, 2H), and 4.10 (s, 3H). IR ($CHCl_3$, $cm^{-1}$) 3200 (br), 1686, 1597, 1442, 1428, 1332, 1286, and 1268. MS (ES) m/e 333 (M+1).

| Elemental Analyses for $C_{20}H_{16}N_2O_3$: | | | |
|---|---|---|---|
| Calculated | C, 72.28; | H, 4.85; | N, 8.43. |

(continued)

| Elemental Analyses for $C_{20}H_{16}N_2O_3$: | | | |
|---|---|---|---|
| Found | C, 72.44; | H, 4.79; | N, 8.44. |

c. 9-[(2-Pyridyl)methyl]-4-hydroxy-5-carbamoyl carbazole

A solution of the 9-[(2-pyridyl)methyl]-4-hydroxy-5-carbomethoxy carbazole (480 mg, 1.43 mmol) in 10 mL THF and 40 mL concentrated aqueous ammonium hydroxide was treated with a stream of $NH_3$ gas to ensure saturation. The reaction vessel was capped and the mixture heated to 35 °C with stirring until tlc indicated complete consumption of starting material (20 hours). The THF was evaporated and the aqueous layer saturated with solid sodium chloride. The mixture was extracted three times with THF. The combined organic layers were dried over anhydrous sodium sulfate, filtered and concentrated. The foam was taken up in hot ethyl acetate and passed over a shoroom temperaturecolumn of silica gel using ethyl acetate as the eluent to afford 247 mg (54%) of the 9-[(2-pyridyl)methyl]-4-hydroxy-5-carbamoyl carbazole as a white solid. $^1H$ NMR (DMSO-d6) δ 10.46 (s, 1H), 8.81 (br s, 1H), 8.46 (d, 1H, J=5.8 Hz), 8.36 (br s, 1H), 7.8 (dd, 1H, J=2.9 and 6.4 Hz), 7.67-7.59 (m, 1H), 7.47-7.41 (m, 2H), 7.30-7.20 (m, 2H), 7.05 (d, 1H, J=7.9 Hz), 6.87 (d, 1H, J=8.3 Hz), 6.57 (d, 1H, J=7.8 Hz), and 5.73 (s, 2H). IR (KBr, $cm^{-1}$) 3404, 3051, 1652, 1618, 1595, 1582, 1567, 1559, 1450, 1436, 1334, 1266, 1226, 776, 763 and 647. MS (ES) m/e 318 (M+1).

| Elemental Analyses for $C_{19}H_{15}N_3O_2$: | | | |
|---|---|---|---|
| Calculated | C, 71.91; | H, 4.76; | N, 13.24. |
| Found | C, 72.11; | H, 4.70; | N, 12.95. |

D. {9-[(2-Pyridyl)methyl]-5-carbamoylcarbazol-4-yl}oxyacetic acid, methyl ester

A mixture of the 9-[(2-pyridyl)methyl]-4-hydroxy-5-carbamoyl carbazole (216 mg, 0.68 mmol) and $Cs_2CO_3$ (550 mg; 1.69 mmol) in 5 mL DMF was treated with methyl bromoacetate (0.08 mL; 0.85 mmol). The reaction was stirred until tlc analysis indicated complete consumption of starting material (2 hours). The mixture was concentrated and the residue taken up in $H_2O$ (50 mL). The aqueous layer was saturated with solid NaCl and was extracted five times with THF. The combined organic layers were dried over anhydrous sodium sulfate, filtered and concentrated. The solid was triturated with EtOAc to afford 205 mg (77%) of the {9-[(2-pyridyl)methyl]-5-carbamoylcarbazol-4-yl} oxyacetic acid, methyl ester as an off white solid. $^1H$ NMR (DMSO-d6) δ 8.47 (d, 1H, J=4.9 Hz), 7.66-7.57 (m, 2H), 7.48 (br s, 1H), 7.38-7.27 (m, 2H), 7.24-7.19 (m, 2H), 7.19 (br s, 1H), 7.04 (d, 1H, J=7.3 Hz), 6.87 (d, 1H, J=7.8 Hz), 6.56 (d, 1H, J=7.8 Hz), 5.71 (s, 2H), 4.89 (s, 2H), and 3.69 (s, 3H). IR ($CHCl_3$, $cm^{-1}$) 3380, 3140, 1737, 1675, 1500, 1457, 1354, 1340, 1242, 1158, 772, and 715. MS (ES) m/e 390 (M+1).

| Elemental Analyses for $C_{22}H_{19}N_3O_4$: | | | |
|---|---|---|---|
| Calculated | C, 67.86; | H, 4.92; | N, 10.79. |
| Found | C, 67.75; | H, 5.08; | N, 10.66. |

E. {9-[(2-Pyridyl)methyl]-5-carbamoylcarbazol-4-yl}oxyacetic acid, hydrochloride

A slurry of the {9-[(2-pyridyl)methyl]-5-carbamoylcarbazol-4-yl}oxyacetic acid, methyl ester (75.0 mg, 0.19 mmol) in 1.3 mL of THF and 0.4 mL of MeOH was treated with 0.4 mL of 1 N aqueous LiOH (0.4 mmol) and the mixture stirred at room temperature for 16 hours. The reaction was concentrated and the residue purified by reverse phase chromatography (Vydac C18 column using a 5% to 40% gradient of 0.01% HCl in acetonitrile in 0.01% HCl in $H_2O$. The fractions containing product were lyopholized to afford 75 mg (96%) of {9-[(2-pyridyl)methyl]-5-carbamoylcarbazol-4-yl}oxyacetic acid hydrochloride as a white powder. $^1H$ NMR (DMSO-d6) δ $^1H$ NMR (DMSO-d6) δ 8.50-8.46 (m, 1H), 7.71 (br s, 1H), 7.62-7.67 (m, 1H), 7.58 (d, 1H, J=8.3 Hz), 7.38 (br s, 1H), 7.42-7.29 (m, 3H), 7.26-7.19 (m, 2H), 7.06 (d, 1H, J=7.3 Hz), 6.87 (d, 1H, J=7.8 Hz), 6.58 (d, 1H, J=8.3 Hz) 5.73 (s, 2H) and 4.80 (s, 2H), no acid proton detected. IR (KBr, $cm^{-1}$) 3381, 1716, 1637, 1593, 1580, 1499, 1454, 1430, 1330, 1287, 1157, 1093, 776 and 720. MS (ES) m/e376 (M+1).

| Elemental Analyses for $C_{21}H_{17}N_3O_4$.HCl: | | | |
|---|---|---|---|
| Calculated | C, 61.24; | H, 4.41; | N, 10.20. |

(continued)

| Elemental Analyses for $C_{21}H_{17}N_3O_4$.HCl: | | | |
|---|---|---|---|
| Found | C, 61.11; | H, 4.25; | N, 10.23. |

EXAMPLE 48

Preparation of {9-[(3-Pyridyl)methyl]-5-carbamoylcarbazol-4-yl}oxyacetic acid

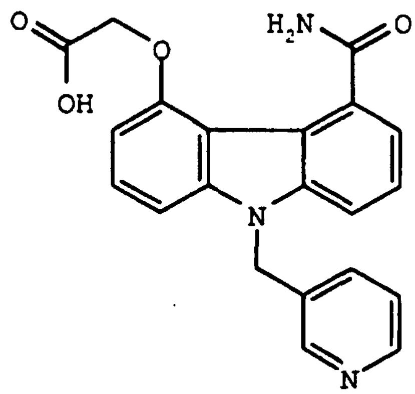

A. 9-[(3-Pyridyl)methyl]-5-carbomethoxy-1,2-dihydrocarbazol-4(3H)-one.

A suspension of 5-carbomethoxy-1,2-dihydro-9H-carbazol-4(3H)-one (500 mg, 2.06 mmol), potassium carbonate (860 mg, 18.8 mmol), and catalytic amount of sodium iodide (ca. 10 mg), was treated with 3-picolyl chloride hydrochloride (500 mg, 3.05 mmol). The reaction was stirred at ambient temperature 19.5 hours. The mixture was poured into $H_2O$ (100 mL) and the mixture extracted four times with ethyl acetate. The combined organic layers were washed four times with $H_2O$, once with saturated brine, dried over anhydrous sodium sulfate, filtered, and concentrated. The residue was purified by flash chromatography on silica gel (elution with 5% MeOH in EtOAc) to afford 550 mg (80%) of the 9-[(3-pyridyl)methyl]-5-carbomethoxy-1,2-dihydrocarbazol-4(3H)-one as a white solid. $^1H$ NMR ($CDCl_3$) δ 8.48 (d, 1H, J=2.9 Hz), 8.43 (br s, 1H), 7.31 (d, 1H, J=7.3 Hz), 7.25-7.09 (m, 5H), 5.29 (s, 2H), 3.97 (s, 3H), 2.80 (t, 2H, J=6.1 Hz), 2.49 (t, 2H, J=6.4 Hz), and 2.20-2.12 (m , 2H). IR ($CHCl_3$, $cm^{-1}$) 1726, 1656, 1464, 1444, 1434, 1289 and 1119. MS (ES) m/e 335 (M+1).

| Elemental Analyses for $C_{20}H_{18}N_2O_3$: | | | |
|---|---|---|---|
| Calculated | C, 71.84; | H, 5.43; | N, 8.38. |
| Found | C, 70.97; | H, 5.89; | N, 8.53. |

B. 9-[(3-Pyridyl)methyl]-4-hydroxy-5-carbomethoxy carbazole.

A solution of the 9-[(3-pyridyl)methyl]-5-carbomethoxy-1,2-dihydrocarbazol-4(3H)-one (456 mg, 1.31 mmol) in 3 mL of dioxane was treated with sodium hydride (128 mg; 3.20 mmol; 60% dispersion in mineral oil) and the mixture stirred for 15 minutes. Methyl benzenesulfinate (0.32 mL; 2.45 mmol) was added dropwise and the reaction heated to 70 °C until tlc analysis indicated complete consumption of starting material (2 hours). The reaction was cooled and diluted with EtOAc (50 mL). The organic layer was separated, washed once with saturated aqueous. $NaHCO_3$ and once with saturated brine, dried over anhydrous sodium sulfate, filtered, and concentrated. The residue was purified by radial chromatography on silica gel (elution with 20% then 30 % then 50% then 75% ethyl acetate in hexanes) to afford 400 mg (92%) of the 9-[(3-pyridyl)methyl]-4-hydroxy-5-carbomethyoxy carbazole as a yellow solid. $^1H$ NMR (DMSO-d6) δ 10.20 (s, 1H), 8.46 (d, 1H, J=2.0 Hz), 8.39 (d, 1H, J=4.9 Hz), 7.73 (d, 1H, J=8.3 Hz), 7.43-7.37 (m, 2H), 7.28-7.21 (m, 2H), 7.15-7.08 (m, 2H), 6.56 (d, 1H, J=7.8 Hz), 5.67 (s, 2H), and 3.80 (s, 3H). IR (KBr, $cm^{-1}$) 1722, 1585, 1459, 1431, 1331, 1321, 1292, 1278, 1136, 781 and 763. MS (ES) m/e 333 (M+1).

| Elemental Analyses for $C_{20}H_{16}N_2O_3$: | | | |
|---|---|---|---|
| Calculated | C, 72.28; | H, 4.85; | N, 8.43. |
| Found | C, 72.37; | H, 4.67; | N, 8.71. |

C. 9-[(3-Pyridyl)methyl]-4-hydroxy-5-carbamoyl carbazole.

A solution of the 9-[(3-pyridyl)methyl]-4-hydroxy-5-carbomethoxy carbazole (362 mg, 1.09 mmol) in 15 mL THF and 60 mL concentrated aqueous ammonium hydroxide was treated with a stream of $NH_3$ gas to ensure saturation. The reaction vessel was capped and the mixture heated to 35 °C with stirring until tlc indicated complete consumption of starting material (48 hours). The mixture was neutralized to pH 8 with 5 N aqueous HCl, saturated with solid sodium chloride, and extracted twice with THF. The combined organic layers were concentrated. The resulting foam was taken up in a minimal amount of THF and loaded onto a silica gel column which had been pre equilibrated with EtOAc. Elution with EtOAc afforded 255 mg (74%) of the 9-[(3-pyridyl)methyl]-4-hydroxy-5-carbamoyl carbazole as a yellow solid. $^1$H NMR (DMSO-d6) δ 10.46 (s, 1H), 8.79 (br s, 1H), 8.43 (d, 1H, J=1.5 Hz), 8.39 (dd, 1H, J=1.4 and 4.9 Hz), 8.35 (br s, 1H), 7.84 (d, 1H, J=7.8 Hz), 7.48-7.22 (m, 6H), 7.13 (d, 1H, J=8.3 Hz), 6.58 (d, 1H, J=7.8 Hz), and 5.73 (s, 2H). IR (KBr, $cm^{-1}$) 3436, 3198 (br), 1629, 1619, 1599, 1580, 1564, 1547, 1444, 1433, 1329, 1263, and 776. MS (ES) m/e 318 (M+1).

| Elemental Analyses for $C_{19}H_{15}N_3O_2$: | | | |
|---|---|---|---|
| Calculated | C, 71.91; | H, 4.76; | N, 13.24. |
| Found | C, 72.10; | H, 4.66; | N, 13.19. |

D. {9-[(3-Pyridyl)methyl]-5-carbamoylcarbazol-4-yl}oxyacetic acid, methyl ester.

A mixture of the 9-[(3-pyridyl)methyl]-4-hydroxy-5-carbamoyl carbazole (225 mg, 0.71 mmol) and $Cs_2CO_3$ (580 mg; 1.78 mmol) in 5 mL DMF was treated with methyl bromoacetate (0.09 mL; 0.95 mmol). The reaction was stirred until tlc analysis indicated complete consumption of starting material (2 hours). The mixture was concentrated and the residue taken up in $H_2O$ (50 mL). The aqueous layer was saturated with solid NaCl and was extracted five times with THF. The combined organic layers were dried over anhydrous sodium sulfate, filtered and concentrated. The solid was triturated with THF then EtOAc to afford 85 mg of the {9-[(2-pyridyl)methyl]-5-carbamoylcarbazol-4-yl} oxyacetic acid, methyl ester as an off white solid. The mother liquors from the triturations were chromatographed over silica gel using radial chromatography (0.5% then 1% then 2% MeOH in $CHCl_3$) to afford an additional 80 mg of product (165 mg total; 60%). $^1$H NMR ($CDCl_3$) δ 8.53 (br, 2H), 7.42-7.39 (m, 4H), 7.20 (d, 1H, J=7.8 Hz), 7.13 (br s, 1H), 6.98 (d, 1H, J=8.3 Hz), 6.56 (d, 1H, J=7.9 Hz), 6.17 (br s, 1H), 5.91 (br s, 1H), 5.49 (s, 2H), 4.88 (s, 2H), and 3.79 (s, 3H). IR (KBr, $cm^{-1}$) 3367, 3161, 1760, 1733, 1673, 1577, 1501, 1458, 1433, 1418, 1328, 1216, 1202, 1180, 1157, 771, and 714. MS (ES) m/e 373 (M+-$NH_2$) and 390 (M+1).

E. {9-[(3-Pyridyl)methyl]-5-carbamoylcarbazol-4-yl}oxyacetic acid, hydrochloride

A slurry of the {9-[(3-pyridyl)methyl]-5-carbamoylcarbazol-4-yl}oxyacetic acid, methyl ester (85.0 mg, 0.22 mmol) in 1.5 mL of THF and 0.48 mL of MeOH was treated with 0.48 mL of 1 N aqueous LiOH (0.48 mmol) and the mixture stirred at room temperature for 16 hours. The reaction was concentrated and the residue purified by reverse phase chromatography (Vydac C18 column using a 5% to 40% gradient of 0.01% HCl in acetonitrile in 0.01% HCl in $H_2O$. The fractions containing product were lyopholized to afford 63 mg (70%) of {9-[(3-pyridyl)methyl]-5-carbamoyl-carbazol-4-yl}oxyacetic acid hydrochloride as a white powder. $^1$H NMR (DMSO-d6) δ 8.62-8.57 (m, 2H), 7.77-7.67 (m, 3H), 7.62-7.54 (m, 1H), 7.43-7.28 (m, 4H), 7.09 (d, 1H, J=6.3 Hz), 6.61 (d, 1H, J=7.8 Hz), 5.81 (s, 2H) and 4.80 (s, 2H), no acid proton detected. IR (KBr, $cm^{-1}$) 3424, 3324, 1728, 1671, 1655, 1616, 1595, 1579, 1500, 1456, 1421, 1328, 1203, 1156, and 772. MS (ES) m/e 374 (M-1), 376 (M+1).

| Elemental Analyses for $C_{22}H_{17}N_3O_4$.HCl: | | | |
|---|---|---|---|
| Calculated | C, 61.24; | H, 4.41; | N, 10.20. |
| Found | C, 61.28; | H, 4.25; | N, 10.28. |

Example 49

Preparation of [9-benzyl-4-carbamoyl-8-methyl-1,2,3,4-tetrahydrocarbazol-5-yl]oxyacetic acid

A. Preparation of ethyl 5-methoxy-8-methyl-1,2,3,4-tetrahydrocarbazole-4-carboxylate

A solution of 1.87 g (13.65 mmol) of 2-methyl-5-methoxyaniline and 3.40 g (13.65 mmol) of 2-carboethoxy-6-bromocyclohexanone (Sheehan and Mumaw, JACS, 72, 2127 (1950)) in 10 ml of anhydrous dimethylformamide was heated at 55°C for 13 hours. The reaction mixture was cooled, poured into brine and extracted twice with diethyl ether. The extracts were washed twice with water and then with brine, dried over magnesium sulfate and concentrated. The residue was chromatographed on silica gel using a 10:1 hexane/ethyl acetate mixture to afford 2.88 g (69 %) of a mixture of diastereomers of N-alkylated material. This mixture was refluxed in 90 ml of benzene with 4.69 g (34.4 mmol) of zinc chloride for 10 hours. The solvent was evaporated and the residue was partitioned between 80 ml of 1 N HCl and 80 ml of ethyl acetate and then extracted once more with ethyl acetate. The organic layers were washed with water and then brine, dried over magnesium sulfate and concentrated to afford 2.60 g (95%) of the subtitled compound. m. p. 119-122°C

| Elemental Analyses | | | |
|---|---|---|---|
| Calculated | C 71.06; | H 7.37; | N 4.87 |
| Found | C 71.35; | H 7.25; | N 4.92 |

B. Preparation of ethyl 9-benzyl-5-methoxy-8-methyl-1,2,3,4-tetrahydrocarbazole-4-carboxylate

A solution of 1.58 g of ethyl 5-methoxy-8-methyl-1,2,3,4-tetrahydrocarbazole-4-carboxylate in 5 ml of dimethylformamide was added to 0.24 g of sodium hydride (60% in mineral oil) in 5 ml of dimethylformamide and stirred for 30 minutes at room temperature. Potassium iodide (90 mg) and 0.75 ml of benzyl bromide were then added and the reaction was stirred overnight. The reaction mixture was poured into 75 ml of saturated ammonium chloride solution and then extracted twice with ether. The extracts were washed with water and then with brine, dried over magnesium sulfate and concentrated. The residue was chromatographed on silica gel using hexane/ethyl acetate mixtures to afford 1.09 g (53%) of the subtitle compound. ESIMS *m/e* 378 ($M^+$+1) NMR (300 MHz, $CDCl_3$): δ 7.28-7.19 (m, 3H); 6.84 (d, J=7.4, 2H); 6.67 (d, J=7.8, 1H); 6.33 (d, J=7.9, 1H); 5.55 and 5.39 (ABq, J=7.8, 2H); 4.17 (q+m, J=6.9, 3H); 3.80 (s, 3H); 2.64 (dt, J=16.1, 5.3, 1H); 2.48 (dt, J=16.6, 6.9,1H); 2.41 (s, 3H); 2.05 (m, 2H); 1.95 (m, 1H); 1.83 (m, 1H); 1.25 (t, J=7.3, 3H).

C. Preparation of 9-benzyl-5-methoxy-8-methyl-1,2,3,4-tetrahydrocarbazole-4-carboxamide

A slurry of 0.38 g of ammonium chloride in 15 ml of dry toluene was cooled in an ice bath and treated with 3.5 ml of a 2.0 M solution of trimethylaluminum in toluene. This mixture was stirred for 1 hour at room temperature, whereupon 0.762 g (2.02 mmol) of ethyl 9-benzyl-5-methoxy-8-methyl-1,2,3,4-tetrahydrocarbazole-4-carboxylate in 10 ml of toluene and 1 ml of dichloromethane was added. The mixture was heated to 50°C overnight, cooled and quenched with 20 ml of aqueous 5% HCl solution. Ethyl acetate extracts (3x 100 ml) were washed with water and then with brine, dried over magnesium sulfate and concentrated to afford 0.693 g (98%) of the subtitle compound. ESIMS *m/e* 349 ($M^+$+1) NMR (300 MHz, $CDCl_3$): δ 7.25 (m, 3H); 6.83 (d, J=7.2, 2H); 6.74 (d, J=7.8, 1H); 6.40 (d, J=7.8, 1H); 5.93 (br, 1H); 5.54 and 5.45 (ABq, J=17.7, 2H); 5.42 (br, 1H); 4.14 (br, 1H); 3.87 (s, 3H); 2.65 (dt, J=16.4, 4.1, 1H); 2.55-2.36 (m,2H); 2.45 (s, 3H); 1.97-1.86 (m, 3H).

D. Preparation of [9-benzyl-4-carbamoyl-8-methyl-1,2,3,4-tetrahydrocarbazol-5-yl]oxyacetic acid methyl ester

A solution of 0.661 g of 9-benzyl-5-methoxy-8-methyl-1,2,3,4-tetrahydrocarbazole-4-carboxamide in 50 ml of dry dichloromethane was cooled to -40°C and treated dropwise with 1.8 ml of neat boron tribromide. The reaction was stirred for 2 hours at room temperature and quenched by pouring into ice and adding 1 N HCl solution. This mixture was extracted twice with dichloromethane and the organic layers were dried over magnesium sulfate and concentrated to afford 0.625 g of the demethylated compound.

A solution of 0.55 g of this intermediate in 10 ml of dimethylformamide was cooled in an ice bath and treated with 1.61 g of cesium carbonate and 0.16 ml of methyl bromoacetate. After stirring for 1 hour at room temperature, the reaction mixture was poured into water and extracted twice with ethyl acetate. The extracts were washed with water and then with brine, dried over magnesium sulfate and concentrated. The residue was chromatographed on silica gel

using methanol/ 0-2% in dichloromethane to afford 0.46 g (69%) of the subtitle compound. m.p. 209°C

| Elemental Analyses | | | |
|---|---|---|---|
| Calculated | C 70.92; | H 6.45; | N 6.89 |
| Found | C 70.85; | H 6.19; | N 6.98 |

E. Preparation of [9-benzyl-4-carbamoyl-8-methyl-1,2,3,4-tetrahydrocarbazol-5-yl]oxyacetic acid ]

A slurry of 64 mg (0.157 mmol) of [9-benzyl-4-carbamoyl-8-methyl-1,2,3,4-tetrahydrocarbazol-5-yl]oxyacetic acid methyl ester in 2 ml of tetrahydrofuran and 7 ml of methanol was treated with 0.5 ml of an aqueous 2 N sodium hydroxide solution and stirred overnight at room temperature. The solvent was evaporated and the residue was partitioned between ethyl acetate and 1 N HCl solution. After another extraction with ethyl acetate, the extracts were washed with brine, dried over magnesium sulfate and concentrated to afford a quantitative yield (62 mg) of the title compound. ESIMS *m/e* 393 ($M^+$+1), 391 ($M^+$-1). NMR (300 MHz, $d^6$-DMSO): δ 12.98 (br, 1H); 7.30-7.18 (m, 3H); 6.82 (d+br, J=7.0, 3H); 6.73 (br, 1H); 6.59 (d, J=7.9, 1H); 6.26 (d, J=7.9, 1H); 5.53 and 5.45 (ABq, J=18.1, 2H); 4.62 (s, 2H); 3.96 (br, 1H); 2.63 (m, 1H); 2.43 (m, 1H); 2.34 (s, 3H); 2.04 (m, 2H); 1.78 (m, 2H).

| Elemental Analyses | | | |
|---|---|---|---|
| Calculated | C 70.39; | H 6.16; | N 7.14 |
| Found | C 70.41; | H 6.44; | N 6.88 |

Example 50

Preparation of [9-benzyl-5-carbamoyl-1-methylcarbazol-4-yl]oxyacetic acid

A. Preparation of 5-carbamoyl-4-methoxy-1-methylcarbazole

A solution of 0.805 g of 9-benzyl-5-methoxy-8-methyl-1,2,3,4-tetrahydrocarbazole-4-carboxamide in 24 ml of carbitol was treated with 1.1 g of 5% palladium on carbon and was refluxed for 6 hours open to the air. After cooling, the solution was filtered thourough a pad of celite and the pad was washed with ethyl acetate. The filtrates were diluted with ether and washed four times with water and dried over magnesium sulfate and concentrated. The residue was chromatographed on silica gel using methanol/0-4% in dichloromethane to afford 0.166 g (28%) of debenzylated carbazole. ESIMS *m/e* 255 ($M^+$+1), 253 ($M^+$-1) NMR (300 MHz, $CDCl_3$): δ 8.13 (br, 1H); 7.51 (d, J=8.1, 1H); 7.40 (t, J=7.6, 1H); 7.32 (d, J=7.2, 1H); 7.18 (d, J=7.8, 1H); 6.60 (d, J=8.0, 1H); 5.68 (br, 2H); 3.99 (s, 3H); 2.50 (s 3H).

B. Preparation of 9-benzyl-5-carbamoyl-4-methoxy-1-methylcarbazole

A solution of 0.148 g of 5-carbamoyl-4-methoxy-1-methylcarbazole in 1.1 ml of dimethylformamide was added to 0.026 g of sodium hydride (60% in mineral oil) in 0.4 ml of dimethylformamide and stirred for 60 minutes at room temperature. Benzyl bromide (0.076 ml) was then added and the reaction was stirred overnight. The reaction mixture was poured into 20 ml of saturated ammonium chloride solution and then extracted twice with ethyl acetate. The extracts were washed with water and then with brine, dried over magnesium sulfate and concentrated. The residue was rinsed with hexane and dissolved in dichloromethane, filtered and concentrated to afford 0.21 g of the subtitle compound. FDMS *m/e* 344 ($M^+$)

| Elemental Analyses | | | |
|---|---|---|---|
| Calculated | C 76.72; | H 5.85; | N 8.13 |
| Found | C 75.20; | H 6.19; | N 7.54 |

C. Preparation of [9-benzyl-5-carbamoyl-1-methylcarbazol-4-yl]oxyacetic acid methyl ester]

A solution of 0.23 g of 9-benzyl-5-carbamoyl-4-methoxy-1-methylcarbazole in 4 ml of dimethylformamide was added to a 1 ml solution of sodium ethane thiolate (prepared from 0.116 g of sodium hydride 60% dispersion and 0.22 ml of ethanethiol under nitrogen) and heated at 110°C for 15 hours. The reaction mixture was cooled, poured into 20 ml of 1 N HCl and extracted twice with ethyl acetate. The extracts were washed twice with water and then with brine,

dried over magnesium sulfate and concentrated. The residue was chromatographed on silica gel using methanol/0-1% in dichloromethane to afford 0.146 g (66%) of the demethylated intermediate. A solution of 0.146 g of this intermediate in 1.5 ml of dimethylformamide was added to 0.021 g of sodium hydride (60% in mineral oil) in 0.5 ml of dimethylformamide. After stirring for 10 minutes at room temperature, 0.054 ml of methyl bromoacetate was added. After stirring for 5 hours at room temperature, the reaction mixture was poured into water and extracted twice with ethyl acetate. The extracts were washed with water and then with brine, dried over magnesium sulfate and concentrated. The residue was chromatographed on silica gel using methanol/ 0-2% in dichloromethane to afford 0.10 g (56%) of the subtitle compound. mp. 228-230°C ESIMS *m/e* 403 ($M^{+}$+1)

| Elemental Analyses | | | |
|---|---|---|---|
| Calculated | C 71.63; | H 5.51; | N 6.96 |
| Found | C 71.34; | H 5.60; | N 6.70 |

D. Preparation of [9-benzyl-5-carbamoyl-1-methylcarbazol-4-yl]oxyacetic acid

A slurry of 32 mg (0.0795 mmol) of [9-benzyl-5-carbamoyl-1-methylcarbazol-4-yl]oxyacetic acid methyl ester in 1 ml of tetrahydrofuran and 3.5 ml of methanol was treated with 0.3 ml of an aqueous 2 N sodium hydroxide solution and stirred overnight at room temperature. The solvent was evaporated and the residue was partitioned between 1:1 ethyl acetate/tetrahydrofuran and 0.2 N HCl solution. After another extraction with 1:1 ethyl acetate/tetrahydrofuran, the extracts were washed with brine, dried over magnesium sulfate and concentrated to afford (27 mg) of the title compound. mp. 253-254°C. ESIMS *m/e* 389 ($M^{+}$+1), 387 ($M^{+}$-1) NMR (300 MHz, $d^6$-DMSO): δ 12.83 (br, 1H); 7.75 (br, 1H); 7.53 (d, J=8.2, 1H); 7.41-7.34 (m, 2H); 7.28-7.17 (m, 3H); 7.07 (m, 2H); 6.90 (d, J=7.2, 2H); 6.49 (d, J=8.1, 1H); 5.89 (s, 2H); 4.79 (s, 2H); 2.52 (s, 3H).

Example 51

Preparation of [9-benzyl-4-carbamoyl-8-fluoro-1,2,3,4-tetrahydrocarbazol-5-yl]oxyacetic acid

A. Preparation of (2-chloro-4-fluorophenyl)- ethyl carbonate

A solution of 19.16 g of 2-chloro-4-fluorophenol in 65.4 ml of 2 N aqueous sodium hydroxide solution was cooled in an ice bath and treated dropwise with 16.3 ml of ethyl chloroformate. After stirring at room temperature overnight, the two-phase reaction mixture was diluted with 100 ml of water and extracted with 300 ml of a 1:1 pentane/ ether mixture. The extract was washed three times with 0.02 N sodium hydroxide solution, water and then brine. After drying and evaporation, 27.63 g (97%) of the subtitle compound were obtained. NMR (300 MHz, $CDCl_3$): δ 7.23-7.18 (m, 2H); 7.00 (dt, J=8.4, 2.7, 1H); 4.35 (q, J=7.1, 2H); 1.40 (t, J=7.1, 3H).

B. Preparation of (2-chloro-4-fluoro-5-nitrophenyl)- ethyl carbonate

A solution of 27.63 g of (2-chloro-4-fluorophenyl)-ethyl carbonate in 60 ml of dichloromethane was cooled in an ice bath and treated dropwise with 31.86 g of a 1:2 mixture of fuming nitric acid (90%) and concentrated sulfuric acid. The reaction was stirred for 2 hours at room temperature and then cooled with ice and treated with another 4.5 g of the same nitrating mixture. The reaction was stirred overnight at room temperature, poured into 200 ml of ice and water,and extracted twice with dichloromethane. The extracts were washed with water and then with brine, dried over magnesium sulfate and concentrated to afford 33.01 g (99%) of the subtitle compound. mp. 50-51 C

| Elemental Analyses | | | | |
|---|---|---|---|---|
| Calculated | C 41.01; | H 2.68; | N 5.31; | Cl 13.45 |
| Found | C 41.03; | H 2.59; | N 5.38; | Cl 13.71 |

C. Preparation of 2-chloro-4-fluoro-5-nitroanisole

A solution of 15.0 g of (2-chloro-4-fluoro-5-nitrophenyl)- ethyl carbonate in 100 ml of dimethyl formamide was treated with 18.6 g of cesium carbonate, 7.1 ml of iodomethane and 7 ml of methanol and stirred overnight at room temperature. The reaction mixture was poured into water and extracted twice with ether. The extracts were washed twice with water and then with brine, dried over magnesium sulfate and concentrated to afford 11.4g of the subtitle

compound. mp. 69-70°C Ex. 57, C.

| Elemental Analyses | | | | |
|---|---|---|---|---|
| Calculated | C 40.90; | H 2.45; | N 6.81; | Cl 17.25 |
| Found | C 41.20; | H 2.48; | N 6.70; | Cl 17.44 |

D. Preparation of 2-fluoro-5-methoxyaniline

A solution of 5.63 g of 2-chloro-4-fluoro-5-nitroanisole in 90 ml of ethanol and 5 ml of triethylamine was hydrogenated at room temperature under 60 pounds per square inch with 1.0 g of 5% palladium on carbon for four hours. The catalyst was filtered off and the solvent was evaporated. The residue was slurried in chloroform and filtered thourough a plug of silica gel and then evaporated. This residue was chromatographed on silica gel using hexane/ chloroform mixtures to afford 2.77 g (72%) of the subtitle compound. mp. 253-254°C. NMR (300 MHz, $CDCl_3$): δ 6.88 (dd, J=10.6, 8.9, 1H); 6.32 (dd, J=7.4, 3.0, 1H); 6.20 (dt, J=8.9, 3.2, 1H); 3.73 (s, 3H); 3.72 (br, 2H).

E. Preparation of N-benzyl-2-fluoro-5-methoxyaniline

This procedure was patterned after that of Tietze and Grote, Chem Ber. 126(12), 2733 (1993). A solution of 2.73 g of 2-fluoro-5-methoxyaniline and 2.67 g of benzaldehyde in 48 ml of methanol was treated with 3.43 g of zinc chloride and then cooled in an ice bath. Sodium cyanoborohydride (1.58 g) was added in small poroom temperature ions over 30 minutes and the reaction was stirred for five hours at room temperature. After evaporation of the solvent, the residue was slurried in 40 ml of 1 N sodium hydroxide solution and then extracted twice with ether. The extracts were washed with water and then with brine, dried over magnesium sulfate and concentrated. The residue was recrystallized from hexane to afford 2.61 g and the mother liquors were chromatographed on silica gel using 20:1 hexane/ ether to afford another 1.4 g of the subtitle compound (90%). mp. 56-58°C

| Elemental Analyses | | | |
|---|---|---|---|
| Calculated | C 72.71; | H 6.10; | N 6.06 |
| Found | C 72.51; | H 6.06; | N 5.99 |

F. Preparation of ethyl 9-benzyl-5-methoxy-8-fluoro-1,2,3,4-tetrahydrocarbazole-4-carboxylate

A solution of 0.62 g of N-benzyl-2-fluoro-5-methoxyaniline in 20 ml of dry tetrahydrofuran was cooled in an ice bath and treated with 11.3 ml of 0.5 M potassium bis(trimethylsilyl)amide in toluene. After stirring for 30 minutes, 0.74 g of 2-carboethoxy-6-bromocyclohexanone (Sheehan and Mumaw, JACS, 72, 2127 (1950)) in 4 ml of tetrahydrofuran was added and the reaction was allowed to warm slowly to room temperature over two hours. The reaction was quenched with saturated ammonium chloride solution and extracted twice with ether. The extracts were washed with water and then with brine, dried over magnesium sulfate and concentrated. This residue was chromatographed on silica gel using hexane/ ether mixtures to afford 0.796 g (74%) of N-alkylated intermediate diastereomers. This mixture was refluxed in 20 ml of benzene with 0.99 g of zinc chloride overnight. The solvent was evaporated and the residue was partitioned between 25 ml of 1 N HCl and 25 ml of ethyl acetate and then extracted once more with ethyl acetate. The organic layers were washed with water and then brine, dried over magnesium sulfate and concentrated to afford 0.734 g (96%) of the subtitled compound. ESIMS *m*/*e* 382 ($M^+$+1)

| Elemental Analyses | | | |
|---|---|---|---|
| Calculated | C 72.42; | H 6.34; | N 3.67 |
| Found | C 72.20; | H 6.26; | N 3.70 |

G. Preparation of 9-benzyl-5-methoxy-8-fluoro-1,2,3,4-tetrahydrocarbazole-4-carboxamide

Ethyl 9-benzyl-5-methoxy-8-fluoro-1,2,3,4-tetrahydrocarbazole-4-carboxylate (0.722 g) was treated similarly as described in Example 49, Part C and chromatographed on silica gel using 1% methanol in dichloromethane to afford 0.482 g (72%) of the subtitle compound. ESIMS *m*/*e* 353 ($M^+$+1)

| Elemental Analyses | | | |
|---|---|---|---|
| Calculated | C 71.57; | H 6.01; | N 7.95 |
| Found | C 71.42; | H 5.83; | N 7.75 |

H. Preparation of [9-benzyl-4-carbamoyl-8-fluoro-1,2,3,4-tetrahydrocarbazol-5-yl]oxyacetic acid methyl ester

9-Benzyl-5-methoxy-8-fluoro-1,2,3,4-tetrahydrocarbazole-4-carboxamide (0.170 g) was converted similarly as described in Example 49, Part D and chromatographed on silica gel using methanol/ 0-1% in dichloromethane to afford 85 mg (50%) of the subtitle compound. mp. 183-185°C

| Elemental Analyses | | | |
|---|---|---|---|
| Calculated | C 67.31; | H 5.65; | N 6.82 |
| Found | C 67.58; | H 5.48; | N 6.95 |

I. Preparation of [9-benzyl-4-carbamoyl-8-fluoro-1,2,3,4-tetrahydrocarbazol-5-yl]oxyacetic acid

[9-Benzyl-4-carbamoyl-8-fluoro-1,2,3,4-tetrahydrocarbazol-5-yl]oxyacetic acid methyl ester (71 mg) was hydrolyzed similarly as described in Example 50, Part D to afford 65 mg of the title compound. ESIMS *m/e* 397 ($M^+$+1), 395 ($M^+$-1) NMR (300 MHz, $d^6$-DMSO): δ 13.03 (br, 1H); 7.31-7.19 (m, 3H); 6.97 (d, J=7.4, 2H); 6.95 (br, 1H); 6.70 (d, J=3.8, 1H); 6.67 (dd, J=12.4, 3.9, 1H); 6.28 (dd, J=8.5, 2.6, 1H); 5.39 (ABq, 2H); 4.64 (s, 2H); 3.92 (br, 1H); 2.71 (m, 1H); 2.44 (m, 1H); 2.02 (m, 2H); 1.76 (m, 2H).

Example 52

Preparation of [9-benzyl-5-carbamoyl-1-fluorocarbazol-4-yl]oxyacetic acid

A. Preparation of 9-benzyl-5-carbamoyl-4-methoxy-1-fluorocarbazole

A solution of 0.458 g of 9-benzyl-5-methoxy-8-fluoro-1,2,3,4-tetrahydrocarbazole-4-carboxamide in 13 ml of dry dioxane under nitrogen was treated with 0.59 g of 2,3-dichloro-5,6-dicyano-1,4-benzoquinone and refluxed for one hour. The reaction mixture was cooled and filtered and the precipitate was washed with 15 ml of dioxane. The filtrate and washing were poured into saturated sodium bicarbonate solution and extracted three times with ethyl acetate. The extracts were washed with saturated sodium bicarbonate, with water and then with brine; dried over magnesium sulfate and concentrated. This residue was chromatographed on silica gel using dichloromethane/ 0-2% methanol to afford 0.45 g of subtitle compound. ESIMS *m/e* 349 ($M^+$+1)

| Elemental Analyses | | | |
|---|---|---|---|
| Calculated | C 72.42; | H 4.92; | N 8.04 |
| Found | C 72.35; | H 4.81; | N 7.88 |

B. Preparation of [9-benzyl-5-carbamoyl-1-fluorocarbazol-4-yl]oxyacetic acid methyl ester

A solution of 0.45 g of 9-benzyl-5-carbamoyl-4-methoxy-1-fluorocarbazole in 25 ml of dichloromethane was cooled in an ice bath treated dropwise with 12 ml of 1.0 M boron tribromide solution in dichloromethane. The reaction was allowed to warm to room temperature slowly over 2 hours and then quenched by pouring into ice and then adding 50 ml of 1 N HCl. The mixture was extracted with dichloromethane (3x200 ml) and the extracts were dried over magnesium sulfate and concentrated to afford 0.35 g (78%) of the demethylated intermediate. This intermediate (0.215 g) was alkylated and purified similarly to Example GH1, Part D to afford 0.166 g (64%) of the subtitle compound. mp. 190-191°C

| Elemental Analyses | | | |
|---|---|---|---|
| Calculated | C 67.97; | H 4.71; | N 6.89 |
| Found | C 67.81; | H 4.94; | N 6.96 |

C. Preparation of [9-benzyl-5-carbamoyl-1-fluorocarbazol-4-yl]oxyacetic acid

[9-Benzyl-5-carbamoyl-1-fluorocarbazol-4-yl]oxyacetic acid methyl ester (56 mg) was hydrolyzed and isolated similarly as described in Example 50, Part D to afford 54 mg of the title compound. FDMS *m/e* 392 ($M^+$); ESIMS *m/e* 393 ($M^+$+1), 391 ($M^+$-1) NMR (300 MHz, $d^6$-DMSO) : δ 12.92 (br, 1H); 7.70 (m, 2H); 7.45 (t, J=7.5, 1H); 7.39 (br, 1H); 7.28-7.17 (m, 4H); 7.12 (d, J=7.2, 1H); 7.07 (d, J=7.0, 2H); 6.51 (dd, J=8.8, 2.7, 1H); 5.77 (s, 2H); 4.80 (s, 2H).

| Elemental Analyses | | | |
|---|---|---|---|
| Calculated | C 67.34; | H 4.37; | N 7.14 |
| Found | C 66.92; | H 4.49; | N 6.77 |

Example 53

Preparation of [9-benzyl-4-carbamoyl-8-chloro-1,2,3,4-tetrahydrocarbazol-5-yl]oxyacetic acid

A. Preparation of ethyl 9-benzyl-5-methoxy-8-chloro-1,2,3,4-tetrahydrocarbazole-4-carboxylate

N-benzyl-2-chloro-5-methoxyaniline was prepared similarly to Example 51, Part E. A solution of 2.07 g of N-benzyl-2-chloro-5-methoxyaniline in 60 ml of dry tetrahydrofuran was converted similarly to Example GH3, Part F and chromatographed on silica gel using 15:1 hexane/ethyl acetate to afford 1.65 g (50%) of the subtitle compound. Ex. 59, A. ESIMS *m/e* 398 ($M^+$+1) NMR (300 MHz, $CDCl_3$): δ 7.29-7.19 (m, 3H); 6.92 (m, 3H); 6.36 (d, J=8.4, 1H); 5.87 (d, J=17.4, 1H); 5.53 (d, J=17.3, 1H); 4.16 m, 3H); 3.81 (s, 3H); 2.66 (dt, J=16.3, 5.4, 1H); 2.49 (dt, J=16.6, 6.6, 1H); 2.05 (m, 2H); 1.98-1.79 (m, 2H); 1.25 (t, 3H).

B. Preparation of 9-benzyl-5-methoxy-8-chloro-1,2,3,4-tetrahydrocarbazole-4-carboxamide

Ethyl 9-benzyl-5-methoxy-8-chloro-1,2,3,4-tetrahydrocarbazole-4-carboxylate (1.65g) was converted similarly to Example 51, Part G to afford 1.54 g (100%) of the subtitle compound. Ex. 59, B. mp. 205-8°C ESIMS *m/e* 3.69 ($M^+$+1).

C. Preparation of [9-benzyl-4-carbamoyl-8-chloro-1,2,3,4-tetrahydrocarbazol-5-yl]oxyacetic acid methyl ester

9-Benzyl-5-methoxy-8-chloro-1,2,3,4-tetrahydrocarbazole-4-carboxamide (0.405 g) was converted similarly to Example 51, Part H and chromatographed on silica gel using dichlormethane/0-1.5% methanol to afford 0.248 g (53%) of the subtitle compound. Ex. 59, C. m.p. 185-186°C.

| Elemental Analyses | | | |
|---|---|---|---|
| Calculated | C 64.71; | H 5.43; | N 6.56 |
| Found | C 64.98; | H 5.39; | N 6.67 |

D. Preparation of [9-benzyl-4-carbamoyl-8-chloro-1,2,3,4-tetrahydrocarbazol-5-yl]oxyacetic acid

[9-Benzyl-4-carbamoyl-8-chloro-1,2,3,4-tetrahydrocarbazol-5-yl]oxyacetic acid methyl ester (58 mg) was hydrolyzed similarly as described in Example 49, Part E to afford 54 mg of the title compound. Ex. 59, D. ESIMS *m/e* 413 ($M^+$+1), 411 ($M^+$-1) NMR (300 MHz, $d^6$-DMSO): δ 13.05 (br, 1H); 7.30-7.18 (m, 3H); 6.90 (d+m, J=7.6, 4H); 6.73 (br, 1H); 6.39 (d, J=8.3, 1H); 5.77 and 5.58 (ABq, J=17.5, 2H); 4.67 (s, 2H); 3.95 (br, 1H); 2.66 (m, 1H); 2.43 (m, 1H); 2.00 (m, 2H); 1.76 (m, 2H).

| Elemental Analyses | | | | |
|---|---|---|---|---|
| Calculated | C 64.00; | H 5.13; | N 6.78; | Cl 8.59 |
| Found | C 62.82; | H 5.34; | N 6.22; | Cl 7.99 |

Example 54

Preparation of [9-benzyl-5-carbamoyl-1-chlorocarbazol-4-yl]oxyacetic acid

A. Preparation of 9-benzyl-5-carbamoyl-4-methoxy-1-chlorocarbazole

A solution of 1.0 g of 9-benzyl-5-methoxy-8-methyl-1,2,3,4-tetrahydrocarbazole-4-carboxamide was oxidized similarly to Example 51, Part A and chromatographed on silica gel using dichloromethane/ 0-1% methanol to afford 0.66 g (67%) of the subtitle compound. FDMS *m/e* 364 ($M^+$)

| Elemental Analyses | | | | |
|---|---|---|---|---|
| Calculated | C 69.14; | H 4.70; | N 7.68; | Cl 9.72 |
| Found | C 69.40; | H 4.64; | N 7.49; | Cl 9.98 |

B. Preparation of 5-carbamoyl-4-hydroxy-1-chlorocarbazole

A solution of 0.66 g of 9-benzyl-5-carbamoyl-4-methoxy-1-chlorocarbazole in 40 ml of dichloromethane was cooled in an ice bath treated dropwise with 14 ml of 1.0 M boron tribromide solution in dichloromethane. The reaction was allowed to warm to room temperature slowly over 2 hours and then quenched by pouring into ice and then adding 50 ml of 1 N HCl. The mixture was extracted with dichloromethane (3x200 ml) and the extracts were washed with brine, dried with magnesium sulfate and concentrated. The aqueous layers exhibited a precipitate and was then extracted twice with ethyl acetate, washed with brine, dried with magnesium sulfate and concentrated to afford 0.287 g of the subtitle compound. The first residue was chromatographed on silica gel using 0.5% methanol in dichloromethane to afford another 93 mg of the subtitle compound. (total yield 80%) ESIMS *m/e* 259 ($M^+$-1) NMR (300 MHz, $d^6$-DMSO): δ 11.79 (s, 1H); 10.76 (s, 1H); 8.87 (br s, 1H); 8.41 (br s, 1H); 7.77 (t, J=4.6, 1H); 7.48 (d, J=4.2, 2H); 7.34 (d, J=8.5, 1H); 6.54 (d, J=8.5, 1H).

C. Preparation of [5-carbamoyl-1-chlorocarbazol-4-yl]oxyacetic acid methyl ester

A solution of 0.28 g of 5-carbamoyl-4-hydroxy-1-chlorocarbazole in 6 ml of tetrahydrofuran was added to 0.043 g of sodium hydride (60% in mineral oil) in 1 ml of tetrahydrofuran and stirred for 60 minutes at room temperature. Methyl bromoacetate (0.11 ml) was then added and the reaction was stirred overnight. The reaction mixture was poured into 20 ml of saturated ammonium chloride solution and then extracted twice with ethyl acetate. The extracts were washed with water and then with brine, dried over magnesium sulfate and concentrated. The residue was chromatographed on silica gel eluting with chloroform and then 2:1 chloroform/ethyl acetate to afford 0.16 g (45%) of the subtitle compound. ESIMS *m/e* 333 ($M^+$+1), 335 ($M^+$+3), 331 ($M^+$-1) NMR (300 MHz, $d^6$-DMSO) : δ 11.73 (s, 1H) ; 7.56 (d, J=8.1, 1H); 7.50 (br s, 1H); 7.43-7.35 (m, 2H); 7.18 (br s, 1H); 7.06 (d, J=7.8, 1H); 6.56 (d, J=8.6, 1H); 4.90 (s, 2H); 3.70 (s, 3H).

D. Preparation of [9-benzyl-5-carbamoyl-1-chlorocarbazol-4-yl]oxyacetic acid methyl ester

A solution of 78 mg of [5-carbamoyl-1-chlorocarbazol-4-yl]oxyacetic acid methyl ester in 0.8 ml of dry dimethylformamide was added to 10 mg sodium hydride (60% in mineral oil) in 0.2 ml of dimethylformamide and stirred for 15 minutes. Benzyl bromide (0.031 ml) was then added and the reaction was stirred overnight. The reaction mixture was poured into water and acidified with 1 ml of 1 N HCl solution and extracted twice with ethyl acetate. The extracts were washed with water (3x) and then with brine, dried over magnesium sulfate and concentrated. The residue was chromatographed on silica gel eluting with methanol/0-2% in dichloromethane to afford 40 mg of the subtitle compound. ESIMS *m/e* 423 ($M^+$+1) 425 ($M^+$+3) NMR (300 MHz, $CDCl_3$): δ 7.43-7.22 (m, 7H); 7.06 (d, J=7.3, 2H); 6.51 (d, J=8.6, 1H); 6.05 (s, 2H); 5.80 (br, 2H); 4.88 (s, 2H); 3.83 (s, 3H).

E. Preparation of [9-benzyl-5-carbamoyl-1-chlorocarbazol-4-yl]oxyacetic acid

[9-Benzyl-5-carbamoyl-1-chlorocarbazol-4-yl]oxyacetic acid methyl ester (15 mg) was hydrolyzed similarly as described in Example 50, Part D to afford 14 mg of the title compound. mp. 240-2°C ESIMS *m/e* 409 ($M^+$+1), 411 ($M^+$+3), 407 ($M^+$-1) NMR (300 MHz, $d^6$-DMSO) : δ 12.94 (br, 1H) ; 7.70 (br, 1H); 7.61 (d, J=8.3, 1H); 7.43 (t, J=7.8, 1H); 7.36 (m, 2H); 7.28-7.19 (m, 3H); 7.13 (d, J=7.2, 1H); 6.99 (d, J=7.4, 2H); 6.63 (d, J=8.6, 1H); 6.08 (s, 2H); 4.83 (s, 2H).

EXAMPLE 55

Preparation of [9-[(Cyclohexyl)methyl]-5-carbamoylcarbazol-4-yl]oxyacetic acid

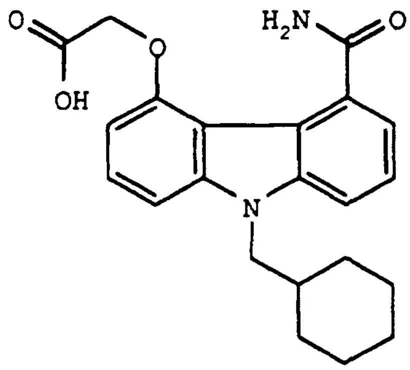

A. 9-[(Cyclohexyl)methyl]-5-carbomethoxy-1,2-dihydrocarbazol-4(3H)-one

A 0 °C suspension of 5-carbomethoxy-1,2-dihydro-9H-carbazol-4(3H)-one (1.0 g, 4.11 mmol), a catalytic amount of NaI (ca. 10 mg) and $K_2CO_3$ (1.1 g, 8.22 mmol) in 10 mL of DMF was treated with cyclohexylmethyl bromide (0.631 mL, 4.52 mmol). After stirring overnight at ambient temperature, an additional 0.63 mL cyclohexylmethylbromide was added, and the resulting mixture was heated at 60 °C for 3 hours. The mixture was poured into $H_2O$ (30 mL) and extracted with EtOAc (2 x 25 mL). The combined organic layers were washed with $H_2O$ (4 x 50 mL), dried over anhydrous $Na_2SO_4$, filtered and concentrated *in vacuo*. The residue was purified by radial chromatography on silica gel (elution with a gradient of 20% to 40% EtOAc/hexanes) to afford 1.36 g (4.01 mmol; 97%) of 9-[(cyclohexyl)methyl]-5-carbomethoxy-1,2-dihydrocarbazol-4(3H)-one as a white foam. IR ($CHCl_3$, $cm^{-1}$) 3011, 2932, 2857, 1725, 1649, 1469, 1446, 1288 and 1120. MS (ES) m/e 340 (M+1), 453 ($M+AcO^-$). FAB HRMS m/e, Calcd for $C_{21}H_{26}NO_3$: 340.1913. Found: 340.1916 (M+1).

| Elemental Analyses for $C_{21}H_{25}NO_3$: | | | |
|---|---|---|---|
| Calculated | C, 74.31; | H, 7.42; | N, 4.13. |
| Found | C, 72.65; | H, 7.39; | N, 4.70. |

B. 9-[(Cyclohexyl)methyl]-9-hydroxy-5-carbomethoxy carbazole

A solution of 9-[(cyclohexyl)methyl]-5-carbomethoxy-1,2-dihydrocarbazol-4(3H)-one (1.16 g, 3.42 mmol) and 2,3-dichloro-5,6-dicyano-1,4-benzoquinone (853 mg, 3.76 mmol) in 20 mL of toluene was heated at 80 °C for 3 hours. The mixture was purified directly by column chromatography on silica gel (elution with $CH_2Cl_2$) to afford 259 mg (0.768 mmol; 22%) of 9-[(cyclohexyl)methyl]-4-hydroxy-5-carbomethoxy carbazole as a yellow oil which slowly solidified. MS (ES) m/e 338 (M+1), 336 (M-1).

| Elemental Analyses for $C_{21}H_{23}NO_3$: | | | |
|---|---|---|---|
| Calculated | C, 74.75; | H, 6.87; | N, 4.15. |
| Found | C, 74.95; | H, 6.99; | N, 4.42. |

C. 9-[(Cyclohexyl)methyl]-9-hydroxy-5-carbamoyl carbazole

A solution of 9-[(cyclohexyl)methyl]-4-hydroxy-5-carbomethoxy carbazole (205 mg, 0.608 mmol) in 5 mL of THF and 20 mL of concentrated aqueous ammonium hydroxide was treated with a stream of $NH_3$ gas to ensure saturation. The reaction vessel was capped, and the mixture was heated at 35 °C with stirring until tlc indicated complete consumption of starting material (20 hrs). The THF was evaporated, and the aqueous layer was filtered. The green

solid precipitate was dissolved in THF and purified by radial chromatography on silica gel (elution with $CH_2Cl_2$). The resultant foam was triturated with ether to afford 138 mg (70%) of the title compound as an off-white solid. IR (KBr, $cm^{-1}$) 3418, 3200, 3131, 1629, 1600, 1443, 1261, 778. 'FAB HRMS m/e, Calcd for $C_{20}H_{23}N_2O_2$: 323.1760. Found: 323.1760 (M+1).

D. [9-[(Cyclohexyl)methyl]-5-carbamoylcarbazol-4-yl]oxyacetic acid, methyl ester

A mixture of 9-[(cyclohexyl)methyl]-4-hydroxy-5-carbamoyl carbazole (60 mg, 0.186 mmol) and $Cs_2CO_3$ (150 mg; 0.460 mmol) in 2 mL of DMF was treated with methyl bromoacetate (0.023 mL; 0.242 mmol). The reaction was stirred for 2 hours at ambient temperature, then it was diluted with EtOAc and $H_2O$ (10 mL each). The aqueous layer was saturated with solid NaCl and extracted with EtOAc (2 x 10 mL). The combined organic layers were washed with $H_2O$ (2 x 25 mL), dried over anhydrous $Na_2SO_4$, filtered and concentrated *in vacuo*. Purification of the crude residue by flash chromatography on silica gel (elution with a gradient of 0% to 90% EtOAc/hexanes) followed by trituration with $Et_2O$/EtOAc afforded 45 mg (0.114 mmol; 61%) of title compound as an off-white solid. MS (ES) m/e 395 (M+1), 378 (M+H-$NH_3$), 453 (M+$AcO^-$).

| Elemental Analyses for $C_{23}H_{26}N_2O_4 \cdot 0.3H_2O$: | | | |
|---|---|---|---|
| Calculated | C, 69.08; | H, 6.71; | N, 7.01. |
| Found | C, 69.13; | H, 6.71; | N, 7.09. |

E. [9-[(Cyclohexyl)methyl]-5-carbamoylcarbazol-4-yl]oxyacetic acid

A slurry of [9-[(cyclohexyl)methyl]-5-carbamoylcarbazol-4-yl]oxyacetic acid, methyl ester (20 mg, 0.051 mmol) in 0.3 mL of THF and 0.1 mL of MeOH was treated with 0.1 mL of 1 N aq LiOH (0.1 mmol), and the mixture stirred at room temperature for 2 h. The reaction was acidified with 0.2 N HCl, and the organics were removed *in vacuo*. The white precipitate was filtered away from the aqueous layer and rinsed with $Et_2O$ to afford 16 mg (0.042 mmol; 83%) the title acid as a white powder. MS (ES) m/e 381 (M+1), 364 (M+H-$NH_3$), 379 (M-1).

| Elemental Analyses for $C_{22}H_{24}N_2O_4$: | | | |
|---|---|---|---|
| Calculated | C, 69.46; | H, 6.36; | N, 7.36. |
| Found | C, 69.34; | H, 6.35; | N, 7.29. |

EXAMPLE 56

Preparation of [9-[(Cyclopentyl)methyl]-5-carbamoylcarbazol-4-yl]oxyacetic acid

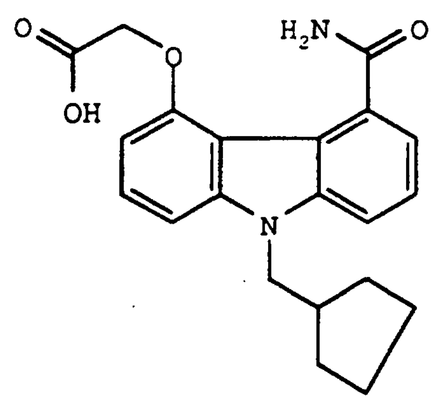

A. 9-[(Cyclopentyl)methyl]-5-carbomethoxy-1,2-dihydrocarbazol-4(3H)-one

A suspension of 5-carbomethoxy-1,2-dihydro-9H-carbazol-4(3H)-one (820 g, 3.37 mmol), a catalytic amount of NaI (ca. 10 mg) and $K_2CO_3$ (930 mg, 6.74 mmol) in 6 mL of DMF was treated with cyclopentylmethyl chloride (*JOC,* 1964, *29,* 421-423; 400 mg, 3.37 mmol). After stirring overnight at ambient temperature, an additional 800 mg of cyclopentylmethyl chloride and 1 g of NaI were added, and the resulting mixture was heated at 80 °C overnight. An additional 800 mg of cyclopentylmethyl chloride and 2.2 g of $Cs_2CO_3$ were added, and the reaction mixture was heated at 80 °C for 24 h. An additional 1.6 g of cyclopentylmethyl chloride was added, and the reaction mixture was heated at 80 °C for 3 d The mixture was poured into $H_2O$ (30 mL) and extracted with EtOAc (3 x 10 mL). The combined organic layers were dried over anhydrous $Na_2SO_4$, filtered, and concentrated *in vacuo*. The residue was purified by radial chromatography on silica gel (elution with gradient of 10% to-40% EtOAc/hexanes) to afford 775 mg (2.38 mmol; 71%) of 9-[(cyclopentyl)methyl]-5-carbomethoxy-1,2-dihydrocarbazol-4(3H)-one as a brown foam. MS (ES) m/e 326 (M+1), 384 ($M+AcO^-$).

| Elemental Analyses for $C_{20}H_{23}NO_3$: | | | |
|---|---|---|---|
| Calculated | C, 73.82; | H, 7.12; | N, 4.30. |
| Found | C, 74.12; | H, 7.21; | N, 4.45. |

B. 9-[(Cyclopentyl)methyl]-4-hydroxy-5-carbomethoxy carbazole

A solution of 9-[(cyclopentyl)methyl]-5-carbomethoxy-1,2-dihydrocarbazol-4(3H)-one (730 mg, 2.24 mmol) and 2,3-dichloro-5,6-dicyano-1,9-benzoquinone (560 mg, 2.47 mmol) in 20 mL of toluene was heated at 80 °C for 3 hours. The mixture was purified directly by column chromatography on silica gel (elution with $CH_2Cl_2$) to afford 140 mg (0.433 mmol; 19%) of 9-[(cyclopentyl)methyl]-4-hydroxy-5-carbomethoxy carbazole as a yellow oil which slowly solidified. MS (ES) m/e 324 (M+1), 322 (M-1).

| Elemental Analyses for $C_{20}H_{21}NO_3 \cdot 0.3H_2O$: | | | |
|---|---|---|---|
| Calculated | C, 73.06; | H, 6.62; | N, 4.26. |
| Found | C, 73.19; | H, 6.44; | N, 4.40. |

C. 9-[(Cyclopentyl)methyl]-4-hydroxy-5-carbamoyl carbazole

A solution of 9-[(cyclopentyl)methyl]-4-hydroxy-5-carbomethoxy carbazole (110 mg, 0.34 mmol) in 3 mL of THF and 20 mL ofconcentrated aqueous ammonium hydroxide was treated with a stream of $NH_3$ gas to ensure saturation. The reaction vessel was capped, and the mixture heated to 35 °C with stirring until tlc indicated complete consumption of starting material (20 h). The THF was evaporated, and the aqueous layer was filtered. The resultant solid was triturated with ether to afford 50 mg (0.162; 48%) of the title compound as a greenish-white solid. IR (KBr, $cm^{-1}$) 3416, 3199, 3126, 1630, 1599. 1442, 1262, 778. 'FAB HRMS m/e, Calcd for $C_{20}H_{21}N_2O_2$: 309.1603. Found: 309.1607 (M+1).'

D. [9-[(Cyclopentyl)methyl]-5-carbamoylcarbazol-4-yl]oxyacetic acid, methyl ester

A mixture of 9-[(cyclopentyl)methyl]-4-hydroxy-5-carbamoyl carbazole (45 mg, 0.146 mmol) and $Cs_2CO_3$ (120 mg; 0.365 mmol) in 2 mL of DMF was treated with methyl bromoacetate (0.018 mL; 0.19 mmol). The reaction was stirred for 2 hours at ambient temperature, then it was diluted with EtOAc and $H_2O$ (10 mL each). The aqueous layer was saturated with solid NaCl extracted with EtOAc (2 x 10 mL). The combined organic layers were washed with $H_2O$ (2 x 25 mL), dried over anhydrous $Na_2SO_4$, filtered and concentrated *in vacuo*. Purification of the crude residue by flash chromatography on silica gel (elution with a gradient of 0% to 100% EtOAc/hexanes) followed by trituration with $Et_2O$/EtOAc afforded 26 mg (0.0683 mmol; 47%) of title compound as a tan solid. MS (ES) m/e 381 (M+1), 364 ($M+H-NH_3$), 439 ($M+AcO^-$).

| Elemental Analyses for $C_{23}H_{26}N_2O_4 \cdot 0.1H_2O$: | | | |
|---|---|---|---|
| Calculated | C, 69.13; | H, 6.38; | N, 7.33. |
| Found | C, 68.99; | H, 6.39; | N, 7.41. |

E. [9-[(Cyclopentyl)methyl]-5-carbamoylcarbazol-4-yl]oxyacetic acid

A slurry of [9-[(cyclopentyl)methyl]-5-carbamoylcarbazol-4-yl}oxyacetic acid, methyl ester (20 mg, 0.065 mmol) in 0.3 mL of THF and 0.1 mL of MeOH was treated with 0.1 mL of 1 N aq LiOH (0.1 mmol), and the mixture stirred at room temperature for 2 hours. The reaction was acidified with 0.2 N HCl, and the organics were removed *in vacuo*. The white precipitate was filtered away from the aqueous layer and rinsed with $Et_2O$ to afford 15 mg (0.0409 mmol; 63%) the title acid as a white powder. MS (ES) m/e 367 (M+1), 350 ($M+H-NH_3$), 365 (M-1).

| Elemental Analyses for $C_{21}H_{22}N_2O_4 \cdot 0.3H_2O$: | | | |
|---|---|---|---|
| Calculated | C, 67.84; | H, 6.13; | N, 7.53. |
| Found | C, 67.73; | H, 5.97; | N, 7.70. |

Example 57

[5-carbamoyl-9-(phenylmethyl)-2-(2-thienyl)carbazol-4-yl]oxyacetic acid

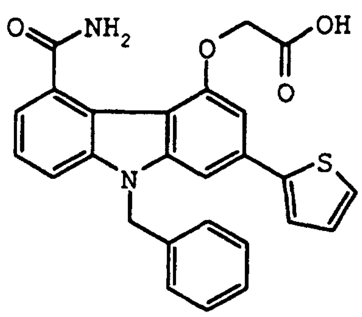

A. Preparation of 3-(2-bromo-3-carbomethoxyanilino)-5-(2-thienyl)cyclohex-2-en-1-one

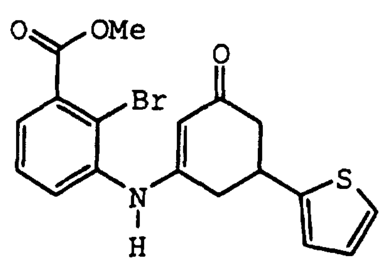

Prepared in 59% yield by the method of Example 17C. $^1H$-NMR ($CDCl_3$): δ 2.63 (dd, *J* = 16.5, 118. Hz, 1H), 2.78-2.96 (m, 3H), 3.71-3.80 (m, 1H), 3.94 (s, 3H), 5.61 (s, 1H), 6.23 (br s, 1H), 6.93 (d, *J* = 3.5 Hz, 1H), 6.97-6.99 (m, 1H), 7.21 (d, *J* = 5.2 Hz, 1H), 7.34 (br t, *J* = 7.8 Hz, 1H), 7.55 (d, *J* = 7.8 Hz, 2H).

B. Preparation of 5-carbomethoxy-1,2-dihydro-2-(2-thienyl)-9H-carbazol-4(3H)-one

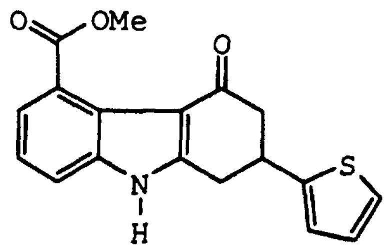

Prepared in 85% yield by the method of Example 17D. $^1$H-NMR ($CDCl_3$): δ 2.73 (dd, *J* = 16.3, 11.8 Hz, 1H), 2.91 (dd, *J* = 16.4, 4.0 Hz, 1H), 3.03 (dd, *J* = 16.6, 10.8 Hz, 1H), 3.24 (dd, *J* = 16.6, 4.5 Hz, 1H), 3.75-3.78 (m, 1H), 4.03 (s, 3H), 6.88 (br s, 1H), 6.93-6.96 (m, 1H), 7.17 (d, *J* = 5.0 Hz, 1H), 7.22-7.26 (m, 1H), 7.36 (d, *J* = 7.4 Hz, 1H), 7.40 (d, *J* = 8.0 Hz, 1H),9.17 (br s, 1H).

C. Preparation of 5-carbomethoxy-1,2-dihydro-9-(phenylmethyl)-2-(2-thienyl)carbazol-4(3H)-one

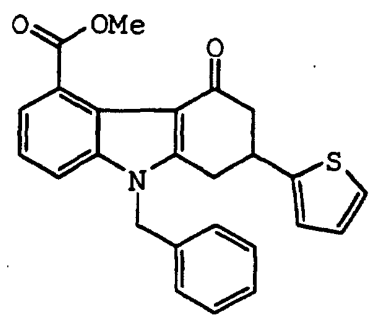

Prepared in 88% yield by the method of Example 17E. $^1$H-NMR ($CDCl_3$): δ 2.84 (dd, J = 16.5, 11.6 Hz, 1H), 2.97-3.10 (m, 2H), 3.34 (dd, *J* = 16.5, 4.5 Hz, 1H), 3.89-3.96 (m, 1H), 4.06 (s, 3H), 5.38 (s, 2H), 6.89-7.00 (m, 4H), 7.18 (d, *J* = 5.3 Hz, 1H), 7.25-7.41 (m, 6H).

D. Preparation of 5-carbomethoxy-4-hydroxy-9-(phenylmethyl)-2-(2-thienyl)carbazole

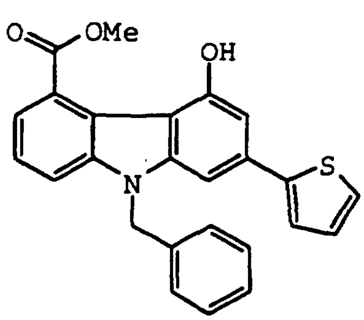

Prepared in 76% yield by the method (b) of Example 17F. $^1$H-NMR ($CDCl_3$): δ 4.11 (s, 3H), 5.55 (s, 2H),

7.07-7.12 (m, 3H), 7.16 (s, 2H), 7.24-7.30 (m, 4H), 7.37-7.42 (m, 2H), 7.56 (d, $J$ = 8.1 Hz, 1H), 8.01 (d, $J$ = 7.6 Hz, 1H).

E. Preparation of 5-carbamoyl-4-hydroxy-9-(phenylmethyl)-2-(2-thienyl)carbazole

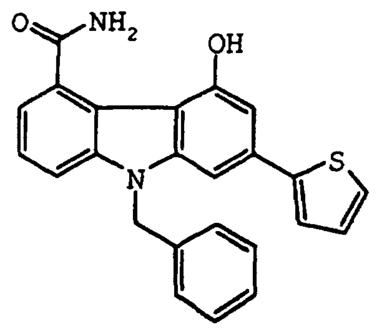

Prepared in 85% yield by the method of Example 17G. $^1$H-NMR (DMSO-$d_6$): δ 5.73 (s, 2H), 6.87 (s, 1H), 7.08-7.26 (m, 6H), 7.41-7.56 (m, 5H), 7.76 (br t, $J$ = 4.5 Hz, 1H), 8.39 (s, 1H), 8.83 (s, 1H), 10.76 (s, 1H).

F. Preparation of [5-carbamoyl-9-(phenylmethyl)-2-(2-thienyl)carbazol-4-yl]oxyacetic acid, methyl ester

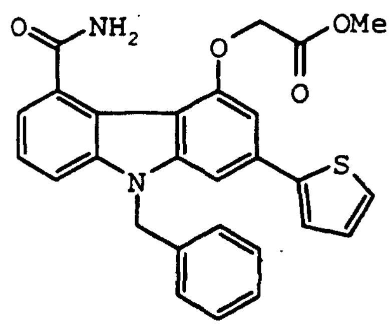

Prepared in 92% yield by the method of Example 17H. $^1$H-NMR (DMSO-$d_6$): δ 3.70 (s, 3H), 4.99 (s, 2H) , 5.71 (s, 2H), 6.85 (s, 1H), 7.04 (d, $J$ = 7.2 Hz, 1H), 7.11-7.26 (m, 7H), 7.35 (br t, $J$ = 7.7 Hz, 1H), 7.50-7.57 (m, 5H).

G. Preparation of [5-carbamoyl-9-(phenylmethyl)-2-(2-thienyl)carbazol-4-yl]oxyacetic acid

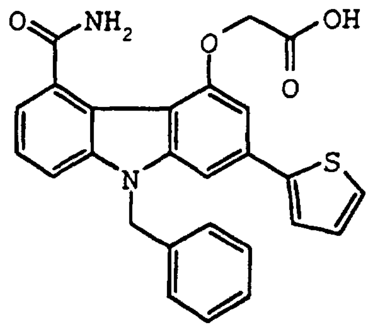

Prepared in 98% yield by the method of Example 17I. $^1$H-NMR (DMSO-$d_6$) : δ 4.90 (s, 2H), 5.72 (s, 2H), 6.85 (s, 1H), 7.04-7.26 (m, 7H), 7.33-7.38 (m, 2H), 7.50-7.59 (m, 4H), 7.71 (s, 1H), 12.99 (br s, 1H).

Example 58

[5-carbamoyl-9-(phenylmethyl)-2-[[(propen-3-yl)oxy]methyl]carbazol-4-yl]oxyacetic acid

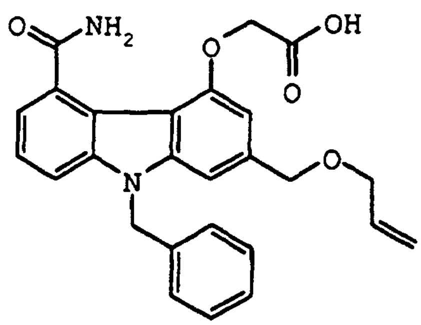

A. Preparation of 5-carbomethoxy-1,2-dihydro-9-(phenylmethyl)-2-[[(propen-3-yl)oxy]methyl]carbazol-4(3H)-one

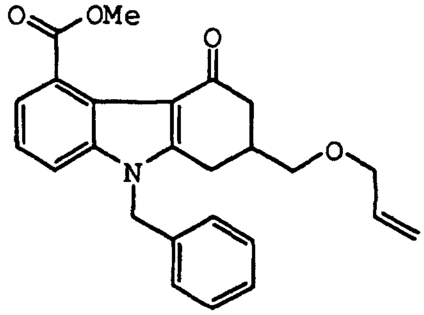

Sodium hydride (63.4 mg, 1.58 mmol) was added to a stirred anhydrous DMF (7 mL) solution containing the

compound of Example 19C (480 mg, 1.32 mmol) and allyl bromide (0.172 mL, 1.98 mmol) under a nitrogen atmosphere. The resultant solution was stirred at ambient temperature for 2 hours. Then the mixture was treated with two drops of acetic acid before it was concentrated in vacuo. The residue was subject to chromatography on silica (gradient 30-70% ethyl acetate in hexane) to provide the subtitled compound (395 mg, 74%) as a white solid. IR (KBr) 1726, 1654 $cm^{-1}$; $^1$H-NMR ($CDCl_3$) : δ 2.40 (dd, *J* = 16.5, 11.1 Hz, 1H), 2.57-2.78 (m, 3H), 3.01-3.08 (m, 1H), 3.41 (dd, *J* = 9.2, 6.9 Hz, 1H), 3.49 (dd, *J* = 9.2, 4.3 Hz, 1H), 3.95 (d, *J* = 5.4 Hz, 2H, $-CH_2O-$), 4.04 (s, 3H, $-OCH_3$), 5.14-5.27 (m, 2H, $=CH_2$), 5.32 (s, 2H, $-NCH_2-$), 5.80-5.92 (m, 1H, -CH=), 6.97-7.02 (m, 2H), 7.20-7.39 (m, 6H); ESIMS m/e 404 ($M^+$+1);

| Elemental Analyses for $C_{25}H_{25}NO_4$: | | |
|---|---|---|
| Calculated: | C, 74.42; | H, 6.25. |
| Found: | C, 74.59; | H, 6.07. |

B. Preparation of 5-carbomethoxy-4-hydroxy-9-(phenylmethyl)-2-[[(propen-3-yl)oxy]methyl]carbazole

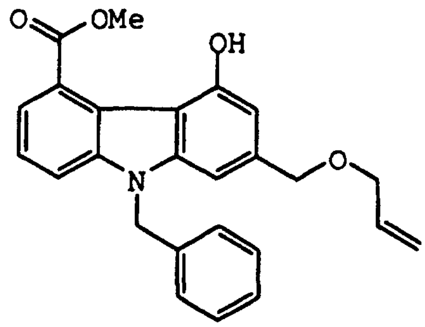

Prepared in a 78% yield by the method (b) of Example 17F. IR ($CHCl_3$) 3200 (br), 1687 $cm^{-1}$; $^1$H-NMR ($CDCl_3$): δ 4.04 (d, *J* = 5.7 Hz, 2H, $-CH_2O-$), 4.11 (s, 3H, $-OCH_3$), 4. 63 (s, 2H, $-OCH_2-$), 5.15-5.31 (m, 2H, $=CH_2$), 5.55 (s, 2H, $-NCH_2-$), 5.88-6.02 (m, 1H, -CH=), 6.81 (s, 1H), 6.99 (s, 1H), 7.05-7.09 (m, 2H), 7.22-7.30 (m, 3H), 7.40 (br t, *J*= 8.0 Hz, 1H), 7.58 (d, *J* = 8.0 Hz, 1H), 8.00 (d, *J* = 8.0 Hz, 1H), 10.48 (s, 1H, -OH); ESIMS m/e 402 ($M^+$+1);

| Elemental Analyses for $C_{25}H_{23}NO_4$: | | |
|---|---|---|
| Calculated | C, 74.80; | H, 5.77. |
| Found | C, 75.08; | H, 5.78. |

C. Preparation of 5-carbamoyl-4-hydroxy-9-(phenylmethyl)-2-[[(propen-3-yl)oxy]methyl]carbazole

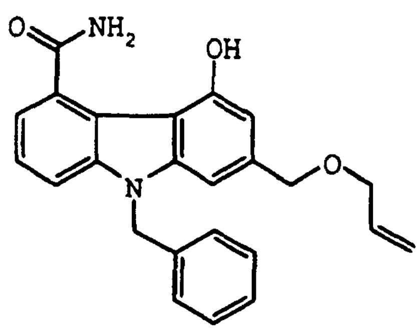

Prepared in a 75% yield by the method of Example 17G. IR (KBr) 3420, 3203 (br), 3121, 1632, 1601

$cm^{-1}$; $^1$H-NMR (DMSO-$d_6$): 8 3.95 (d, $J$ = 5.3 Hz, 2H, $-CH_2O-$), 4.50 (s, 2H, $-OCH_2-$), 5.08-5.25 (m, 2H, $=CH_2$), 5.65 (s, 2H, $-NCH_2-$), 5.83-5.93 (m, 1H, -CH=), 6.54 (s, 1H), 7.02-7.05 (m, 3H), 7.14-7.25 (m, 3H), 7.39-7.45 (m, 2H), 7.73-7.77 (m, 1H), 8.34 (s, 1H, -NH), 8.79 (s, 1H, -NH), 10.53 (s, 1H, -OH); ESIMS m/e 387 ($M^+$+1);

| Elemental Analyses for $C_{24}H_{22}N_2O_3$: | | |
|---|---|---|
| Calculated | C, 74.59; | H, 5.74. |
| Found | C, 74.85; | H, 5.93. |

D. Preparation of [5-carbamoyl-9-(phenylmethyl)-2-[[(propen-3-yl)oxy]methyl]carbazol-4-yl]oxyacetic acid,

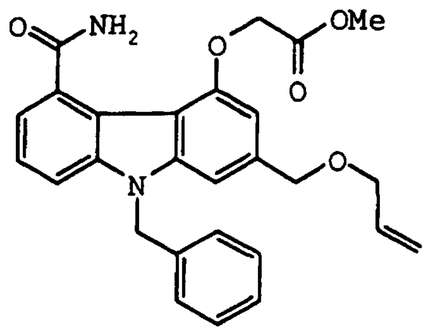

methyl ester

Prepared in a 90% yield by the method of Example 17H. IR (KBr) 3360, 3167, 1758, 1639 $cm^{-1}$; $^1$H-NMR ($CDCl_3$): δ 3.83 (s, 3H, $-OCH_3$), 4.00 (d, $J$ = 5.7 Hz, 2H, $-CH_2O-$), 4.62 (s, 2H, $-OCH_2-$), 4.91 (s, 2H, $-OCH_2-$), 5.16-5.30 (m, 2H, $=CH_2$), 5.52 (s, 2H, $-NCH_2-$), 5.88-6.00 (m, 1H, -CH=), 6.05 (br s, 2H, $-NH_2$), 6.59 (s, 1H), 7.05 (s, 1H), 7.06-7.10 (m, 2H), 7.22-7.41 (m, 6H); ESIMS m/e 459 ($M^+$+1).

E. Preparation of [5-carbamoyl-9-(phenylmethyl)-2-[[(propen-3-yl)oxy]methyl]carbazol-4-yl]oxyacetic acid

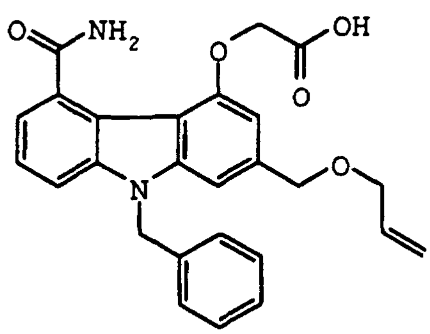

Prepared in a 89% yield by the method of Example 17I. IR (KBr) 3453, 3421, 3332, 3220, 2580 (br), 1740, 1724, 1631 $cm^{-1}$; $^1$H-NMR (DMSO-$d_6$): δ 3.95 (d, $J$ = 4.7 Hz, 2H, $-CH_2O-$), 4.53 (s, 2H, $-OCH_2-$), 4.79 (s, 2H, $-OCH_2-$), 5.10-5.26 (m, 2H, $=CH_2$), 5.64 (s, 2H, $-NCH_2-$), 5.80-6.00 (m, 1H, -CH=), 6.56 (s, 1H), 7.04-7.40 (m, 9H), 7.57 (d, $J$ = 8.1 Hz, 1H), 7.70 (s, 1H, -NH), 12.94 (br s, 1H, $-CO_2H$); ESIMS m/e 445 ($M^+$+1);

| Elemental Analyses for $C_{26}H_{24}N_2O_5$: | | |
|---|---|---|
| Calculated | C, 70.26; | H, 5.44. |
| Found | C, 70.00; | H, 5.42. |

Example 59

[5-carbamoyl-9-(phenylmethyl)-2-[(propyloxy)methyl]carbazol-4-yl]oxyacetic acid

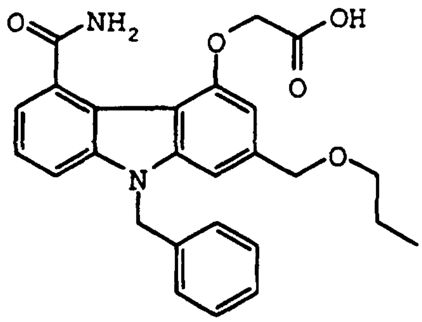

A. Preparation of [5-carbamoyl-9-(phenylmethyl)-2-[(propyloxy)methyl]carbazol-4-yl]oxyacetic acid, methyl ester

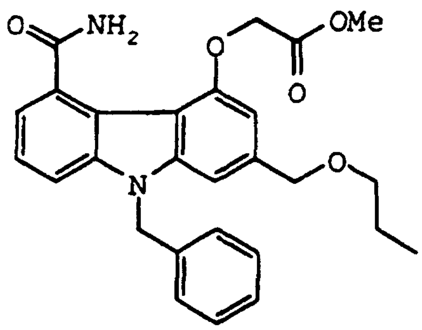

Platinum oxide (30 mg) was added to a stirred THF (30 mL) solution containing the compound of Example IID (120 mg, 0.262 mmol) under a nitrogen atmosphere. The mixture was then stirred under a hydrogen atmosphere for 30 minutes. After filtration and concentration, the residue was chromatographed on silica (gradient 0-6% methanol in methylene chloride) to afford the subtitled compound (117 mg, 97%) as a white solid. IR (KBr) 3364, 3166, 1758, 1742, 1642 $cm^{-1}$; $^1H$-NMR ($CDCl_3$): δ 0.91 (t, *J* = 7.4 Hz, 3H, $-CH_3$), 1.57-1.65 (m, 2H), 3.40 (t, *J* = 6.6 Hz, 2H, $-OCH_2-$), 3.83 (s, 3H, $-OCH_3$), 4.60 (s, 2H, $-OCH_2-$), 4.91 (s, 2H, $-OCH_2-$), 5.52 (s, 2H, $-NCH_2-$), 5.95 (br s, 1H, -NH), 6.06 (br s, 1H, - NH), 6.58 (s, 1H), 7.04 (s, 1H), 7.07-7.10 (m, 2H), 7.20-7.41 (m, 6H); ESIMS m/e 461 ($M^+$+1).

B. Preparation of [5-carbamoyl-9-(phenylmethyl)-2-[(propyloxy)methyl]carbazol-4-yl]oxyacetic acid

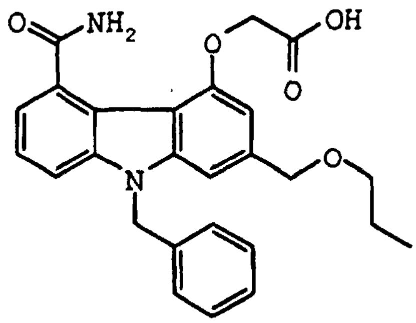

Prepared in a 99% yield by the method of Example 17I. IR (KBr) 3458, 3413, 3332, 3232, 2500 (br), 1716, 1627 $cm^{-1}$; $^1H$-NMR (DMSO-$d_6$): δ 0.82 (t, J = 7.3 Hz, 3H, $-CH_3$), 1.45-1.53 (m, 2H), 3.33 (t, *J* = 6.3 Hz, 2H, $-OCH_2-$), 4.51 (s, 2H, $-CH_2O-$) 4.78 (s, 2H, $-OCH_2-$), 5.64 (s, 2H, $-NCH_2-$), 6.54 (s, 1H), 7.03-7.39 (m, 9H), 7.57 (d, *J* = 8.2 Hz, 1H), 7.70 (s, 1H, -NH), 12.93 (s, 1H, $-CO_2H$); ESIMS m/e 447 ($M^+$+1);

| Elemental Analyses for $C_{26}H_{26}N_2O_5$: | | |
|---|---|---|
| Calculated | C, 69.94; | H, 5.87. |
| Found | C, 70.00; | H, 5.88. |

Example 60

Preparation of 9-benzyl-7-methoxy-5-((carboxamidomethyl)oxy)-1,2,3,4-tetrahydrocarbazole-4-carboxamide

To 195mg (0.5mmol) of 9-benzyl-7-methoxy-5-cyanomethyloxy-1,2,3,4-tetrahydrocarbazole-4-carboxamide in 3ml $CH_2Cl_2$ was added 34mg (0.1mmol) tetrabutylammonium sulfate. After cooling to 0°C, 0.25ml 30% $H_2O_2$ and 3ml 20% NaOH were added. The reaction was allowed to warm to room temperature and stirred for 18h. The mixture was diluted with $CH_2Cl_2$, washed with water, washed with brine, dried over sodium sulfate, and evaporated *in vacuo*. The residue was chromatographed on silica gel eluting with a gradient of 2 to 10% isopropanol in methylene chloride to give the titled product (36.7mg 19%). An analytical sample was crystallized from methanol.
MS (ES+) 408

| Elemental analysis for $C_{23}H_{25}N_3O_4$: | | | |
|---|---|---|---|
| Calculated | C 67.80; | H 6.18; | N 10.31 |
| Theory | C 67.91; | H 6.17; | N 10.44 |

Example 61

Preparation of 9-benzyl-7-methoxy-5-cyanomethyloxycarbazole-4-carboxamide

To a stirred solution of 9-benzyl-5-hydroxy-7-methoxycarbazole-4-carboxamide (0.75g, 2.17mmol) in DMF (76ml) and THF (16ml) and added 60% NaH (0.11g, 2.71mmol). After 15 min bromoacetonitrile (0.20ml, 2.93mmol) was added and the reaction was allowed to stir for 4h. The reaction was diluted with EtOAc, extracted with water, then brine, dried ($Na_2SO_4$), and chromatographed on silica gel using a $CH_2Cl_2$-EtOAc-methanol gradient to give the titled compound (0.52g, 63%). MS (ES+) 386

| Elemental analysis for $C_{23}H_{19}N_3O_3$: | | | |
|---|---|---|---|
| Calculated | C 71.68; | H 4.97; | N 10.90 |
| Theory | C 71.67; | H 4.72; | N 10.65 |

Example 62

Preparation of 9-benzyl-7-methoxy-5-((1H-tetrazol-5-yl-methyl)oxy)-carbazole-4-carboxamide

0.20gram (0.52mmol) of 9-benzyl-7-methoxy-5-cyanomethyloxy-carbazole-4-carboxamide was heated with 2.2ml tri-n-butyl tin hydride at 95°C for 3.5 h. The reaction was then added to a mixture of 56 ml acetonitrile, 11ml tetrahydrofuran, and 22ml acetic acid and stirred for 2 h. The mixture was extracted 4 times with hexane and the residue evaporated *in vacuo*. The residue was chromatographed on silica gel using a $CH_2Cl_2$-methanol gradient, then 1% HOAc in EtOAc. Crystallization from acetone and hexane afforded the titled compound (0.047g, 21%). MS (ES+) 412, 429

| Elemental analysis for $C_{23}H_{24}N_6O_3$: | | | |
|---|---|---|---|
| Calculated | C 64.48; | H 4.71; | N 19.61 |
| Theory | C 64.58; | H 4.67; | N 19.68 |

Example 63

Preparation of 9-benzyl-7-methoxy-5-((carboxamidomethyl)oxy)-carbazole-4-carboxamide

To 200mg (0.52mmol) of 9-benzyl-7-methoxy-5-cyanomethyloxy-carbazole-4-carboxamide in 6ml $CH_2Cl_2$ was added 35mg (0.1mmol) tetrabutylammonium sulfate. After cooling to 0°C, 0.26ml 30% $H_2O_2$ and 6ml 20% NaOH. were added. The reaction was allowed to warm to room temperature and stirred for 18h. The mixture was diluted with $CH_2Cl_2$ and methanol, washed with water, washed with brine, dried over sodium sulfate, and evaporated *in vacuo*. The residue was chromatographed on silica gel using a $CH_2Cl_2$-ethanol gradient. Crystallization from methanol afforded the titled compound (22.5mg, 11%). MS (FD) 403

| Elemental analysis for $C_{23}H_{21}N_3O_4$: | | | |
|---|---|---|---|
| Calculated | C 68.47; | H 5.25; | N 10.42 |
| Theory | C 68.65; | H 4.99; | N 10.40 |

Example 64

Preparation of [9-Benzyl-4-carbamoyl-1,2,3,4-tetrahydrocarbazole-5-yl]oxyacetic acid

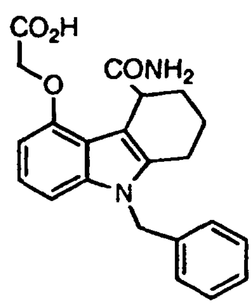

A. 9-Benzyl-4-carboxy-5-methoxy-1,2,3,4-tetrahydrocarbazole, Ethyl Ester.

A solution of 1.50 g (4.02 mmol) of 9-Benzyl-4-carboxy-8-chloro-5-methoxy-1,2,3,4-tetrahydrocarbazole, ethyl ester and 0.45 g (4.40 mmol) of $Et_3N$ in 25 mL of EtOH was treated with 0.24 g of 5% Pd-C and the mixture hydrogenated at 60 psi for 16 hrs. The reaction was filtered and concentrated in vacuo to give 1.40 g of a tan solid. $^1H$ NMR ($CDCl_3$) δ 7.30-7.19 (m, 3H), 7.03-6.95 (m, 3H), 6.80 (d, 1H, J=8.1 Hz), 6.44 (d, 1H, J=7.7 Hz), 5.22 (d, 2H, J=5.9 Hz), 4.22-4.11 (m, 3H), 3.82 (s, 3H), 2.75-2.64 (m, 1H), 2.59-2.48 (m, 1H), 2.11-1.64 (m, 4H), and 1.25 (t, 3H, J=7.0 Hz). IR ($CHCl_3$) 2959, 1725, 1499, 1453, 1260, 1178, 1128 cm-1;

| Elemental Analysis for $C_{23}H_{25}NO_3$: | |
|---|---|
| Calculated | 363.1836 |
| Found | 363.1834. |

B. 9-Benzyl-4-carbamoyl-5-hydroxy-1,2,3,4-tetrahydrocarbazole.

A 0 °C solution of 1.00 g (2.80 mmol) 9-benzyl-4-carboxy-5-methoxy-1,2,3,4-tetrahydrocarbazole, ethyl ester in 15 mL of $CH_2Cl_2$ was treated with 22.40 mL (22.40 mmol; 1M in $CH_2Cl_2$) of $BBr_3$. The cold bath was removed and the reaction stirred until tlc analysis (10% EtOAc in hexanes) indicated complete consumption of starting material (1.5 hrs). The reaction was cooled to 0 °C and was quenched with 5.0 mL of MeOH. The mixture was stirred at ambient temperature for 18 hrs and was concentrated *in vacuo*. The black oil was taken up in 200 mL of $CH_2Cl_2$ and the solution washed with H20 (100 mL) and sat'd aq $NaHCO_3$ (100mL). Evaporation of the solvent *in vacuo* afforded 700 mg of a black oil. Purification by radial chromatography (10 % EtOAc in hexanes) afforded 400 mg of 9-benzyl-4-carboxy-5-hydroxy-1,2,3,4-tetrahydrocarbazole, ethyl ester which was taken on directly to the next reaction.

The phenol was taken up in 40 ml of THF and the solution treated with 10 mL of $NH_4OH$. The reaction vessel was capped and the mixture stirred vigorously for 13 days. The reaction was poured into $H_2O$ and the mixture extracted with EtOAc (3 x 150 mL). The combined organic layers were dried ($Na_2SO_4$), filtered and concentrated *in vacuo* to give 300 mg of a brown foam. Radial chromatography (3% MeOH in $CH_2Cl_2$) afforded 50 mg of starting phenol and 80 mg (0.03 mmol; 22 %) of 9-benzyl-4-carbamoyl-5-hydroxy-1,2,3,4-tetrahydrocarbazole. $^1H$ NMR ($CDCl_3$) δ 7.33-7.24 (m, 3H), 7.06-6.97 (m, 3H), 6.81 (d, 1H, J=8.1 Hz), 6.56 (d, 1H, J=7.5 Hz), 5.22 (d, 2H, J=2.2 Hz), 4.20-4.15 (m, 1H), 2.78-2.67 (m, 1H), 2.63-2.51 (m, 1H), 2.35-2.27 (m, 1H), and 2.09-1.91 (m, 3H), no phenol proton detected. IR (CHCl3) 3007, 1667, 1586, 1567, 1496, 1266 cm-1;

| Elemental Analysis for $C_{20}H_{21}N_2O_2$: | |
|---|---|
| Calculated | 321.1603. |
| Found | 321.1607. |

C. [9-Benzyl-4-carbamoyl-1,2,3,4-tetrahydrocarbaole-5-yl]oxyacetic acid, Methyl Ester.

A solution of 80 mg (0.25 mmol) of 9-benzyl-4-carbamoyl-5-hydroxy-1,2,3,4-tetrahydrocarbazole in 2.5 mL of DMF was treated with 61 mg (0.30 mmol) of $Cs_2CO_3$ followed by 26 mg (0.30 mmol) of methyl bromoacetate. The mixture was stirred at room temperature until tlc indicated complete consumption of starting material (2 hrs). The reaction was diluted with $H_2O$ (10 mL) and was extracted with EtOAc (3x10 mL). The combined organic layers were washed with $H_2O$ (3 x2 0 mL), dried over $Na_2SO_4$, filtered, and concentrated *in vacuo*. The residue was purified by radial chromatography ($SiO_2$; 2.5% MeOH in $CH_2Cl_2$) to afford 50 mg (0.13 mmol; 51%) of [9-benzyl-4-carbamoyl-1,2,3,4-tetrahydrocarbaole-5-yl]oxyacetic acid, methyl ester as an oil. $^1H$ NMR ($CDCl_3$) δ 7.33-7.21 (m, 3H), 7.05-6.98 (m, 3H), 6.98 (d, 1H, J=7.4 Hz), 6.46 (br s, 1H), 6.37 (d, 1H, J=7.7 Hz), 5.52 (br s, 1H), 5.23 (d, 1H, J=4.9 Hz) 4.79-4.70 (m, 2H), 4.20-4.15 (m, 1H), 3.81 (s, 3H), 2.79-2.69 (m, 1H), 2.63-2.49 (m, 1H), 2.43-2.35 (m, 1H), 2.25-2.09 (m, 1H), and 1.99-1.78 (m, 2H). IR ($CHCl_3$, $cm^{-1}$) 1759, 1670, 1497, 1453, 1440, and 1132. MS (ES) m/e 393 (M+1).

| Elemental Analysis for $C_{23}H_{24}N_2O_4$: | | | |
|---|---|---|---|
| Calculated | C, 70.39; | H, 6.16; | N, 7.14. |
| Found | C, 70.29; | H, 6.31; | N, 7.08. |

D. [9-Benzyl-4-carbamoyl-1,2,3,4-tetrahydrocarbaole-5-yl]oxyacetic acid

A solution of 30 mg (0.076 mmol) of [9-benzyl-4-carbamoyl-1,2,3,4-tetrahydrocarbaole-5-yl]oxyacetic acid, methyl ester in 1.0 mL of THF and 1.0 mL of MeOH was treated with 0.2 mL of 1 N aq LiOH (0.2 mmol). The mixture was stirred for 18 hrs. An additional 0.2 mL of 1 N aq LiOH (0.2 mmol) was added and stirring continued. After 1 hr, the mixture was concentrated *in vacuo*. The residue was dissolved in 2.0 mL of $H_2O$ and the solution acidified with 0.2 N aq HCl. The solid was filtered and dried to afford 25 mg of [9-benzyl-4-carbamoyl-1,2,3,4-tetrahydrocarbaole-5-yl] oxyacetic acid as a white solid. $^1H$ NMR (DMSO-d6) δ 7.36-7.12 (m, 5H), 7.05-6.83 (m, 5H), 6.71 (br s, 1H), 6.35 (d, 1H, J=7.6 Hz), 5.27 (s, 2H), 4.64 (s, 2H), 3.93-3.84 (m, 2H), 2.75-2.64 (m, 1H), 2.16-1.95 (m, 2H), 1.81-1.64 (m, 2H) and 1 proton masked by $H_2O$ peak between 2.58-2.40. IR (KBr, $cm^{-1}$) 3435, 2936, 1722, 1644, 1586, 1566, 1495,

1451, 1354, 1227, 1134, 730, 716, and 698. MS (ES) m/e 377 (M-1) and 379 (M+1).

| Elemental Analysis for $C_{22}H_{22}N_2O_4$: | | | |
|---|---|---|---|
| Calculated | C, 69.83; | H, 5.86; | N, 7.40. |
| Found | C, 70.11; | H, 5.76; | N, 7.12. |

The compounds described herein are believed to achieve their beneficial therapeutic action principally by direct inhibition of human $sPLA_2$, and not by acting as antagonists for arachidonic acid, nor other active agents below arachidonic acid in the arachidonic acid cascade, such as 5-lipoxygenases, cyclooxygenases, etc.

The use of the invention for inhibiting $sPLA_2$ mediated release of fatty acids comprises the manufacture of a medicament adapted for contacting $sPLA_2$ with a compound as identified in Claim 1 or 2 below.

The compounds of the invention may be used for the manufacture of a medicament for treating a mammal (e.g., a human) to alleviate the pathological effects of septic shock, adult respiratory distress syndrome, pancreatitus, trauma, bronchial asthma, allergic rhinitis, and rheumatoid arthritis; wherein the medicament is adapted for administering to the mammal a compound as identified in Claim 1 or 2 below in a therapeutically effective amount. A "therapeutically effective" amount is an amount sufficient to inhibit $sPLA_2$ mediated release of fatty acid and to thereby inhibit or prevent the arachidonic acid cascade and its deleterious products. The therapeutic amount of compound of the invention needed to inhibit $sPLA_2$ may be readily determined by taking a sample of body fluid and assaying it for $sPLA_2$ content by conventional methods.

Throughout this document, the person or animal to be treated will be described as a "mammal", and it will be understood that the most preferred subject is a human. However it must be noted that the study of adverse conditions of the central nervous system in non-human animals is only now beginning, an that some instances of such treatments are coming into use. Accordingly, use of the present compounds in non-human animals is contemplated. It will be understood that the dosage ranges for other animals will necessarily be quite different from the doses administered to humans, and accordingly that the dosage ranges described be recalculated. For example, a small dog may be only $1/10^{th}$ of a typical human's size, and it will therefore be necessary for a much smaller dose to be used. The determination of an effective amount for a certain non-human animal is carried out in the same manner described below in the case of humans, and veterinarians are well accustomed to such determinations.

As previously noted the compounds of this invention are useful for inhibiting $sPLA_2$ mediated release of fatty acids such as arachidonic acid. By the term, "inhibiting" is meant the prevention or therapeutically significant reduction in release of $sPLA_2$ initiated fatty acids by the compounds of the invention. By "pharmaceutically acceptable" it is meant the carrier, diluent or excipient must be compatible with the other ingredients of the formulation and not deleterious to the recipient thereof.

In general, the compounds of the invention are most desirably administered at a dose that will generally afford effective results without causing any serious side effects and can be administered either as a single unit dose, or if desired, the dosage may be divided into convenient subunits administered at suitable times throughout the day.

The specific dose of a compound administered according to this invention to obtain therapeutic or prophylactic effects will, of course, be determined by the particular circumstances surrounding the case, including, for example, the route of administration, the age, weight and response of the individual patient, the condition being treated and the severity of the patient's symptoms. Typical daily doses will contain a non-toxic dosage level of from about 0.01 mg/kg to about 50 mg/kg of body weight of an active compound of this invention.

Preferably the pharmaceutical formulation is in unit dosage form. The unit dosage form can be a capsule or tablet itself, or the appropriate number of any of these. The quantity of active ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

A "chronic" condition means a deteriorating condition of slow progress and long continuance. As such, it is treated when it is diagnosed and continued throughout the course of the disease. An "acute" condition is an exacerbation of short course followed by a period of remission. In an acute event, compound is administered at the onset of symptoms and discontinued when the symptoms disappear.

Pancreatitis, trauma-induced shock, bronchial asthma, allergic rhinitis and rheumatoid arthritis may occur as an acute event or a chronic event. Thus, the treatment of these conditions contemplates both acute and chronic forms. Septic shock and adult respiratory distress, on the other hand, are acute conditions treated when diagnosed.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the compounds of the invention together with a pharmaceutically acceptable carrier or diluent therefor.

The present pharmaceutical formulations are prepared by known procedures using well known and readily available ingredients.

In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from about 1 to about 99 weight percent of the active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The active ingredient can be dissolved or suspended in a pharmaceutically acceptable carrier, such as sterile water, sterile organic solvent or a mixture of both. The active ingredient can often be dissolved in a suitable organic solvent, for instance aqueous propylene glycol. Other compositions can be made by dispersing the finely divided active ingredient in aqueous starch or sodium carboxymethyl cellulose solution or in a suitable oil.

The following pharmaceutical formulations 1 through 8 are illustrative only and are not intended to limit the scope of the invention in any way. "Active ingredient", refers to a compound according to Claim 1 or 2 below or a pharmaceutically acceptable salt, solvate, or prodrug thereof.

Formulation 1

Hard gelatin capsules are prepared using the following ingredients:

| | Quantity (mg/capsule) |
|---|---|
| Compound of Example 5 | 250 |
| Starch, dried | 200 |
| Magnesium stearate | 10 |
| Total | 460 mg |

Formulation 2

A tablet is prepared using the ingredients below:

| | Quantity (mg/tablet) |
|---|---|
| Compound of Example 10 | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

Formulation 3

An aerosol solution is prepared containing the following components:

| | Weight |
|---|---|
| Compound of Example 15 | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | |
|---|---|
| Compound of Example 25 | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| Compound of Example 30 | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| Compound of Example 35 | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, each containing 50 mg of active ingredient per 5 ml dose, are made as follows:

| | |
|---|---|
| Compound of Example 40 | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| Compound of Example 45 | 100 mg |
| Isotonic saline | 1,000 ml |

The solution of the above ingredients generally is administered intravenously to a subject at a rate of 1 ml per minute.

Assay Example 1

The following chromogenic assay procedure was used to identify and evaluate inhibitors of recombinant human secreted phospholipase $A_2$. The assay described herein has been adapted for high volume screening using 96 well microtiter plates. A general description of this assay method is found in the article, "Analysis of Human Synovial Fluid Phospholipase $A_2$ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, Analytical Biochemistry, 204, pp. 190-197, 1992 (the disclosure of which is incorporated herein by reference):
Reagents:

REACTION BUFFER -

$CaCl_2.2H_2O$ (1.47 g/L)
KCl (7.455 g/L)
Bovine Serum Albumin (fatty acid free) (1 g/L) (Sigma A-7030, product of Sigma Chemical Co. St. Louis MO, USA)
TRIS HCl (3.94 g/L)
pH 7.5 (adjust with NaOH)

ENZYME BUFFER -

0.05 $NaOAc.3H_2O$, pH 4.5
0.2 NaCl
Adjust pH to 4.5 with acetic acid

DTNB -

5,5'-dithiobis-2-nitrobenzoic acid

RACEMIC DIHEPTANOYL THIO - PC

racemic 1,2-bis(heptanoylthio)-1,2-dideoxy-*sn*-glycero-3-phosphorylcholine
TRITON X-100™ prepare at 6.249 mg/ml in reaction buffer to equal 10uM
TRITON X-100™ is a polyoxy ethylene non-ionic detergent supplied by
Pierce Chemical Company,
3747 N. Meridian Road, Rockford, Illinois 61101.

REACTION MIXTURE -

A measured volume of racemic dipheptanoyl thio PC supplied in chloroform at a concentration of 100 mg/ml is taken to dryness and redissolved in 10 millimolar TRITON X-100™ nonionic detergent aqueous solution. Reaction Buffer is added to the solution, then DTNB to give the Reaction Mixture.
The reaction mixture thus obtained contains 1mM diheptanoly thio-PC substrate, 0.29 mm Triton X-100™ detergent, and 0.12 mm DTMB in a buffered aqueous solution at pH 7.5.

Assay Procedure:

1. Add 0.2 ml reaction mixture to all wells;
2. Add 10 ul test compound (or solvent blank) to appropriate wells, mix 20 seconds;
3. Add 50 nanograms of $sPLA_2$ (10 microliters) to appropriate wells;
4. Incubate plate at 40°C for 30 minutes;
5. Read absorbance of wells at 405 nanometers with an automatic plate reader.

All compounds were tested in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, $IC_{50}$ values were determined. Typically, the $IC_{50}$ values were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of $IC_{50}$ values. $IC_{50}$ were determined by plotting log concentration versus inhibition values in the range from 10-90% inhibition.
Compounds of the instant invention were tested in Assay Example 1 and were found to be effective at concentrations of less than 100µM.

Assay Example 2

Method:

Male Hartley strain guinea pigs (500-700g) were killed by cervical dislocation and their heart and lungs removed intact and placed in aerated (95% $O_2$:5% $CO_2$) Krebs buffer. Dorsal pleural strips (4x1x25mm) were dissected from intact parenchymal segments (8x4x25mm) cut parallel to the outer edge of the lower lung lobes. Two adjacent pleural strips, obtained from a single lobe and representing a single tissue sample, were tied at either end and independently attached to a metal support rod. One rod was attached to a Grass force-displacement transducer Model FTO3C, product of Grass Medical Instruments Co., Quincy, MA, USA). Changes in isometric tension were displayed on a monitor and thermal recorder (product of Modular Instruments, Malvern, PA). All tissues were placed in 10 ml

jacketed tissue baths maintained at 37°C. The tissue baths were continuously aerated and contained a modified Krebs solution of the following composition (millimolar) NaCl, 118.2; KCl, 4.6; $CaCl_2 \cdot 2H_2O$, 2.5; $MgSO_4 \cdot 7H_2O$, 1.2; $NaHCO_3$, 24.8; $KH_2PO_4$, 1.0; and dextrose, 10.0. Pleural strips from the opposite lobes of the lung were used for paired experiments. Preliminary data generated from tension/response curves demonstrated that resting tension of 800mg was optimal. The tissues were allowed to equilibrate for 45 min. as the bath fluid was changed periodically.

Cumulative concentration-response curves:

Initially tissues were challenged 3 times with KCl (40 mM) to test tissue viability and to obtain a consistent response. After recording the maximal response to KCl, the tissues were washed and allowed to return to baseline before the next challenge. Cumulative concentration-response curves were obtained from pleural strips by increasing the agonist concentration ($sPLA_2$) in the tissue bath by half-$log_{10}$ increments while the previous concentration remained in contact with the tissues (Ref.1, supra.). Agonist concentration was increased after reaching the plateau of the contraction elicited by the preceding concentration. One concentration-response curve was obtained from each tissue. To minimize variability between tissues obtained from different animals, contractile responses were expressed as a percentage of the maximal response obtained with the final KCl challenge. When studying the effects of various drugs on the contractile effects of $sPLA_2$, the compounds and their respective vehicles were added to the tissues 30 minutes prior to starting the $sPLA_2$ concentration-response curves.

Statistical analysis:

Data from different experiments were pooled and presented as a percentage of the maximal KCl responses (mean ± S.E.). To estimate the drug induced rightward shifts in the concentration response curves, the curves were analyzed simultaneously using statistical nonlinear modeling methods similar to those described by Waud (1976), Equation 26, p. 163, (Ref.2). The model includes four parameters: the maximum tissue response which was assumed the same for each curve, the $ED_{50}$ for the control curve, the steepness of the curves, and the $pA_2$, the concentration of antagonist that requires a two-fold increase in agonist to achieve an equivalent response. The Schild slope was determined to be 1, using statistical nonlinear modeling methods similar to those described by Waud (1976), Equation 27, p. 164 (Ref. 2). The Schild slope equal to 1 indicates the model is consistent with the assumptions of a competitive antagonist; therefore, the pA2 may be interpreted as the apparent $K_B$, the dissociation constant of the inhibitor.

To estimate the drug-induced suppression of the maximal responses, $sPLA_2$ responses (10 ug/ml) were determined in the absence and presence of drug, and percent suppression was calculated for each pair of tissues. Representative examples of inhibitory activities are presented in Table 2, below.

Ref. 1 - Van, J.M.: Cumulative dose-response curves. II. Technique for the making of dose-response curves in isolated organs and the evaluation of drug parameters. Arch. Int. Pharmacodyn. Ther., 143: 299-330, 1963.

Ref. 2 - Waud, D.: Analysis of dose-response relationships. in Advances in General and Cellular Pharmacology eds Narahashi, Bianchi 1:145-178, 1976.

Compounds of the instant invention were tested in Assay Example 2 and were found to be effective at concentrations below 20μM.

Assay Example 3

$sPLA_2$ Transgenic Mice Assay

Materials & Methods

The mice utilized in these studies were mature, 6-8 month old, $ZnSO_4$-stimulated, hemizygous line 2608[a] transgenic mice (Fox et. al. 1996). Transgenic mice from this line express human $sPLA_2$ in the liver and other tissues and typically achieve levels of human $sPLA_2$ in their circulation of approximately 173 ± 10 ng/ml when maximally stimulated with $ZnSO_4$ (Fox, *et al*. 1996). The mice were housed under constant humidity and temperature and received food and water ad libitum. Animal room lighting was maintained on a 12-hour light/dark cycle and all experiments were performed at the same time of the day during the early morning light period.

For intravenous testing, compounds or vehicle were administered as an IV bolus via the tail vein in a volume of 0.15 ml. Vehicle consisted of 1-5% dimethylsulfoxide, 1-5% ethanol and 10-30% polyethylene glycol 300 in $H_2O$; the concentrations of these ingredients were adjusted according to the solubility of the compound. Mice were bled retro-orbitally prior to drug or vehicle administration and 30 minutes, 2 and 4 hours thereafter. Three to six mice were used for each dose. $PLA_2$ catalytic activity in the serum was assayed with a modified phosphatidylcholine/deoxycholine mixed micelle assay (Fox, *et al*. 1996, Schadlich, *et al*., 1987) utilizing 3 mM sodium deoxycholate and 1 mM 1-palmitoyl-2-oleoyl-sn-glycero-3-phosphocholine.

For oral testing, compounds were dissolved in 1-5% ethanol/10-30% polyethylene glycol 300 in $H_2O$ or were suspended in 5% dextrose in $H_2O$ and administered by oral gavage. Serum was prepared from retro-orbital blood and assayed for $PLA_2$ catalytic activity as above.

References

Fox, N., M. Song, J. Schrementi, J. D. Sharp, D. L. White, D. W. Snyder, L. W. Hartley, D. G. Carlson, N. J. Bach, R. D. Dillard, S. E. Draheim, J. L. Bobbitt, L. Fisher and E. D. Mihelich. 1996.

Eur. J. Pharmacol. 308: 195.

Schadlich, H.R., M. Buchler, and H. G. Beger, 1987, J. Clin. Chem. Clin. Biochem. 25, 505.

Compounds of the instant invention were tested in Assay Example 3 and were found to be effective.

While the present invention has been illustrated above by certain specific embodiments, it is not intended that these specific examples should limit the scope of the invention as described in the appended claims.

## Claims

**1.** A compound which is selected from the group consisting of;

9-benzyl-5,7-dimethoxy-1,2,3,4-tetrahydrocarbazole-4-carboxylic acid hydrazide;
9-benzyl-5,7-dimethoxy-1,2,3,4-tetrahydrocarbazole-4-carboxamide;
[9-benzyl-4-carbamoyl-7-methoxy-1,2,3,4-tetrahydrocarbazol-5-yl]oxyacetic acid;
[9-benzyl-4-carbamoyl-7-methoxycarbazol-5-yl]oxyacetic acid;
methyl [9-benzyl-4-carbamoyl-7-methoxycarbazol-5-yl]oxyacetic acid;
9-benzyl-7-methoxy-5-cyanomethyloxy-1,2,3,4-tetrahydrocarbazole-4-carboxamide;
9-benzyl-7-methoxy-5-(1H-tetrazol-5-yl-methyl)oxy)-1,2,3,4-tetrahydrocarbazole-4-carboxamide;
{9-[(phenyl)methyl]-5-carbamoyl-2-methyl-carbazol-4-yl}oxyacetic acid;
{9-[(3-fluorophenyl)methyl]-5-carbamoyl-2-methylcarbazol-4-yl}oxyacetic acid;
{9-[(3-methylphenyl)methyl]-5-carbamoyl-2-methylcarbazol-4-yl}oxyacetic acid;
{9-[(phenyl)methyl]-5-carbamoyl-2-(4-trifluoromethylphenyl)-carbazol-4-yl}oxyacetic acid;
9-benzyl-5-(2-methanesulfonamido)ethyloxy-7-methoxy-1,2,3,4-tetrahydrocarbazole-4-carboxamide;
9-benzyl-4-(2-methanesulfonamido)ethyloxy-2-methoxycarbazole-5-carboxamide;
9-benzyl-4-(2-trifluoromethanesulfonamido)ethyloxy-2-methoxycarbazole-5-carboxamide;
9-benzyl-5-methanesulfonamidoylmethyloxy-7-methoxy-1,2,3,4-tetrahydrocarbazole-4-carboxamide;
9-benzyl-4-methanesulfonamidoylmethyloxy-carbazole-5-carboxamide;
[5-carbamoyl-2-pentyl-9-(phenylmethyl)carbazol-4-yl]oxyacetic acid;
[5-carbamoyl-2-(1-methylethyl)-9-(phenylmethyl)carbazol-4-yl]oxyacetic acid;
[5-carbamoyl-9-(phenylmethyl)-2-[(tri(-1-methylethyl)silyl)oxymethyl]carbazol-4-yl]oxyacetic acid;
[5-carbamoyl-2-phenyl-9-(phenylmethyl)carbazol-4-yl]oxyacetic acid;
[5-carbamoyl-2-(4-chlorophenyl)-9-(phenylmethyl)carbazol-4-yl]oxyacetic acid;
[5-carbamoyl-2-(2-furyl)-9-(phenylmethyl)carbazol-4-yl]oxyacetic acid;
[5-carbamoyl-9-(phenylmethyl)-2-[(tri(-1-methylethyl)silyl)oxymethyl]carbazol-4-yl]oxyacetic acid;
{9-[(2-Fluorophenyl)methyl]-5-carbamoylcarbazol-4-yl}oxyacetic acid;
{9-[(2-trifluoromethylphenyl)methyl]-5-carbamoylcarbazol-4-yl}oxyacetic acid;
{9-[(2-benzylphenyl)methyl]-5-carbamoylcarbazol-4-yl}oxyacetic acid;
{9-[(1-naphthyl)methyl]-5-carbamoylcarbazol-4-yl}oxyacetic acid;
{9-[(2-cyanophenyl)methyl]-5-carbamoylcarbazol-4-yl}oxyacetic acid;
{9-[(3-cyanophenyl)methyl]-5-carbamoylcarbazol-4-yl}oxyacetic acid;
{9-[(3,5-dimethylphenyl)methyl]-5-carbamoylcarbazol-4-yl}oxyacetic acid;
{9-[(3-iodophenyl)methyl]-5-carbamoylcarbazol-4-yl}oxyacetic acid;
{9-[(2-Chlorophenyl)methyl]-5-carbamoylcarbazol-4-yl}oxyacetic acid;
{9-[(2,3-difluorophenyl)methyl]-5-carbamoylcarbazol-4-yl}oxyacetic acid;
{9-[(2,6-difluorophenyl)methyl]-5-carbamoylcarbazol-4-yl}oxyacetic acid;
{9-[(2,6-dichlorophenyl)methyl]-5-carbamoylcarbazol-4-yl}oxyacetic acid;
{9-[(2-biphenyl)methyl]-5-carbamoylcarbazol-4-yl}oxyacetic acid;
{9-[(2-Biphenyl)methyl]-5-carbamoylcarbazol-4-yl}oxyacetic acid methyl ester;

[9-Benzyl-4-carbamoyl-1,2,3,4-tetrahydrocarbazole-5-yl]oxyacetic acid;
{9-[(2-Pyridyl)methyl]-5-carbamoylcarbazol-4-yl}oxyacetic acid;
{9-[(3-Pyridyl)methyl]-5-carbamoylcarbazol-4-yl}oxyacetic acid;
[9-benzyl-4-carbamoyl-8-methyl-1,2,3,4-tetrahydrocarbazol-5-yl]oxyacetic acid;
[9-benzyl-5-carbamoyl-1-methylcarbazol-4-yl]oxyacetic acid;
[9-benzyl-4-carbamoyl-8-fluoro-1,2,3,4-tetrahydrocarbazol-5-yl]oxyacetic acid;
[9-benzyl-4-carbamoyl-8-chloro-1,2,3,4-tetrahydrocarbazol-5-yl]oxyacetic acid;
[5-carbamoyl-9-(phenylmethyl)-2-[[(propen-3-yl)oxy]methyl]carbazol-4-yl]oxyacetic acid;
[5-carbamoyl-9-(phenylmethyl)-2-[(propyloxy)methyl]carbazol-4-yl]oxyacetic acid;
9-benzyl-7-methoxy-5-((carboxamidomethyl)oxy)-1,2,3,4-tetrahydrocarbazole-4-carboxamide;
9-benzyl-7-methoxy-5-cyanomethyloxy-carbazole-4-carboxamide;
9-benzyl-7-methoxy-5-((1H-tetrazol-5-yl-methyl)oxy)-carbazole-4-carboxamide;
9-benzyl-7-methoxy-5-((carboxamidomethyl)oxy)-carbazole-4-carboxamide; and
[9-Benzyl-4-carbamoyl-1,2,3,4-tetrahydrocarbazole-5-yl]oxyacetic acid

or a pharmaceutically acceptable racemate, solvate, tautomer, optical isomer, prodrug derivative selected from the methyl, ethyl, propyl, isopropyl, butyl, sec-butyl, tert-butyl, morpholinoethyloxy, diethylglycolamide or diethylaminocarbonyl methoxy ester or salt, thereof.

**2.** A compound which is selected from the group consisting of;

{9-[(phenyl)methyl]-5-carbamoyl-2-methyl-carbazol-4-yl}oxyacetic acid;
{9-[(3-fluorophenyl)methyl]-5-carbamoyl-2-methylcarbazol-4-yl}oxyacetic acid;
{9-[(3-methylphenyl)methyl]-5-carbamoyl-2-methylcarbazol-4-yl}oxyacetic acid;
{9-[(phenyl)methyl]-5-carbamoyl-2-(4-trifluoromethylphenyl)-carbazol-4-yl}oxyacetic acid;
9-benzyl-5-(2-methanesulfonamido)ethyloxy-7-methoxy-1,2,3,4-tetrahydrocarbazole-4-carboxamide;
9-benzyl-4-(2-methanesulfonamido)ethyloxy-2-methoxycarbazole-5-carboxamide;
9-benzyl-4-(2-trifluoromethanesulfonamido)ethyloxy-2-methoxycarbazole-5-carboxamide;
9-benzyl-5-methanesulfonamidoylmethyloxy-7-methoxy-1,2,3,4-tetrahydrocarbazole-4-carboxamide;
9-benzyl-4-methanesulfonamidoylmethyloxy-carbazole-5-carboxamide;
[5-carbamoyl-2-pentyl-9-(phenylmethyl)carbazol-4-yl]oxyacetic acid;
[5-carbamoyl-2-(1-methylethyl)-9-(phenylmethyl)carbazol-4-yl]oxyacetic acid;
[5-carbamoyl-9-(phenylmethyl)-2-[(tri(-1-methylethyl)silyl)oxymethyl]carbazol-4-yl]oxyacetic acid;
[5-carbamoyl-2-phenyl-9-(phenylmethyl)carbazol-4-yl]oxyacetic acid;
[5-carbamoyl-2-(4-chlorophenyl)-9-(phenylmethyl)carbazol-4-yl]oxyacetic acid;
[5-carbamoyl-2-(2-furyl)-9-(phenylmethyl)carbazol-4-yl]oxyacetic acid;
[5-carbamoyl-9-(phenylmethyl)-2-[(tri(-1-methylethyl)silyl)oxymethyl]carbazol-4-yl]oxyacetic acid;
{9-[(phenyl)methyl]-5-carbamoylcarbazol-4-yl}oxyacetic acid;
{9-[(3-fluorophenyl)methyl]-5-carbamoylcarbazol-4-yl}oxyacetic acid;
{9-[(3-chlorophenyl)methyl]-5-carbamoylcarbazol-4-yl}oxyacetic acid;
{9-[(3-phenoxyphenyl)methyl]-5-carbamoylcarbazol-4-yl}oxyacetic acid;
{9-[(2-Fluorophenyl)methyl]-5-carbamoylcarbazol-4-yl}oxyacetic acid;
{9-[(2-trifluoromethylphenyl)methyl]-5-carbamoylcarbazol-4-yl}oxyacetic acid;
{9-[(2-benzylphenyl)methyl]-5-carbamoylcarbazol-4-yl}oxyacetic acid;
{9-[(3-trifluoromethylphenyl)methyl]-5-carbamoylcarbazol-4-yl}oxyacetic acid;
{9-[(1-naphthyl)methyl]-5-carbamoylcarbazol-4-yl}oxyacetic acid;
{9-[(2-cyanophenyl)methyl]-5-carbamoylcarbazol-4-yl}oxyacetic acid;
{9-[(3-cyanophenyl)methyl]-5-carbamoylcarbazol-4-yl}oxyacetic acid;
{9-[(2-methylphenyl)methyl]-5-carbamoylcarbazol-4-yl}oxyacetic acid;
{9-[(3-methylphenyl)methyl]-5-carbamoylcarbazol-4-yl}oxyacetic acid;
{9-[(3,5-dimethylphenyl)methyl]-5-carbamoylcarbazol-4-yl}oxyacetic acid;
{9-[(3-iodophenyl)methyl]-5-carbamoylcarbazol-4-yl}oxyacetic acid;
{9-[(2-Chlorophenyl)methyl]-5-carbamoylcarbazol-4-yl}oxyacetic acid;
{9-[(2,3-difluorophenyl)methyl]-5-carbamoylcarbazol-4-yl}oxyacetic acid;
{9-[(2,6-difluorophenyl)methyl]-5-carbamoylcarbazol-4-yl}oxyacetic acid;
{9-[(2,6-dichlorophenyl)methyl]-5-carbamoylcarbazol-4-yl}oxyacetic acid;
{9-[(3-trifluoromethoxyphenyl)methyl]-5-carbamoylcarbazol-4-yl}oxyacetic acid;
{9-[(2-biphenyl)methyl]-5-carbamoylcarbazol-4-yl}oxyacetic acid;

{9-[(2-Biphenyl)methyl]-5-carbamoylcarbazol-4-yl}oxyacetic acid methyl ester;
[9-Benzyl-4-carbamoyl-1,2,3,4-tetrahydrocarbazole-5-yl]oxyacetic acid;
{9-[(2-Pyridyl)methyl]-5-carbamoylcarbazol-4-yl}oxyacetic acid;
{9-[(3-Pyridyl)methyl]-5-carbamoylcarbazol-4-yl}oxyacetic acid;
[9-benzyl-4-carbamoyl-8-methyl-1,2,3,4-tetrahydrocarbazol-5-yl]oxyacetic acid;
[9-benzyl-5-carbamoyl-1-methylcarbazol-4-yl]oxyacetic acid;
[9-benzyl-4-carbamoyl-8-fluoro-1,2,3,4-tetrahydrocarbazol-5-yl]oxyacetic acid;
[9-benzyl-5-carbamoyl-1-fluorocarbazol-4-yl]oxyacetic acid;
[9-benzyl-4-carbamoyl-8-chloro-1,2,3,4-tetrahydrocarbazol-5-yl]oxyacetic acid;
[9-benzyl-5-carbamoyl-1-chlorocarbazol-4-yl]oxyacetic acid;
[9-[(Cyclohexyl)methyl]-5-carbamoylcarbazol-4-yl]oxyacetic acid;
[9-[(Cyclopentyl)methyl]-5-carbamoylcarbazol-4-yl]oxyacetic acid;
[5-carbamoyl-9-(phenylmethyl)-2-(2-thienyl)carbazol-4-yl]oxyacetic acid;
[5-carbamoyl-9-(phenylmethyl)-2-[[(propen-3-yl)oxy]methyl]carbazol-4-yl]oxyacetic acid;
[5-carbamoyl-9-(phenylmethyl)-2-[(propyloxy)methyl]carbazol-4-yl]oxyacetic acid;
9-benzyl-7-methoxy-5-((carboxamidomethyl)oxy)-1,2,3,4-tetrahydrocarbazole-4-carboxamide;
9-benzyl-7-methoxy-5-cyanomethyloxy-carbazole-4-carboxamide;
9-benzyl-7-methoxy-5-((1H-tetrazol-5-yl-methyl)oxy)-carbazole-4-carboxamide;
9-benzyl-7-methoxy-5-((carboxamidomethyl)oxy)-carbazole-4-carboxamide; and
[9-Benzyl-4-carbamoyl-1,2,3,4-tetrahydrocarbazole-5-yl]oxyacetic acid

or a pharmaceutically acceptable racemate, solvate, tautomer, optical isomer, prodrug derivative selected from the methyl, ethyl, propyl, isopropyl, butyl, sec-butyl, tert-butyl, morpholinoethyloxy, diethylglycolamide or diethylaminocarbonyl-methoxy ester or salt, thereof.

**3.** A compound as claimed in Claim 1 or 2 wherein the prodrug derivative is a methyl, ethyl, propyl, isopropyl, butyl, morpholinoethyloxy or diethylglycolamide ester.

**4.** A pharmaceutical formulation comprising a compound as claimed in Claim 1 or 2 together with a pharmaceutically acceptable carrier or diluent therefor.

**5.** A pharmaceutical formulation adapted for the treatment of a condition associated with inhibiting $sPLA_2$, containing a compound as claimed in Claim 1 or 2 together with a pharmaceutically acceptable carrier or diluent therefor.

**6.** The use of a compound of Claim 1 or 2 or a pharmaceutically acceptable racemate, solvate, tautomer, optical isomer, prodrug derivative selected from the methyl, ethyl, propyl, isopropyl, butyl, sec-butyl, tert-butyl, morpholinoethyloxy, diethylglycolamide or diethylaminocarbonyl-methoxy ester or salt, thereof for the manufacture of a medicament for selectively inhibiting $sPLA_2$ in a mammal.

**7.** The use as claimed in Claim 6 wherein the mammal is a human.

**8.** The use of a compound as claimed in Claim 1 or 2 for the manufacture of a medicament adapted for the administration to a mammal of said compound in an amount sufficient to inhibit $sPLA_2$ mediated release of fatty acid and to thereby inhibit or prevent the arachidonic acid cascade and its deleterious products, for alleviating the pathological effects of $sPLA_2$ related diseases.

**9.** The use of a compound as claimed in Claim 1 or 2 for the manufacture of a medicament for alleviating the pathological effects of $sPLA_2$ related diseases.

**10.** The use of a compound as claimed in Claim 1 or 2 for the manufacture of a medicament adapted for contacting $sPLA_2$ with said compound, for inhibiting $sPLA_2$.

**11.** The use of a compound as claimed in Claim 1 or 2 or a pharmaceutically acceptable racemate, solvate, tautomer, optical isomer, prodrug derivative selected from the methyl, ethyl, propyl, isopropyl, butyl, sec-butyl, tert-butyl, morpholinoethyloxy, diethylglycolamide or diethylaminocarbonyl-methoxy ester or salt, thereof, for the manufacture of a medicament for treating sepsis, septic shock, rheumatoid arthritis, osteoarthritis, stroke, apoptosis, asthma, chronic bronchitis, acute bronchitis, cystic fibrosis, inflammatory bowel disease, or pancreatitis.

**12.** The use as claimed in any one of Claims 6 to 11 for alleviating the pathological effects of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis;
and related diseases.

**Patentansprüche**

**1.** Verbindung die ausgewählt ist aus der Gruppe, welche besteht aus

9-Benzyl-5,7-dimethoxy-1,2,3,4-tetrahydrocarbazol-4-carbonsäurehydrazid,
9-Benzyl-5,7-dimethoxy-1,2,3,4-tetrahydrocarbazol-4-carboxamid,
[9-Benzyl-4-carbamoyl-7-methoxy-1,2,3,4-tetrahydrocarbazol-5-yl]oxyessigsäure,
[9-Benzyl-4-carbamoyl-7-methoxycarbazol-5-yl]oxyessigsäure,
Methyl-[9-benzyl-4-carbamoyl-7-methoxycarbazol-5-yl]oxyessigsäure,
9-Benzyl-7-methoxy-5-cyanomethyloxy-1,2,3,4-tetrahydrocarbazol-4-carboxamid,
9-Benzyl-7-methoxy-5-(1H-tetrazol-5-yl-methyl)oxy-1,2,3,4-tetrahydrocarbazol-4-carboxamid,
{9-[(Phenyl)methyl]-5-carbamoyl-2-methyl-carbazol-4-yl}oxyessigsäure,
{9-[(3-Fluorphenyl)methyl]-5-carbamoyl-2-methyl-carbazol-4-yl}oxyessigsäure,
{9-[(3-Methylphenyl)methyl]-5-carbamoyl-2-methyl-carbazol-4-yl}oxyessigsäure,
{9-[(Phenyl)methyl]-5-carbamoyl-2-(4-trifluormethylphenyl)-carbazol-4-yl}oxyessigsäure,
9-Benzyl-5-(2-methansulfonamido)ethyloxy-7-methoxy-1,2,3,4-tetrahydrocarbazol-4-carboxamid,
9-Benzyl-4-(2-methansulfonamido)ethyloxy-2-methoxycarbazol-5-carboxamid,
9-Benzyl-4-(2-trifluormethansulfonamido)ethyloxy-2-methoxycarbazol-5-carboxamid,
9-Benzyl-5-methansulfonamidoylmethyloxy-7-methoxy-1,2,3,4-tetrahydrocarbazol-4-carboxamid,
9-Benzyl-4-methansulfonamidoylmethyloxy-carbazol-5-carboxamid,
[5-Carbamoyl-2-pentyl-9-(phenylmethyl)carbazol-4-yl]oxyessigsäure,
[5-Carbamoyl-2-(1-methylethyl)-9-(phenylmethyl)carbazol-4-yl]oxyessigsäure,
[5-Carbamoyl-9-(phenylmethyl)-2-[(tri(1-methylethyl)silyl)oxymethyl]carbazol-4-yl]oxyessigsäure,
[5-Carbamoyl-2-phenyl-9-(phenylmethyl)carbazol-4-yl]oxyessigsäure,
[5-Carbamoyl-2-(4-chlorphenyl)-9-(phenylmethyl)carbazol-4-yl]oxyessigsäure,
[5-Carbamoyl-2-(2-furyl)-9-(phenylmethyl)carbazol-4-yl]oxyessigsäure,
[5-Carbamoyl-9-(phenylmethyl)-2-[(tri(1-methylethylsilyl)oxymethyl]carbazol-4-yl]oxyessigsäure,
{9-[(2-Fluorphenyl)methyl]-5-carbamoylcarbazol-4-yl}oxyessigsäure,
{9-[(2-Trifluormethylphenyl)methyl]-5-carbamoylcarbazol-4-yl}oxyessigsäure,
{9-[(2-Benzylphenyl)methyl]-5-carbamoylcarbazol-4-yl}oxyessigsäure,
{9-[(1-Naphthyl)methyl]-5-carbamoylcarbazol-4-yl}oxyessigsäure,
{9-[(2-Cyanophenyl)methyl]-5-carbamoylcarbazol-4-yl}oxyessigsäure,
{9-[(3-Cyanophenyl)methyl]-5-carbamoylcarbazol-4-yl}oxyessigsäure,
{9-[(3,5-Dimethylphenyl)methyl]-5-carbamoylcarbazol-4-yl}oxyessigsäure,
{9-[(3-Iodphenyl)methyl]-5-carbamoylcarbazol-4-yl}oxyessigsäure,
{9-[(2-Chlorphenyl)methyl]-5-carbamoylcarbazol-4-yl}oxyessigsäure,
{9-[(2,3-Difluorphenyl)methyl]-5-carbamoylcarbazol-4-yl}oxyessigsäure,
{9-[(2,6-Difluorphenyl)methyl]-5-carbamoylcarbazol-4-yl}oxyessigsäure,
{9-[(2,6-Dichlorphenyl)methyl]-5-carbamoylcarbazol-4-yl}oxyessigsäure,
{9-[(2-Biphenyl)methyl]-5-carbamoylcarbazol-4-yl}oxyessigsäure,
{9-[(2-Biphenyl)methyl]-5-carbamoylcarbazol-4-yl}oxyessigsäuremethylester,

[9-Benzyl-4-carbamoyl-1,2,3,4-tetrahydrocarbazol-5-yl]oxyessigsäure,
{9-[(2-Pyridyl)methyl]-5-carbamoylcarbazol-4-yl}oxyessigsäure,
{9-[(3-Pyridyl)methyl]-5-carbamoylcarbazol-4-yl}oxyessigsäure,
[9-Benzyl-4-carbamoyl-8-methyl-1,2,3,4-tetrahydrocarbazol-5-yl]oxyessigsäure,
[9-Benzyl-5-carbamoyl-1-methylcarbazol-4-yl]oxyessigsäure,
[9-Benzyl-4-carbamoyl-8-fluor-1,2,3,4-tetrahydrocarbazol-5-yl]oxyessigsäure,
[9-Benzyl-4-carbamoyl-8-chlor-1,2,3,4-tetrahydrocarbazol-5-yl]oxyessigsäure,
[5-Carbamoyl-9-(phenylmethyl)-2-[[(propen-3-yl)oxy]methyl]carbazol-4-yl]oxyessigsäure,
[5-Carbamoyl-9-(phenylmethyl)-2-[(propyloxy)methyl]carbazol-4-yl]oxyessigsäure,
9-Benzyl-7-methoxy-5-((carboxamidomethyl)oxy)-1,2,3,4-tetrahydrocarbazol-4-carboxamid,
9-Benzyl-7-methoxy-5-cyanomethyloxycarbazol-4-carboxamid,
9-Benzyl-7-methoxy-5-((1H-tetrazol-5-yl-methyl)oxy)-carbazol-4-carboxamid,
9-Benzyl-7-methoxy-5-((carboxamidomethyl)oxy)-carbazol-4-carboxamid und
[9-Benzyl-4-carbamoyl-1,2,3,4-tetrahydrocarbazol-5-yl]oxyessigsäure

oder ein pharmazeutisch annehmbares Razemat, Solvat, Tautomer, optisches Isomer, Prodrugderivat, ausgewählt aus den Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyestern oder einem Salz hiervon.

**2.** Verbindung, die aus der Gruppe ausgewählt ist, die besteht aus:

{9-[(Phenyl)methyl]-5-carbamoyl-2-methyl-carbazol-4-yl}oxyessigsäure,
{9-[(3-Fluorphenyl)methyl]-5-carbamoyl-2-methyl-carbazol-4-yl}oxyessigsäure,
{9-[(3-Methylphenyl)methyl]-5-carbamoyl-2-methyl-carbazol-4-yl}oxyessigsäure,
{9-[(Phenyl)methyl]-5-carbamoyl-2-(4-trifluormethylphenyl)-carbazol-4-yl}oxyessigsäure,
9-Benzyl-5-(2-methansulfonamido)ethyloxy-7-methoxy-1,2,3,4-tetrahydrocarbazol-4-carboxamid,
9-Benzyl-4-(2-methansulfonamido)ethyloxy-2-methoxycarbazol-5-carboxamid,
9-Benzyl-4-(2-trifluormethansulfonamido)ethyloxy-2-methoxycarbazol-5-carboxamid,
9-Benzyl-5-methansulfonamidoylmethyloxy-7-methoxy-1,2,3,4-tetrahydrocarbazol-4-carboxamid,
9-Benzyl-4-methansulfonamidoylmethyloxy-carbazol-5-carboxamid,
[5-Carbamoyl-2-pentyl-9-(phenylmethyl)carbazol-4-yl]oxyessigsäure,
[5-Carbamoyl-2-(1-methylethyl)-9-(phenylmethyl)carbazol-4-yl]oxyessigsäure,
[5-Carbamoyl-9-(phenylmethyl)-2-[(tri(1-methylethyl)silyl)oxymethyl]carbazol-4-yl]oxyessigsäure,
[5-Carbamoyl-2-phenyl-9-(phenylmethyl)carbazol-4-yl]oxyessigsäure,
[5-Carbamoyl-2-(4-chlorphenyl)-9-(phenylmethyl)carbazol-4-yl]oxyessigsäure,
[5-Carbamoyl-2-(2-furyl)-9-(phenylmethyl)carbazol-4-yl]oxyessigsäure,
[5-Carbamoyl-9-(phenylmethyl)-2-[(tri(1-methylethylsilyl)oxymethyl]carbazol-4-yl]oxyessigsäure,
{9-[(Phenyl)methyl]-5-carbamoylcarbazol-4-yl}oxyessigsäure,
{9-[(3-Fluorphenyl)methyl]-5-carbamoylcarbazol-4-yl}oxyessigsäure,
{9-[(3-Chlorphenyl)methyl]-5-carbamoylcarbazol-4-yl}oxyessigsäure,
{9-[(3-Phenoxyphenyl)methyl]-5-carbamoylcarbazol-4-yl}oxyessigsäure,
{9-[(2-Fluorphenyl)methyl]-5-carbamoylcarbazol-4-yl}oxyessigsäure,
{9-[(2-Trifluormethylphenyl)methyl]-5-carbamoylcarbazol-4-yl}oxyessigsäure,
{9-[(2-Benzylphenyl)methyl]-5-carbamoylcarbazol-4-yl}oxyessigsäure,
{9-[(3-Trifluormethylphenyl)methyl]-5-carbamoylcarbazol-4-yl}oxyessigsäure,
{9-[(1-Naphthyl)methyl]-5-carbamoylcarbazol-4-yl}oxyessigsäure,
{9-[(2-Cyanophenyl)methyl]-5-carbamoylcarbazol-4-yl}oxyessigsäure,
{9-[(3-Cyanophenyl)methyl]-5-carbamoylcarbazol-4-yl}oxyessigsäure,
{9-[(2-Methylphenyl)methyl]-5-carbamoylcarbazol-4-yl}oxyessigsäure,
{9-[(3-Methylphenyl)methyl]-5-carbamoylcarbazol-4-yl}oxyessigsäure,
{9-[(3,5-Dimethylphenyl)methyl]-5-carbamoylcarbazol-4-yl}oxyessigsäure,
{9-[(3-Iodphenyl)methyl]-5-carbamoylcarbazol-4-yl}oxyessigsäure,
{9-[(2-Chlorphenyl)methyl]-5-carbamoylcarbazol-4-yl}oxyessigsäure,
{9-[(2,3-Difluorphenyl)methyl]-5-carbamoylcarbazol-4-yl}oxyessigsäure,
{9-[(2,6-Difluorphenyl)methyl]-5-carbamoylcarbazol-4-yl}oxyessigsäure,
{9-[(2,6-Dichlorphenyl)methyl]-5-carbamoylcarbazol-4-yl}oxyessigsäure,
{9-[(3-Trifluormethoxyphenyl)methyl]-5-carbamoylcarbazol-4-yl}oxyessigsäure,
{9-[(2-Biphenyl)methyl]-5-carbamoylcarbazol-4-yl}oxyessigsäure,

{9-[(2-Biphenyl)methyl]-5-carbamoylcarbazol-4-yl}oxyessigsäuremethylester,
[9-Benzyl-4-carbamoyl-1,2,3,4-tetrahydrocarbazol-5-yl]oxyessigsäure,
{9-[(2-Pyridyl)methyl]-5-carbamoylcarbazol-4-yl}oxyessigsäure,
{9-[(3-Pyridyl)methyl]-5-carbamoylcarbazol-4-yl}oxyessigsäure,
[9-Benzyl-4-carbamoyl-8-methyl-1,2,3,4-tetrahydrocarbazol-5-yl]oxyessigsäure,
[9-Benzyl-5-carbamoyl-1-methylcarbazol-4-yl]oxyessigsäure,
[9-Benzyl-4-carbamoyl-8-fluor-1,2,3,4-tetrahydrocarbazol-5-yl]oxyessigsäure,
[9-Benzyl-5-carbamoyl-1-fluorcarbazol-4-yl]oxyessigsäure,
[9-Benzyl-4-carbamoyl-8-chlor-1,2,3,4-tetrahydrocarbazol-5-yl]oxyessigsäure,
[9-Benzyl-5-carbamoyl-1-chlorcarbazol-4-yl]oxyessigsäure,
{9-[(Cyclohexyl)methyl]-5-carbamoylcarbazol-4-yl}oxyessigsäure,
{9-[(Cyclopentyl)methyl]-5-carbamoylcarbazol-4-yl}oxyessigsäure,
[5-Carbamoyl-9-(phenylmethyl)-2-(2-thienyl)carbazol-4-yl]oxyessigsäure,
[5-Carbamoyl-9-(phenylmethyl)-2-[[(propen-3-yl)oxy]methyl]carbazol-4-yl]oxyessigsäure,
[5-Carbamoyl-9-(phenylmethyl)-2-[(propyloxy)methyl]carbazol-4-yl]oxyessigsäure,
9-Benzyl-7-methoxy-5-((carboxamidomethyl)oxy)-1,2,3,4-tetrahydrocarbazol-4-carboxamid,
9-Benzyl-7-methoxy-5-cyanomethyloxy-carbazol-4-carboxamid,
9-Benzyl-7-methoxy-5-((1H-tetrazol-5-yl-methyl)oxy)-carbazol-4-carboxamid,
9-Benzyl-7-methoxy-5-((carboxamidomethyl)oxy)carbazol-4-carboxamid und
[9-Benzyl-4-carbamoyl-1,2,3,4-tetra-hydrocarbazol-5-yl]oxyessigsäure,

oder ein pharmazeutisch annehmbares Razemat, Solvat, Tautomer, optisches Isomer, Prodrugderivat, ausgewählt aus den Methyl-, Ethyl-, Propyl-, Isopropyl-, Butyl-, sek-Butyl, tert-Butyl-, Morpholinoethyloxy-, Diethylglycolamid- oder Diethylaminocarbonylmethoxyestern oder einem Salz hiervon.

3. Verbindung nach Anspruch 1 oder 2, worin das Prodrugderivat ein Methyl-, Ethyl-, Propyl-, Isopropyl-, Butyl-, Morpholinoethylethoxy- oder Diethylglycolamidester ist.

4. Pharmazeutische Formulierung, die eine Verbindung nach Anspruch 1 oder 2 zusammen mit einem pharmazeutisch annehmbaren Träger oder Verdünnungsmittel hierfür enthält.

5. Pharmazeutische Formulierung, die zur Behandlung eines Zustands angepasst ist, der mit der Hemmung der $sPLA_2$ assoziiert ist, welche eine Verbindung nach Anspruch 1 oder 2 zusammen mit einem pharmazeutisch annehmbaren Träger oder Verdünnungsmittel hierfür enthält.

6. Verwendung einer Verbindung nach Anspruch 1 oder 2 oder eines pharmazeutisch annehmbaren Razemats, Solvats, Tautomers, optischen Isomers, Prodrugderivats, ausgewählt aus dem Methyl-, Ethyl-, Propyl-, Isopropyl-, Butyl-, sek-Butyl, tert-Butyl-, Morpholinoethyloxy-, Diethylglycolamid- oder Diethylaminocarbonylmethoxyester oder eines Salzes hiervon zur Herstellung eines Arzneimittels zur selektiven Hemmung der $sPLA_2$ bei einem Säuger.

7. Verwendung nach Anspruch 6, worin der Säuger ein Mensch ist.

8. Verwendung einer Verbindung nach Anspruch 1 oder 2 zur Herstellung eines Arzneimittels, das zur Verabreichung der Verbindung in einer Menge an den Säuger angepasst ist, die ausreicht, um die durch $sPLA_2$ vermittelte Freisetzung der Fettsäure zu hemmen und hierdurch die Arachidonsäurekaskade und ihre schädlichen Produkte zu hemmen oder zu verhindern und die pathologischen Effekte von $sPLA_2$ bedingten Erkrankungen zu lindern.

9. Verwendung einer Verbindung nach Anspruch 1 oder 2 zur Herstellung eines Arzneimittels zur Linderung der pathologischen Effekte der durch $sPLA_2$ bedingten Erkrankungen.

10. Verwendung einer Verbindung nach Anspruch 1 oder 2 zur Herstellung eines Arzneimittels, das zur Kontaktierung von $sPLA_2$ mit dieser Verbindung und zur Hemmung der $sPLA_2$ angepasst ist.

11. Verwendung einer Verbindung nach Anspruch 1 oder 2 oder eines pharmazeutisch annehmbaren Razemats, Solvats, Tautomers, optischen Isomers, Prodrugderivats, ausgewählt aus den Methyl-, Ethyl-, Propyl-, Isopropyl-, Butyl-, sek-Butyl, tert-Butyl-, Morpholinoethyloxy-, Diethylglycolamid- oder Diethylaminocarbonylmethoxyestern oder eines Salzes hiervon zur Herstellung eines Arzneimittels zur Behandlung von Sepsis, septischem Schock,

rheumatoider Arthritis, Osteoarthritis, Schlaganfall, Apoptose, Asthma, chronischer Bronchitis, akuter Bronchitis, cystischer Fibrose, entzündlicher Darmerkrankung oder Pankreatitis.

**12.** Verwendung nach einem der Ansprüche 6 bis 11 zur Linderung der pathologischen Effekte von Sepsis, septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Bronchiolitis, chronischer Bronchiolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikulärem Rheuma, Bursitis, Tenosynovitis, Epicondylitis (Tennisellenbogen), Carpaltunnelsyndrom, Verletzung durch wiederkehrende Tätigkeit (Tippen), verschiedenen Formen der Arthritis, neuropathischer Gelenkserkrankung (Charcotgelenk und Gelenke), Hämarthrose (hämarthrotisch), Purpura Schönlein-Hennoch, hypertropher Osteoarthropathie, multizentrischer Reticulohistiocytose, Arthritis, die mit bestimmten Erkrankungen assoziiert ist, Surcoilose, Hämochromatose, Sichelzellerkrankung und anderen Hämoglobinopathien, Hyperlipoproteinämie, Hypogammaglobulinämie, Hyperparathyreoidismus, Akromegalie, familiärem Mittelmeerfieber, Behat Erkrankung, systemischem Lupus erythematosis oder Polychondritisrückfall und verwandten Krankheiten.

## Revendications

**1.** Composé choisi dans le groupe constitué de :

hydrazide d'acide 9-benzyl-5,7-diméthoxy-1,2,3,4-tétrahydrocarbazole-4-carboxylique ;
9-benzyl-5,7-diméthoxy-1,2,3,4-tétrahydrocarbazole-4-carboxamide ;
acide [9-benzyl-4-carbamoyl-7-méthoxy-1,2,3,4-tétrahydrocarbazol-5-yl]-oxyacétique ;
acide [9-benzyl-4-carbamoyl-7-méthoxycarbazol-5-yl]-oxyacétique ;
acide méthyl-[9-benzyl-4-carbamoyl-7-méthoxycarbazol-5-yl]-oxy-acétique ;
9-benzyl-7-méthoxy-5-cyanométhoxy-1,2,3,4-tétrahydrocarbazole-4-carboxamide ;
9-benzyl-7-méthoxy-5-(1H-tétrazol-5-yl-méthyl)oxy)-1,2,3,4-tétrahydrocarbazole-4-carboxamide ;
acide {9-[(phényl)méthyl]-5-carbamoyl-2-méthylcarbazol-4-yl}oxy-acétique ;
acide {9-[(3-fluorophényl)méthyl]-5-carbamoyl-2-méthylcarbazol-4-yl}oxy-acétique ;
acide {9-[(3-méthylphényl)méthyl]-5-carbamoyl-2-méthylcarbazol-4-yl}oxyacétique ;
acide {9-[(phényl)méthyl]-5-carbamoyl-2-(4-trifluorométhylphényl}-carbazol-4-yl)oxyacétique ;
9-benzyl-5-(2-méthanesulfonamido)éthyloxy-7-méthoxy-1,2,3,4-tétrahydrocarbazole-4-carboxamide ;
9-benzyl-4-(2-méthanesulfonamido)éthyloxy-2-méthoxycarbazole-5-carboxamide ;
9-benzyl-4-(2-trifluorométhanesulfonamido)éthyloxy-2-méthoxy-carbazole-5-carboxamide ;
9-benzyl-5-méthanesulfonamidoylméthyloxy-7-méthoxy-1,2,3,4-tétrahydrocarbazole-4-carboxamide ;
9-benzyl-4-méthanesulfonamidoylméthyloxy-carbazole-5-carboxamide ;
acide [5-carbamoyl-2-pentyl-9-(phénylméthyl)-carbazol-4-yl]oxyacétique ;
acide [5-carbamoyl-2-(1-méthyléthyl)-9-(phénylméthyl)-carbazol-4-yl]-oxyacétique ;
acide [5-carbamoyl-9-(phénylméthyl)-2-[(tri(1-méthyléthyl)silyl)-oxy-méthyl]carbazol-4-yl]oxyacétique ;
acide [5-carbamoyl-2-phényl-9-(phénylméthyl)-carbazol-4-yl]-oxy-acétique ;
acide [5-carbamoyl-2-(4-chlorophényl)-9-(phénylméthyl)-carbazol-4-yl]-oxyacétique ;
acide [5-carbamoyl-2-(2-furyl)-9-(phénylméthyl)-carbazol-4-yl]-oxy-acétique ;
acide [5-carbamoyl-9-(phénylméthyl)-2-[(tri(1-méthyléthyl)silyl)-oxyméthyl]carbazol-4-yl]oxyacétique ;
acide {9-[(2-fluorophényl)méthyl]-5-carbamoylcarbazol-4-yl}oxyacétique ;
acide {9-[(2-trifluorométhylphényl)méthyl]-5-carbamoylcarbazol-4-yl}oxy-acétique ;
acide {9-[(2-benzylphényl)méthyl]-5-carbamoylcarbazol-4-yl}oxy-acétique ;
acide {9-[(1-naphtyl)méthyl]-5-carbamoylcarbazol-4-yl}oxyacétique ;
acide {9-[(2-cyanophényl)méthyl]-5-carbamoylcarbazol-4-yl}oxyacétique ;
acide {9-[(3-cyanophényl)méthyl]-5-carbamoylcarbazol-4-yl}oxyacétique ;
acide {9-[(3,5-diméthylphényl)méthyl]-5-carbamoylcarbazol-4-yl}oxy-acétique ;
acide {9-[(3-iodophényl)méthyl]-5-carbamoylcarbazol-4-yl}oxyacétique ;
acide {9-[(2-chlorophényl)méthyl]-5-carbamoylcarbazol-4-yl}oxyacétique ;

acide {9-[(2,3-difluorophényl)méthyl]-5-carbamoylcarbazol-4-yl}oxy-acétique ;
acide {9-[(2,6-difluorophényl)méthyl]-5-carbamoylcarbazol-4-yl}oxy-acétique ;
acide {9-[(2,6-dichlorophényl)méthyl]-5-carbamoylcarbazol-4-yl}oxy-acétique ;
acide {9-[(2-biphényl)méthyl]-5-carbamoylcarbazol-4-yl}oxyacétique ;
ester méthylique de l'acide {9-[(2-biphényl)méthyl]-5-carbamoylcarbazol-4-yl}oxyacétique ;
acide [9-benzyl-4-carbamoyl-1,2,3,4-tétrahydrocarbazol-5-yl]-oxy-acétique ;
acide {9-[(2-pyridyl)méthyl]-5-carbamoylcarbazol-4-yl}oxyacétique ;
acide {9-[(3-pyridyl)méthyl]-5-carbamoylcarbazol-4-yl}oxyacétique ;
acide [9-benzyl-4-carbamoyl-8-méthyl-1,2,3,4-tétrahydrocarbazol-5-yl]-oxyacétique ;
acide [9-benzyl-5-carbamoyl-1-méthylcarbazol-4-yl]-oxyacétique ;
acide [9-benzyl-4-carbamoyl-8-fluoro-1,2,3,4-tétrahydrocarbazol-5-yl]-oxyacétique ;
acide [9-benzyl-4-carbamoyl-8-chforo-1,2,3,4-tétrahydrocarbazol-5-yl]-oxyacétique ;
acide [5-carbamoyl-9-(phénylméthyl)-2-[[(propén-3-yl)oxy]méthyl]-carbazol-4-yl]oxyacétique ;
acide [5-carbamoyl-9-(phénylméthyl)-2-[(propyloxy)méthyl]carbazol-4-yl]-oxyacétique ;
9-benzyl-7-méthoxy-5-((carboxamidométhyl)oxy)-1,2,3,4-tétrahydrocarbazole-4-carboxamide ;
9-benzyl-7-méthoxy-5-cyanométhyloxy-carbazole-4-carboxamide ;
9-benzyl-7-méthoxy-5-((1H-tétrazol-5-yl-méthyl)oxy)carbazole-4-carboxamide ;
9-benzyl-7-méthoxy-5-((carboxamidométhyl)oxy)-carbazole-4-carboxamide ; et
acide [9-benzyl-4-carbamoyl-1,2,3,4-tétrahydrocarbazol-5-yl]-oxyacétique ;

ou un racémate, un produit de solvatation, un tautomère, un isomère optique, un pro-médicament dérivé choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

**2.** Composé choisi dans le groupe constitué de :

acide {9-[(phényl)méthyl]-5-carbamoyl-2-méthylcarbazol-4-yl}-oxyacétique ;
acide {9-[(3-fluorophényl)méthyl]-5-carbamoyl-2-méthylcarbazol-4-yl}-oxyacétique ;
acide {9-[(3-méthylphényl)méthyl]-5-carbamoyl-2-méthylcarbazol-4-yl}-oxyacétique ;
acide {9-[(phényl)méthyl]-5-carbamoyl-2-(4-trifluorométhylphényl)-carbazol-4-yl}oxyacétique ;
9-benzyl-5-(2-méthanesulfonamido)éthyloxy-7-méthoxy-1,2,3,4-tétrahydrocarbazole-4-carboxamide ;
9-benzyl-4-(2-méthanesulfonamido)éthyloxy-2-méthoxycarbazole-5-carboxamide ;
9-benzyl-4-(2-trifluorométhanesulfonamido)éthyloxy-2-méthoxy-carbazole-5-carboxamide ;
9-benzyl-5-méthanesulfonamidoylméthyloxy-7-méthoxy-1,2,3,4-tétrahydrocarbazole-4-carboxamide ;
9-benzyl-4-méthanesulfonamidoylméthyloxy-carbazole-5-carboxamide ;
acide [5-carbamoyl-2-pentyl-9-(phénylméthyl)carbazol-4-yl]-oxyacétique ;
acide [5-carbamoyl-2-(1-méthyléthyl)-9-(phénylméthyl)carbazol-4-yl]-oxy-acétique ;
[5-carbamoyl-9-(phénylméthyl)-2-[(tri(1-méthyléthyl)silyl)-oxyméthyl]-carbazol-4-yl]oxyacétique ;
acide [5-carbamoyl-2-phényl-9-(phénylméthyl)-carbazol-4-yl]-oxy-acétique ;
acide [5-carbamoyl-2-(4-chlorophényl)-9-(phénylméthyl)-carbazol-4-yl]-oxyacétique ;
acide [5-carbamoyl-2-(2-furyl)-9-(phénylméthyl)-carbazol-4-yl]-oxy-acétique ;
acide [5-carbamoyl-9-(phénylméthyl)-2-[(tri(1-méthyléthyl)silyl)-oxyméthyl]carbazol-4-yl]oxyacétique ;
acide {9-[(phényl)méthyl]-5-carbamoylcarbazol-4-yl}oxyacétique ;
acide {9-[(3-fluorophényl)méthyl]-5-carbamoylcarbazol-4-yl}oxyacétique ;
acide {9-[(3-chlorophényl)méthyl]-5-carbamoylcarbazol-4-yl}oxyacétique ;
acide {9-[(3-phénoxyphényl)méthyl]-5-carbamoylcarbazol-4-yl}oxy-acétique ;
acide {9-[(2-fluorophényl)méthyl]-5-carbamoylcarbazol-4-yl}oxyacétique ;
acide {9-[(2-trifluorométhylphényl)méthyl]-5-carbamoylcarbazol-4-yl}-oxy-acétique ;
acide {9-[(2-benzylphényl)méthyl]-5-carbamoylcarbazol-4-yl}-oxy-acétique ;
acide {9-[(3-trifluorométhylphényl)méthyl]-5-carbamoylcarbazol-4-yl}-oxy-acétique ;
acide {9-[(1-naphtyl)méthyl]-5-carbamoylcarbazol-4-yl}oxyacétique ;
acide {9-[(2-cyanophényl)méthyl]-5-carbamoylcarbazol-4-yl}oxyacétique ;
acide {9-[(3-cyanophényl)méthyl]-5-carbamoylcarbazol-4-yl}oxyacétique ;
acide {9-[(2-méthylphényl)méthyl]-5-carbamoylcarbazol-4-yl}oxy-acétique ;
acide {9-[(3-méthylphényl)méthyl]-5-carbamoylcarbazol-4-yl}oxy-acétique ;
acide {9-[(3,5-diméthylphényl)méthyl]-5-carbamoylcarbazol-4-yl}oxy-acétique ;
acide {9-[(3-iodophényl)méthyl]-5-carbamoylcarbazol-4-yl}oxyacétique ;

acide {9-[(2-chlorophényl)méthyl]-5-carbamoylcarbazol-4-yl}oxyacétique ;
acide {9-[(2,3-difluorophényl)méthyl]-5-carbamoylcarbazol-4-yl}oxy-acétique ;
acide {9-[(2,6-difluorophényl)méthyl]-5-carbamoylcarbazol-4-yl}oxy-acétique ;
acide {9-[(2,6-dichlorophényl)méthyl]-5-carbamoylcarbazol-4-yl}oxy-acétique ;
acide {9-[(3-trifluorométhoxyphényl)méthyl]-5-carbamoylcarbazol-4-yl}-oxyacétique ;
acide {9-[(2-biphényl)méthyl]-5-carbamoylcarbazol-4-yl}oxyacétique ;
ester méthylique de l'acide {9-[(2-biphényl)méthyl]-5-carbamoylcarbazol-4-yl}oxyacétique ;
acide [9-benzyl-4-carbamoyl-1,2,3,4-tétrahydrocarbazol-5-yl]-oxy-acétique ;
acide {9-[(2-pyridyl)méthyl]-5-carbamoylcarbazol-4-yl}oxyacétique ;
acide {9-[(3-pyridyl)méthyl]-5-carbamoylcarbazol-4-yl}oxyacétique ;
acide [9-benzyl-4-carbamoyl-8-méthyl-1,2,3,4-tétrahydrocarbazol-5-yl]-oxyacétique ;
acide [9-benzyl-5-carbamoyl-1-méthylcarbazol-4-yl]-oxyacétique ;
acide [9-benzyl-4-carbamoyl-8-fluoro-1,2,3,4-tétrahydrocarbazol-5-yl]-oxyacétique ;
acide [9-benzyl-5-carbamoyl-1-fluorocarbazol-4-yl]-oxyacétique ;
acide [9-benzyl-4-carbamoyl-8-chloro-1,2,3,4-tétrahydrocarbazol-5-yl]-oxyacétique ;
acide [9-benzyl-5-carbamoyl-1-chlorocarbazol-4-yl]-oxyacétique ;
acide [9-[(cyclohexyl)méthyl]-5-carbamoylcarbazol-4-yl]-oxyacétique ;
acide [9-[(cyclopentyl)méthyl]-5-carbamoylcarbazol-4-yl]-oxyacétique ;
acide [5-carbamoyl-9-(phénylméthyl)-2-(2-thiényl)carbazol-4-yl]-oxy-acétique ;
acide [5-carbamoyl-9-(phénylméthyl)-2-[[(propén-3-yl)-oxy]méthyl]-carbazol-4-yl]oxyacétique ;
acide [5-carbamoyl-9-(phénylméthyl)-2-[(propyloxy)méthyl]carbazol-4-yl]-oxyacétique ;
9-benzyl-7-méthoxy-5-((carboxamidométhyl)oxy)-1,2,3,4-tétrahydrocarbazole-4-carboxamide ;
9-benzyl-7-méthoxy-5-cyanométhyloxy-carbazole-4-carboxamide ;
9-benzyl-7-méthoxy-5-((1H-tétrazol-5-yl-méthyl)oxy)-carbazole-4-carboxamide ;
9-benzyl-7-méthoxy-5-((carboxamidométhyl)oxy)-carbazole-4-carboxamide ; et

acide [9-benzyl-4-carbamoyl-1,2,3,4-tétrahydrocarbazol-5-yl]-oxy-acétique ;
ou un racémate, un produit de solvatation, un tautomère, un isomère optique, un pro-médicament dérivé choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

**3.** Composé selon la revendication 1 ou 2, dans lequel le pro-médicament dérivé est un ester méthylique, éthylique, propylique, isopropylique, butylique, morpholinoéthyloxy ou diéthylglycolamide.

**4.** Formulation pharmaceutique comprenant un composé selon la revendication 1 ou 2 et un support ou un diluant pharmaceutiquement acceptable.

**5.** Formulation pharmaceutique adaptée au traitement d'une pathologie liée à l'inhibition de $sPLA_2$, comprenant un composé selon la revendication 1 ou 2 et un support ou un diluant pharmaceutiquement acceptable.

**6.** Utilisation d'un composé selon la revendication 1 ou 2 ou d'un racémate, d'un produit de solvatation, d'un tautomère, d'un isomère optique, d'un pro-médicament dérivé choisi parmi un ester méthylique, éthylique, propylique, isopropylique, butylique, sec-butylique, tert-butylique, morpholinoéthyloxy, diéthylglycolamide ou diéthylaminocarbonylméthoxy ou d'un sel pharmaceutiquement acceptable de celui-ci, pour la fabrication d'un médicament destiné à inhiber sélectivement $sPLA_2$ chez un mammifère.

**7.** Utilisation selon la revendication 6, dans laquelle le mammifère est un être humain.

**8.** Utilisation d'un composé selon la revendication 1 ou 2 pour la fabrication d'un médicament adapté pour l'administration à un mammifère dudit composé en une quantité suffisante pour inhiber la libération médiée par $sPLA_2$ d'acide gras et pour ainsi inhiber ou empêcher la cascade de l'acide arachidonique et ses produits nuisibles, afin de soulager les effets pathologiques de maladies liées à $sPLA_2$.

**9.** Utilisation d'un composé selon la revendication 1 ou 2 pour la fabrication d'un médicament destiné à soulager les effets pathologiques de maladies liées à $sPLA_2$.

**10.** Utilisation d'un composé selon la revendication 1 ou 2 pour la fabrication d'un médicament adapté pour mettre en

contact $sPLA_2$ avec ledit composé, afin d'inhiber $sPLA_2$.

**11.** Utilisation d'un composé selon la revendication 1 ou 2 ou d'un racémate, d'un produit de solvatation, d'un tautomère, d'un isomère optique, d'un pro-médicament dérivé choisi parmi un ester méthylique, éthylique, propylique, isopropylique, butylique, sec-butylique, tert-butylique, morpholinoéthyloxy, diéthylglycolamide ou diéthylaminocarbonylméthoxy ou d'un sel pharmaceutiquement acceptable de celui-ci, pour la fabrication d'un médicament destiné à traiter une septicémie, un choc septique, une polyarthrite rhumatoïde, une arthrose, une attaque, une apoptose, un asthme, une bronchite chronique, une bronchite aiguë, une fibrose kystique, une maladie du côlon inflammatoire ou une pancréatite.

**12.** Utilisation selon l'une quelconque des revendications 6 à 11 pour soulager les effets pathologiques d'une septicémie, d'un choc septique, d'un syndrome de détresse respiratoire aigu de l'adulte, d'une pancréatite, d'un choc provoqué par un traumatisme, d'un asthme bronchique, d'une rhinite allergique, d'une polyarthrite rhumatoïde, d'une fibrose kystique, d'une attaque, d'une bronchite aiguë, d'une bronchite chronique, d'une bronchiolite aiguë, d'une bronchiolite chronique, d'une arthrose, d'une goutte, d'une spondylarthropathie, d'une pelvispondylite rhumatismale, du syndrome de Reiter, d'une arthropathie psoriasique, d'une spondylite entéropathique, d'une arthropathie juvénile, ou d'une pelvispondylite rhumatismale juvénile, d'une arthropathie réactive, d'une arthrite infectieuse ou post-infectieuse, d'une arthrite gonococcique, d'une arthrite tuberculeuse, d'une arthrite virale, d'une arthrite fongique, d'une arthrite syphilique, de la maladie de Lyme, d'une arthrite associée aux « syndromes vasculitiques », d'une périartérite noueuse, d'une angéite d'hypersensibilité, d'une granulomatose de Luegenec, d'une pseudopolyarthrite rhizomélique, d'une artérite cellulaire des articulations, d'une arthropathie liée à un dépôt de cristaux de calcium, d'une chondrocalcinose articulaire, d'un rhumatisme non articulaire, d'une bursite, d'une ténosynovite, d'une épicondylite (« tennis elbow : épicondylite des joueurs de tennis), du syndrome du canal carpien, d'un trouble lié à une utilisation répétée (dactylographie), de diverses formes d'arthrite, d'une maladie des articulations névrotiques (charco et articulations), d'une hémarthrose (hémarthrosique), d'un purpura rhumatoïde de Henoch-Schonlein, d'une ostéo-arthropathie hypertrophiante, d'une réticulo-histiocytose multicentrique, d'une arthrite associée à certaines maladies, d'une surcoilose, d'une hémochromatose, d'une drépanocyte et d'autres hémoglobinopathies, d'une hyperlipidémie, d'une hypogammaglobulinémie, d'une hyperparathyroïdie, d'une acromégalie, d'une brucellose familiale, de la maladie de Behat, d'un lupus érythémateux disséminé ou d'une polychondrite atrophiante ; et d'autres maladies apparentées.